# EUROPEAN PATENT SPECIFICATION

(11) **EP 3 969 479 B1**
(45) Date of publication and mention of the grant of the patent: **30.07.2025**
(21) Application number: 20805976.6
(22) Date of filing: 14.05.2020
(51) Int. Cl.: C07K 16/28, C12N 15/62, A61P 35/00

(54) **EPCAM BINDING PROTEINS AND METHODS OF USE**
EPCAM-BINDENDE PROTEINE UND VERFAHREN ZUR VERWENDUNG
PROTÉINES DE LIAISON À EPCAM ET MÉTHODES D'UTILISATION

(30) Priority: 14.05.2019 US 201962847778 P; 14.05.2019 WO PCT/US2019/032224; 14.05.2019 WO PCT/US2019/032307; 14.05.2019 WO PCT/US2019/032302; 14.05.2019 WO PCT/US2019/032306
(43) Date of publication of application: 23.03.2022
(73) Proprietor: Harpoon Therapeutics, Inc., South San Francisco, CA 94080 (US)
(72) Inventor: WESCHE, Holger, South San Francisco, California 94080 (US); AUSTIN, Richard J., South San Francisco, California 94080 (US); LIN, Shuoyen Jack, South San Francisco, California 94080 (US); LEMON, Bryan, South San Francisco, California 94080 (US); WRIGHT, Kevin, South San Francisco, California 94080 (US); ROCHA, Sony, South San Francisco, California 94080 (US); KWANT, Kathryn, South San Francisco, California 94080 (US)
(74) Representative: Merck Sharp & Dohme LLC
(86) International application number: PCT/US2020/032985
(87) International publication number: WO 2020/232303

(56) References cited:
- WO-A1-2005/040220
- WO-A1-2017/157305
- WO-A1-2019/222278
- WO-A2-2012/158818
- US-A1- 2010 311 119
- US-A1- 2012 039 899
- US-A1- 2016 032 011
- US-A1- 2019 092 862
- JIANG Z C ET AL: "protritac: a protease cleavable T cell engager platform", SCIENTIFIC REPORTS, 29 January 2018 (2018-01-29), England, pages 19991, XP093004389, Retrieved from the Internet <URL:https://calidibio.com/wp-content/uploads/2019/10/609-Abstract-_SITC-2018.pdf> [retrieved on 20221202], DOI: 10.1038/srep19991

## Description

### CROSS-REFERENCE

This application claims the benefit of U.S. Provisional Application No. 62/847,778 filed on May 14, 2019, PCT Application No. PCT/US2019/032307 filed on May 14, 2019, PCT Application No. PCT/US2019/032224 filed on May 14, 2019, PCT Application No. PCT/US2019/US032302 filed on May 14, 2019, and PCT Application No. PCT/US2019/032306 filed on May 14, 2019.

### BACKGROUND OF THE INVENTION

Studies have shown that epithelial cell adhesion/activating molecule (EpCAM/CD326) is expressed on essentially all human adenocarcinoma, on certain squamous cell carcinoma, on retinoblastoma, and on hepatocellular carcinoma. *See, e.g.,* Baeuerle PA, Gires O. EpCAM (CD326) finding its role in cancer. EpCAM is part of the signature of cancer-propagating cells in numerous solid tumors and of normal progenitor and stem cells. *See Id.* EpCAM has been causally implicated in proliferation, migration, and signal transduction in cancers, *e.g.,* in breast cancer. *See, e.g.,* Osta WA, Chen Y, Mikhitarian K, et al. EpCAM is overexpressed in breast cancer and is a potential target for breast cancer gene therapy. Cancer Res 2004; 64: 5818-24.

Edrecolomab, an anti-EpCAM antibody showed limited efficacy in the phase III studies. *See* Eyvazi S, Farajnia S, Dastmalchi S, Kanipour F, Zarredar H, Bandehpour M. Antibody Based EpCAM Targeted Therapy of Cancer, Review and Update. Curr Cancer Drug Targets. 2018;18(9):857-868. Other anti-EpCAM antibodies used in various clinical studies include Adecatumumab (a wholly human monoclonal antibody), Catumaxomab (a chimeric antibody), Oportuzumab Monatox (an scFv antibody conjugated to Pseudomonas exotoxin A (ETA), Citatuzumab Bogatox (Fab fragment with bouganin toxin), immono-conjugate antibody Tucotuzumab (monoclonal antibody with IL2). *See Id.*

There is a need for a greater choice of treatment options which allows physicians to select the therapeutic with the best side effect profile for an individual patient. The present disclosure provides novel polypeptides and protein therapeutics useful in methods of treatment, particularly for treatment of conditions associated with abnormal expression of EpCAM. Lin SJ et al, J. ImmunoTher Cancer, 2018, 6(Suppl.1): 115, P608 describes a polypeptide with an anti-albumin binding domain, an anti CD3 epsilon binding domain, and an anti-tumor-associated antigen binding domain.

### SUMMARY OF THE INVENTION

**In** one aspect, the invention provides a multispecific protein comprising (a) an EpCAM binding domain, (b) a CD3ε binding domain, and (c) a bulk serum protein binding domain that binds to a human serum albumin protein, wherein the multispecific protein comprises an amino acid sequence of any one of SEQ ID NOS: 576, 577, and 578.
In another aspect, the invention provides a pharmaceutical composition comprising (i) a multispecific protein of the invention and (ii) a pharmaceutically acceptable carrier.
In another aspect, the invention provides a polynucleotide encoding a multispecific protein of the invention.
In yet another aspect, the invention provides a multispecific protein, pharmaceutical composition or vector encoding a polynucleotide of the invention, for use in the treatment of a cancer in a subject in need thereof.

The following paragraphs are useful for understanding the invention, which is defined in the claims. In some cases, the EpCAM binding domain is part of a multispecific protein. In some cases, the multispecific protein further comprises a CD3 binding domain. In some cases, the multispecific protein comprises an active drug format. In some cases, the multispecific protein further comprises a bulk serum protein binding domain. In some cases, the bulk serum protein comprises a serum albumin protein. In some cases, the serum albumin protein comprises a human serum albumin protein. In some cases, the bulk serum protein binding domain comprises a sequence that is at least 75% identical the sequence as set forth in SEQ ID No. 378. In some cases, the CD3 binding domain comprises a sequence that is at least 75% identical the sequence as set forth in SEQ ID No. 379. In some cases, the multispecific protein comprises a sequence that is at least about 75% identical to the sequence as set forth in SEQ ID No. 492. In some cases, the bulk serum protein binding domain is a binding moiety comprising a linker and a masking moiety, wherein the masking moiety is capable of masking the binding of the EpCAM binding domain or the CD3 binding domain, to their respective targets. In some cases, the multispecific protein comprises a non-cleavable prodrug format. In some cases, the masking moiety comprises a sequence selected from the group consisting of: SEQ ID Nos. 380-424, or a sequence comprising one or more substitutions in a sequence selected from the group consisting of: SEQ ID Nos. 380-424. In some cases, the linker comprises a sequence selected from the group consisting of: SEQ ID Nos. 425-471, 503-507, and 581, or a sequence comprising one or more substitutions in a sequence selected from the group consisting of: SEQ ID Nos. 425-471, 503-507, and 581. In some cases, the bulk serum protein binding domain comprises a sequence that is at least 75% identical the sequence selected from the group consisting of SEQ ID Nos. 472-473 and 482-483. In some cases, the CD3 binding domain comprises a sequence that is at least 75% identical to the sequence as set forth in SEQ ID No. 474.

In some cases, the multispecific protein comprises a sequence that is at least 75% identical to a sequence selected from the group consisting of SEQ ID Nos. 495, 498-502, 569-570, 572, 573, 575, 576, 577, and 578. In some cases, the multispecific protein comprises a sequence that is at least 75% identical to a sequence selected from the group consisting of SEQ ID Nos. 495 and 502. In some cases, the multispecific protein comprises a sequence that is at least 75% identical to a sequence selected from the group consisting of SEQ ID Nos. 498-501, 569-570, 572, 573, 575, 576, 577, and 578. In some cases, the active drug comprises a sequence that is at least 75% identical to a sequence selected from the group consisting of SEQ ID Nos. 153-179, 180-206, 210-212, 494, 571, and 574.

In some cases, the EpCAM binding domain is part of a chimeric antigen receptor (a CAR) or a conditionally activatable chimeric antigen receptor (a ProCAR), wherein the CAR further comprises at least one of a transmembrane domain, a costimulatory domain, and an intracellular signaling domain. In some cases, the EpCAM binding domain is part of the ProCAR and the ProCAR further comprises (a) a binding moiety comprising a non-CDR loop and a cleavable linker; (b) a transmembrane domain; and (c) an intracellular signaling domain; wherein the binding moiety is capable of masking the binding of the EpCAM binding domain to its target. In some cases, the binding moiety further comprises one or more complementarity determining regions (CDRs). In some cases, the CDR loop provides a binding site specific for a bulk serum protein. In some cases, the bulk serum protein comprises at least one of: a serum albumin, a transferrin, an IgG1, an IgG2, an IgG4, an IgG3, an IgA monomer, a Factor XIII, a fibrinogen, a pentameric IgM. In some cases, the bulk serum protein comprises the serum albumin. In some cases, the serum albumin is a human serum albumin. In some cases, the ProCAR further comprising a costimulatory domain, wherein the costimulatory domain is a functional signaling domain of a protein selected from the group consisting of OX40, CD2, CD27, CD28, CDS, ICAM-1, LFA-1 (CD11a/CD18), ICOS (CD278), and 4-1BB (CD137), and amino acid sequences thereof having at least one but not more than 20 modifications thereto. In some cases, the at least one but not more than 20 modifications thereto comprises a modification of an amino acid that mediates cell signaling or a modification of an amino acid that is phosphorylated in response to a ligand binding to the encoded T-cell receptor fusion protein. In some cases, the transmembrane domain comprises a transmembrane domain of a protein selected from the group consisting of a TCR alpha chain, a TCR beta chain, a TCR zeta chain, a CD3 epsilon TCR subunit, a CD3 gamma TCR subunit, a CD3 delta TCR subunit, CD45, CD4, CDS, CD8, CD9, CD16, CD22, CD33, CD28, CD37, CD64, CD80, CD86, CD134, CD137, CD154, functional fragments thereof, and amino acid sequences thereof having at least one but not more than 20 modifications thereto. In some cases, the intracellular signaling domain is derived from CD3 epsilon CD3 gamma, CD3 delta, CD3 alpha, CD3 beta, or a combination thereof. In some cases, the EpCAM binding domain is part of the ProCAR, and wherein the ProCAR comprises a sequence that is at least about 75% identical to a sequence selected from the group consisting of SEQ ID Nos. 485-491.

The references to the methods of treatment by therapy or surgery or in vivo diagnosis methods in this description are to be interpreted as references to compounds of the present invention for use in those methods. One embodiment provides a method for the treatment or amelioration of a proliferative disease, or a tumorous disease, comprising the administration of a claimed protein, pharmaceutical composition or vector to a subject in need thereof. The disease to be treated is a cancer. In some embodiments, the subject is human.

The following paragraphs are useful for understanding the invention, which is defined in the claims. One case provides a conditionally active chimeric antigen receptor (ProCAR) that comprises a single polypeptide chain, comprising (a) a binding moiety comprising a non-CDR loop and a cleavable linker; (b) an EpCAM binding domain, wherein the EpCAM binding domain comprises a complementarity determining region 1 (CDR1), a CDR2, and a CDR3, wherein the CDR1 comprises a sequence selected from the group consisting of SEQ ID Nos. 39-76, or a sequence comprising one or more substitutions in a sequence selected from the group consisting of SEQ ID Nos. 39-76; the CDR2 comprising a sequence selected from the group consisting of SEQ ID Nos. 77-114 or a sequence comprising one or more substitutions in a sequence selected from the group consisting of SEQ ID Nos. 77-114; and the CDR3 comprising a sequence selected from the group consisting of SEQ ID Nos. 115-152 or a sequence comprising one or more substitutions in a sequence selected from the group consisting of SEQ ID Nos. 115-152; (c) a transmembrane domain; and (d) an intracellular signaling domain; wherein the binding moiety is capable of masking the binding of the EpCAM binding domain to its target. In some cases, the EpCAM binding domain comprises a sequence that is at least about 75% identical to a sequence selected from the group consisting of SEQ ID Nos. 1-38, 207-209, and 496-497.

One case provides a conditionally active EpCAM binding protein comprising a binding moiety (M) which comprises a non-CDR loop, a cleavable linker (L), a first target antigen binding domain (T1), and a second target antigen binding domain (T2), wherein at least one of the first target antigen binding domain (T1) and the second target antigen binding domain (T2) comprises an EpCAM binding domain, wherein the EpCAM binding domain comprises a complementarity determining region 1 (CDR1), a CDR2, and a CDR3, wherein the CDR1 comprises a sequence selected from the group consisting of SEQ ID Nos. 39-76, or a sequence comprising one or more substitutions in a sequence selected from the group consisting of SEQ ID Nos. 39-76; the CDR2 comprising a sequence selected from the group consisting of SEQ ID Nos. 77-114 or a sequence comprising one or more substitutions in a sequence selected from the group consisting of SEQ ID Nos. 77-114; and the CDR3 comprising a sequence selected from the group consisting of SEQ ID Nos. 115-152 or a sequence comprising one or more substitutions in a sequence selected from the group consisting of SEQ ID Nos. 115-152, wherein the non-CDR loop is capable of binding to the EpCAM binding domain or the second target antigen binding domain, and wherein the binding moiety is capable of masking the binding of the EpCAM binding domain or the second target antigen binding domain to its target. In some cases, the binding moiety comprises a masking moiety and wherein the masking moiety comprises a sequence selected from the group consisting of SEQ ID Nos. 380-424, or a sequence comprising one or more substitutions relative to a sequence selected from the group consisting of SEQ ID Nos. 380-424. In some cases, the linker comprises a sequence selected from the group consisting of: SEQ ID Nos. 425-471, 503-506, and 581, or a sequence comprising one or more substitutions relative to a sequence selected from the group consisting of: SEQ ID Nos. 425-471, 503-506, and 581. In some cases, the binding moiety comprises a sequence that is at least 75% identical the sequence selected from the group consisting of SEQ ID Nos. 472-473 and 482-483. In some cases, the second target antigen binding domain (T2) comprises a CD3 binding domain. In some cases, the CD3 binding domain comprises a sequence that is at least 75% identical to the sequence as set forth in SEQ ID No. 474.

In some cases, the binding domain is a humanized antibody or an antigen binding fragment thereof. In some cases, the binding domain is a single domain antibody, a VHH domain, a scFv, a VH domain, a VL domain, a Fab, a Fab', a non-Ig domain, a ligand, a knottin, or a small molecule entity. In some cases, the binding domain comprises the single domain antibody. In some cases, the binding domain binds to EpCAM with a binding affinity (Kd) of about 0.001 nM to about 500 nM. In some cases, the binding domain binds to human EpCAM, mouse EpCAM, cynomolgus EpCAM, or a combination thereof.
One case provides a multispecific protein comprising an EpCAM binding domain, wherein the EpCAM binding domain is according to this disclosure. In some cases, the multispecific protein comprises the EpCAM binding domain according to this disclosure (anti-EpCAM domain), and a CD3 binding domain (anti-CD3 domain). In some cases, the anti-EpCAM domain and the anti-CD3 domain are in an anti-EpCAM:anti-CD3 orientation. In some cases, the anti-EpCAM domain and the anti-CD3 domain are in an anti-CD3: anti-EpCAM orientation. In some cases, the EpCAM binding domain according to any one of claims 1-3 and 49-53 (anti-EpCAM domain), the CD3 binding domain (anti-CD3 domain), and an albumin binding domain (anti-ALB domain). In some cases, the anti-CD3 domain comprises an amino acid as set forth in SEQ ID No. 379 or SEQ ID No. 474. In some cases, the anti-ALB domain comprises an amino acid sequence as set forth in SEQ ID No. 375, SEQ ID No. 472, SEQ ID No. 473, SEQ ID No. 482, or SEQ ID No. 483.
In some cases, the anti-EpCAM domain, the anti-CD3 domain, and the anti-ALB domain are in an anti-CD3: anti-ALB: anti-EpCAM orientation. In some cases, the anti-EpCAM domain, the anti-CD3 domain, and the anti-ALB domain are in an anti-EpCAM: anti-ALB: anti-CD3 orientation. In some cases, the anti-EpCAM domain, the anti-CD3 domain, and the anti-ALB domain are in an anti-ALB: anti-EpCAM: anti-CD3 orientation. In some cases, the anti-EpCAM domain, the anti-CD3 domain, and the anti-ALB domain are in an anti-CD3: anti-EpCAM: anti-ALB orientation. In some cases, the anti-EpCAM domain, the anti-CD3 domain, and the anti-ALB domain are in an anti-ALB: anti-CD3: anti-EpCAM orientation. In some cases, the anti-EpCAM domain, the anti-CD3 domain, and the anti-ALB domain are in an anti-EpCAM: anti-CD3: anti-ALB orientation.

One case provides a multivalent protein comprising a sequence as set forth in any one of SEQ ID Nos. 495, 498-502, 569-570, 572, 573, 575, 576, 577, and 578. One case provides an active drug comprising a sequence as set forth in any one of SEQ ID Nos. 494, 571, and 574. One case provides a multivalent protein comprising a sequence as set forth in any one of SEQ ID Nos. 485-491.

One case provides a pharmaceutical composition comprising (i)(a) an EpCAM binding domain according to this disclosure; (i)(b) a conditionally active chimeric antigen receptor according to this disclosure; (i)(c) a conditionally active EpCAM binding protein according to this disclosure; (i)(d) a multispecific protein according to this disclosure; (i)(e) a multivalent protein according to this disclosure; or (i)(f) an active drug according to this disclosure, and (ii) a pharmaceutically acceptable carrier.

One case provides a process for the production of an EpCAM binding domain according to this disclosure, said process comprising culturing a host transformed or transfected with a vector comprising a nucleic acid sequence encoding the EpCAM binding domain according to this disclosure, under conditions allowing the expression of the EpCAM binding domain and recovering and purifying the produced protein from the culture.

One embodiment provides a process for the production of a multispecific protein according to this disclosure, said process comprising culturing a host transformed or transfected with a vector comprising one or more nucleic acid sequences encoding the domains of the multispecific EpCAM binding protein according to this disclosure under conditions allowing the expression of the multispecific protein and recovering and purifying the produced protein from the culture.

One embodiment provides a method for the treatment or amelioration of a proliferative disease as defined by the claims. In some embodiments, the subject is human. In some embodiments, the method further comprises administration of an agent in combination with a multispecific protein according to this disclosure, or a pharmaceutical composition according to this disclosure. In some embodiments, the EpCAM binding domain selectively binds to tumor cells expressing EpCAM. In some embodiments, the tumorous disease comprises a solid tumor disease. In some embodiments, the solid tumor disease is metastatic. In some embodiments, the tumorous disease comprises at least one of: a colorectal cancer, a prostate cancer, a neuroendocrine cancer, a thyroid cancer, a non-small cell lung cancer, a small cell lung cancer, a gastric cancer, an ovarian cancer, an endometrial cancer, a pancreatic cancer, a biliary track cancer, a gall bladder cancer, an esophageal cancer, a breast cancer, an adenocarcinoma.

The following paragraphs are useful for understanding the invention, which is defined in the claims. One case provides a process for the production of a conditionally active chimeric antigen receptor according to this disclosure, said process comprising culturing a host transformed or transfected with a vector comprising a nucleic acid sequence encoding the conditionally active chimeric antigen receptor according to this disclosure, under conditions allowing the expression of the conditionally active chimeric antigen receptor and recovering and purifying the produced protein from the culture. One case provides a process for the production of a conditionally active EpCAM binding protein according to this disclosure, said process comprising culturing a host transformed or transfected with a vector comprising one or more nucleic acid sequences encoding the domains of the conditionally active EpCAM binding protein according to this disclosure, under conditions allowing the expression of the conditionally active EpCAM binding protein and recovering and purifying the produced protein from the culture. One case provides a process for the production of a multivalent protein according this disclosure, said process comprising culturing a host transformed or transfected with a vector comprising one or more nucleic acid sequences encoding the domains of the multivalent protein according to this disclosure, under conditions allowing the expression of the multivalent protein and recovering and purifying the produced protein from the culture. One case provides a process for the production of an active drug according to this disclosure, said process comprising culturing a host transformed or transfected with a vector comprising one or more nucleic acid sequences encoding the domains of the active drug according to this disclosure, under conditions allowing the expression of the active drug and recovering and purifying the produced drug from the culture.

One case provides a cell comprising a CAR according to this disclosure.
One case provides a cell comprising a ProCAR according to this disclosure. In some cases, the cell is a T cell or an NK cell. One case provides a method comprising transfecting a cell according to this disclosure, with a vector or an RNA comprising a nucleotide sequence encoding the CAR or the ProCAR.

One case provides a conditionally active chimeric antigen receptor that has a greater therapeutic index compared to a chimeric antigen receptor (CAR) that does not comprise the (a) binding moiety but is otherwise identical to the conditionally active chimeric antigen receptor. In some cases, the conditionally active chimeric antigen receptor has a therapeutic index that is at least about 5-fold to about 100-fold greater than that of a chimeric antigen receptor (CAR) that does not comprise the (a) binding moiety but is otherwise identical to the conditionally active chimeric antigen receptor. In some cases, the protein comprises a sequence that is at least about 75% identical to a sequence selected from the group consisting of SEQ ID Nos. 498-501, 569-570, 572, 573, 575, 576, 577, and 578. In some cases, the protein comprises a sequence that is at least about 75% identical to the sequence of SEQ ID No. 576. In some cases, the protein comprises a sequence as set forth in SEQ ID No. 576. In some cases, the conditionally active EpCAM binding protein has a greater therapeutic index compared to an EpCAM binding protein that does not comprise the binding moiety (M) or the cleavable linker (L) but is otherwise identical to the conditionally active EpCAM binding protein. In some cases, the conditionally active EpCAM binding protein has a therapeutic index that is at least about 5-fold to about 100-fold greater than that of an EpCAM binding protein that does not comprise the binding moiety (M) or the cleavable linker (L) but is otherwise identical to the conditionally active EpCAM binding protein.

One case provides a pharmaceutical composition comprising: (i)(a) a conditionally active chimeric antigen receptor according to this disclosure; or (i)(b) a conditionally active EpCAM binding protein according to this disclosure, and (ii) a pharmaceutically acceptable carrier. One case provides a method of for the treatment or amelioration of a proliferative disease, or a tumorous disease, comprising the administration of the conditionally active chimeric antigen receptor according to this disclosure, or a pharmaceutical composition comprising the same, to a subject in need thereof.

One case provides a method of for the treatment or amelioration of a proliferative disease, or a tumorous disease, comprising the administration of the conditionally active EpCAM binding protein according to this disclosure, or a pharmaceutical composition comprising the same, to a subject in need thereof. In some cases, the subject is human. In some cases, the tumorous disease comprises at least one of: a colorectal cancer, a prostate cancer, a neuroendocrine cancer, a thyroid cancer, a non-small cell lung cancer, a small cell lung cancer, a gastric cancer, an ovarian cancer, an endometrial cancer, a pancreatic cancer, a biliary track cancer, a gall bladder cancer, an esophageal cancer, a breast cancer, an adenocarcinoma.

One case provides a method of increasing a therapeutic index of an EpCAM binding domain, the method comprising conjugating the EpCAM binding domain to a binding moiety comprising a cleavable linker and a non-CDR loop,
- wherein the non-CDR loop comprises a binding site specific for the EpCAM binding domain,
- wherein the EpCAM binding domain is masked from binding its target by the binding moiety,
- wherein the EpCAM binding domain is able to bind its target upon cleavage of the cleavable linker.

In some cases, the EpCAM binding domain comprises a complementarity determining region 1 (CDR1), a CDR2, and a CDR3, wherein the CDR1 comprises a sequence selected from the group consisting of SEQ ID Nos. 39-76, or a sequence comprising one or more substitutions in a sequence selected from the group consisting of SEQ ID Nos. 39-76; the CDR2 comprising a sequence selected from the group consisting of SEQ ID Nos. 77-114 or a sequence comprising one or more substitutions in a sequence selected from the group consisting of SEQ ID Nos. 77-114; and the CDR3 comprising a sequence selected from the group consisting of SEQ ID Nos. 115-152 or a sequence comprising one or more substitutions in a sequence selected from the group consisting of SEQ ID Nos. 115-152. In some cases, the EpCAM binding domain conjugated to the binding moiety is part of a conditionally active multispecific protein, wherein the multispecific protein further comprises a CD3 binding domain. In some cases, the binding moiety comprises a sequence that is at least about 75% identical to a sequence selected from the group consisting of SEQ ID Nos. 472-473 and 482-483. In some cases, the CD3 binding domain comprises a sequence that is at least about 75% identical to a sequence selected from the group consisting of SEQ ID No. 379 and 474. In some cases, the cleavable linker comprises a sequence selected from the group consisting of: SEQ ID Nos. 425-471, 503-506, and 581, or a sequence comprising one or more substitutions in a sequence selected from the group consisting of: SEQ ID Nos. 425-471, 503-506, and 581.

In some cases, the EpCAM binding domain comprises a sequence that is at least 75% identical to a sequence selected from the group consisting of SEQ ID Nos. 1-38, 207-209, and 496-497. In some cases, the conditionally active multispecific protein comprises a sequence that is at least 75% identical to a sequence selected from the group consisting of SEQ ID Nos. 498-501, 569-570, 572, 573, 575, 576, 577, and 578. In some cases, the conditionally active multispecific protein comprises a sequence that is at least 75% identical to SEQ ID No. 576. In some cases, the conditionally active multispecific protein comprises a sequence as set forth in SEQ ID No. 576. In some cases, the EpCAM binding domain conjugated to the binding moiety is part of a conditionally active chimeric antigen receptor, wherein the conditionally active chimeric antigen receptor further comprises at least one of: a transmembrane domain, an intracellular signaling domain, and a costimulatory domain. In some cases, the EpCAM binding domain comprises a complementarity determining region 1 (CDR1), a CDR2, and a CDR3, wherein the CDR1 comprises a sequence selected from the group consisting of SEQ ID Nos. 39-76, or a sequence comprising one or more substitutions in a sequence selected from the group consisting of SEQ ID Nos. 39-76; the CDR2 comprising a sequence selected from the group consisting of SEQ ID Nos. 77-114 or a sequence comprising one or more substitutions in a sequence selected from the group consisting of SEQ ID Nos. 77-114; and the CDR3 comprising a sequence selected from the group consisting of SEQ ID Nos. 115-152 or a sequence comprising one or more substitutions in a sequence selected from the group consisting of SEQ ID Nos. 115-152. In some cases, the binding moiety comprises a sequence that is at least about 75% identical to a sequence selected from the group consisting of SEQ ID Nos. 472-473 and 482-483. In some cases, the EpCAM binding domain comprises a sequence that is at least 75% identical to a sequence selected from the group consisting of SEQ ID Nos. 1-38, 207-209, and 496-497. In some cases, the conditionally active chimeric antigen receptor comprises a sequence that is at least about 75% identical to a sequence selected from the group consisting of SEQ ID Nos. 485-491.

One case provides a method of increasing a therapeutic index of an EpCAM binding protein comprising a first target antigen binding domain and a second target antigen binding domain, wherein at least one of the first and the second target antigen binding domain comprises an EpCAM binding domain, the method comprising conjugating the first or the second target antigen binding domain to a binding moiety comprising a cleavable linker and a non-CDR loop,
- wherein the non-CDR loop comprises a binding site specific for the first or the second target antigen binding domain,
- wherein at least one of the first or the second target antigen binding domain is masked from binding its target by the binding moiety, and
- wherein the first or the second target antigen binding domain that is masked, is able to bind its target upon cleavage of the cleavable linker.

In some cases, the EpCAM binding domain comprises a complementarity determining region 1 (CDR1), a CDR2, and a CDR3, wherein the CDR1 comprises a sequence selected from the group consisting of SEQ ID Nos. 39-76, or a sequence comprising one or more substitutions in a sequence selected from the group consisting of SEQ ID Nos. 39-76; the CDR2 comprising a sequence selected from the group consisting of SEQ ID Nos. 77-114 or a sequence comprising one or more substitutions in a sequence selected from the group consisting of SEQ ID Nos. 77-114; and the CDR3 comprising a sequence selected from the group consisting of SEQ ID Nos. 115-152 or a sequence comprising one or more substitutions in a sequence selected from the group consisting of SEQ ID Nos. 115-152. In some cases, the non-CDR loop comprises a binding site specific for the EpCAM binding domain. In some cases, at least one of the first or the second target antigen binding domain comprises a CD3 binding domain. In some cases, the non-CDR loop comprises a binding site specific for the CD3 binding domain. In some cases, the CD3 binding domain comprises a sequence that is at least about 75% identical to SEQ ID No. 474. In some cases, the binding moiety comprises a sequence that is at least about 75% identical to a sequence selected from the group consisting of SEQ ID Nos. 472-473 and 482-483. In some cases, the EpCAM binding domain comprises a sequence that is at least 75% identical to a sequence selected from the group consisting of SEQ ID Nos. 1-38, 207-209, and 496-497. In some cases, the cleavable linker comprises a sequence selected from the group consisting of: SEQ ID Nos. 425-471, 503-506, and 581, or a sequence comprising one or more substitutions in a sequence selected from the group consisting of: SEQ ID Nos. 425-471, 503-506, and 581. In some cases, the conditionally active multispecific protein comprises a sequence that is at least 75% identical to a sequence selected from the group consisting of SEQ ID Nos. 498-501, 569-570, 572, 573, 575, 576, 577, and 578. In some cases, the conditionally active multispecific protein comprises a sequence that is at least 75% identical to SEQ ID No. 576. In some cases, the conditionally active multispecific protein comprises a as set forth in SEQ ID No. 576.

One case provides a method of increasing a therapeutic index of an EpCAM binding protein comprising an EpCAM binding domain and a CD3 binding domain, the method comprising conjugating CD3 binding domain to a binding moiety comprising a cleavable linker and a non-CDR loop, wherein the non-CDR loop comprises a binding site specific for CD3 binding domain. In some cases, the EpCAM binding domain comprises a complementarity determining region 1 (CDR1), a CDR2, and a CDR3, wherein the CDR1 comprises a sequence selected from the group consisting of SEQ ID Nos. 39-76, or a sequence comprising one or more substitutions in a sequence selected from the group consisting of SEQ ID Nos. 39-76; the CDR2 comprising a sequence selected from the group consisting of SEQ ID Nos. 77-114 or a sequence comprising one or more substitutions in a sequence selected from the group consisting of SEQ ID Nos. 77-114; and the CDR3 comprising a sequence selected from the group consisting of SEQ ID Nos. 115-152 or a sequence comprising one or more substitutions in a sequence selected from the group consisting of SEQ ID Nos. 115-152. In some cases, the CD3 binding domain comprises a sequence that is at least about 75% identical to SEQ ID No. 474. In some cases, the binding moiety comprises a sequence that is at least about 75% identical to a sequence selected from the group consisting of SEQ ID Nos. 472-473 and 482-483. In some cases, the EpCAM binding domain comprises a sequence that is at least 75% identical to a sequence selected from the group consisting of SEQ ID Nos. 1-38, 207-209, and 496-497. In some cases, the cleavable linker comprises a sequence selected from the group consisting of: SEQ ID Nos. 425-471, 503-506, and 581, or a sequence comprising one or more substitutions in a sequence selected from the group consisting of: SEQ ID Nos. 425-471, 503-506, and 581. In some cases, the conditionally active multispecific protein comprises a sequence that is at least 75% identical to a sequence selected from the group consisting of SEQ ID Nos. 498-501, 569-570, 572, 573, 575, 576, 577, and 578. In some cases, the conditionally active multispecific protein comprises a sequence that is at least 75% identical to SEQ ID No. 576. In some cases, the conditionally active multispecific protein comprises a as set forth in SEQ ID No. 576.

One case provides an EpCAM targeting conditionally active multispecific protein, comprising: an EpCAM binding domain, a CD3 binding domain, an albumin binding domain, wherein the albumin binding domain comprises a non-CDR loops that comprises a binding site specific for the CD3 binding domain and a cleavable linker, wherein the EpCAM binding domain comprises a complementarity determining region 1 (CDR1), a CDR2, and a CDR3, wherein the CDR1 comprises a sequence selected from the group consisting of SEQ ID Nos. 39-76, or a sequence comprising one or more substitutions in a sequence selected from the group consisting of SEQ ID Nos. 39-76; the CDR2 comprising a sequence selected from the group consisting of SEQ ID Nos. 77-114 or a sequence comprising one or more substitutions in a sequence selected from the group consisting of SEQ ID Nos. 77-114; and the CDR3 comprising a sequence selected from the group consisting of SEQ ID Nos. 115-152 or a sequence comprising one or more substitutions in a sequence selected from the group consisting of SEQ ID Nos. 115-152. In some cases, the albumin binding domain comprises a sequence that is at least about 75% identical to a sequence selected from the group consisting of SEQ ID Nos. 472-473 and 482-483. In some cases, the the EpCAM binding domain comprises a sequence that is at least 75% identical to a sequence selected from the group consisting of SEQ ID Nos. 1-38, 207-209, and 496-497. In some cases, the cleavable linker comprises a sequence selected from the group consisting of: SEQ ID Nos. 425-471, 503-506, and 581, or a sequence comprising one or more substitutions in a sequence selected from the group consisting of: SEQ ID Nos. 425-471, 503-506, and 581. In some cases, the conditionally active multispecific protein comprises a sequence that is at least 75% identical to a sequence selected from the group consisting of SEQ ID Nos. 498-501, 569-570, 572, 573, 575, 576, 577, and 578. In some cases, the conditionally active multispecific protein comprises a sequence that is at least 75% identical to SEQ ID No. 576. In some cases, the conditionally active multispecific protein comprises a as set forth in SEQ ID No. 576.

One case provides a pharmaceutical composition comprising an EpCAM targeting conditionally active multispecific protein of this disclosure. In some cases, the pharmaceutical composition further comprises a pharmaceutically acceptable carrier. One case provides a process for the production of EpCAM targeting conditionally active multispecific protein of this disclosure, said process comprising culturing a host transformed or transfected with a vector comprising one or more nucleic acid sequences encoding the domains of the EpCAM targeting conditionally active multispecific protein this disclosure, under conditions allowing the expression of the EpCAM targeting conditionally active multispecific protein and recovering and purifying the produced protein from the culture.

One case provides a method for the treatment or amelioration of a proliferative disease, or a tumorous disease, comprising the administration of an of EpCAM targeting conditionally active multispecific protein of this disclosure, or a pharmaceutical composition according to claim 149 or 150, to a subject in need thereof. In some cases, the tumorous disease comprises a solid tumor disease. In some cases, the solid tumor disease is metastatic. In some cases, the tumorous disease comprises at least one of: a colorectal cancer, a prostate cancer, a neuroendocrine cancer, a thyroid cancer, a non-small cell lung cancer, a small cell lung cancer, a gastric cancer, an ovarian cancer, an endometrial cancer, a pancreatic cancer, a biliary track cancer, a gall bladder cancer, an esophageal cancer, a breast cancer, an adenocarcinoma.

One case provides an EpCAM binding domain comprising a sequence that is at least about 75% identical to a sequence selected from the group consisting of SEQ ID Nos. 1-38. In some cases, the EpCAM binding domain comprises a CDR1, a CDR2, and a CDR3. In some cases, the CDR1 comprises a sequence selected from the group consisting of SEQ ID Nos. 39-76, or one or more amino acid substitutions relative to a sequence selected from the group consisting of SEQ ID Nos. 39-76. In some cases, the CDR2 comprises a sequence selected from the group consisting of SEQ ID Nos. 77-114, or one or more amino acid substitutions relative to a sequence selected from the group consisting of SEQ ID Nos. 77-114. In some cases, the CDR3 comprises a sequence selected from the group consisting of SEQ ID Nos. 115-152, or one or more substitutions relative to a sequence selected from SEQ ID Nos. 115-152. In some cases, the EpCAM binding domain comprises a sequence that is at least about 75% identical to a sequence selected from the group consisting of SEQ ID Nos. 1-38. In some cases, the EpCAM binding domain comprises a sequence selected from the group consisting of SEQ ID Nos. 1-38. In some cases, the EpCAM binding domain is a humanized antibody or an antigen binding fragment thereof. In some cases, the EpCAM binding domain is a single domain antibody, a VHH domain, a scFv, a VH domain, a VL domain, a Fab, a Fab', a non-Ig domain, a ligand, a knottin, or a small molecule entity. In some cases, the EpCAM binding domain comprises the single domain antibody. In some cases, the binding domain binds to EpCAM with a binding affinity (Kd) of about 0.001 nM to about 500 nM. In some cases, the EpCAM binding domain binds to human EpCAM, mouse EpCAM, cynomolgus EpCAM, or a combination thereof.

One case provides a multispecific protein comprising an EpCAM binding domain, wherein the EpCAM binding domain is according to this disclosure. In some cases, the multispecific protein comprises the EpCAM binding domain according to this disclosure (anti-EpCAM domain), and a CD3 binding domain (anti-CD3 domain). In some cases, the anti-EpCAM domain and the anti-CD3 domain are in an anti-EpCAM:anti-CD3 orientation. In some cases, the anti-EpCAM domain and the anti-CD3 domain are in an anti-CD3: anti-EpCAM orientation. In some cases, the multispecific protein comprises an EpCAM binding domain according to this disclosure (anti-EpCAM domain), the CD3 binding domain (anti-CD3 domain), and an albumin binding domain (anti-ALB domain). In some cases, the anti-CD3 domain comprises an amino acid as set forth in SEQ ID No. 379 or SEQ ID No. 474. In some cases, the anti-ALB domain comprises an amino acid sequence as set forth in SEQ ID No. 375, SEQ ID No. 472, SEQ ID No. 473, SEQ ID No. 482, or SEQ ID No. 483. In some cases, the anti-EpCAM domain, the anti-CD3 domain, and the anti-ALB domain are in an anti-CD3: anti-ALB: anti-EpCAM orientation. In some cases, the anti-EpCAM domain, the anti-CD3 domain, and the anti-ALB domain are in an anti-EpCAM: anti-ALB: anti-CD3 orientation. In some cases, the anti-EpCAM domain, the anti-CD3 domain, and the anti-ALB domain are in an anti-ALB: anti-EpCAM: anti-CD3 orientation. In some cases, the anti-EpCAM domain, the anti-CD3 domain, and the anti-ALB domain are in an anti-ALB: anti-EpCAM: anti-CD3 orientation. In some cases, the anti-EpCAM domain, the anti-CD3 domain, and the anti-ALB domain are in an anti-CD3: anti-EpCAM: anti-ALB orientation. In some cases, the anti-EpCAM domain, the anti-CD3 domain, and the anti-ALB domain are in an anti-ALB: anti-CD3: anti-EpCAM orientation. In some cases, the anti-EpCAM domain, the anti-CD3 domain, and the anti-ALB domain are in an anti-EpCAM: anti-CD3: anti-ALB orientation.

One case provides a multivalent protein comprising a sequence as set forth in any one of SEQ ID Nos. 485-491. One case provides a pharmaceutical composition comprising (i)(a) an EpCAM binding domain according to this disclosure, (i)(b) a multispecific protein according to this disclosure, or (i)(c) a multivalent protein according to this disclosure; and (ii) a pharmaceutically acceptable carrier.

One case a process for the production of an EpCAM binding domain according to this disclosure, said process comprising culturing a host transformed or transfected with a vector comprising a nucleic acid sequence encoding the EpCAM binding domain according to this disclosure under conditions allowing the expression of the EpCAM binding domain and recovering and purifying the produced protein from the culture. One case provides a process for the production of a multispecific protein according to any this disclosure, said process comprising culturing a host transformed or transfected with a vector comprising one or more nucleic acid sequences encoding the domains of the multispecific EpCAM binding protein according to this disclosure under conditions allowing the expression of the multispecific protein and recovering and purifying the produced protein from the culture.

One case provides a method for the treatment or amelioration of a proliferative disease, or a tumorous disease, comprising the administration of an EpCAM binding domain according to this disclosure, or a pharmaceutical composition according to this disclosure, to a subject in need thereof.

One case provides a method for the treatment or amelioration of a proliferative disease, or a tumorous disease, comprising the administration of the multispecific protein according to this disclosure, a multivalent protein according to this disclosure, or a pharmaceutical composition according to this disclosure, to a subject in need thereof. In some cases, the subject is human. In some cases, the method further comprises administration of an agent in combination with an EpCAM binding domain according to this disclosure, a multispecific protein according to this disclosure, a multivalent protein according this disclosure, or a pharmaceutical composition according to this disclosure. In some cases, the EpCAM binding domain selectively binds to tumor cells expressing EpCAM. In some cases, the tumorous disease comprises a solid tumor disease. In some cases, the solid tumor disease is metastatic.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
**FIG 1** provides results from a representative T cell dependency cellular cytotoxicity assay with NCI-H508 cells using exemplary fusion proteins of this disclosure containing an anti-EpCAM domain as described herein and an anti-CD3 domain.
**FIG. 2** provides results from a representative T cell dependency cellular cytotoxicity assay using exemplary fusion proteins of this disclosure containing an anti-EpCAM domain as described herein and an anti-CD3 domain.
**FIG. 3** provides results from a representative T cell dependency cellular cytotoxicity assay using exemplary fusion proteins of this disclosure containing an anti-EpCAM domain as described herein and an anti-CD3 domain.
**FIG. 4** provides results from a representative T cell dependency cellular cytotoxicity assay using exemplary fusion proteins of this disclosure containing an anti-EpCAM domain as described herein and an anti-CD3 domain.
**FIG. 5** provides results from a representative T cell dependency cellular cytotoxicity assay using exemplary fusion proteins of this disclosure containing a humanized anti-EpCAM domain as described herein and an anti-CD3 domain.

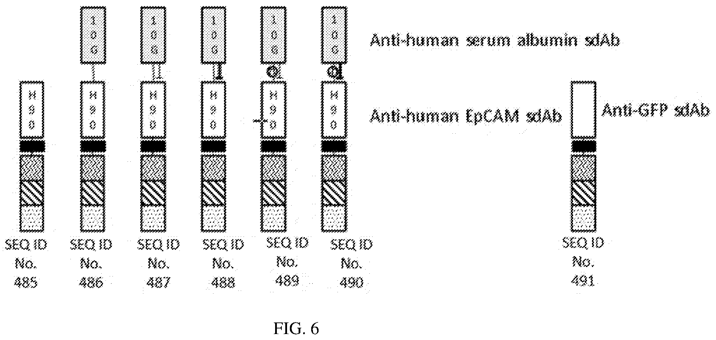

**FIG. 6** illustrate exemplary ProCAR constructs. One exemplary construct includes an anti-human EpCAM sdAb, a FLAG epitope, a CD8 hinge/transmembrane domain, a 4-1BB intracellular domain, and a CD3 zeta intracellular domain (SEQ ID NO: 485). One exemplary construct includes an anti-human serum albumin sdAb, an anti-human EpCAM sdAb, a FLAG epitope, a CD8 hinge/transmembrane domain, a 4-1BB intracellular domain, and a CD3 zeta intracellular domain (SEQ ID NO: 486). One exemplary construct includes an anti-human serum albumin sdAb, an anti-human EpCAM sdAb, a FLAG epitope, a CD8 hinge/transmembrane domain, a 4-1BB intracellular domain, and a CD3 zeta intracellular domain (SEQ ID NO: 487). One exemplary construct includes an anti-human serum albumin sdAb, an anti-human EpCAM sdAb, a FLAG epitope, a CD8 hinge/transmembrane domain, a 4-1BB intracellular domain, and a CD3 zeta intracellular domain (SEQ ID NO: 488). One exemplary construct includes an anti-human serum albumin sdAb, a protease cleavage site 3, an anti-human EpCAM sdAb, a FLAG epitope, a CD8 hinge/transmembrane domain, a 4-1BB intracellular domain, and a CD3 zeta intracellular domain (SEQ ID NO: 489). One exemplary construct includes an anti-human serum albumin sdAb, a protease cleavage site 3, an anti-human EpCAM sdAb, a FLAG epitope, a CD8 hinge/transmembrane domain, a 4-1BB intracellular domain, and a CD3 zeta intracellular domain (SEQ ID NO: 490). One exemplary construct includes an anti-GFP sdAb, a FLAG epitope, a CD8 hinge/transmembrane domain, a 4-1BB intracellular domain, and a CD3 zeta intracellular domain (SEQ ID NO: 491).
**FIG. 7** demonstrates steric blocking of the anti-EpCAM sdAb H90 by HSA of the indicated constructs at ratios 10:1, 5:1, 2.5:1, and 1.25:1 CAR-T:Target cells.
**FIGS. 8A-8C** provide histograms of EpCAM-Fc/Alexa Fluor 647 staining of CAR-T cells from FIG. 6 that have been grouped into low (**FIG. 8A**), medium (**FIG. 8B**), or high (**FIG. 8C**) CAR expression based on anti-FLAG staining that demonstrate the efficacy of EpCAM mask 1 in blocking ProCAR EpCAM-binding activity. The numbers refer to sequence identifiers (*e.g.,* 491 = SEQ ID No. 491).
**FIGS. 9A-9C** provide histograms of EpCAM-Fc/Alexa Fluor 647 staining of CAR-T cells from FIG. 6 that have been grouped into low (**FIG. 9A**), medium (**FIG. 9B**), or high (**FIG. 9C**) CAR expression based on anti-FLAG staining that demonstrate the efficacy of EpCAM mask 2 in blocking ProCAR EpCAM-binding activity. The numbers refer to sequence identifiers (*e.g.,* 488 = SEQ ID No. 488).
**FIG. 10** demonstrates masking of the anti-EpCAM sdAb H90 of constructs of FIG. 6 at ratios 10:1, 5:1, 2.5:1, and 1.25:1 CAR-T:Target cells.
**FIG. 11** demonstrates protease site-dependent activation of EpCAM ProCAR mask 2 cell killing activity.
**FIGS. 12A-12C** illustrate protease activation of EpCAM Mask 1 ProCAR antigen binding activity at low (**FIG. 12A**), medium (**FIG. 12B**), or high (**FIG. 12C**) CAR expression based on anti-FLAG staining. The numbers refer to sequence identifiers (*e.g.,* 489 = SEQ ID No. 489).
**FIGS. 13A-13C** illustrate protease activation of EpCAM Mask 2 ProCAR antigen binding activity at low (**FIG. 13A**), medium (**FIG. 13B**), or high (**FIG. 13C**) CAR expression based on anti-FLAG. The numbers refer to sequence identifiers (*e.g.,* 485 = SEQ ID No. 485).
**FIGS. 14A-14C** show body weight percent change in mice, following administering exemplary EpCAM ProTriTAC molecules (EpCAM ProTriTAC containing linker L040 in **FIG.14B****;** EpCAM ProTriTAC containing a non-cleavable linker in **FIG. 14C**) and EpCAM TriTAC molecules (**FIG. 14A**) of this disclosure.
**FIG. 15A-15C** show binding kinetics for various EpCAM binding domains; H13 (**FIG. 15A**), H90 (**FIG. 15B**), and H90.2 (**FIG. 15C**).
**FIGS. 16A-16C** show results of T cell dependent cytotoxicity assay (TDCC assay), on HCT116 cells, using ProTriTAC, TriTAC proteins, or active drugs (CT) containing EpCAM binding domains H13 (**FIG. 16A**), H90.2 (**FIG. 16B**), and H138.2 (**FIG. 16C**).
**FIGS. 17A-17C** show results of T cell dependent cytotoxicity assay (TDCC assay), on NCI-H929 cells, using ProTriTAC, TriTAC proteins, or active drugs (CT) containing EpCAM binding domains H13 (**FIG. 17A**), H90.2 (**FIG. 17B**), and H138.2 (**FIG. 17C**).
**FIGS. 18A-18B** show results of T cell dependent cytotoxicity assay (TDCC assay), on HCT116 cells (**FIG. 18A**) and HCT116 (EpCAM-knock out; KO) (**FIG. 18B**), using TriTAC, containing EpCAM binding domains H13 and H90.2.
**FIG. 19** shows results of a flow cytometry assay for measuring binding of HCT116 cells wild-type (WT) and HCT116 (EpCAM-knock out; KO) cells and EpCAM binding domains H13 and H90.2.
**FIG. 20** shows results of a TDCC assay on SKBR3 cells, using a non-cleavable prodrug or active drug (CT) versions containing EpCAM binding domains H13 or H90.2
**FIGS. 21A-21B** **illustrates** representative plots demonstrating the masking effect achieved by the non-cleavable prodrug versions containing EpCAM binding domains H13 (**FIG. 21A**) or H90.2 (**FIG. 21B**), compared to active drugs containing the same.
**FIGS. 22A-22H** show results of a TDCC assay on various cell lines, using a non-cleavable prodrug (NCLV), ProTriTAC (L040), or active drug (CT) versions containing EpCAM binding domains H13 or H90.2. **FIG. 22A** shows the results for CAPAN2 cells; **FIG. 22B** shows the results for DMS53 cells, **FIG. 22C** shows the results for HepG2 cells; **FIG. 22D** shows the results for KMRC3 cells; **FIG. 22E** shows the results for MDAPCA2b cells; **FIG. 22F** shows the results for OVCAR8 cells; **FIG. 22G** shows the results for PECAPJ41 cells; **FIG. 22H** shows the results for SKBR3 cells.
**FIGS. 23A-23B** show efficacy of ProTriTAC (**FIG. 23B**) and TriTAC (**FIG. 23A**) versions containing EpCAM binding domain H13, in a mouse tumor model.
**FIGS. 24A-24D** show cytokine profiles following administration of ProTriTAC and TriTAC versions containing EpCAM binding domain H13, in cynomolgus monkeys; **FIG. 24A** illustrates IFN-gamma levels; **FIG. 24B** illustrates IL-2 levels; **FIG. 24C** illustrates IL-6 levels, and **FIG. 24D** illustrates IL-10 levels.
**FIGS. 25A-25B** show plasma concentrations following administration of ProTriTAC and TriTAC versions containing EpCAM binding domain H13 (**FIG. 25A**) or H90.2 (**FIG. 25B**), in cynomolgus monkeys.
**FIG. 26** illustrates a variable domain of an immunoglobulin molecule, comprising complementarity determining regions (CDR1, CDR2, and CDR3), and non-CDR loops connecting the beta strand (AB, CC', C" D, EF, and DE).
**FIG. 27** illustrates an exemplary arrangement of a target antigen binding domain (aTarget 1), cleavable linker, and a binding moiety (aTarget 2) of the present disclosure.
**FIG. 28** illustrates an example of a conditionally active receptor as described herein.

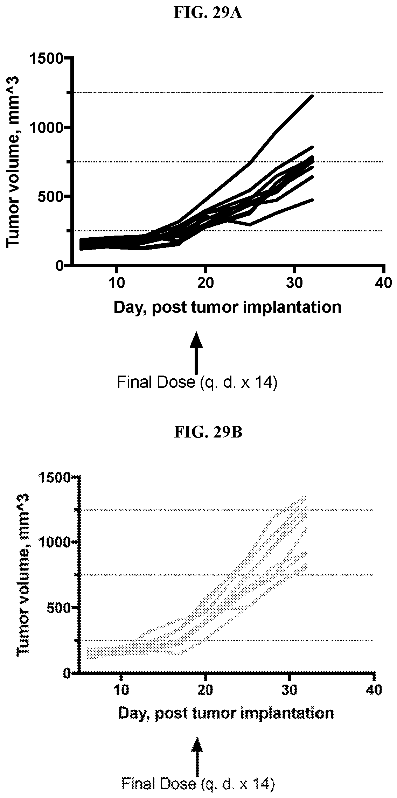

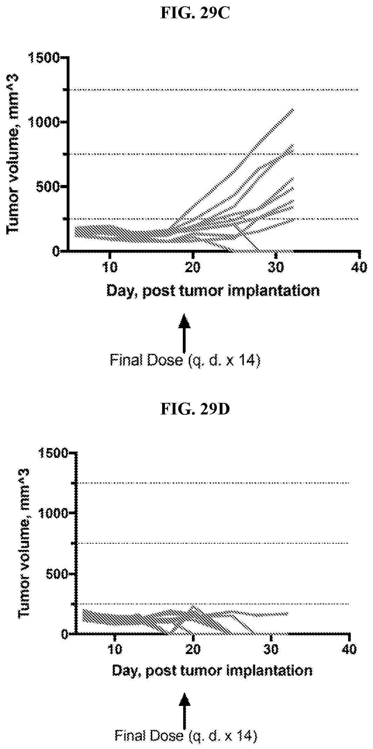

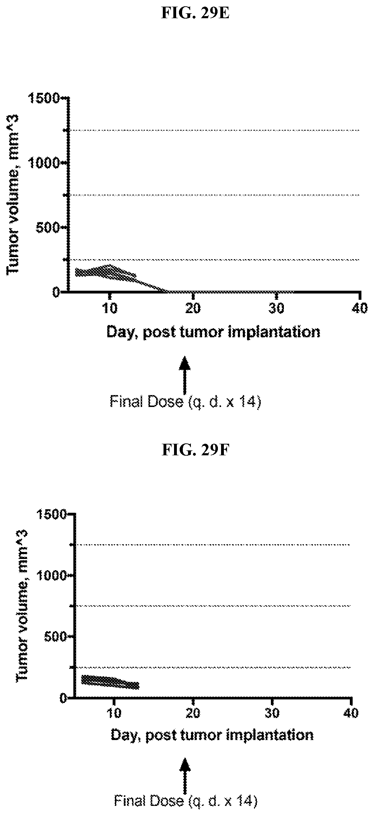

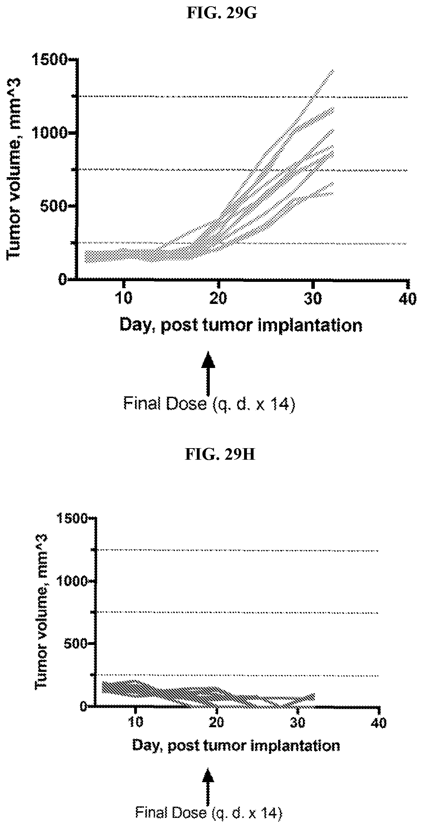

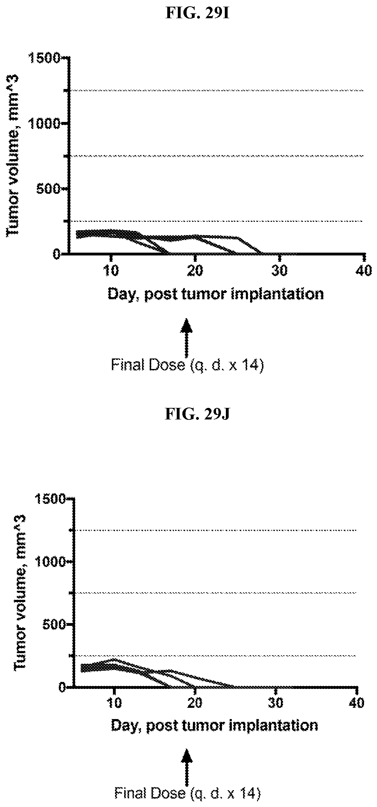

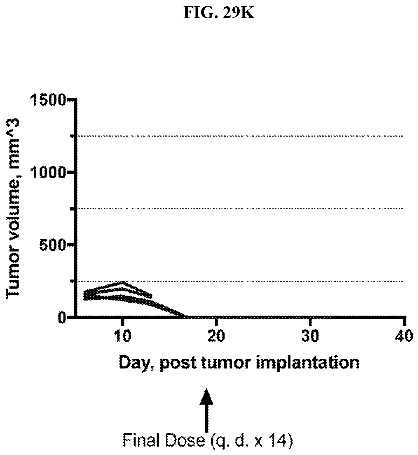

**FIGS. 29A-29K** show efficacy of Control TriTAC, EpCAM ProTriTAC and EpCAM TriTAC proteins in an established LoVo (colon cancer) tumor model; **FIG. 29A** shows results for control TriTAC; **FIG. 29B** shows the results for EpCAM TriTAC at 0.003 mg/kg; **FIG. 29C** shows the results for EpCAM TriTAC at 0.01 mg/kg; **FIG. 29D** shows the results for EpCAM TriTAC at 0.03 mg/kg; **FIG. 29E** shows the results for EpCAM TriTAC at 0.1 mg/kg; **FIG. 29F** shows the results for EpCAM TriTAC at 0.1 mg/kg; **FIG. 29G** shows the results for EpCAM ProTriTAC at 0.03 mg/kg; **FIG. 29H** shows the results for EpCAM ProTriTAC at 0.1 mg/kg; **FIG. 29I** shows the results for EpCAM ProTriTAC at 0.3 mg/kg; **FIG. 29J** shows the results for EpCAM ProTriTAC at 1 mg/kg; and **FIG. 29K** shows the results for EpCAM ProTriTAC at 3 mg/kg.
**FIGS. 30A-30E** show percent survival (**FIGS. 30A-30B**), alanine transaminase (ALT) (**FIG. 30C**), aspartate transaminase (AST) (**FIG. 30D**), and total bilirubin (**FIG. 30E**) levels following administering a ProTriTAC or TriTAC versions containing EpCAM binding domain.
**FIG. 31** shows the results and summary for a histopathological study using a control GFP TriTAC, an EpCAM TriTAC, or an EpCAM ProTriTAC.
**FIG. 32** provides a schematic representation of an EpCAM targeting trispecific protein.

### DETAILED DESCRIPTION OF THE INVENTION

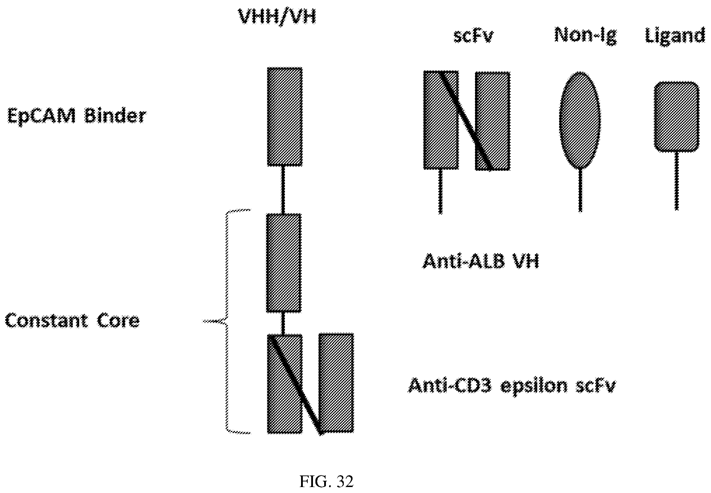

Described herein are trispecific proteins that target EpCAM, pharmaceutical compositions thereof, as well as nucleic acids, recombinant expression vectors and host cells for making such proteins thereof. Also provided are methods of using the disclosed EpCAM targeting trispecific proteins in the prevention, and/or treatment of diseases, conditions and disorders. The EpCAM targeting trispecific proteins are capable of specifically binding to EpCAM as well as CD3 and have a half-life extension domain, such as a domain binding to human albumin (ALB). **Fig. 32** depicts one non-limiting example of a trispecific EpCAM-binding protein.
The references to the methods of treatment by therapy or surgery or in vivo diagnosis methods in this description are to be interpreted as references to compounds of the present invention for use in those methods.

### Certain definitions

The terminology used herein is for the purpose of describing particular cases only and is not intended to be limiting. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

The term "about" or "approximately" means within an acceptable error range for the particular value as determined by one of ordinary skill in the art, which will depend in part on how the value is measured or determined, *e.g.*, the limitations of the measurement system. For example, "about" can mean within 1 or more than 1 standard deviation, per the practice in the given value. Where particular values are described in the application and claims, unless otherwise stated the term "about" should be assumed to mean an acceptable error range for the particular value.

The terms "individual," "patient," or "subject" are used interchangeably. None of the terms require or are limited to situation characterized by the supervision (*e.g.* constant or intermittent) of a health care worker (*e.g.* a doctor, a registered nurse, a nurse practitioner, a physician's assistant, an orderly, or a hospice worker).

An "antibody" typically refers to a Y-shaped tetrameric protein comprising two heavy (H) and two light (L) polypeptide chains held together by covalent disulfide bonds and noncovalent interactions. Human light chains comprise a variable domain (VL) and a constant domain (CL) wherein the constant domain may be readily classified as kappa or lambda based on amino acid sequence and gene loci. Each heavy chain comprises one variable domain (VH) and a constant region, which in the case of IgG, IgA, and IgD, comprises three domains termed CH1, CH2, and CH3 (IgM and IgE have a fourth domain, CH4). In IgG, IgA, and IgD classes the CH1 and CH2 domains are separated by a flexible hinge region, which is a proline and cysteine rich segment of variable length (generally from about 10 to about 60 amino acids in IgG). The variable domains in both the light and heavy chains are joined to the constant domains by a "J" region of about 12 or more amino acids and the heavy chain also has a "D" region of about 10 additional amino acids. Each class of antibody further comprises inter-chain and intra-chain disulfide bonds formed by paired cysteine residues. There are two types of native disulfide bridges or bonds in immunoglobulin molecules: interchain and intrachain disulfide bonds. The location and number of interchain disulfide bonds vary according to the immunoglobulin class and species. Interchain disulfide bonds are located on the surface of the immunoglobulin, are accessible to solvent and are usually relatively easily reduced. In the human IgG1 isotype there are four interchain disulfide bonds, one from each heavy chain to the light chain and two between the heavy chains. The interchain disulfide bonds are not required for chain association. As is well known the cysteine rich IgG1 hinge region of the heavy chain has generally been held to consist of three parts: an upper hinge, a core hinge, and a lower hinge. Those skilled in the art will appreciate that that the IgG1 hinge region contain the cysteines in the heavy chain that comprise the interchain disulfide bonds (two heavy/heavy, two heavy/light), which provide structural flexibility that facilitates Fab movements. The interchain disulfide bond between the light and heavy chain of IgG1 are formed between C214 of the kappa or lambda light chain and C220 in the upper hinge region of the heavy chain. The interchain disulfide bonds between the heavy chains are at positions C226 and C229 (all numbered per the EU index according to Kabat, et al., infra.)

As used herein the term "antibody" includes polyclonal antibodies, multiclonal antibodies, monoclonal antibodies, chimeric antibodies, deimmunized, humanized and primatized antibodies, CDR grafted antibodies, human antibodies, recombinantly produced antibodies, intrabodies, multispecific antibodies, bispecific antibodies, monovalent antibodies (*e.g.,* a monovalent IgG), multivalent antibodies, anti-idiotypic antibodies, synthetic antibodies, including muteins and variants thereof, immunospecific antibody fragments such as: hcIgG, a V-NAR, Fv, Fd, Fab, F(ab')2, F(ab'), Fab2, Fab3 fragments, single-chain fragments (*e.g.,* di-scFv, scFv, scFvFc, scFv-zipper, scFab), disulfide-linked Fvs (sdFv), a Fd fragment consisting of the VH and CH1 domains, linear antibodies, single domain antibodies such as nanobodies or single variable domain antibodies comprising merely one variable domain such as sdAb (VH, VL, or VHH domains), "r IgG" ("half antibody"), diabodies, single chain diabodies, tandem diabodies (Tandab's), tandem di-scFv, tandem tri-scFv, "minibodies" are in some instances exemplified by a structure which is as follows: (VH-VL-CH3)2, (scFv-CH3)2, ((scFv)2-CH3+CH3), ((scFv)2-CH3) or (scFv-CH3-scFv)2, multibodies such as triabodies or tetrabodies,; and derivatives thereof including Fc fusions and other modifications, and any other immunoreactive molecule so long as it comprises a domain having a binding site for preferential association or binding with an EpCAM-protein. Moreover, unless dictated otherwise by contextual constraints the term further comprises all classes of antibodies (*i.e.* IgA, IgD, IgE, IgG, and IgM) and all subclasses (*i.e.,* IgG1, IgG2, IgG3, IgG4, IgA1, and IgA2). Heavy-chain constant domains that correspond to the different classes of antibodies are typically denoted by the corresponding lower case Greek letter alpha, delta, epsilon, gamma, and mu, respectively. Light chains of the antibodies from any vertebrate species can be assigned to one of two clearly distinct types, called kappa (kappa) and lambda (lambda ), based on the amino acid sequences of their constant domains. In some embodiments, the EpCAM binding proteins comprise a heavy chain only antibody, such as a VH or a VHH domain. In some cases, the EpCAM binding proteins comprise a heavy chain only antibody that is an engineered human VH domain. In some examples, the engineered human VH domain is produced by panning of phage display libraries. In some embodiments, the EpCAM binding proteins comprise a VHH. The term "VHH," as used herein, refers to single chain antibody binding domain devoid of light chain. In some cases, a VHH is derived from an antibody of the type that can be found in Camelidae or cartilaginous fish which are naturally devoid of light chains or to a synthetic and non-immunized VHH which can be constructed accordingly. Each heavy chain comprises a variable region encoded by V-, D- and J exons. A VHH, in some cases, is a natural VHH, such as a Camelid-derived VHH, or a recombinant protein comprising a heavy chain variable domain. In some embodiments, the VHH is derived from a species selected from the group consisting of camels, llamas, vicunas, guanacos, and cartilaginous fish (such as, but not limited to, sharks). In another embodiment, the VHH is derived from an alpaca (such as, but not limited to, a Huacaya Alpaca or a Suri alpaca).

As used herein, "Variable region" or "variable domain" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the βsheet structure. The CDRs in each chain are held together in close proximity by the FR regions and, with the CDRs from the other chain, contribute to the formation of the antigen-binding site of antibodies (see Kabat et al., Sequences of Proteins of Immunological Interest, Fifth Edition, National Institute of Health, Bethesda, Md. (1991)). The constant domains are not involved directly in binding an antibody to an antigen, but exhibit various effector functions, such as participation of the antibody in antibody-dependent cellular toxicity. The assignment of amino acids to each domain, framework region and CDR is, in some embodiments, in accordance with one of the numbering schemes provided by Kabat et al. (1991) Sequences of Proteins of Immunological Interest (5th Ed.), US Dept. of Health and Human Services, PHS, NIH, NIH Publication no. 91-3242; Chothia et al., 1987, PMID: 3681981; Chothia et al., 1989, PMID: 2687698; MacCallum et al., 1996, PMID: 8876650; or Dubel, Ed. (2007) Handbook of Therapeutic Antibodies, 3rd Ed., Wily-VCH Verlag GmbH and Co or AbM (Oxford Molecular/MSI Pharmacopia) unless otherwise noted. In some embodiments of this disclosure, the EpCAM binding proteins comprise heavy chain only antibodies, such as VH or VHH domains, and comprise three CDRs. Such heavy chain only antibodies, in some embodiments, bind EpCAM as a monomer with no dependency on dimerisation with a VL (light chain variable) region for optimal binding affinity.

"Variable domain residue numbering as in Kabat" or "amino acid position numbering as in Kabat," and variations thereof, refers to the numbering system used for heavy chain variable domains or light chain variable domains of the compilation of antibodies in Kabat et al., Sequences of Proteins of Immunological Interest, 5th Ed. Public Health Service, National Institutes of Health, Bethesda, Md. (1991). Using this numbering system, the actual linear amino acid sequence may contain fewer or additional amino acids corresponding to a shortening of, or insertion into, a FR or CDR of the variable domain. For example, a heavy chain variable domain may include a single amino acid insert (residue 52a according to Kabat) after residue 52 of H2 and inserted residues (*e.g.,* residues 82a, 82b, and 82c, etc according to Kabat) after heavy chain FR residue 82. The Kabat numbering of residues may be determined for a given antibody by alignment at regions of homology of the sequence of the antibody with a "standard" Kabat numbered sequence. It is not intended that CDRs of the present disclosure necessarily correspond to the Kabat numbering convention.

The term "Framework" or "FR" residues (or regions) refer to variable domain residues other than the CDR or hypervariable region residues as herein defined. A "human consensus framework" is a framework which represents the most commonly occurring amino acid residue in a selection of human immunoglobulin VL or VH framework sequences.

The term "epitope," as used herein, refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

As used herein, the term "Percent (%) amino acid sequence identity" with respect to a sequence is defined as the percentage of amino acid residues in a candidate sequence that are identical with the amino acid residues in the specific sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer softwares such as EMBOSS MATCHER, EMBOSS WATER, EMBOSS STRETCHER, EMBOSS NEEDLE, EMBOSS LALIGN, BLAST, BLAST-2, ALIGN or Megalign (DNASTAR) software. Those skilled in the art can determine appropriate parameters for measuring alignment, including any algorithms needed to achieve maximal alignment over the full length of the sequences being compared.

As used herein, "elimination half-time" is used in its ordinary sense, as is described in Goodman and Gillman's The Pharmaceutical Basis of Therapeutics 21-25 (Alfred Goodman Gilman, Louis S. Goodman, and Alfred Gilman, eds., 6th ed. 1980). Briefly, the term is meant to encompass a quantitative measure of the time course of drug elimination. The elimination of most drugs is exponential (*i.e.,* follows first-order kinetics), since drug concentrations usually do not approach those required for saturation of the elimination process. The rate of an exponential process may be expressed by its rate constant, k, which expresses the fractional change per unit of time, or by its half-time, t_{1/2} the time required for 50% completion of the process. The units of these two constants are time-1 and time, respectively. A first-order rate constant and the half-time of the reaction are simply related (k×t_{1/2}=0.693) and may be interchanged accordingly. Since first-order elimination kinetics dictates that a constant fraction of drug is lost per unit time, a plot of the log of drug concentration versus time is linear at all times following the initial distribution phase (*i.e.,* after drug absorption and distribution are complete). The half-time for drug elimination can be accurately determined from such a graph.

As used herein, the term "binding affinity" refers to the affinity of the proteins described in the disclosure to their binding targets, and is expressed numerically using "Kd" values. If two or more proteins are indicated to have comparable binding affinities towards their binding targets, then the Kd values for binding of the respective proteins towards their binding targets, are within ±2-fold of each other. If two or more proteins are indicated to have comparable binding affinities towards single binding target, then the Kd values for binding of the respective proteins towards said single binding target, are within ±2-fold of each other. If a protein is indicated to bind two or more targets with comparable binding affinities, then the Kd values for binding of said protein to the two or more targets are within ±2-fold of each other. In general, a higher Kd value corresponds to a weaker binding. In some embodiments, the "Kd" is measured by a radiolabeled antigen binding assay (RIA) or surface plasmon resonance assays using a BIAcore^{™}-2000 or a BIAcore^{™}-3000 (BIAcore, Inc., Piscataway, N.J.). In certain embodiments, an "on-rate" or "rate of association" or "association rate" or "kon" and an "off-rate" or "rate of dissociation" or "dissociation rate" or "koff" are also determined with the surface plasmon resonance technique using a BIAcore^{™}-2000 or a BIAcore^{™}-3000 (BIAcore, Inc., Piscataway, N.J.). In additional embodiments, the "Kd", "kon", and "koff" are measured using the OCTET^{®} Systems (Pall Life Sciences). In an exemplary method for measuring binding affinity using the OCTET^{®} Systems, the ligand, *e.g.*, biotinylated human or cynomolgus EpCAM, is immobilized on the OCTET^{®} streptavidin capillary sensor tip surface which streptavidin tips are then activated according to manufacturer's instructions using about 20-50 µg/ml human or cynomolgus EpCAM protein. A solution of PBS/Casein is also introduced as a blocking agent. For association kinetic measurements, EpCAM binding protein variants are introduced at a concentration ranging from about 10 ng/mL to about 100 µg/mL, about 50 ng/mL to about 5 µg/mL, or about 2 ng/mL to about 20 µg/mL. In some embodiments, the EpCAM binding single domain proteins are used at a concentration ranging from about 2 ng/mL to about 20 µg/mL. Complete dissociation is observed in case of the negative control, assay buffer without the binding proteins. The kinetic parameters of the binding reactions are then determined using an appropriate tool, *e.g.*, ForteBio software.

As used herein, in some embodiments, "treatment" or "treating" or "treated" refers to therapeutic treatment wherein the object is to slow (lessen) an undesired physiological condition, disorder or disease, or to obtain beneficial or desired clinical results. For the purposes described herein, beneficial or desired clinical results include, but are not limited to, alleviation of symptoms; diminishment of the extent of the condition, disorder or disease; stabilization (*i.e.,* not worsening) of the state of the condition, disorder or disease; delay in onset or slowing of the progression of the condition, disorder or disease; amelioration of the condition, disorder or disease state; and remission (whether partial or total), whether detectable or undetectable, or enhancement or improvement of the condition, disorder or disease. Treatment includes eliciting a clinically significant response without excessive levels of side effects. Treatment also includes prolonging survival as compared to expected survival if not receiving treatment. In other embodiments, "treatment" or "treating" or "treated" refers to prophylactic measures, wherein the object is to delay onset of or reduce severity of an undesired physiological condition, disorder or disease, such as, for example is a person who is predisposed to a disease (*e.g.,* an individual who carries a genetic marker for a disease such as breast cancer).

A "TriTAC," an "EpCAM targeting TriTAC," or an "EpCAM targeting trispecific protein," as used herein refers to a trispecific binding protein that is not conditionally activated, and comprises a binding moiety that is specific for a bulk serum protein, a first target antigen binding domain, and a second target antigen binding domain, wherein at least one of the first target antigen binding domain and the second target antigen binding domain comprises an EpCAM binding protein as described herein, and at least one of the first target antigen binding domain and the second target antigen binding domain comprises a domain that binds a CD3, such as a human CD3.

A "ProTriTAC," or an "EpCAM targeting protrispecific protein," as used herein refers to a trispecific binding protein that is conditionally activated, and comprises (i) a cleavable linker (*e.g.,* comprising a sequence as set forth in SEQ ID Nos. 425-471 and SEQ ID Nos. 503-506)), (ii) a binding moiety that is specific for a bulk serum protein and also comprises a masking moiety (*e.g.,* comprising a sequence as set forth in SEQ ID Nos. 380-424) which prohibits the binding of a first target antigen binding domain or a second target antigen binding domain to its target, wherein at least one of the first target antigen binding domain and the second target antigen binding domain comprises an EpCAM binding protein as described herein. The ProTriTAC proteins of this disclosure are, in some cases, activated from a masked state to an active state by cleavage of the cleavable linker, for example, in a protease rich environment, such as in a tumor microenvironment, to form an active drug. An active drug, as provided herein, in some instances, comprises an EpCAM binding domain of the disclosure and a CD3 binding domain of the disclosure. An example of an active drug is provided in SEQ ID Nos. 153-179, 180-206, 210-212, 494, 571, and 574, or a sequence that is at least about 75% to 100% identical to a sequence selected from the group consisting of SEQ ID Nos. 153-179, 180-206, 210-212, 494, 571, and 574 such as about 75%, about 80%, about 85%, about 90%, about 91%, about 92%, about 93%, about 94%, about 95%, about 96%, about 97%, about 98%, about 99%, or 100% identical to a sequence selected from the group consisting of SEQ ID Nos. 153-179, 180-206, 210-212, 494, 571, and 574.

A "non-cleavable prodrug," as used herein refers to a ProTriTAC as described above where the cleavable linker is replaced by a non-cleavable linker (*e.g.,* a linker as in SEQ ID No. 507). An example of an active drug is provided in SEQ ID Nos. 495 and 502, or a sequence that is at least about 75% to 100% identical to a sequence selected from the group consisting of SEQ ID Nos. 495 and 502, such as about 75%, about 80%, about 85%, about 90%, about 91%, about 92%, about 93%, about 94%, about 95%, about 96%, about 97%, about 98%, about 99%, or 100% identical to a sequence selected from the group consisting of SEQ ID Nos. 495 and 502.

In a non-beta-sandwich scaffold (*e.g.,* a DARPin, an affimer, an affibody), the "non-CDR loops" refer to an area that is (1) amenable for sequence randomization to allow engineered specificities to a second antigen, and (2) distal to the primary specificity determining region(s) typically used on the scaffold to allow simultaneous engagement of the scaffold to both antigens without steric interference. For this purpose, the primary specificity determining region(s) can be defined using the framework established in the Skrlec 2015 publication (Trends in Biotechnol, 33:408-418). An excerpt of the framework is listed below:

| **Scaffold** | **Primary specificity determining region(s)** |
|---|---|
| Affibody | 13 residues in two helices |
| Affimer | 12-36 residues |
| Anticalin | Four loops (up to 24 aa) |
| Avimer | 11 residues |
| Centyrin | 13 residues |
| DARPin | 7 residues in each n-repeat, or |
| | 8 residues in each n-repeat |
| Fynomer | 6 residues in the RT- and n-Src-loop |
| Kunitz domain | 1-2 loops |

"Chimeric antigen receptor" or "CAR" or "CARs", as used herein, refers to engineered receptors which provide antigen specificity to cells (for example T cells). CARs comprise multiple domains, for example, at least one target antigen binding domain, a transmembrane domain, one or more co-stimulatory domains, and an intracellular signaling domain. Each domain may be connected by a linker. A "ProCAR," as used herein, refers to a conditionally activatable CAR, comprising an EpCAM binding domain of this disclosure.

### EpCAM binding proteins

The multispecific proteins of the invention comprise an EpCAM binding domain in accordance with the claims. The following paragraphs are useful for understanding this aspect of the invention. Described herein are proteins that bind EpCAM, pharmaceutical compositions thereof, as well as nucleic acids, recombinant expression vectors and host cells for making such proteins thereof. Also provided are methods of using the disclosed EpCAM binding proteins in the prevention, and/or treatment of diseases, conditions and disorders. In some cases, the EpCAM binding proteins are part of multispecific (*e.g.,* trispecific) proteins that comprise an EpCAM binding domain as described herein.

The epithelial cell adhesion molecule (EpCAM) is a membrane glycoprotein that is expressed in most normal human epithelia and overexpressed in most carcinomas. This molecule is responsible for cell-to-cell adhesion and additionally participates in signaling, cell migration, proliferation and differentiation. Therefore, EpCAM has been the target of immunotherapy in clinical trials of several solid tumors. It has been found to play an important role in the detection and isolation of circulating tumor cells (CTCs). EpCAM has been shown in various studies to be beneficial in diagnosis and therapy of various carcinomas. Furthermore, in many cases, tumor cells were observed to express EpCAM to a much higher degree than their parental epithelium or less aggressive forms of said cancers. For example, EpCAM expression was shown to be significantly higher on neoplastic tissue and in adenocarcinoma than on normal prostate epithelium (n=76; p<0.0001), suggesting that increased EpCAM expression represents an early event in the development of prostate cancer. *See* Poczatek, J Urol., 1999, 162, 1462-1644. In addition, in the majority of both squamous and adenocarcinomas of the cervix a strong EpCAM expression has been shown to correlate with an increased proliferation and the disappearance of markers for terminal differentiation. *See* Litvinov, Am. J. Pathol. 1996, 148, 865-75. One example is breast cancer where overexpression of EpCAM on tumor cells is a predictor of survival. *See* Gastl, Lancet. 2000, 356, 1981-1982. Furthermore, EpCAM has been described as a marker for the detection of disseminated tumor cells in patients suffering from squamous cell carcinoma of the head, neck and lung. *See* Chaubal, Anticancer Res 1999, 19, 2237-2242, Piyathilake, Hum Pathol. 2000, 31, 482-487. Normal squamous epithelium, as found in epidermis, oral cavity, epiglottis, pharynx, larynx and esophagus did not significantly express EpCAM. *See* Quak, Hybridoma, 1990, 9, 377-387.

EpCAM is contemplated to serve to adhere epithelial cells in an oriented and highly ordered fashion. *See* Litvinov, J Cell Biol. 1997, 139, 1337-1348. Upon malignant transformation of epithelial cells the rapidly growing tumor cells are believed to abandon the high cellular order of epithelia. Consequently, the surface distribution of EpCAM is contemplated to become less restricted and the molecule better exposed on tumor cells. Due to their epithelial cell origin, tumor cells from most carcinomas are expected to express EpCAM on their surface.

EpCAM, is a 40-kDa membrane-integrated glycoprotein of 314 amino acids with specific expression in certain epithelia and on many human carcinomas. *See, e.g.,* in Balzar, J. Mol. Med. 1999, 77, 699-712). EpCAM was discovered and subsequently cloned through its recognition by the murine monoclonal antibody 17-1A/edrecolomab. *See* Goettlinger, Int J Cancer. 1986; 38, 47-53 and Simon, Proc. Natl. Acad. Sci. USA. 1990; 87, 2755-2759. Monoclonal antibody 17-1A was generated by immunization of mice with human colon carcinoma cells. *See* Koprowski, Somatic Cell Genet. 1979, 5, 957-971. The EGF-like repeats of EpCAM were shown to mediate lateral and reciprocal interactions in homophilic cell adhesion. *See, e.g.,* Balzar, Mol. Cell. Biol. 2001, 21, 2570-2580) and, for that reason, is predominantly located between epithelial cells (Litvinov, J Cell Biol. 1997, 139, 1337-1348, Balzar, J Mol Med. 1999, 77, 699-712 and Trebak, J Biol Chem. 2001, 276, 2299-2309).

EpCAM is also known by the following alternate names: Epithelial Cell Adhesion Molecule, Tumor-Associated Calcium Signal Transducer, Major Gastrointestinal Tumor-Associated Protein GA733-2, Adenocarcinoma-Associated Antigen, Cell Surface Glycoprotein Trop-1, Epithelial Glycoprotein 314, TACSTD1, EGP314, MIC18, TROP1, M4S1, KSA, Membrane Component Chromosome 4 Surface marker (35 kD glycoprotein), Antigen identified by monoclonal antibody AUA-1, human epithelial glycoprotein-2, epithelial cell surface antigen, epithelial glycoprotein, KS 1/4Antigen, CD326 Antigen, GA722-2, HEGP314, HNPCC8, EpCAM, DIAR5, EGP-2, EGP40, KS ¼, MK-1, M1S2, ESA, and EGP. Exemplary protein sequences for EpCAM is provided in UniProtkB ID Nos. P16422 and B5MCA4. In some cases, the EpCAM binding proteins of this disclosure binds to an EpCAM sequence provided in UniProtkB ID Nos. P16422 (SEQ ID No.478) or B5MCA4 (SEQ ID No.475).

In some cases, the EpCAM binding domain binds to an extracellular domain of the mature EpCAM protein. The human extracellular domain sequence is provided in SEQ ID No. 479; the cynomolgus extracellular domain sequence is provided in SEQ ID No. 480; and the mouse extracellular domain sequence is provided in SEQ ID No. 481.

In some cases, the EpCAM binding domain binds to a protein comprising a truncated sequence compared to SEQ ID No. 475. In some cases, the EPCAM binding domain binds to a protein comprising the sequence of SEQ ID No. 475. In some cases, the EpCAM binding domain binds to a protein comprising a truncated sequence compared to SEQ ID No. 476. In some cases, the EpCAM binding domain binds to a protein comprising the sequence of SEQ ID No. 476. In some cases, the EpCAM binding domain binds to a protein comprising a truncated sequence compared to SEQ ID No. 477. In some cases, the EpCAM binding domain binds to a protein comprising a truncated sequence compared to SEQ ID No. 477. In some cases, the EpCAM binding domain binds to a protein comprising a truncated sequence compared to SEQ ID No. 478. In some cases, the EpCAM binding domain binds to a protein comprising a truncated sequence compared to SEQ ID No. 478. In some cases, the EpCAM binding domain binds to a protein comprising a truncated sequence compared to SEQ ID No. 479. In some cases, the EpCAM binding domain binds to a protein comprising a truncated sequence compared to SEQ ID No. 479. In some cases, the EpCAM binding domain binds to a protein comprising a truncated sequence compared to SEQ ID No. 480. In some cases, the EpCAM binding domain binds to a protein comprising a truncated sequence compared to SEQ ID No. 480. In some cases, the EpCAM binding domain binds to a protein comprising a truncated sequence compared to SEQ ID No. 481. In some cases, the EpCAM binding domain binds to a protein comprising a truncated sequence compared to SEQ ID No. 481.

In some cases, the EpCAM binding domains disclosed herein recognize full-length EpCAM. In certain instances, the EpCAM binding domains disclosed herein recognize an epitope within EpCAM, such as, in some cases the EpCAM binding proteins interact with one or more amino acids found within a domain of human EpCAM. The epitope to which the antibodies bind may consist of a single contiguous sequence of 3 or more (*e.g.,* 3, 4, 5, 6, 7, 8, 9, 10, 11, 12, 13, 14, 15, 16, 17, 18, 19, 20 or more) amino acids located within a domain of EpCAM. Alternatively, the epitope may consist of a plurality of non-contiguous amino acids (or amino acid sequences) located within a domain of EpCAM.

In some cases, the EpCAM binding proteins of this disclosure binds to the full length EpCAM protein or to a fragment thereof, such as epitope containing fragments within the full length EpCAM protein, as described above. In some cases, the epitope containing fragment comprises antigenic or immunogenic fragments and derivatives thereof of the EpCAM protein. Epitope containing fragments, including antigenic or immunogenic fragments, are, in some cases, 12 amino acids or more, *e.g.,* 20 amino acids or more, 50 or 100 amino acids or more. The EpCAM fragments, in some cases, comprises 95% or more of the length of the full protein, 90% or more, 75% or 50% or 25% or 10% or more of the length of the full protein. In some cases, the epitope-containing fragments of EpCAM including antigenic or immunogenic fragments are capable of eliciting a relevant immune response in a patient. Derivatives of EpCAM include, in some cases, variants on the sequence in which one or more (*e.g.,* 1-20 such as 15 amino acids, or up to 20% such as up to 10% or 5% or 1% by number of amino acids based on the total length of the protein) deletions, insertions or substitutions have been made to the EpCAM sequence provided in SEQ ID Nos. 475-481.

In some cases, substitutions comprise conservative substitutions. Derivatives and variants of, in some examples, have essentially the same biological function as the protein from which they are derived. For instance, derivatives and variants of EpCAM are, in some cases, comparably antigenic or immunogenic to the protein from which they are derived, have either the ligand-binding activity, or the active receptor-complex forming ability, or preferably both, of the protein from which they are derived, and have the same tissue distribution as EpCAM.

In some cases, the EpCAM binding protein specifically binds EpCAM with equivalent or better affinity as that of a reference EpCAM binding protein, and the EpCAM binding protein in such cases comprises an affinity matured EpCAM binding molecule, and is derived from the EpCAM binding parental molecule, comprising one or more amino acid mutations (*e.g.,* a stabilizing mutation, a destabilizing mutation) with respect to the EpCAM binding parental molecule. In some cases, the affinity matured EpCAM binding molecule has superior stability with respect to selected destabilizing agents, as that of a reference EpCAM binding parental molecule. In some cases, the affinity matured EpCAM binding molecule is identified in a process comprising panning of one or more pre-candidate EpCAM binding molecules derived from one or more EpCAM binding parental molecule, expressed in a phage display library, against an EpCAM protein, such as a human EpCAM protein. The pre-candidate EpCAM binding molecule comprises, in some cases, amino acid substitutions in the variable regions, CDRs, or framework residues, relative to a parental molecule.

As used herein, "Phage display" refers to a technique by which variant polypeptides are displayed as fusion proteins to at least a portion of a coat protein on the surface of phage, *e.g.*, filamentous phage, particles. A utility of phage display lies in the fact that large libraries of randomized protein variants can be rapidly and efficiently selected for those sequences that bind to a target molecule with high affinity. Display of peptide and protein libraries on phage has been used for screening millions of polypeptides for ones with specific binding properties. Polyvalent phage display methods have been used for displaying small random peptides and small proteins through fusions to either gene III or gene VIII of filamentous phage. *See e.g.,* Wells and Lowman, Curr. Opin. Struct. Biol, 3:355-362 (1992), and references cited therein. In monovalent phage display, a protein or peptide library is fused to a gene III or a portion thereof, and expressed at low levels in the presence of wild type gene III protein so that phage particles display one copy or none of the fusion proteins. Avidity effects are reduced relative to polyvalent phage so that selection is on the basis of intrinsic ligand affinity, and phagemid vectors are used, which simplify DNA manipulations. *See e.g.,* Lowman and Wells, Methods: A companion to Methods in Enzymology, 3:205-0216 (1991).

In some cases, the panning comprises using varying binding times and concentrations to identify EpCAM binding molecules with increased or decreased on-rates, from pre-candidate EpCAM binding molecules. In some cases, the panning comprises using varying wash times to identify EpCAM binding molecules with increased or decreased off-rates, from pre-candidate EpCAM molecules. In some cases, the panning comprises using both varying binding times and varying wash times. In some cases, one or more stabilizing mutations are combined to increase the stability of the affinity matured EpCAM binding molecule, for example, by shuffling to create a second-stage combinatorial library from such mutants and conducting a second round of panning followed by a binding selection.

In some cases, the affinity matured EpCAM binding molecule comprises an equivalent or better affinity to a EpCAM protein (such as human EpCAM protein) as that of a EpCAM binding parental molecule, but that has reduced cross reactivity, or in some cases, increased cross reactivity, with selected substances, such as ligands, proteins, antigens, or the like, other than the EpCAM epitope for which the EpCAM binding parental molecule is specific, or is designed to be specific for. In regard to the latter, an affinity matured EpCAM binding molecule, in some cases, is more successfully tested in animal models if the affinity matured EpCAM binding molecule is reacted with both human EpCAM and the corresponding target of the animal model, *e.g.* mouse EpCAM or cynomolgus EpCAM. In some cases, the parental EpCAM binding molecule binds to human EpCAM with an affinity of about 500 nM or less, 400 nM or less, 300 nM or less, 200 nM or less, 100 nM or less, 50 nM or less, 10 nM or less, and to cynomolgus EpCAM with an affinity of about 500 nM or less, 400 nM or less, 300 nM or less, 200 nM or less, 100 nM or less, 50 nM or less, 15 nM or less, or 10 nM or less. In some cases, the affinity matured EpCAM binding molecule, identified after one round of panning, binds to human EpCAM with an affinity of about 5 nM or less, such as 1 nM or less, and to cynomolgus EpCAM with an affinity of about 7.5 nM or less, such as 1 nM or less. In some cases, the affinity matured EpCAM binding molecule, identified after two rounds of panning, binds to human EpCAM with an affinity of about 2.5 nM or less, and to cynomolgus EpCAM with an affinity of about 3.5 nM or less.

In some cases, the EpCAM binding protein comprises an antigen-specific binding domain polypeptide that specifically bind to targets, such as targets on diseased cells, or targets on other cells that support the diseased state, such as targets on stromal cells that support tumor growth or targets on immune cells that support disease-mediated immunosuppression. In some examples, the antigen-specific binding domain includes antibodies, single chain antibodies, Fabs, Fv, T-cell receptor binding domains, ligand binding domains, receptor binding domains, domain antibodies, single domain antibodies, minibodies, nanobodies, peptibodies, or various other antibody mimics (such as affimers, affitins, alphabodies, atrimers, CTLA4-based molecules, adnectins, anticalins, Kunitz domain-based proteins, avimers, knottins, fynomers, darpins, affibodies, affilins, monobodies and armadillo repeat protein-based proteins).

In some cases, the EpCAM binding domain is an anti-EpCAM antibody or an antigen binding fragment thereof, or an antibody variant of the EpCAM binding domain or an antigen binding fragment thereof. As used herein, the term "antibody variant" refers to variants and derivatives of an antibody or an antigen binding fragment as described herein. In certain cases, amino acid sequence variants of the anti-EpCAM antibodies or antigen binding fragments thereof, as described herein, are contemplated. For example, in certain cases amino acid sequence variants of anti-EpCAM antibodies or antigen binding fragments thereof, as described herein, are contemplated to improve the binding affinity and/or other biological properties of the same. Exemplary method for preparing amino acid variants include, but are not limited to, introducing appropriate modifications into the nucleotide sequence encoding the antibody or antigen binding fragment thereof, or by peptide synthesis. Such modifications include, for example, deletions from, and/or insertions into and/or substitutions of residues within the amino acid sequences of the antibody or antigen binding fragments thereof.

Any combination of deletion, insertion, and substitution can be made to arrive at the final construct, provided that the final construct possesses the desired characteristics, *e.g.*, antigen- binding. In certain cases, variants having one or more amino acid substitutions are provided. Sites of interest for substitution mutagenesis include the CDRs and framework regions. Examples of such substitutions are described below. Amino acid substitutions may be introduced into an antibody or antigen binding fragments thereof of interest and the products screened for a desired activity, *e.g.*, retained/improved antigen binding, decreased immunogenicity, altered Antibody dependent cellular cytotoxicity (ADCC), or improved T-cell mediated cytotoxicity (TDCC). Both conservative and non-conservative amino acid substitutions are contemplated for preparing the antibody variants.

In another example of a substitution to create a variant anti-EpCAM antibody or antigen binding fragments thereof, one or more hypervariable region residues of a parent antibody are substituted. In general, variants are then selected based on improvements in desired properties compared to a parent antibody or antigen binding fragments thereof, for example, increased affinity, reduced affinity, reduced immunogenicity, increased pH dependence of binding.

In some cases, the EpCAM binding domain is a single domain antibody (sdAb) such as a heavy chain variable domain (VH), a variable domain (VHH) of a llama derived sdAb, a peptide, a ligand or a small molecule entity specific for EpCAM. In some cases, the EpCAM binding domain described herein is any domain that binds to EpCAM including but not limited to domains from a monoclonal antibody, a polyclonal antibody, a recombinant antibody, a human antibody, a humanized antibody. In certain cases, the EpCAM binding domain is a single-domain antibody. In other cases, the EpCAM binding domain is a peptide. In further cases, the EpCAM binding domain is a small molecule.

Generally, it should be noted that the term single domain antibody as used herein in its broadest sense is not limited to a specific biological source or to a specific method of preparation. Single domain antibodies are antibodies whose complementary determining regions are part of a single domain polypeptide. Examples include, but are not limited to, heavy chain antibodies, antibodies naturally devoid of light chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, goat, rabbit, bovine. For example, in some cases, the single domain antibodies of the disclosure are obtained: (1) by isolating the VHH domain of a naturally occurring heavy chain antibody; (2) by expression of a nucleotide sequence encoding a naturally occurring VHH domain; (3) by "humanization" of a naturally occurring VHH domain or by expression of a nucleic acid encoding a such humanized VHH domain; (4) by "camelization" of a naturally occurring VH domain from any animal species, and in particular from a species of mammal, such as from a human being, or by expression of a nucleic acid encoding such a camelized VH domain; (5) by "camelization" of a "domain antibody" or "Dab," or by expression of a nucleic acid encoding such a camelized VH domain; (6) by using synthetic or semi-synthetic techniques for preparing proteins, polypeptides or other amino acid sequences; (7) by preparing a nucleic acid encoding a single domain antibody using techniques for nucleic acid synthesis known in the field, followed by expression of the nucleic acid thus obtained; and/or (8) by any combination of one or more of the foregoing.

In one case, a single domain antibody corresponds to the VHH domains of naturally occurring heavy chain antibodies directed against EpCAM. As further described herein, such VHH sequences can generally be generated or obtained by suitably immunizing a species of Llama with EpCAM, (*i.e.,* so as to raise an immune response and/or heavy chain antibodies directed against EpCAM), by obtaining a suitable biological sample from said Llama (such as a blood sample, serum sample or sample of B-cells), and by generating VHH sequences directed against EpCAM, starting from said sample, using any suitable technique known in the field.

In another case, such naturally occurring VHH domains against EpCAM, are obtained from naive libraries of Camelid VHH sequences, for example by screening such a library using EpCAM, or at least one part, fragment, antigenic determinant or epitope thereof using one or more screening techniques known in the field. Such libraries and techniques are for example described in WO 99/37681, WO 01/90190, WO 03/025020 and WO 03/035694. Alternatively, improved synthetic or semi-synthetic libraries derived from naive VHH libraries are used, such as VHH libraries obtained from naive VHH libraries by techniques such as random mutagenesis and/or CDR shuffling, as for example described in WO 00/43507.

In a further case, yet another technique for obtaining VHH sequences directed against EpCAM, involves suitably immunizing a transgenic mammal that is capable of expressing heavy chain antibodies (i.e., so as to raise an immune response and/or heavy chain antibodies directed against EpCAM), obtaining a suitable biological sample from said transgenic mammal (such as a blood sample, serum sample or sample of B-cells), and then generating VHH sequences directed against EpCAM, starting from said sample, using any suitable technique known in the field. For example, for this purpose, the heavy chain antibody-expressing rats or mice and the further methods and techniques described in WO 02/085945 and in WO 04/049794 can be used.

In some cases, an anti-EpCAM single domain antibody of this disclosure comprises a single domain antibody with an amino acid sequence that corresponds to the amino acid sequence of a non-human antibody and/or a naturally occurring VHH domain, *e.g.,* a llama anti-EpCAM antibody, but that has been "humanized," *i.e.,* by replacing one or more amino acid residues in the amino acid sequence of said non-human anti-EpCAM and/or the naturally occurring VHH sequence (and in particular in the framework sequences) by one or more of the amino acid residues that occur at the corresponding position(s) in a VH domain from a conventional 4-chain antibody from a human being (*e.g.,* as indicated above). This can be performed in a manner known in the field, which will be clear to the skilled person, for example on the basis of the further description herein. Again, it should be noted that such humanized anti-EpCAM single domain antibodies of the disclosure are obtained in any suitable manner known per se (i.e., as indicated under points (1)-(8) above) and thus are not strictly limited to polypeptides that have been obtained using a polypeptide that comprises a naturally occurring VHH domain as a starting material. In some additional cases, a single domain anti-EpCAM antibody, as described herein, comprises a single domain antibody with an amino acid sequence that corresponds to the amino acid sequence of a naturally occurring VH domain, but that has been "camelized" i.e., by replacing one or more amino acid residues in the amino acid sequence of a naturally occurring VH domain from a conventional 4-chain antibody by one or more of the amino acid residues that occur at the corresponding position(s) in a VHH domain of a heavy chain antibody. Such "camelizing" substitutions are preferably inserted at amino acid positions that form and/or are present at the VH-VL interface, and/or at the so-called Camelidae hallmark residues. *See, e.g.,* WO 94/04678 and Davies and Riechmann (1994 and 1996)). Preferably, the VH sequence that is used as a starting material or starting point for generating or designing the camelized single domain is preferably a VH sequence from a mammal, more preferably the VH sequence of a human being, such as a VH3 sequence. However, it should be noted that such camelized anti-EpCAM single domain antibodies of the disclosure, in certain cases, are obtained in any suitable manner known in the field (*i.e.,* as indicated under points (1)-(8) above) and thus are not strictly limited to polypeptides that have been obtained using a polypeptide that comprises a non-human anti-EpCAM antibody and/or the naturally occurring VH domain as a starting material. For example, as further described herein, both "humanization" and "camelization" is performed by providing a nucleotide sequence that encodes a naturally occurring VHH domain or VH domain, respectively, and then changing, one or more codons in said nucleotide sequence in such a way that the new nucleotide sequence encodes a "humanized" or "camelized" single domain antibody, respectively. This nucleic acid can then be expressed, so as to provide a desired anti-EpCAM single domain antibody of the disclosure. Alternatively, in other cases, based on the amino acid sequence of a naturally occurring VHH domain or VH domain, respectively, the amino acid sequence of the desired humanized or camelized anti-EpCAM single domain antibody of the disclosure, respectively, are designed and then synthesized *de novo* using known techniques for peptide synthesis. In some cases, based on the amino acid sequence or nucleotide sequence of a naturally occurring VHH domain or VH domain, respectively, a nucleotide sequence encoding the desired humanized or camelized anti-EpCAM single domain antibody of the disclosure, respectively, is designed and then synthesized *de novo* using known techniques for nucleic acid synthesis, after which the nucleic acid thus obtained is expressed in using known expression techniques, so as to provide the desired anti-EpCAM single domain antibody of the disclosure.

Other suitable methods and techniques for obtaining the anti-EpCAM single domain antibody of the disclosure and/or nucleic acids encoding the same, starting from naturally occurring VH sequences or VHH sequences for example comprises combining one or more parts of one or more naturally occurring VH sequences (such as one or more framework (FR) sequences and/or complementarity determining region (CDR) sequences), one or more parts of one or more naturally occurring VHH sequences (such as one or more FR sequences or CDR sequences), and/or one or more synthetic or semi-synthetic sequences, in a suitable manner, so as to provide an anti-EpCAM single domain antibody of the disclosure or a nucleotide sequence or nucleic acid encoding the same.

In some cases, the EpCAM binding domain is an anti-EpCAM specific antibody comprising a heavy chain variable complementarity determining region CDR1, a heavy chain variable CDR2, a heavy chain variable CDR3, a light chain variable CDR1, a light chain variable CDR2, and a light chain variable CDR3. In some cases, the EpCAM binding domain comprises any domain that binds to EpCAM including but not limited to domains from a monoclonal antibody, a polyclonal antibody, a recombinant antibody, a human antibody, a humanized antibody, or antigen binding fragments such as single domain antibodies (sdAb), Fab, Fab', F(ab)2, and Fv fragments, fragments comprised of one or more CDRs, single-chain antibodies (*e.g.,* single chain Fv fragments (scFv)), disulfide stabilized (dsFv) Fv fragments, heteroconjugate antibodies (*e.g.,* bispecific antibodies), pFv fragments, heavy chain monomers or dimers, light chain monomers or dimers, and dimers consisting of one heavy chain and one light chain. In some cases, the EpCAM binding domain is a single domain antibody. In some cases, the anti-EpCAM single domain antibody comprises heavy chain variable complementarity determining regions (CDR), CDR1, CDR2, and CDR3.

In some cases, the EpCAM binding domain is a polypeptide comprising an amino acid sequence that is comprised of four framework regions/sequences (f1-f4) interrupted by three complementarity determining regions/sequences, as represented by the formula: f1-r1-f2-r2-f3-r3-f4, wherein r1, r2, and r3 are complementarity determining regions CDR1, CDR2, and CDR3, respectively, and f1, f2, f3, and f4 are framework residues. The framework residues of the EpCAM binding protein of the present disclosure comprise, for example, 75, 76, 77, 78, 79, 80, 81, 82, 83, 84, 85, 86, 87, 88, 89, 90, 91, 92, 93, or 94 amino acid residues, and the complementarity determining regions comprise, for example, 24, 25, 26, 27, 28, 29, 30, 31, 32, 33, 34, 35, or 36 amino acid residues. In some cases, the EpCAM binding domain comprises an amino acid sequence selected from SEQ ID NOs: 1-38, 207-209, and 496-497.

In some cases, the binding proteins described herein comprise a polypeptide having a sequence selected from SEQ ID Nos. 1-38, subsequences thereof, and variants thereof. In some cases, the EpCAM binding protein comprises at least 70%-95% or more homology to a sequence selected from SEQ ID Nos. 1-38, subsequences thereof, and variants thereof. In some cases, the EpCAM binding protein comprises at least 60%, 61%, 62%, 63%, 63%, 65%, 66%, 67%, 68%, 69%, 70%, 75%, 80%, 85%, 90%, 95%, 96%, 97%, 98%, 99%, or more homology to a sequence selected from SEQ ID Nos. 1-38, 207-209, and 496-497, subsequences thereof, and variants thereof. In some cases, the EpCAM binding protein comprises at least 70%-95% or more identity to a sequence selected from SEQ ID Nos. 1-38, 207-209, and 496-497, subsequences thereof, and variants thereof. In some cases, the EpCAM binding protein comprises at least 60%, 61%, 62%, 63%, 63%, 65%, 66%, 67%, 68%, 69%, 70%, 75%, 80%, 85%, 90%, 95%, 96%, 97%, 98%, 99% or more identity to a sequence selected from SEQ ID Nos. 1-38, 207-209, and 496-497, subsequences thereof, and variants thereof.

In some cases, the CDR1 comprises the amino acid sequence as set forth in any one of SEQ ID Nos. 39-76 or a sequence comprising one or more substitutions compared to a sequence selected from the group consisting of SEQ ID Nos. 39-76. In some cases, the CDR2 comprises a sequence as set forth in any one of SEQ ID Nos.77-114 or a sequence comprising one or more substitutions compared to a sequence selected from the group consisting of SEQ ID Nos. 77-114. In some cases, the CDR3 comprises a sequence as set forth in any one of SEQ ID Nos. 115-152 a sequence comprising one or more substitutions compared to a sequence selected from the group consisting of SEQ ID Nos. 115-152.

In various cases, the EpCAM binding domain of the present disclosure is at least about 60%, about 61%, at least about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% identical to an amino acid sequence selected from SEQ ID Nos. 1-38, 207-209, and 496-497.

In various cases, a complementarity determining region of the EpCAM binding domain of the present disclosure is at least about 10%, about 20%, about 30%, about 40%, about 50%, about 60%, about 70%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% identical to the amino acid sequence set forth in SEQ ID Nos. 39-76.

In various cases, a complementarity determining region of the EpCAM binding domain of the present disclosure is at least about 10%, about 20%, about 30%, about 40%, about 50%, about 60%, about 70%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% identical to the amino acid sequence set forth in SEQ ID Nos. 77-114.

In various cases, a complementarity determining region of the EpCAM binding domain of the present disclosure is at least about 10%, about 20%, about 30%, about 40%, about 50%, about 60%, about 70%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% identical to the amino acid sequence set forth in SEQ ID Nos. 115-152.

In some cases, the EpCAM binding domain is cross-reactive with human cynomolgus and mouse EpCAM. In some cases, the EpCAM binding domain is specific for human EpCAM. In certain cases, the EpCAM binding domains disclosed herein bind to human EpCAM with a human Kd (hKd). In certain cases, the EpCAM binding domains disclosed herein bind to cynomolgus EpCAM with a cyno Kd (cKd). In certain cases, the EpCAM binding domains disclosed herein bind to cynomolgus EpCAM with a mouse Kd (mKd). In certain cases, the EpCAM binding domains disclosed herein bind to both cynomolgus EpCAM and a human EpCAM, with a cyno Kd (cKd) and a human Kd (hKd), respectively. In certain cases, the EpCAM binding domains disclosed herein bind to cynomolgus EpCAM, mouse EpCAM, and a human EpCAM, with a cyno Kd (cKd), mouse Kd (mKd), and a human Kd (hKd), respectively. In some cases, the EpCAM binding protein binds to human, mouse and cynomolgus EpCAM with comparable binding affinities (*i.e.*, hKd, mKd and cKd values do not differ by more than ± 10%). In some cases, the hKd, mKd and the cKd range from about 0.001 nM to about 500 nM. In some cases, the hKd, mKd and the cKd range from about 0.001 nM to about 450 nM. In some cases, the hKd, mKd and the cKd range from about 0.001 nM to about 400 nM. In some cases, the hKd, mKd and the cKd range from about 0.001 nM to about 350 nM. In some cases, the hKd, mKd and the cKd range from about 0.001 nM to about 300 nM. In some cases, the hKd, mKd and the cKd range from about 0.001 nM to about 250 nM. In some cases, the hKd, mKd and the cKd range from about 0.001 nM to about 200 nM. In some cases, the hKd, mKd and the cKd range from about 0.001 nM to about 150 nM. In some cases, the hKd, mKd and the cKd range from about 0.001 nM to about 100 nM. In some cases, the hKd, mKd and the cKd range from about 0.1 nM to about 90 nM. In some cases, the hKd, mKd and the cKd range from about 0.2 nM to about 80 nM. In some cases, the hKd, mKd and the cKd range from about 0.3 nM to about 70 nM. In some cases, the hKd, mKd and the cKd range from about 0.4 nM to about 50 nM. In some cases, the hKd, mKd and the cKd range from about 0.5 nM to about 30 nM. In some cases, the hKd, mKd and the cKd range from about 0.6 nM to about 10 nM. In some cases, the hKd, mKd and the cKd range from about 0.7 nM to about 8 nM. In some cases, the hKd, mKd and the cKd range from about 0.8 nM to about 6 nM. In some cases, the hKd, mKd and the cKd range from about 0.9 nM to about 4 nM. In some cases, the hKd, mKd and the cKd range from about 1 nM to about 2 nM.

In some cases, any of the foregoing EpCAM binding domains (*e.g.,* anti-EpCAM single domain antibodies of SEQ ID Nos. 1-38) are affinity peptide tagged for ease of purification. In some cases, the affinity peptide tag is six consecutive histidine residues, also referred to as 6X-his (SEQ ID No. 377).

In certain cases, the EpCAM binding domains of the present disclosure preferentially bind membrane bound EpCAM over soluble EpCAM Membrane bound EpCAM refers to the presence of EpCAM in or on the cell membrane surface of a cell that expresses EpCAM. Soluble EpCAM refers to EpCAM that is no longer on in or on the cell membrane surface of a cell that expresses or expressed EpCAM. In certain instances, the soluble EpCAM is present in the blood and/or lymphatic circulation in a subject. In one case, the EpCAM binding domains bind membrane-bound EpCAM at least 5 fold, 10 fold, 15 fold, 20 fold, 25 fold, 30 fold, 40 fold, 50 fold, 100 fold, 500 fold, or 1000 fold greater than soluble EpCAM. In one case, the EpCAM binding proteins of the present disclosure preferentially bind membrane-bound EpCAM 30 fold greater than soluble EpCAM. Determining the preferential binding of an antigen binding protein to membrane bound EpCAM over soluble EpCAM can be readily determined using binding assays.

It is contemplated that in some cases the EpCAM binding protein is fairly small and no more than 25 kDa, no more than 20 kDa, no more than 15 kDa, or no more than 10 kDa in some cases. In certain instances, the EpCAM binding protein is 5 kDa or less if it is a peptide or small molecule entity.

In other cases, the EpCAM binding proteins described herein comprise small molecule entity (SME) binders for EpCAM. SME binders are small molecules averaging about 500 to 2000 Da in size and are attached to the EpCAM binding proteins by known methods, such as sortase ligation or conjugation. In these instances, the EpCAM binding protein comprises a domain comprising a sortase recognition sequence, *e.g.*, LPETG (SEQ ID No. 376). To attach a SME binder to EpCAM binding protein comprising a sortase recognition sequence, the protein is incubated with a sortase and a SME binder whereby the sortase attaches the SME binder to the recognition sequence. In yet other cases, the EpCAM binding proteins described herein comprise a knottin peptide for binding EpCAM. Knottins are disufide-stabilized peptides with a cysteine knot scaffold and have average sizes about 3.5 kDa. Knottins have been contemplated for binding to certain tumor molecules such as EpCAM. In further cases, the EPCAM binding proteins described herein comprise a natural EpCAM ligand.

In some cases, the EpCAM binding protein comprises more than one domain and are of a single-polypeptide design with flexible linkage of the domains. This allows for facile production and manufacturing of the EpCAM binding proteins as they can be encoded by single cDNA molecule to be easily incorporated into a vector. Further, in some cases where the EpCAM binding proteins described herein are a monomeric single polypeptide chain, there are no chain pairing issues or a requirement for dimerization. It is contemplated that, in such cases, the EpCAM binding proteins described herein have a reduced tendency to aggregate.

In the EpCAM binding proteins comprising more than one domain, the domains are linked by one or more internal linker. In certain cases, the internal linkers are "short," *i.e.,* consist of 0, 1, 2, 3, 4, 5, 6, 7, 8, 9, 10, 11 or 12 amino acid residues. Thus, in certain instances, the internal linkers consist of about 12 or less amino acid residues. In the case of 0 amino acid residues, the internal linker is a peptide bond. In certain cases, the internal linkers are "long," *i.e.,* consist of 15, 20 or 25 amino acid residues. In some cases, the internal linkers consist of about 3 to about 15, for example 8, 9 or 10 contiguous amino acid residues. Regarding the amino acid composition of the internal linkers, peptides are selected with properties that confer flexibility to the EpCAM binding proteins, do not interfere with the binding domains as well as resist cleavage from proteases. For example, glycine and serine residues generally provide protease resistance. Examples of internal linkers suitable for linking the domains in the EpCAM binding proteins include but are not limited to (GS)ₙ (SEQ ID No. 365), (GGS)ₙ (SEQ ID No. 366), (GGGS)ₙ (SEQ ID No. 367), (GGSG)ₙ (SEQ ID No. 368), (GGSGG)ₙ (SEQ ID No. 369), (GGGGS)ₙ (SEQ ID No. 370), (GGGGG)ₙ (SEQ ID No. 371), or (GGG)ₙ (SEQ ID No. 372), wherein n is 1, 2, 3, 4, 5, 6, 7, 8, 9, or 10. In one case, the linker is (GGGGSGGGGSGGGGSGGGGS) (SEQ ID No. 373), (GGGGSGGGGSGGGGS) (SEQ ID No. 374), or (GGGGSGGGS) (SEQ ID No. 375).

In some cases, where the EpCAM binding protein comprises more than one domain, the domains within the EpCAM binding proteins are conjugated using an enzymatic site-specific conjugation method which involves the use of a mammalian or bacterial transglutaminase enzyme. Microbial transglutaminases (mTGs) are versatile tools in modern research and biotechnology. The availability of large quantities of relatively pure enzymes, ease of use, and lack of regulation by calcium and guanosine-5'-triphosphate (GTP) has propelled mTG to be the main cross-linking enzyme used in both the food industry and biotechnology. Currently, mTGs are used in many applications to attach proteins and peptides to small molecules, polymers, surfaces, DNA, as well as to other proteins. *See, e.g.,* Pavel Strp, Veracity of microbial transglutaminase, Bioconjugate Chem. 25, 5, 855-862.

In some examples are provided EpCAM binding proteins comprising more than one domain, wherein one of the domains comprises an acceptor glutamine in a constant region, which can then be conjugated to another domain *via* a lysine-based linker *(e.g.,* any primary amine chain which is a substrate for TGase, *e.g.* comprising an alkylamine, oxoamine) wherein the conjugation occurs exclusively on one or more acceptor glutamine residues present in the targeting moiety outside of the antigen combining site (*e.g.,* outside a variable region, in a constant region). Conjugation thus does not occur on a glutamine, *e.g.* an at least partly surface exposed glutamine, within the variable region. The EpCAM binding protein, in some examples, is formed by reacting one of the domains with a lysine-based linker in the presence of a TGase.

In some cases, where one or more domains within the EpCAM binding proteins are directly joined, a hybrid vector is made where the DNA encoding the directly joined domains are themselves directly ligated to each other. In some cases, where linkers are used, a hybrid vector is made where the DNA encoding one domain is ligated to the DNA encoding one end of a linker moiety and the DNA encoding another domain is ligated to the other end of the linker moiety.

In some cases, the EpCAM binding protein is a single chain variable fragments (scFv), single-domain antibody such as a heavy chain variable domain (VH), a light chain variable domain (VL) and a variable domain (VHH) of camelid derived single domain antibody. In other cases, the EpCAM binding protein is a non-Ig binding domain, *i.e.,* an antibody mimetic, such as anticalins, affilins, affibody molecules, affimers, affitins, alphabodies, avimers, DARPins, fynomers, kunitz domain peptides, and monobodies. In further cases, the EpCAM binding protein is a ligand or peptide that binds to or associates with EpCAM. In yet further cases, the EpCAM binding protein is a knottin. In yet further cases, the binding domain to EpCAM is a small molecular entity.

In certain cases, the EpCAM binding proteins according to the present disclosure may be incorporated into EpCAM targeting trispecific proteins. In some cases, the trispecific proteins comprise a CD3 binding domain, a half-life extension domain, and an EpCAM binding domain according to this disclosure. In some cases, the EpCAM binding trispecific protein comprises a trispecific antibody.

### Multispecific EpCAM targeting proteins, such as EpCAM targeting trispecific proteins (also referred to herein as EpCAM targeting TriTAC proteins or molecules)

The invention provides a multispecific protein as defined by the claims. The following paragraphs are useful for understanding this aspect of the invention. In some cases, the multispecific protein further comprises a domain which specifically binds to CD3. In some cases, the multispecific protein further comprises a domain which specifically binds to human CD3. In some cases, the multispecific protein further comprises a domain which specifically binds to CD3-gamma. In some cases, the multispecific protein further comprises a domain which specifically binds to CD3-delta. In some cases, the multispecific protein further comprises a domain which specifically binds to CD3-epsilon.

In additional cases, the multispecific protein further comprises a domain which specifically binds to the T cell receptor (TCR). In some cases, the multispecific protein further comprises a domain which specifically binds the alpha chain of the TCR. In some cases, the multispecific protein further comprises a domain which specifically binds the β chain of the TCR.

In certain cases, the CD3 binding domain of the multispecific protein exhibits not only potent CD3 binding affinities with human CD3, but also shows excellent cross-reactivity with the respective cynomolgus monkey CD3 proteins. In some instances, the CD3 binding domain of the multispecific proteins are cross-reactive with CD3 from cynomolgus monkey. In certain instances, human:cynomolgous KD (hKd: cKd) ratios for CD3 binding are between 20:1 and 1:2.

In some cases, the CD3 binding domain of the multispecific protein is any domain that binds to CD3 including but not limited to domains from a monoclonal antibody, a polyclonal antibody, a recombinant antibody, a human antibody, a humanized antibody, or antigen binding fragments of the CD3 binding antibodies, such as single domain antibodies (sdAb), Fab, F(ab')2, and Fv fragments, fragments comprised of one or more CDRs, single-chain antibodies (*e.g.,* single chain Fv fragments (scFv)), disulfide stabilized (dsFv) Fv fragments, heteroconjugate antibodies (*e.g.,* bispecific antibodies), pFv fragments, heavy chain monomers or dimers, light chain monomers or dimers, and dimers consisting of one heavy chain and one light chain. In some instances, it is beneficial for the CD3 binding domain to be derived from the same species in which the multispecific protein comprising a single domain serum albumin binding protein described herein will ultimately be used in. For example, for use in humans, it may be beneficial for the CD3 binding domain of the multispecific protein comprising an EpCAM binding protein described herein to comprise human or humanized residues from the antigen binding domain of an antibody or antibody fragment. Exemplary amino acid sequence for the CD3 binding domain of a multispecific (*e.g.,* trispecific) EpCAM targeting TriTAC protein of this disclosure is provided as SEQ ID No. 379, or a sequence that is at least about 75% to 100% identical to SEQ ID No. 379, such as at least about 75%, about 80%, about 85%, about 90%, about 91%, about 92%, about 93%, about 94%, about 95%, about 96%, about 97%, about 98%, about 99%, or 100% identical to SEQ ID NO. 379.

In some cases, the serum albumin binding domain (also referred to herein as the half-life extension domain) of a multispecific protein comprising an EpCAM binding protein as described herein can be any domain that binds to serum albumin including but not limited to domains from a monoclonal antibody, a polyclonal antibody, a recombinant antibody, a human antibody, a humanized antibody. In some cases, the serum albumin binding domain is a single chain variable fragments (scFv), single-domain antibody such as a heavy chain variable domain (VH), a light chain variable domain (VL) and a variable domain (VHH) of camelid derived sdAb, or antigen binding fragments of the HSA binding antibodies, such as Fab, F(ab')2, and Fv fragments, fragments comprised of one or more CDRs, single-chain antibodies (*e.g.,* single chain Fv fragments (scFv)), disulfide stabilized (dsFv) Fv fragments, heteroconjugate antibodies (*e.g.,* bispecific antibodies), pFv fragments, heavy chain monomers or dimers, light chain monomers or dimers, and dimers consisting of one heavy chain and one light chain, peptide, ligand or small molecule entity specific for serum albumin. In certain cases, the HSA binding domain is a single-domain antibody. In other cases, the serum albumin binding domain is a peptide. In further cases, the serum albumin binding domain is a small molecule. It is contemplated that the serum albumin binding domain of the multispecific binding protein comprising a single chain variable fragment CD3 binding protein is fairly small and no more than 25 kD, no more than 20 kD, no more than 15 kD, or no more than 10 kD in some cases. In certain instances, the serum albumin binding is 5 kD or less if it is a peptide or small molecule entity. Exemplary amino acid sequence for a serum albumin binding domain of a multispecific (*e.g.,* trispecific) EpCAM targeting TriTAC protein of this disclosure is provided as SEQ ID No. 378, or a sequence that is at least about 75% to 100% identical to SEQ ID No. 378, such as at least about 75%, about 80%, about 85%, about 90%, about 91%, about 92%, about 93%, about 94%, about 95%, about 96%, about 97%, about 98%, about 99%, or 100% identical to SEQ ID NO. 378.

The half-life extension domain of a multispecific binding protein, as described herein, comprising a single chain variable fragment CD3 binding protein provides for altered pharmacodynamics and pharmacokinetics of the single chain variable fragment CD3 binding protein itself. As above, the half-life extension domain extends the elimination half-time. The half-life extension domain also alters pharmacodynamic properties including alteration of tissue distribution, penetration, and diffusion of the single chain variable fragment CD3 binding protein. In some cases, the half-life extension domain provides for improved tissue (including tumor) targeting, tissue distribution, tissue penetration, diffusion within the tissue, and enhanced efficacy as compared with a protein without a half-life extension domain. In one case, therapeutic methods effectively and efficiently utilize a reduced amount of the multispecific binding protein comprising a single chain variable fragment CD3 binding protein, resulting in reduced side effects, such as reduced off-target, such as non-tumor cell cytotoxicity.

Further, the binding affinity of the half-life extension domain, in some cases, is selected so as to target a specific elimination half-time in a particular multispecific binding protein comprising an EpCAM binding protein as described herein. Thus, in some cases, the half-life extension domain has a high binding affinity. In other cases, the half-life extension domain has a medium binding affinity. In yet other cases, the half-life extension domain has a low or marginal binding affinity. Exemplary binding affinities include K_{d} of 10 nM or less (high), between 10 nM and 100 nM (medium), and greater than 100 nM (low). As above, binding affinities to serum albumin are determined by known methods such as Surface Plasmon Resonance (SPR).

An EpCAM targeting multispecific protein of this disclosure, in certain cases, comprises (A) a first domain which binds to a CD3; (B) a second domain which is an half-life extension domain; and (C) a third domain which is an EpCAM binding protein as described herein. In certain cases, the first domain comprises an scFv that specifically binds the CD3. The CD3, for instance, is a human CD3 protein. In certain cases, the second domain comprises an sdAb that specifically binds a bulk serum protein. In some instances, the bulk serum protein is albumin, such as, a serum albumin, such as, a human serum albumin.

The domains (A), (B), and (C), are, in some cases, linked *via* linkers L1 and L2, in any one of the following orientations: H₂N-(A)-L1-(C)-L2-(B)-COOH, H₂N-(B)-L1-(A)-L2-(C)-COOH, H₂N-(C)-L1-(B)-L2-(A)-COOH, H₂N-(C)-L1-(A)-L2-(B)-COOH, H₂N-(A)-L1-(B)-(C)-L2-COOH, or H₂N-(B)-(C)-(A)-COOH.

An EpCAM targeting multispecific protein of this disclosure, in some cases, comprises an amino acid sequence that is at least about 70% to about 100% identical to a sequence selected from the group consisting of SEQ ID Nos. 153-206 and 210-212. In some cases, an EpCAM targeting multispecific protein of this disclosure comprises an amino acid sequence that is at least about 70%, at least about 75%, at least about 76%, at least about 77%, about 78%, at least about 79%, at least about 80%, at least about 81%, at least about 82%, at least about 83%, at least about 84%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, to about 100% identical to a sequence selected from the group consisting of SEQ ID Nos. 153-206 and 210-212.

### Conditionally active multispecific EpCAM targeting proteins, such as conditionally active EpCAM targeting trispecific proteins (also referred to herein as EpCAM targeting ProTriTAC or protrispecific proteins or molecules)

The invention provides a multispecific protein as defined by the claims. The following paragraphs are useful for understanding this aspect of the invention. One case of this disclosure provides a conditionally active multispecific protein comprising an EpCAM binding domain as disclosed herein (for example, in some case this disclosure provides an EpCAM targeting protrispecific/ProTriTAC protein comprising an EpCAM binding domain of this disclosure).

In some cases, the conditionally active multispecific protein further comprises a domain which specifically binds to a CD3 and a binding moiety which specifically binds to a bulk serum protein, such as a human serum albumin. In some cases, the binding moiety is capable of masking the interaction of the EpCAM binding domain or the CD3 binding domain, to their targets. In some cases, a binding moiety of this disclosure comprises a masking moiety and a cleavable linker, such as a protease cleavable linker. Exemplary sequences for masking moiety within a binding moiety are provided in SEQ ID Nos. 380-424, or a sequence comprising one or more substitutions relative to a sequence selected from the group consisting of SEQ ID Nos. 380-424. In some cases, the binding moiety comprises a modified non-CDR loop sequence and a cleavable linker. In some cases, the cleavable linker comprises a sequence selected from the group consisting of SEQ ID Nos. 425-471, 503-506, 508-550, and 581, or a sequence that comprises one or substitutions relative to a sequence selected from the group consisting of SEQ ID Nos. 425-425-471, 503-506, 508-550, and 581. In some cases, the masking moiety comprises a modified non-CDR loop sequence and a non-cleavable linker. In some cases the non-cleavable linker comprises a sequence as set forth in SEQ ID No. 507, or a sequence comprising one or more substitutions relative to SEQ ID No. 507. In some cases, a binding moiety comprises a sequence that is at least about 75%, about 80%, about 85%, about 90%, about 91%, about 92%, about 93%, about 94%, about 95%, about 96%, about 97%, about 98%, about 99%, or about 100% identical to a sequence selected from the group consisting of SEQ ID Nos. 472-473, and 482-483. In some cases, a CD3 binding domain of an EpCAM ProTriTAC of this disclosure comprises a sequence that is at least about 75%, about 80%, about 85%, about 90%, about 91%, about 92%, about 93%, about 94%, about 95%, about 96%, about 97%, about 98%, about 99%, or about 100% identical to SEQ ID No. 474.

In some cases, an EpCAM targeting ProTriTAC of this disclosure, comprises, from N-terminal to C-terminal, comprise a binding moiety that is an anti-ALB domain comprising a non-CDR loop with a binding site for a CD3 binding domain (*e.g.,* a CD3 binding domain having the sequence of SEQ ID No. 474, or at least about 75% identity to the same), a cleavable linker, the CD3 binding domain, and on the C-terminal end the anti-EpCAM binding domain. The EpCAM binding domain of the ProTriTAC, in some cases, is at least about 60%, about 61%, at least about 62%, about 63%, about 64%, about 65%, about 66%, about 67%, about 68%, about 69%, about 70%, about 71%, about 72%, about 73%, about 74%, about 75%, about 76%, about 77%, about 78%, about 79%, about 80%, about 81%, about 82%, about 83%, about 84%, about 85%, about 86%, about 87%, about 88%, about 89%, about 90%, about 91%, about 92%, about 93%, about 94%, about 95%, about 96%, about 97%, about 98%, about 99%, or about 100% identical to an amino acid sequence selected from SEQ ID Nos. 1-38, 207-209, and 496-497

In some cases, an EpCAM targeting ProTriTAC of this disclosure comprises an amino acid sequence that is at least about at least about 75%, about 80%, about 85%, about 90%, about 91%, about 92%, about 93%, about 94%, about 95%, about 96%, about 97%, about 98%, about 99%, or about 100% identical to a sequence selected from the group consisting of: SEQ ID Nos. 495, 498, 499, 500, 502, 569, 570, 572, 573, 575, 576, 577, and 578. In some cases, an EpCAM targeting ProTriTAC of this discloses comprises an amino acid sequence as set forth in SEQ ID No. 576, a pharmaceutical composition comprising the same, and method of using the same for treating a disease, such as a tumorous disease as described herein.

In some cases, an EpCAM targeting ProTriTAC of this disclosure, in a non-cleavable prodrug format, comprises an amino acid sequence that is at least about at least about 75%, about 80%, about 85%, about 90%, about 91%, about 92%, about 93%, about 94%, about 95%, about 96%, about 97%, about 98%, about 99%, or about 100% identical to a sequence selected from the group consisting of: SEQ ID Nos. 495 and 502.

An exemplary sequence for an active EpCAM targeting drug (CT), as described herein, is a sequence that is at least about 75%, about 80%, about 85%, about 90%, about 91%, about 92%, about 93%, about 94%, about 95%, about 96%, about 97%, about 98%, about 99%, or about 100% identical to SEQ ID No. 153-179, 180-206, 210-212, 494, 571, and 574.

The binding moiety is capable of synergistically expanding a therapeutic window of a conditionally active EpCAM targeting protrispecific protein, by both steric masking and specific masking. In some cases, the binding moiety combines both steric masking (for example, *via* binding to a bulk serum albumin) and specific masking (for example, *via* non-CDR loops binding to the CDRs of an anti-EpCAM domain or an anti-CD3 scFv domain). In some cases, modifying the non-CDR loops within the binding moiety does not affect albumin binding. The protease cleavable linker, in some cases, enables activation of an EpCAM targeting protrispecific protein in a single proteolytic event, thereby allowing more efficient conversion of the protrispecific molecule in tumor microenvironment. Further, tumor-associated proteolytic activation, in some cases, reveals active T cell engager with minimal off-tumor activity after activation. The present disclosure, in some cases, provides a half-life extended T cell engager format (ProTriTAC) comprising an EpCAM binding moiety as described herein, which in some cases represents a new and improved approach to engineer conditionally active T cell engagers.

The half-life of the EpCAM binding domain in a conditionally active protrispecific format is, in some cases, extended in systemic circulation by using the binding moiety as described above which acts as a safety switch that keeps the multispecific protein in the pro format in an inert state until it reaches the tumor microenvironment where it is conditionally activated by cleavage of the linker and is able to bind its target antigen(s). The safety switch, in certain instances, provides several advantages: some examples including (i) expanding the therapeutic window of the conditionally active EpCAM targeting protein; (ii) reducing target-mediated drug disposition by maintaining the conditionally active EpCAM targeting protein in systemic circulation; (iii) reducing the concentration of undesirable activated protein in systemic circulation, thereby minimizing the spread of chemistry, manufacturing, and controls related impurities, *e.g.,* pre-activated drug product, endogenous viruses, host-cell proteins, DNA, leachables, anti-foam, antibiotics, toxins, solvents, heavy metals; (iv) reducing the concentration of undesirable activated proteins in systemic circulation, thereby minimizing the spread of product related impurities, aggregates, breakdown products, product variants due to: oxidation, deamidation, denaturation, loss of C-term Lys in MAbs; (v) preventing aberrant activation in circulation; (vi) reducing the toxicities associated with the leakage of activated species from diseased tissue or other pathophysiological conditions, *e.g.,* tumors, autoimmune diseases, inflammations, viral infections, tissue remodeling events (such as myocardial infarction, skin wound healing), or external injury (such as X-ray, CT scan, UV exposure); and (vii) reducing non-specific binding of the conditionally active EpCAM targeting protein. Furthermore, post-activation, or in other words post breaking of the safety switch, the conditionally active EpCAM targeting protein is separated from the safety switch which provided extended half-life, and thus is cleared from circulation. For instance, if the drug is inadvertently activated outside of a tumor environment or if it leaks out of a tumor environment after activation, it is likely to be cleared rapidly and is less likely to cause damage to normal tissues, thus reducing toxicity.

In some cases, a conditionally active multispecific EpCAM binding protein as described herein has an improved therapeutic index as compared to that of an EpCAM binding protein that is not conditionally active, but is rather constitutively active. For instance, an EpCAM ProTriTAC, in some cases, has an increased therapeutic index than an EpCAM TriTAC. The increase in therapeutic index, in some cases, is from at least about 2-fold to about 1000-fold, such as about 4-fold to about 800-fold, about 6-fold to about 800-fold, about 6-fold to about 600-fold, about 10-fold to about 400-fold, about 20-fold to about 200-fold, about 30-fold to about 150-fold, about 50-fold to about 100-fold. The increase in therapeutic index, in some cases, is attributed to the conjugation of the EpCAM binding domain to a binding moiety as described above, with the non-CDR loop and the cleavable linker.

A "therapeutic index" (TI) (also referred to as "therapeutic window") is, in some cases, a comparison of the minimum amount of a therapeutic agent (*e.g.,* an EpCAM TriTAC, an EpCAM ProTriTAC, an EpCAM CAR, an EpCAM ProCAR) that causes the therapeutic effect (*e.g.,* improved survival of a patient with an EpCAM expressing cancer, treated with a therapeutic agent as mentioned above) to minimum tolerated dose. An example of therapeutic index expansion of a ProTriTAC is shown in **Table 14**. In some instances, the therapeutic index improvement is manifested in terms of an improved EC₅₀ of an EpCAM TriTAC compared to an EpCAM ProTriTAC, in T cell mediated killing of cancer cells.

In some cases, the conditionally active EpCAM targeting protein format gives the EpCAM binding domain a significantly longer serum half-life and reduces the likelihood of its undesirable activation in circulation, thereby producing a "biobetter" version.

A binding moiety as described herein comprises at least one non-CDR loop. In some cases, a non-CDR loop provides a binding site for binding of the binding moiety to an EpCAM binding domain of this disclosure. In some cases, the binding moiety masks binding of the EpCAM binding domain to its target antigen, *e.g., via* steric occlusion, *via* specific intermolecular interactions, or a combination of both.

In some cases, a binding moiety as described herein further comprises complementarity determining regions (CDRs), for instance, specific for binding a bulk serum protein (*e.g.,* a human serum albumin). In some instances, a binding moiety of this disclosure is a domain derived from an immunoglobulin molecule (Ig molecule). The Ig may be of any class or subclass (IgG1, IgG2, IgG3, IgG4, IgA, IgE, IgM etc). A polypeptide chain of an Ig molecule folds into a series of parallel beta strands linked by loops. In the variable region, three of the loops constitute the "complementarity determining regions" (CDRs) which determine the antigen binding specificity of the molecule. An IgG molecule comprises at least two heavy (H) chains and two light (L) chains inter-connected by disulfide bonds, or an antigen binding fragment thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDRs) with are hypervariable in sequence and/or involved in antigen recognition and/or usually form structurally defined loops, interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4.

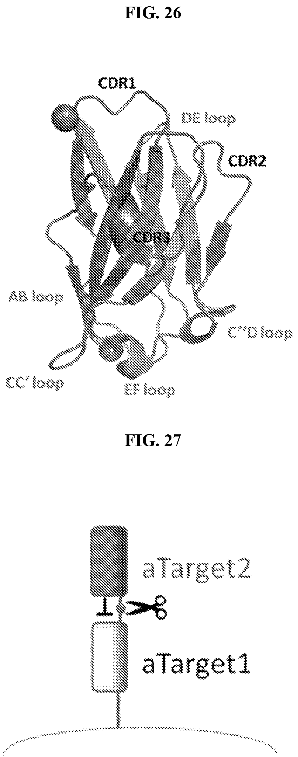

In some cases, a binding moiety of this disclosure is a heavy chain only antibody. As shown in **FIGS. 26****,** the variable domain of a heavy chain only antibody has several beta strands that are arranged in two sheets. The variable domain of a heavy chain only antibody contains three hypervariable loops, or complementarity-determining regions (CDRs) and framework regions FR1, FR2, FR3, and FR4. The three CDRs of the variable domain (CDR1, CDR2, CDR3) cluster at one end of the beta barrel. The CDRs are the loops that connect beta strands B-C, C'-C", and F-G of the immunoglobulin fold, whereas the bottom loops that connect beta strands AB, CC', C" -D and E-F of the immunoglobulin fold, and the top loop that connects the D-E strands of the immunoglobulin fold are the non-CDR loops.

In some cases of this disclosure at least some or all of the amino acid sequences of FR1, FR2, FR3, and FR4 are part of the "non-CDR loop" of the binding moieties described herein, such as a binding moiety that is a heavy chain only antibody. In some cases of this disclosure, at least some amino acid residues of a constant domain, CH1, CH2, or CH3, are part of the "non-CDR loop" of the binding moieties described herein. Non-CDR loops comprise, in some cases, one or more of AB, CD, EF, and DE loops of a C1-set domain of an Ig or an Ig-like molecule; AB, CC', EF, FG, BC, and EC' loops of a C2-set domain of an Ig or an Ig-like molecule; DE, BD, GF, A(A1A2)B, and EF loops of I(Intermediate)-set domain of an Ig or Ig-like molecule.

Within the variable domain, the CDRs are believed to be responsible for antigen recognition and binding, while the FR residues are considered a scaffold for the CDRs. However, in certain cases, some of the FR residues play an important role in antigen recognition and binding. Framework region residues that affect Ag binding are divided into two categories. The first are FR residues that contact the antigen, thus are part of the binding-site, and some of these residues are close in sequence to the CDRs. Other residues are those that are far from the CDRs in sequence, but are in close proximity to it in the 3-D structure of the molecule, *e.g.,* a loop in heavy chain.

In some cases, the non-CDR loop is modified to generate an antigen binding site specific for a bulk serum protein, such as albumin. In some cases, the non-CDR loop is modified to generate an antigen binding site specific for an EpCAM binding domain as described herein. In some cases, the non-CDR loop is modified to generate an antigen binding site specific for a CD3 binding domain as described herein.

It is contemplated that various techniques can be used for modifying the non-CDR loop, *e*.*g*., site-directed mutagenesis, random mutagenesis, insertion of at least one amino acid that is foreign to the non-CDR loop amino acid sequence, amino acid substitution. An antigen peptide is inserted into a non-CDR loop, in some examples. In some examples, an antigenic peptide is substituted for the non-CDR loop. The modification, to generate an antigen binding site, is in some cases in only one non-CDR loop. In other instances, more than one non-CDR loop are modified. For instance, the modification is in any one of the non-CDR loops shown in FIG. 26, *i.e.,* AB, CC', C" D, EF, and D-E. In some cases, the modification is in the DE loop. In other cases the modifications are in all four of AB, CC', C"D, E-F loops.

In certain examples, the binding moieties described herein are bound to the EpCAM binding domain *via* their AB, CC', C" D, or EF loop and are bound to a bulk-serum protein, such as albumin, *via* their B-C, C'-C", or F-G loop. In certain examples, the binding moiety is bound to the EpCAM binding domain *via* its AB, CC', C" D, and EF loop and is bound to a bulk-serum protein, such as albumin, *via* its BC, C'C", and FG loop. In certain examples, the binding moiety is bound to the EpCAM binding domain *via* one or more of AB, CC', C" D, and E-F loop and is bound to a bulk-serum protein, such as albumin, *via* one or more of BC, C'C", and FG loop. In certain examples, the binding moiety is bound to a bulk serum protein, such as albumin, *via* its AB, CC', C" D, or EF loop and is bound to the EpCAM binding domain *via* its BC, C'C", or FG loop. In certain examples, the binding moiety is bound to a bulk serum protein, such as albumin, *via* its AB, CC', C" D, and EF loop and is bound to the EpCAM binding domain *via* its BC, C'C", and FG loop. In certain examples, the binding moiety of the first case is bound to a bulk serum protein, such as albumin, *via* one or more of AB, CC', C" D, and E-F loop and is bound to the EpCAM binding protein, *via* one or more of BC, C'C", and FG loop. In certain examples, the binding moieties described herein are bound to a CD3 binding domain *via* their AB, CC', C" D, or EF loop and are bound to a bulk-serum protein, such as albumin, *via* their B-C, C'-C", or F-G loop. In certain examples, the binding moieties described herein are bound to a bulk serum protein, such as albumin, *via* their AB, CC', C" D, or EF loop and are bound to a CD3 binding domain, *via* their B-C, C'-C", or F-G loop. In certain examples, the binding moieties described herein are bound to a CD3 binding domain *via* their AB, CC', C" D, or EF loop and are bound to an EpCAM binding domain, *via* their B-C, C'-C", or F-G loop. In certain examples, the binding moieties described herein are bound to an EpCAM binding domain *via* their AB, CC', C" D, or EF loop and are bound to a CD3 binding domain, *via* their B-C, C'-C", or F-G loop.

The bulk serum protein comprises, for example, albumin, fibrinogen, or a globulin. In some cases, the binding moieties are engineered scaffolds. Engineered scaffolds comprise, for example, sdAb, a scFv, a Fab, a VHH, a fibronectin type III domain, immunoglobulin-like scaffold (as suggested in Halaby et al., 1999. Prot Eng 12(7):563-571), DARPin, cystine knot peptide, lipocalin, three-helix bundle scaffold, protein G-related albumin-binding module, or a DNA or RNA aptamer scaffold.

In some cases, the binding moieties comprise a binding site for the bulk serum protein. In some cases, the CDRs within the binding moieties provide a binding site for the bulk serum protein. The bulk serum protein is, in some examples, a globulin, albumin, transferrin, IgG1, IgG2, IgG4, IgG3, IgA monomer, Factor XIII, Fibrinogen, IgE, or pentameric IgM. In some cases, the binding moieties comprise a binding site for an immunoglobulin light chain. In some cases, the CDRs provide a binding site for the immunoglobulin light chain. The immunoglobulin light chain is, in some examples, an Igκ free light chain or an Igλ free light chain.

In additional cases, the binding moieties are any kinds of polypeptides. For example, in certain instances the binding moieties are natural peptides, synthetic peptides, or fibronectin scaffolds, or engineered bulk serum proteins. In some examples, the binding moieties comprise any type of binding domain, including but not limited to, domains from a monoclonal antibody, a polyclonal antibody, a recombinant antibody, a human antibody, a humanized antibody. In some cases, the binding moiety is a single chain variable fragment (scFv), a soluble TCR fragment, a single-domain antibody such as a heavy chain variable domain (VH), a light chain variable domain (VL) and a variable domain (VHH) of camelid derived nanobody. In other cases, the binding moieties are non-Ig binding domains, *i.e.,* antibody mimetic, such as anticalins, affilins, affibody molecules, affimers, affitins, alphabodies, avimers, DARPins, fynomers, kunitz domain peptides, and monobodies.

It is contemplated herein that the binding moiety described herein comprises at least one cleavable linker. In one aspect, the cleavable linker comprises a polypeptide having a sequence recognized and cleaved in a sequence-specific manner. The binding moiety described herein, in some cases, comprises a protease cleavable linker recognized and cleaved in a sequence-specific manner. In some cases, the protease cleavable linker is recognized in a sequence-specific manner by a matrix metalloprotease (MMP), for example a MMP9. In some cases, the protease cleavable linker recognized by a MMP9 comprises a polypeptide having an amino acid sequence PR(S/T)(L/I)(S/T) . In some cases, the protease cleavable linker recognized by a MMP9 comprises a polypeptide having an amino acid sequence LEATA . In some cases, the protease cleavable linker is recognized in a sequence-specific manner by a MMP11.

Proteases are proteins that cleave proteins, in some cases, in a sequence-specific manner. Proteases include but are not limited to serine proteases, cysteine proteases, aspartate proteases, threonine proteases, glutamic acid proteases, metalloproteases, asparagine peptide lyases, serum proteases, cathepsins, Cathepsin B, Cathepsin C, Cathepsin D, Cathepsin E, Cathepsin K, Cathepsin L, kallikreins, hK1, hK10, hK15, plasmin, collagenase, Type IV collagenase, stromelysin, Factor Xa, chymotrypsin-like protease, trypsin-like protease, elastase-like protease, subtilisin-like protease, actinidain, bromelain, calpain, caspases, caspase-3, Mir1-CP, papain, HIV-1 protease, HSV protease, CMV protease, chymosin, renin, pepsin, matriptase, legumain, plasmepsin, nepenthesin, metalloexopeptidases, metalloendopeptidases, matrix metalloproteases (MMP), MMP1, MMP2, MMP3, MMP8, MMP9, MMP13, MMP11, MMP14, urokinase plasminogen activator (uPA), enterokinase, prostate-specific antigen (PSA, hK3), interleukin-1β converting enzyme, thrombin, FAP (FAP-α), dipeptidyl peptidase, and dipeptidyl peptidase IV (DPPIV/CD26).

**Table 1: Exemplary proteases and protease recognition sequences**

| **Protease** | **Cleavage Domain Sequence** | **SEQ ID NO:** |
|---|---|---|
| MMP7 | KRALGLPG | 508 |
| MMP7 | (DE)₈RPLALWRS(DR)₈ | 509 |
| MMP9 | PR(S/T)(L/I)(S/T) | 510 |
| MMP9 | LEATA | 511 |
| MMP11 | GGAANLVRGG | 512 |
| MMP14 | SGRIGFLRTA | 513 |
| MMP | PLGLAG | 514 |
| MMP | PLGLAX | 515 |
| MMP | PLGC(me)AG | 516 |
| MMP | ESPAYYTA | 517 |
| MMP | RLQLKL | 518 |
| MMP | RLQLKAC | 519 |
| MMP2, MMP9, MMP14 | EP(Cit)G(Hof)YL | 520 |
| Urokinase plasminogen activator (uPA) | SGRSA | 521 |
| Urokinase plasminogen activator (uPA) | DAFK | 523 |
| Urokinase plasminogen activator (uPA) | GGGRR | 524 |
| Lysosomal Enzyme | GFLG | 525 |
| Lysosomal Enzyme | ALAL | 526 |
| Lysosomal Enzyme | FK | 527 |
| Cathepsin B | NLL | 528 |
| Cathepsin D | PIC(Et)FF | 529 |
| Cathepsin K | GGPRGLPG | 530 |
| Prostate Specific Antigen | HSSKLQ | 531 |
| Prostate Specific Antigen | HSSKLQL | 532 |
| Prostate Specific Antigen | HSSKLQEDA | 533 |
| Herpes Simplex Virus Protease | LVLASSSFGY | 534 |
| HIV Protease | GVSQNYPIVG | 535 |
| CMV Protease | GVVQASCRLA | 536 |
| Thrombin | F(Pip)RS | 537 |
| Thrombin | DPRSFL | 538 |
| Thrombin | PPRSFL | 539 |
| Caspase-3 | DEVD | 540 |
| Caspase-3 | DEVDP | 541 |
| Caspase-3 | KGSGDVEG | 542 |
| Interleukin 1β converting enzyme | GWEHDG | 543 |
| Enterokinase | EDDDDKA | 544 |
| FAP | KQEQNPGST | 545 |
| Kallikrein 2 | GKAFRR | 546 |
| Plasmin | DAFK | 547 |
| Plasmin | DVLK | 548 |
| Plasmin | DAFK | 549 |
| TOP | ALLLALL | 550 |

Proteases are known to be secreted by some diseased cells and tissues, for example tumor or cancer cells, creating a microenvironment that is rich in proteases or a protease-rich microenvironment. In some case, the blood of a subject is rich in proteases. In some cases, cells surrounding the tumor secrete proteases into the tumor microenvironment. Cells surrounding the tumor secreting proteases include but are not limited to the tumor stromal cells, myofibroblasts, blood cells, mast cells, B cells, NK cells, regulatory T cells, macrophages, cytotoxic T lymphocytes, dendritic cells, mesenchymal stem cells, polymorphonuclear cells, and other cells. In some cases, proteases are present in the blood of a subject, for example proteases that target amino acid sequences found in microbial peptides. This feature allows for targeted therapeutics such as antigen binding proteins to have additional specificity because T cells will not be bound by the antigen binding protein except in the protease rich microenvironment of the targeted cells or tissue.

Other non-limiting examples of linkers that may be utilized in constructs described herein are provided in the Sequence Listing below.

### Integration into chimeric antigen receptors (CAR)

The EpCAM binding proteins of the present disclosure can, in certain examples, be incorporated into a chimeric antigen receptor (CAR), or to a ProCAR. An engineered immune effector cell, *e.g.,* a T cell or NK cell, can be used to express a CAR that includes an EpCAM binding protein containing, for example, an anti-EpCAM single domain antibody as described herein. In one case, the CAR including the EpCAM binding protein as described herein is connected to a transmembrane domain via a hinge region, and further a costimulatory domain, *e.g.,* a functional signaling domain obtained from OX40, CD27, CD28, CD5, ICAM-1, LFA-1 (CD11a/CD18), ICOS (CD278), or 4-1BB. In some cases, the CAR further comprises a sequence encoding an intracellular signaling domain, such as 4-1BB and/or CD3 zeta. Exemplary sequences for a ProCAR comprising an EpCAM binding domain is provided in SEQ ID Nos.485-491, or sequences that are at least about 75% to 100% identical to a sequence selected from SEQ ID Nos. 485-491, such as about 75%, about 80%, about 85%, about 90%, about 91%, about 92%, about 93%, about 94%, about 95%, about 96%, about 97%, about 98%, about 99%, or 100%.

The conditionally active receptors described herein comprise at least one binding moiety comprising a non-CDR loop. In one aspect, the binding moiety masks binding of the EpCAM binding domain, until activation. The cleavable linker, for example, comprises a protease cleavage site or a pH dependent cleavage site. The cleavable linker, in certain instances, is cleaved only in a tumor microenvironment. Thus, the binding moiety, connected to the cleavable linker, and further bound to the EpCAM binding domain, in some examples, maintains the EpCAM binding domain in an inert state in circulation until the cleavable linker is cleaved off in a tumor microenvironment. In some cases, the binding moiety binds to the EpCAM binding domain. In some cases, a non-CDR loop provides a binding site for binding of the moiety to the EpCAM binding domain. In some cases, the binding moiety masks binding of the EpCAM binding domain to its target antigen, *e*.*g*. via steric occlusion, via specific intramolecular interactions, such as interactions within the different domains of the polypeptide comprising the binding moiety. In some cases, the binding moiety further comprises complimentary determining regions (CDRs).

In some instances, the binding moiety of a CAR or a proCAR as described herein is a domain derived from an immunoglobulin molecule (Ig molecule), as described above in the section corresponding to conditionally active multispecific EpCAM targeting proteins of this disclosure.

**FIG. 27** shows a schematic representation of a portion of an example cleavable conditionally active receptor of the present disclosure. The conditionally active receptor comprises an EpCAM binding domain (aTarget1), a cleavable linker, and a binding moiety (aTarget2). The EpCAM binding domain has specificity for a first target (EpCAM), while the binding moiety has specificity for a second target. The binding moiety also has a modified non-CDR loop, which inhibits binding of the EpCAM binding domain to its target. Once cleaved at the cleavable linker, the binding moiety can be released, enabling binding of the EpCAM binding domain.

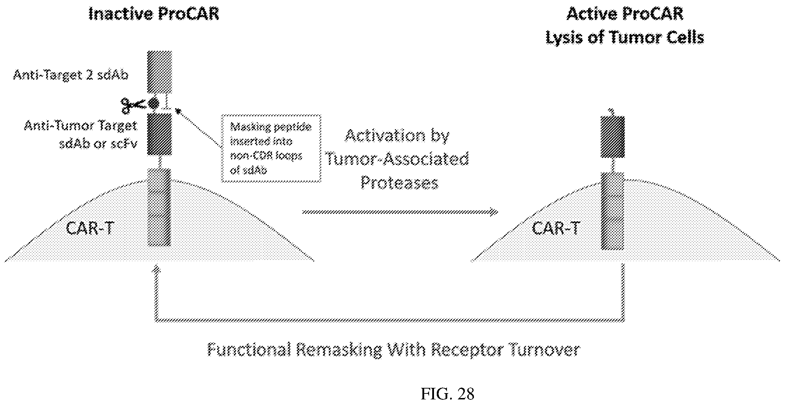

**FIG. 28** shows a schematic representation of the activation of an example conditionally active receptor of the present disclosure. Inactive receptor (Inactive ProCAR) comprises an EpCAM binding domain (Anti-Tumor Target sdAb or scFv) connected to a binding moiety (Anti-Target 2 sdAb) via a linker comprising a protease cleavage site. The binding moiety comprises a masking peptide/moiety, inserted into one or more non-CDR loops, such that the binding moiety binds to and inhibits the EpCAM antigen binding domain. In some cases, the binding moiety has specificity for a given target, as described further elsewhere herein. The receptor also comprises a transmembrane domain and an intracellular signaling domain. The receptor is provided in a T-cell (CAR-T). Upon exposure to a tumor environment, the protease cleavage site is cleaved by tumor-associated proteases, thereby activating the receptor, generating the active receptor which does not comprise the binding moiety. The receptor now comprises an active antigen-binding domain. As the receptor is internalized by the cell, new receptors are generated which comprise the binding moiety and are inactive.

The cleavable linker of the binding moiety, in some cases, comprises a protease cleavable site similar to what is described above with respect to the conditionally active multispecific proteins comprising an EpCAM binding domain of this disclosure. For instance, the cleavable linker in some cases comprises a sequence selected from Table 1 or from other linker sequences provided in the sequence table.

### Transmembrane domain

The conditionally active chimeric antigen receptors, T-cell receptor fusion proteins, and T-cell receptors of the present disclosure include a transmembrane domain for insertion into a eukaryotic cell membrane. In some cases, the transmembrane domain is interposed between the EpCAM binding domain and the intracellular domain. In some cases, the transmembrane domain is interposed between the EpCAM binding domain and the costimulatory domain.

Any transmembrane (TM) domain that provides for insertion of a polypeptide into the cell membrane of a eukaryotic (*e*.*g*., mammalian) cell is suitable for use. As one non-limiting example, the TM sequence IYIWAPLAGTCGVLLLSLVITLYC (SEQ ID NO: 551) can be used. Additional non-limiting examples of suitable TM sequences include: a) CD8 beta derived: GLLVAGVLVLLVSLGVAIHLCC (SEQ ID NO: 552); b) CD4 derived: ALIVLGGVAGLLLFIGLGIFFCVRC (SEQ ID NO: 553); c) CD3 zeta derived: LCYLLDGILFIYGVILTALFLRV (SEQ ID NO: 554); d) CD28 derived: WVLVVVGGVLACYSLLVTVAFIIFWV (SEQ ID NO: 555); e) CD134 (OX40) derived: AAILGLGLVLGLLGPLAILLALYLL (SEQ ID NO: 556); and f) CD7 derived: ALPAALAVISFLLGLGLGVACVLA (SEQ ID NO: 557).

### Hinge Region

In some cases, the conditionally active chimeric antigen receptors, T-cell receptor fusion proteins, and T-cell receptors of the present disclosure comprise a hinge region (also referred to herein as a "spacer"), where the hinge region is interposed between the EpCAM binding domain and the transmembrane domain. In some cases, the hinge region is an immunoglobulin heavy chain hinge region. In some cases, the hinge region is a hinge region polypeptide derived from a receptor (*e.g.,* a CD8-derived hinge region).

The hinge region can have a length of from about 4 amino acids to about 50 amino acids (aa), *e.g.,* from about 4 aa to about 10 aa, from about 10 aa to about 15 aa, from about 15 aa to about 20 aa, from about 20 aa to about 25 aa, from about 25 aa to about 30 aa, from about 30 aa to about 40 aa, or from about 40 aa to about 50 aa.

Suitable spacers can be readily selected and can be of any of a number of suitable lengths, such as from 1 amino acid (*e.g.,* Gly) to 20 amino acids, from 2 amino acids to 15 amino acids, from 3 amino acids to 12 amino acids, including 4 amino acids to 10 amino acids, 5 amino acids to 9 amino acids, 6 amino acids to 8 amino acids, or 7 amino acids to 8 amino acids, and can be 1, 2, 3, 4, 5, 6, or 7 amino acids.

Exemplary spacers include glycine polymers (G)n, glycine- serine polymers (including, for example, (GS)n, (GSGGS)n and (GGGS)n, where n is an integer of at least one), glycine-alanine polymers, alanine-serine polymers, and other flexible linkers known in the art. Glycine and glycine-serine polymers can be used; both Gly and Ser are relatively unstructured, and therefore can serve as a neutral tether between components. Glycine polymers can be used; glycine accesses significantly more phi-psi space than even alanine, and is much less restricted than residues with longer side chains (*see* Scheraga, Rev. Computational Chem. 11173-142 (1992)). Exemplary spacers comprise amino acid sequences including, but not limited to, GGSG, GGSGG, GSGSG, GSGGG, GGGSG, GSSSG, and the like.

Immunoglobulin hinge region amino acid sequences are known in the art; see, *e.g.,* Tan et al. (1990) Proc. Natl. Acad. Sci. USA 87: 162; and Huck et al. (1986) Nucl. Acids Res. 14: 1779. As non-limiting examples, an immunoglobulin hinge region can include one of the following amino acid sequences: DKTHT; CPPC; CPEPKSCDTPPPCPR (SEQ ID NO: 558); *see, e.g.,* Glaser et al. (2005) J. Biol. Chem. 280:41494); ELKTPLGDTTHT (SEQ ID NO: 559); KSCDKTHTCP (SEQ ID NO: 560); KCCVDCP (SEQ ID NO: 561); KYGPPCP (SEQ ID NO: 562); EPKSCDKTHTCPPCP (SEQ ID NO: 563); human IgG1 hinge); ERKCCVECPPCP (SEQ ID NO: 564); human IgG2 hinge); ELKTPLGDTTHTCPRCP (SEQ ID NO: 565); human IgG3 hinge); SPNMVPHAHHAQ (SEQ ID NO: 566); human IgG4 hinge); and the like.

In some cases, the hinge region comprises an amino acid sequence of a human IgG1, IgG2, IgG3, or IgG4, hinge region. The hinge region can include one or more amino acid substitutions and/or insertions and/or deletions compared to a wild-type (naturally-occurring) hinge region. For example, His229 of human IgG1 hinge can be substituted with Tyr, so that the hinge region comprises the sequence EPKSCDKTYTCPPCP (SEQ ID NO: 567); *see, e.g.,* Yan et al. (2012) J. Biol. Chem. 287:5891.

In some cases, the hinge region comprises an amino acid sequence derived from human CD8; *e.g.,* the hinge region comprises the amino acid sequence:
TTTPAPRPPTPAPTIASQPLSLRPEACRPAAGGAVHTRGLDFACD (SEQ ID NO: 568), or a variant thereof.

### Conditionally Active Chimeric Antigen Receptors

In one case, the disclosure provides a conditionally active chimeric antigen receptor (CAR). A CAR generally comprises multiple domains, including a target antigen binding domain, a transmembrane domain, and an intracellular signaling domain. The conditionally active CAR of the present disclosure comprises multiple domains, including a binding moiety, a target antigen binding domain which binds EpCAM, a transmembrane domain, and an intracellular signaling domain. In some cases, the intracellular signaling domain is a signaling domain of a protein including, but not limited to, ZAP70, CD3 zeta, and 4-1BB.

In some cases, the conditionally active chimeric antigen receptor further comprises a costimulatory domain. In some cases, the costimulatory domain is a functional signaling domain of a protein including, but not limited to, OX40, CD2, CD27, CD28, CDS, ICAM-1, LFA-1 (CD11a/CD18), ICOS (CD278), and 4-1BB (CD137), and amino acid sequences thereof having at least one, two, or three modifications but not more than 20, 10, or 5 modifications thereto.

In some cases, the transmembrane domain comprises a transmembrane domain of a protein including, but not limited to, a TCR alpha chain, a TCR beta chain, a TCR zeta chain, a CD3 epsilon TCR subunit, a CD3 gamma TCR subunit, a CD3 delta TCR subunit, CD45, CD4, CDS, CD8, CD9, CD16, CD22, CD33, CD28, CD37, CD64, CD80, CD86, CD134, CD137, CD154, functional fragments thereof, and amino acid sequences thereof having at least one but not more than 20 modifications thereto.

In one aspect, the disclosure provides a cell (*e.g.,* T-cell) engineered to express a CAR. In one aspect a cell is transformed with the CAR and the CAR is expressed on the cell surface. In some cases, the cell (*e.g.,* T-cell) is transduced with a viral vector encoding a CAR. In some cases, the viral vector is a retroviral vector. In some cases, the viral vector is a lentiviral vector. In some such cases, the cell may stably express the CAR. In another case, the cell (*e.g.,* T cell) is transfected with a nucleic acid, *e*.*g*., mRNA, cDNA, DNA, encoding a CAR. In some such cases, the cell may transiently express the CAR.

### Conditionally Active T-cell Receptor Fusion Proteins

In one case, the disclosure provides a conditionally active T-cell receptor fusion protein. As used herein, a "T-cell receptor (TCR) fusion protein" or "TFP" includes a recombinant polypeptide derived from the various polypeptides comprising the TCR that is generally capable of i) binding to a surface antigen on target cells and ii) interacting with other polypeptide components of the intact TCR complex, typically when co-located in or on the surface of a T-cell.

The conditionally active TFP comprises a binding moiety, an EpCAM binding domain, and a T-cell receptor subunit. In some cases, the T-cell receptor subunit further comprises at least a portion of a T-cell receptor extracellular domain, a transmembrane domain, and a T-cell receptor intracellular domain. In some cases, the transmembrane domain comprises a transmembrane domain of a protein including, but not limited to, a TCR alpha chain, a TCR beta chain, a TCR zeta chain, a CD3 epsilon TCR subunit, a CD3 gamma TCR subunit, a CD3 delta TCR subunit, CD45, CD4, CDS, CD8, CD9, CD16, CD22, CD33, CD28, CD37, CD64, CD80, CD86, CD134, CD137, CD154, functional fragments thereof, or amino acid sequences thereof having at least one, two, or three modifications but not more than 20, 10, or 5 modifications thereto.

In some cases, the T-cell receptor intracellular domain comprises a stimulatory domain. The stimulatory domain may be from T-cell receptor subunit, including but not limited to the beta subunit, alpha subunit, delta subunit, gamma subunit, epsilon subunit, or a combination thereof. In some cases, the stimulatory domain comprises an immunoreceptor tyrosine-based activation motif (ITAM) or portion thereof including, but not limited to, CD3 zeta TCR subunit, CD3 epsilon TCR subunit, CD3 gamma TCR subunit, CD3 delta TCR subunit, TCR zeta chain, Fc epsilon receptor 1 chain, Fc epsilon receptor 2 chain, Fc gamma receptor 1 chain, Fc gamma receptor 2a chain, Fc gamma receptor 2b 1 chain, Fc gamma receptor 2b2 chain, Fc gamma receptor 3a chain, Fc gamma receptor 3b chain, Fc beta receptor 1 chain, TYROBP (DAP12), CDS, CD16a, CD16b, CD22, CD23, CD32, CD64, CD79a, CD79b, CD89, CD278, CD66d, functional fragments thereof, and amino acid sequences thereof having at least one, two, or three modifications but not more than 20, 10, or 5 modifications thereto.

In some cases, the conditionally active TFP further comprises a costimulatory domain. In some cases, the costimulatory domain is a functional signaling domain of a protein including, but not limited to, OX40, CD2, CD27, CD28, CDS, ICAM-1, LFA-1 (CD11a/CD18), ICOS (CD278), and 4-1BB (CD137), and amino acid sequences thereof having at least one, two, or three modifications but not more than 20, 10, or 5 modifications thereto.

In some cases, the EpCAM binding domain is connected to the T-cell receptor extracellular domain by a linker sequence. In some instances, the encoded linker sequence comprises (G₄S)n, wherein n=1 to 4. In some instances, the encoded linker sequence comprises a long linker (LL) sequence. In some instances, the encoded long linker sequence comprises (G4S)n, wherein n=2 to 4. In some instances, the encoded linker sequence comprises a short linker (SL) sequence. In some instances, the encoded short linker sequence comprises (G₄S)n, wherein n=1 to 3.

In one aspect, the disclosure provides a cell (*e.g*., T-cell) engineered to express a conditionally active T-cell receptor fusion protein (TFP). In one aspect a cell is transformed with the conditionally active TFP and the conditionally active TFP is expressed on the cell surface. In some cases, the cell (*e.g.,* T-cell) is transduced with a viral vector encoding a conditionally active TFP. In some cases, the viral vector is a retroviral vector. In some cases, the viral vector is a lentiviral vector. In some such cases, the cell may stably express the conditionally active TFP. In another case, the cell (*e.g.,* T cell) is transfected with a nucleic acid, *e.g.,* mRNA, cDNA, DNA, encoding a conditionally active TFP. In some such cases, the cell may transiently express the conditionally active TFP.

### Conditionally Active T-cell Receptors

In one case, the disclosure provides a conditionally active T-cell receptor. A T-cell receptor generally comprises multiple subunits, including alpha, beta, delta, gamma, epsilon, and zeta subunits. The conditionally active T-cell receptor of the present disclosure comprises a binding moiety. In some cases, the binding moiety is attached to a T-cell receptor subunit including, but not limited to, the alpha subunit, beta subunit, or a combination thereof.

In some cases, the binding moiety is capable of masking the binding of the T-cell receptor to its target. In some cases, the binding moiety binds to T-cell receptor. In some cases, a non-CDR loop provides a binding site for binding of the moiety to the T-cell receptor. In some cases, the non-CDR loop provides a binding site specific for T-cell receptor alpha, T-cell receptor beta, or a combination thereof. In some cases, the binding moiety masks binding of the T-cell receptor to its target, *e.g.* via steric occlusion, via specific intermolecular interactions.

In one aspect, the disclosure provides a cell (*e.g.,* T-cell) engineered to express a conditionally active T-cell receptor (TCR). In one aspect a cell is transformed with the conditionally active TCR and the conditionally active TCR is expressed on the cell surface. In some cases, the cell (*e.g.,* T-cell) is transduced with a viral vector encoding a conditionally active TCR. In some cases, the viral vector is a retroviral vector. In some cases, the viral vector is a lentiviral vector. In some such cases, the cell may stably express the conditionally active TCR. In another case, the cell (*e.g.,* T cell) is transfected with a nucleic acid, *e.g.,* mRNA, cDNA, DNA, encoding a conditionally active TCR. In some such cases, the cell may transiently express the conditionally active TCR.

### Cells

In one case, the present disclosure provides a cell comprising the chimeric antigen receptor or the conditionally active chimeric antigen receptor, conditionally active T-cell receptor fusion protein, or conditionally active T-cell receptor of the present disclosure. The cell may be a mammalian cell.

Suitable mammalian cells include primary cells and immortalized cell lines. Suitable mammalian cell lines include human cell lines, non-human primate cell lines, rodent (*e.g*., mouse, rat) cell lines, and the like. Suitable mammalian cell lines include, but are not limited to, HeLa cells (*e.g*., American Type Culture Collection (ATCC) No. CCL-2), CHO cells (*e.g*., ATCC Nos. CRL9618, CCL61, CRL9096), 293 cells (*e.g*., ATCC No. CRL-1573), Vero cells, NIH 3T3 cells (*e.g*., ATCC No. CRL-1658), Huh-7 cells, BHK cells (*e.g*., ATCC No. CCL10), PC12 cells (ATCC No. CRL1721), COS cells, COS-7 cells (ATCC No. CRL1651), RAT1 cells, mouse L cells (ATCC No. CCLI.3), human embryonic kidney (HEK) cells (ATCC No. CRL1573), HLHepG2 cells, HuT-78, Jurkat, HL-60, NK cell lines (*e.g.,* NKL, NK92, and YTS), and the like.

In some instances, the cell is not an immortalized cell line, but is instead a cell (*e.g*., a primary cell) obtained from an individual. For example, in some cases, the cell is an immune cell obtained from an individual. As an example, the cell is a T lymphocyte obtained from an individual. As another example, the cell is a cytotoxic cell obtained from an individual. As another example, the cell is a stem cell or progenitor cell obtained from an individual.

In recent studies, CAR constructs have been used to direct natural killer (NK) cell activity, reviewed by Hermanson & Kaufman (2015, Front Immunol 6:195) and Carlsten & Childs (2015, Front Immunol 6:266). Similar to T cells, NK cells can be transfected with CAR expression constructs and used to induce an immune response. Because NK cells do not require HLA matching, they can be used as allogeneic effector cells (Harmanson & Kaufman, 2015). Also, peripheral blood NK cells (PB-NK), of use for therapy, may be isolated from donors by a simple blood draw. The CAR constructs of use may contain similar elements to those used to make CAR-T cells.

As such, in some cases, the present disclosure provides a cell comprising an NK cell comprising the chimeric antigen receptor, the conditionally active chimeric antigen receptor, conditionally active T-cell receptor fusion protein, or conditionally active T-cell receptor of the present disclosure.

As discussed above in the context of a conditionally active EpCAM binding protein (*e.g*., an EpCAM ProTriTAC), in some cases, a conditionally active chimeric antigen receptor as described herein has an improved therapeutic index as compared to that of a chimeric antigen receptor comprising the same EpCAM binding domain as the conditionally active variant but is constitutively active instead of being conditionally active. For instance, an EpCAM ProCAR, in some cases, has an increased therapeutic index than an EpCAM CAR. The increased, in some cases, is from at least about 2-fold to about 1000-fold, such as about 4-fold to about 800-fold, about 6-fold to about 800-fold, about 6-fold to about 600-fold, about 10-fold to about 400-fold, about 20-fold to about 200-fold, about 30-fold to about 150-fold, about 50-fold to about 100-fold. The increase in therapeutic index, in some cases, is attributed to the conjugation of the EpCAM binding domain to a binding moiety as described above, with the non-CDR loop and the cleavable linker.

### Methods of Generating a Cell Comprising a Conditionally Active Receptor

The present disclosure provides a method of generating a cell comprising a conditionally active chimeric antigen receptor, T-cell receptor fusion protein, or T-cell receptor. The method generally involves genetically modifying a mammalian cell with an expression vector, or an RNA (*e.g*., in vitro transcribed RNA), comprising nucleotide sequences encoding a conditionally active chimeric antigen receptor, T-cell receptor fusion protein, or T-cell receptor of the present disclosure. The genetic modification can be carried out in *vivo, in vitro,* or *ex vivo.* The cell can be, for example, an immune cell (*e.g*., a T lymphocyte or NK cell), a stem cell, or a progenitor cell.

In some cases, the genetic modification is carried out *ex vivo.* For example, a T lymphocyte, a stem cell, or an NK cell is obtained from an individual; and the cell obtained from the individual is genetically modified to express a conditionally active chimeric antigen receptor, T-cell receptor fusion protein, or T-cell receptor of the present disclosure.

### Sources of T-Cells

In some cases, a source of T-cells is obtained from a subject. The term "subject," as used throughout this disclosure, is intended to include living organisms in which an immune response can be elicited (*e*.*g*., mammals). Examples of subjects include humans, dogs, cats, mice, rats, and transgenic species thereof. T-cells can be obtained from a number of sources, including, but not limited to, allogenic T cells (*e.g*., allogeneic donor-derived CAR T cells), natural killer cells (*e.g*., donor derived natural killer cells), peripheral blood mononuclear cells, bone marrow, lymph node tissue, cord blood, thymus tissue, tissue from a site of infection, ascites, pleural effusion, spleen tissue, and tumors. In certain cases of the present disclosure, any number of T-cell lines available in the art, may be used. In certain cases of the present disclosure, T-cells can be obtained from a unit of blood collected from a subject using any number of techniques known to the skilled artisan, such as FICOLL^{™} separation. In one case, cells from the circulating blood of an individual are obtained by apheresis. The apheresis product typically contains lymphocytes, including T-cells, monocytes, granulocytes, B cells, other nucleated white blood cells, red blood cells, and platelets. In one case, the cells collected by apheresis are washed to remove the plasma fraction and to place the cells in an appropriate buffer or media for subsequent processing steps. In one case of the disclosure, the cells are washed with phosphate buffered saline (PBS). In an alternative case, the wash solution lacks calcium and may lack magnesium or may lack many if not all divalent cations. Initial activation steps in the absence of calcium can lead to magnified activation. As those of ordinary skill in the art would readily appreciate a washing step may be accomplished by methods known to those in the art, such as by using a semi-automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

In one case, T-cells are isolated from peripheral blood lymphocytes by lysing the red blood cells and depleting the monocytes, for example, by centrifugation through a PERCOLL^{™} gradient or by counterflow centrifugal elutriation. A specific subpopulation of T-cells, such as CD3+, CD28+, CD4+, CD8+, CD45RA+, and CD45RO+ T-cells, can be further isolated by positive or negative selection techniques. For example, in one case, T-cells are isolated by incubation with anti-CD3/anti-CD28 (*e.g*., 3×28)-conjugated beads, such as DYNABEADS^{®} M-450 CD3/CD28 T, for a time period sufficient for positive selection of the desired T-cells. In one case, the time period is about 30 minutes. In a further case, the time period ranges from 30 minutes to 36 hours or longer and all integer values there between. In a further case, the time period is at least 1, 2, 3, 4, 5, or 6 hours. In yet another case, the time period is 10 to 24 hours. In one case, the incubation time period is 24 hours. Longer incubation times may be used to isolate T-cells in any situation where there are few T-cells as compared to other cell types, such in isolating tumor infiltrating lymphocytes (TIL) from tumor tissue or from immunocompromised individuals. Further, use of longer incubation times can increase the efficiency of capture of CD8+ T-cells. Thus, by simply shortening or lengthening the time T-cells are allowed to bind to the CD3/CD28 beads and/or by increasing or decreasing the ratio of beads to T-cells (as described further herein), subpopulations of T-cells can be preferentially selected for or against at culture initiation or at other time points during the process. Additionally, by increasing or decreasing the ratio of anti-CD3 and/or anti-CD28 antibodies on the beads or other surface, subpopulations of T-cells can be preferentially selected for or against at culture initiation or at other desired time points. Multiple rounds of selection can also be used in the context of this disclosure. In certain cases, it may be desirable to perform the selection procedure and use the "unselected" cells in the activation and expansion process. "Unselected" cells can also be subjected to further rounds of selection.

Enrichment of a T-cell population by negative selection can be accomplished with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail typically includes antibodies to CD14, CD20, CD11b, CD16, HLA-DR, and CD8. In certain cases, it may be desirable to enrich for or positively select for regulatory T-cells which typically express CD4+, CD25+, CD62Lhi, GITR+, and FoxP3+. Alternatively, in certain cases, T regulatory cells are depleted by anti-CD25 conjugated beads or other similar method of selection.

In one case, a T-cell population can be selected that expresses one or more of IFN-γ, TNFα, IL-17A, IL-2, IL-3, IL-4, GM-CSF, IL-10, IL-13, granzyme B, and perforin, or other appropriate molecules, *e*.*g*., other cytokines. Methods for screening for cell expression can be determined, *e*.*g*., by the methods described in PCT Publication No. WO 2013/126712.

For isolation of a desired population of cells by positive or negative selection, the concentration of cells and surface (*e*.*g*., particles such as beads) can be varied. In certain cases, it may be desirable to significantly decrease the volume in which beads and cells are mixed together (*e*.*g*., increase the concentration of cells), to ensure maximum contact of cells and beads. For example, in one case, a concentration of 2 billion cells/ml is used. In one case, a concentration of 1 billion cells/ml is used. In a further case, greater than 100 million cells/ml is used. In a further case, a concentration of cells of 10, 15, 20, 25, 30, 35, 40, 45, or 50 million cells/ml is used. In yet one case, a concentration of cells from 75, 80, 85, 90, 95, or 100 million cells/ml is used. In further cases, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T-cells, or from samples where there are many tumor cells present (*e*.*g*., leukemic blood, tumor tissue, etc.). Such populations of cells may have therapeutic value and would be desirable to obtain. For example, using high concentration of cells allows more efficient selection of CD8+ T-cells that normally have weaker CD28 expression.

In another case, it may be desirable to use lower concentrations of cells. By significantly diluting the mixture of T-cells and surface (*e*.*g*., particles such as beads), interactions between the particles and cells is minimized. This selects for cells that express high amounts of desired antigens to be bound to the particles. For example, CD4+ T-cells express higher levels of CD28 and are more efficiently captured than CD8+ T-cells in dilute concentrations. In one case, the concentration of cells used is 5×10e6/ml. In other cases, the concentration used can be from about 1×10⁵/ml to 1×10⁶/ml and any integer value in between.

In other cases, the cells may be incubated on a rotator for varying lengths of time at varying speeds at either 2-10° C. or at room temperature.

T-cells for stimulation can also be frozen after a washing step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80° C. at a rate of 1° per minute and stored in the vapor phase of a liquid nitrogen storage tank. Other methods of controlled freezing may be used as well as uncontrolled freezing immediately at -20° C. or in liquid nitrogen.

In certain cases, cryopreserved cells are thawed and washed as described herein and allowed to rest for one hour at room temperature prior to activation using the methods of the present disclosure.

Also contemplated in the context of the disclosure is the collection of blood samples or apheresis product from a subject at a time period prior to when the expanded cells as described herein might be needed. As such, the source of the cells to be expanded can be collected at any time point necessary, and desired cells, such as T-cells, isolated and frozen for later use in T-cell therapy for any number of diseases or conditions that would benefit from T-cell therapy, such as those described herein. In one case a blood sample or an apheresis is taken from a generally healthy subject. In certain cases, a blood sample or an apheresis is taken from a generally healthy subject who is at risk of developing a disease, but who has not yet developed a disease, and the cells of interest are isolated and frozen for later use. In certain cases, the T-cells may be expanded, frozen, and used at a later time. In certain cases, samples are collected from a patient shortly after diagnosis of a particular disease as described herein but prior to any treatments. In a further case, the cells are isolated from a blood sample or an apheresis from a subject prior to any number of relevant treatment modalities, including but not limited to treatment with agents such as natalizumab, efalizumab, antiviral agents, chemotherapy, radiation, immunosuppressive agents, such as cyclosporin, azathioprine, methotrexate, mycophenolate, and FK506, antibodies, or other immunoablative agents such as CAMPATH, anti-CD3 antibodies, cytoxan, fludarabine, cyclosporin, FK506, rapamycin, mycophenolic acid, steroids, FR901228, and irradiation.

In a further case of the present disclosure, T-cells are obtained from a patient directly following treatment that leaves the subject with functional T-cells. In this regard, it has been observed that following certain cancer treatments, in particular treatments with drugs that damage the immune system, shortly after treatment during the period when patients would normally be recovering from the treatment, the quality of T-cells obtained may be optimal or improved for their ability to expand *ex vivo.* Likewise, following *ex vivo* manipulation using the methods described herein, these cells may be in a preferred state for enhanced engraftment and *in vivo* expansion. Thus, it is contemplated within the context of the present disclosure to collect blood cells, including T-cells, dendritic cells, or other cells of the hematopoietic lineage, during this recovery phase. Further, in certain cases, mobilization (for example, mobilization with GM-CSF) and conditioning regimens can be used to create a condition in a subject wherein repopulation, recirculation, regeneration, and/or expansion of particular cell types is favored, especially during a defined window of time following therapy. Illustrative cell types include T-cells, B cells, dendritic cells, and other cells of the immune system.

### Activation and Expansion of T-Cells

T-cells may be activated and expanded generally using methods as described, for example, in U.S. Pat. Nos. 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

Generally, the T-cells of the disclosure may be expanded by contact with a surface having attached thereto an agent that stimulates a CD3/TCR complex associated signal and a ligand that stimulates a costimulatory molecule on the surface of the T-cells. In particular, T-cell populations may be stimulated as described herein, such as by contact with an anti-CD3 antibody, or antigen binding fragment thereof, or an anti-CD2 antibody immobilized on a surface, or by contact with a protein kinase C activator (*e*.*g*., bryostatin) in conjunction with a calcium ionophore. For co-stimulation of an accessory molecule on the surface of the T-cells, a ligand that binds the accessory molecule is used. For example, a population of T-cells can be contacted with an anti-CD3 antibody and an anti-CD28 antibody, under conditions appropriate for stimulating proliferation of the T-cells. To stimulate proliferation of either CD4+ T-cells or CD8+ T-cells, an anti-CD3 antibody and an anti-CD28 antibody. Examples of an anti-CD28 antibody include 9.3, B-T3, XR-CD28 (Diaclone, Besancon, France) can be used as can other methods commonly known in the art (Berg et al., Transplant Proc. 30(8):3975-3977, 1998; Haanen et al., J. Exp. Med. 190(9):13191328, 1999; Garland et al., J. Immunol. Meth. 227(1-2):53-63, 1999).

In certain cases, the primary stimulatory signal and the costimulatory signal for the T-cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one case, the agent providing the costimulatory signal is bound to a cell surface and the agent providing the primary activation signal is in solution or coupled to a surface. In certain cases, both agents can be in solution. In one case, the agents may be in soluble form, and then cross-linked to a surface, such as a cell expressing Fc receptors or an antibody or other binding agent which will bind to the agents. In this regard, see for example, U.S. Patent Application Publication Nos. 20040101519 and 20060034810 for artificial antigen presenting cells (aAPCs) that are contemplated for use in activating and expanding T-cells in the present disclosure.

In one case, the two agents are immobilized on beads, either on the same bead, *i.e.,* "cis," or to separate beads, i.e., "trans." By way of example, the agent providing the primary activation signal is an anti-CD3 antibody or an antigen binding fragment thereof and the agent providing the costimulatory signal is an anti-CD28 antibody or antigen binding fragment thereof; and both agents are co-immobilized to the same bead in equivalent molecular amounts. In one case, a 1:1 ratio of each antibody bound to the beads for CD4+ T-cell expansion and T-cell growth is used. In certain cases of the present disclosure, a ratio of anti CD3:CD28 antibodies bound to the beads is used such that an increase in T-cell expansion is observed as compared to the expansion observed using a ratio of 1:1. In one particular case an increase of from about 1 to about 3 fold is observed as compared to the expansion observed using a ratio of 1:1. In one case, the ratio of CD3:CD28 antibody bound to the beads ranges from 100:1 to 1:100 and all integer values there between. In one case of the present disclosure, more anti-CD28 antibody is bound to the particles than anti-CD3 antibody, i.e., the ratio of CD3:CD28 is less than one. In certain cases of the disclosure, the ratio of anti CD28 antibody to anti CD3 antibody bound to the beads is greater than 2:1. In one particular case, a 1:100 CD3:CD28 ratio of antibody bound to beads is used. In one case, a 1:75 CD3:CD28 ratio of antibody bound to beads is used. In a further case, a 1:50 CD3:CD28 ratio of antibody bound to beads is used. In one case, a 1:30 CD3:CD28 ratio of antibody bound to beads is used. In one case, a 1:10 CD3:CD28 ratio of antibody bound to beads is used. In one case, a 1:3 CD3:CD28 ratio of antibody bound to the beads is used. In yet one case, a 3:1 CD3:CD28 ratio of antibody bound to the beads is used.

Ratios of particles to cells from 1:500 to 500:1 and any integer values in between may be used to stimulate T-cells or other target cells. As those of ordinary skill in the art can readily appreciate, the ratio of particles to cells may depend on particle size relative to the target cell. For example, small sized beads could only bind a few cells, while larger beads could bind many. In certain cases the ratio of cells to particles ranges from 1:100 to 100:1 and any integer values in-between and in further cases the ratio comprises 1:9 to 9:1 and any integer values in between, can also be used to stimulate T-cells. The ratio of anti-CD3- and anti-CD28-coupled particles to T-cells that result in T-cell stimulation can vary as noted above, however certain values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one preferred ratio being at least 1:1 particles per T-cell. In one case, a ratio of particles to cells of 1:1 or less is used. In one particular case, a particle: cell ratio is 1:5. In further cases, the ratio of particles to cells can be varied depending on the day of stimulation. For example, in one case, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In one particular case, the ratio of particles to cells is 1:1 on the first day of stimulation and adjusted to 1:5 on the third and fifth days of stimulation. In one case, particles are added on a daily or every other day basis to a final ratio of 1:1 on the first day, and 1:5 on the third and fifth days of stimulation. In one case, the ratio of particles to cells is 2:1 on the first day of stimulation and adjusted to 1:10 on the third and fifth days of stimulation. In one case, particles are added on a daily or every other day basis to a final ratio of 1:1 on the first day, and 1:10 on the third and fifth days of stimulation. One of skill in the art will appreciate that a variety of other ratios may be suitable for use in the present disclosure. In particular, ratios will vary depending on particle size and on cell size and type.

In further cases of the present disclosure, the cells, such as T-cells, are combined with agent-coated beads, the beads and the cells are subsequently separated, and then the cells are cultured. In an alternative case, prior to culture, the agent-coated beads and cells are not separated but are cultured together. In a further case, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3×28 beads) to contact the T-cells. In one case the cells (for example, 10⁴ to 10⁹ T-cells) and beads (for example, DYNABEADS^{®} M-450 CD3/CD28 T paramagnetic beads at a ratio of 1:1) are combined in a buffer, for example PBS (without divalent cations such as, calcium and magnesium). Again, those of ordinary skill in the art can readily appreciate any cell concentration may be used. For example, the target cell may be very rare in the sample and comprise only 0.01% of the sample or the entire sample (i.e., 100%) may comprise the target cell of interest. Accordingly, any cell number is within the context of the present disclosure. In certain cases, it may be desirable to significantly decrease the volume in which particles and cells are mixed together *(i.e.,* increase the concentration of cells), to ensure maximum contact of cells and particles. For example, in one case, a concentration of about 2 billion cells/ml is used. In one case, greater than 100 million cells/ml is used. In a further case, a concentration of cells of 10, 15, 20, 25, 30, 35, 40, 45, or 50 million cells/ml is used. In yet one case, a concentration of cells from 75, 80, 85, 90, 95, or 100 million cells/ml is used. In further cases, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T-cells. Such populations of cells may have therapeutic value and would be desirable to obtain in certain cases. For example, using high concentration of cells allows more efficient selection of CD8+ T-cells that normally have weaker CD28 expression.

In one case of the present disclosure, the mixture may be cultured for several hours (about 3 hours) to about 14 days or any hourly integer value in between. In one case, the mixture may be cultured for 21 days. In one case of the disclosure the beads and the T-cells are cultured together for about eight days. In one case, the beads and T-cells are cultured together for 2-3 days. Several cycles of stimulation may also be desired such that culture time of T-cells can be 60 days or more. Conditions appropriate for T-cell culture include an appropriate media (*e.g*., Minimal Essential Media or RPMI Media 1640 or, X-vivo 15, (Lonza)) that may contain factors necessary for proliferation and viability, including serum (*e*.*g*., fetal bovine or human serum), interleukin-2 (IL-2), insulin, IFN-γ, IL-4, IL-7, GM-CSF, IL-10, IL-12, IL-15, TGFβ, and TNF-α or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T-cells. Antibiotics, *e*.*g*., penicillin and streptomycin, are included only in experimental cultures, not in cultures of cells that are to be infused into a subject. The target cells are maintained under conditions necessary to support growth, for example, an appropriate temperature (*e.g*., 37° C.) and atmosphere (*e.g.,* air plus 5% CO₂).

T-cells that have been exposed to varied stimulation times may exhibit different characteristics. For example, typical blood or apheresed peripheral blood mononuclear cell products have a helper T-cell population (TH, CD4+) that is greater than the cytotoxic or suppressor T-cell population (TC, CD8+). *Ex vivo* expansion of T-cells by stimulating CD3 and CD28 receptors produces a population of T-cells that prior to about days 8-9 consists predominately of TH cells, while after about days 8-9, the population of T-cells comprises an increasingly greater population of TC cells.

### EpCAM Binding Protein Modifications

The invention provides a multispecific protein as defined by the claims. The following paragraphs are useful for understanding this aspect of the invention. The EpCAM binding proteins described herein, including EpCAM binding domains (*e*.*g*., an EpCAM binding sdAb of this disclosure) and EpCAM targeting multispecific proteins (*e.g*., an EpCAM targeting trispecific or protrispecific protein as described herein) encompass derivatives or analogs in which (i) an amino acid is substituted with an amino acid residue that is not one encoded by the genetic code, (ii) the mature polypeptide is fused with another compound such as polyethylene glycol, or (iii) additional amino acids are fused to the protein, such as a leader or secretory sequence or a sequence for purification of the protein.

Typical modifications include, but are not limited to, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent crosslinks, formation of cystine, formation of pyroglutamate, formylation, gamma carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Modifications are made anywhere in the EpCAM binding proteins described herein, including the peptide backbone, the amino acid side-chains, and the amino or carboxyl termini. Certain common peptide modifications that are useful for modification of the EpCAM binding proteins include glycosylation, lipid attachment, sulfation, gamma-carboxylation of glutamic acid residues, hydroxylation, blockage of the amino or carboxyl group in a polypeptide, or both, by a covalent modification, and ADP-ribosylation.

In some cases, derivatives of the EpCAM binding proteins as described herein comprise immunoreactive modulator derivatives and antigen binding molecules comprising one or more modifications.

In some cases, the EpCAM binding proteins of the disclosure are monovalent or multivalent bivalent, trivalent, etc.). As used herein, the term "valency" refers to the number of potential target binding sites associated with an antibody. Each target binding site specifically binds one target molecule or specific position or locus on a target molecule. When an antibody is monovalent, each binding site of the molecule will specifically bind to a single antigen position or epitope. When an antibody comprises more than one target binding site (multivalent), each target binding site may specifically bind the same or different molecules (*e*.*g*., may bind to different ligands or different antigens, or different epitopes or positions on the same antigen).

In some cases, the EpCAM binding proteins as set forth above are fused to an Fc region from any species, including but not limited to, human immunoglobulin, such as human IgG1, a human IgG2, a human IgG3, human IgG4, to generate Fc-fusion EpCAM binding proteins. In some cases, the Fc-fusion EpCAM binding proteins of this disclosure have extended half-life compared to an otherwise identical EpCAM binding protein. In some cases, the Fc-fusion EpCAM binding proteins of this disclosure contain *inter alia* one or more additional amino acid residue substitutions, mutations and/or modifications, *e*.*g*., in the Fc region. which result in a binding protein with preferred characteristics including, but not limited to: altered pharmacokinetics, extended serum half-life.

In some cases, such Fc-fused EpCAM binding proteins provide extended half-lives in a mammal, such as in a human, of greater than 5 days, greater than 10 days, greater than 15 days, greater than 20 days, greater than 25 days, greater than 30 days, greater than 35 days, greater than 40 days, greater than 45 days, greater than 2 months, greater than 3 months, greater than 4 months, or greater than 5 months. The increased half-life, in some cases, results in a higher serum titer which thus reduces the frequency of the administration of the EpCAM binding proteins and/or reduces the concentration of the antibodies to be administered. Binding to human FcRn *in vivo* and serum half-life of human FcRn high affinity binding polypeptides is assayed, in some examples, in transgenic mice or transfected human cell lines expressing human FcRn, or in primates to which the polypeptides with a variant Fc region are administered.

The EpCAM binding proteins, in some cases, are differentially modified during or after production, *e*.*g*., by glycosylation, acetylation, phosphorylation, amidation, derivatization by known protecting/blocking groups, proteolytic cleavage, linkage to an antibody molecule or other cellular ligand, etc. Any of numerous chemical modifications are carried out by techniques, including but not limited, to specific chemical cleavage by cyanogen bromide, trypsin, chymotrypsin, papain, V8 protease, NaBH4, acetylation, formylation, oxidation, reduction, metabolic synthesis in the presence of tunicamycin, etc.

Various post-translational modifications of the EpCAM binding proteins also encompassed by the disclosure include, for example, N-linked or O-linked carbohydrate chains, processing of N-terminal or C-terminal ends, attachment of chemical moieties to the amino acid backbone, chemical modifications of N-linked or O-linked carbohydrate chains, and addition or deletion of an N-terminal methionine residue as a result of prokaryotic host cell expression. Moreover, the EpCAM binding proteins are, in some cases, modified with a detectable label, such as an enzymatic, fluorescent, radioisotopic or affinity label to allow for detection and isolation of the modulator.

### Polynucleotides Encoding EpCAM Binding Proteins

The invention provides a polynucleotide as defined by the claims. The following paragraphs are useful for understanding this aspect of the invention. In some cases, the polynucleotide molecules are provided as a DNA construct. In other cases, the polynucleotide molecules are provided as a messenger RNA transcript.

The polynucleotide molecules are constructed by known methods such as by combining the genes encoding a single domain EpCAM binding protein or gene encoding various domains of EpCAM binding proteins comprising more than one domain. In some cases, the gene encoding the domains are either separated by peptide linkers or, in other cases, directly linked by a peptide bond, into a single genetic construct operably linked to a suitable promoter, and optionally a suitable transcription terminator, and expressing it in bacteria or other appropriate expression system such as, for example CHO cells. Depending on the vector system and host utilized, any number of suitable transcription and translation elements, including constitutive and inducible promoters, may be used. The promoter is selected such that it drives the expression of the polynucleotide in the respective host cell.

In some cases, the polynucleotide coding for an EpCAM binding protein as described herein is inserted into a vector, preferably an expression vector, which represents a further case. This recombinant vector can be constructed according to known methods. Vectors of particular interest include plasmids, phagemids, phage derivatives, virii (*e*.*g*., retroviruses, adenoviruses, adeno-associated viruses, herpes viruses, lentiviruses, and the like), and cosmids.

A variety of expression vector/host systems may be utilized to contain and express the polynucleotide encoding the polypeptide of the described EpCAM binding protein. Examples of expression vectors for expression in *E.coli* are pSKK (Le Gall et al., J Immunol Methods. (2004) 285(1):111-27) or pcDNA5 (Invitrogen) for expression in mammalian cells. Thus, the EpCAM binding proteins as described herein, in some cases, are produced by introducing a vector encoding the protein as described above into a host cell and culturing said host cell under conditions whereby the protein domains are expressed, may be isolated and, optionally, further purified.

### Pharmaceutical Compositions

The invention provides a pharmaceutical composition as defined by the claims. The following paragraphs are useful for understanding this aspect of the invention. Also provided, in some cases, are pharmaceutical compositions comprising an anti-EpCAM binding protein described herein, a vector comprising the polynucleotide encoding the polypeptide of the EpCAM binding proteins or a host cell transformed by this vector and at least one pharmaceutically acceptable carrier. The term "pharmaceutically acceptable carrier" includes, but is not limited to, any carrier that does not interfere with the effectiveness of the biological activity of the ingredients and that is not toxic to the patient to whom it is administered. Examples of suitable pharmaceutical carriers are well known in the art and include phosphate buffered saline solutions, water, emulsions, such as oil/water emulsions, various types of wetting agents, sterile solutions etc. Such carriers can be formulated by conventional methods and can be administered to the subject at a suitable dose. Preferably, the compositions are sterile. These compositions may also contain adjuvants such as preservative, emulsifying agents and dispersing agents. Prevention of the action of microorganisms may be ensured by the inclusion of various antibacterial and antifungal agents. A further case provides one or more of the above described EpCAM binding proteins packaged in lyophilized form, or packaged in an aqueous medium.

In some cases of the pharmaceutical compositions, the EpCAM binding proteins described herein are encapsulated in nanoparticles. In some cases, the nanoparticles are fullerenes, liquid crystals, liposome, quantum dots, superparamagnetic nanoparticles, dendrimers, or nanorods. In other cases of the pharmaceutical compositions, the EpCAM binding protein is attached to liposomes. In some instances, the EpCAM binding proteins are conjugated to the surface of liposomes. In some instances, the EpCAM binding proteins are encapsulated within the shell of a liposome. In some instances, the liposome is a cationic liposome.

The EpCAM binding proteins described herein are contemplated for use as a medicament. Administration is effected by different ways, e.g. by intravenous, intraperitoneal, subcutaneous, intramuscular, topical or intradermal administration. In some cases, the route of administration depends on the kind of therapy and the kind of compound contained in the pharmaceutical composition. The dosage regimen will be determined by the attending physician and other clinical factors. Dosages for any one patient depends on many factors, including the patient's size, body surface area, age, sex, the particular compound to be administered, time and route of administration, the kind of therapy, general health and other drugs being administered concurrently. An "effective dose" refers to amounts of the active ingredient that are sufficient to affect the course and the severity of the disease, leading to the reduction or remission of such pathology and may be determined using known methods.

In some cases, the EpCAM binding proteins of this disclosure are administered at a dosage of up to 10 mg/kg at a frequency of once a week. In some cases, the dosage ranges from about 1 ng/kg to about 10 mg/kg, for example about 1 ng/kg to about 70 ng/kg. In some cases, the dose is from about 1 ng/kg to about 10 ng/kg, about 5 ng/kg to about 15 ng/kg, about 12 ng/kg to about 20 ng/kg, about 18 ng/kg to about 30 ng/kg, about 25 ng/kg to about 50 ng/kg, about 35 ng/kg to about 60 ng/kg, about 45 ng/kg to about 70 ng/kg, about 65 ng/kg to about 85 ng/kg, about 80 ng/kg to about 1 µg/kg, about 0.5 µg/kg to about 5 µg/kg, about 2 µg/kg to about 10 µg/kg, about 7 µg/kg to about 15 µg/kg, about 12 µg/kg to about 25 µg/kg, about 20 µg/kg to about 50 µg/kg, about 35 µg/kg to about 70 µg/kg, about 45 µg/kg to about 80 µg/kg, about 65 µg/kg to about 90 µg/kg, about 85 µg/kg to about 0.1 mg/kg, about 0.095 mg/kg to about 10 mg/kg. In some cases, the dosage is about 0.1 mg/kg to about 0.2 mg/kg; about 0.25 mg/kg to about 0.5 mg/kg, about 0.45 mg/kg to about 1 mg/kg, about 0.75 mg/kg to about 3 mg/kg, about 2.5 mg/kg to about 4 mg/kg, about 3.5 mg/kg to about 5 mg/kg, about 4.5 mg/kg to about 6 mg/kg, about 5.5 mg/kg to about 7 mg/kg, about 6.5 mg/kg to about 8 mg/kg, about 7.5 mg/kg to about 9 mg/kg, or about 8.5 mg/kg to about 10 mg/kg. The frequency of administration, in some cases, is about less than daily, every other day, less than once a day, twice a week, weekly, once in 7 days, once in two weeks, once in three weeks, once in four weeks, or once a month. In some cases, the frequency of administration is weekly. In some cases, the frequency of administration is weekly and the dosage is up to 10 mg/kg. In some cases, duration of administration is from about 1 day to about 4 weeks or longer.

### Methods of treatments and Tumor growth reduction properties

The invention provides multispecific proteins, pharmaceutical compositions and vectors for use in methods of treatment, as defined by the claims. The following paragraphs are useful for understanding this aspect of the invention. The references to the methods of treatment by therapy or surgery or in vivo diagnosis methods in this description are to be interpreted as references to compounds of the present invention for use in those methods. Also provided in certain cases are methods of treating a condition associated with malignant cells expressing EpCAM in a subject comprising administering to a subject in need thereof an effective amount of an EpCAM binding domains or multispecific proteins (including conditionally active multispecific proteins) comprising an EpCAM binding domain of this disclosure, or a CAR or a ProCAR comprising an EpCAM binding protein as described herein, or a pharmaceutical composition comprising the same. In some cases, the condition is a cancer.

In another aspect, the disclosure provides a method of inhibiting tumor growth or progression in a subject who has malignant cells expressing EpCAM, comprising administering to the subject in need thereof an effective amount of an EpCAM binding domains or multispecific proteins comprising an EpCAM binding domain of this disclosure, or a CAR comprising an EpCAM binding protein as described herein, or a pharmaceutical composition comprising the same. In another aspect, the disclosure provides a method of inhibiting metastasis of malignant cells expressing EpCAM in a subject, comprising administering to the subject in need thereof an effective amount of an EpCAM binding domains or multispecific proteins comprising an EpCAM binding domain of this disclosure, or a pharmaceutical composition comprising the same. In another aspect, the disclosure provides a method of inducing tumor regression in a subject who has malignant cells expressing EpCAM, comprising administering to the subject in need thereof an effective amount of an EpCAM binding domains or multispecific proteins comprising an EpCAM binding domain of this disclosure, or a pharmaceutical composition comprising the same. In some cases, the methods as described herein further comprise administering an effective amount of a second therapeutic agent. In some cases, the second therapeutic agent is a biotherapeutic agent, for example, an antibody. In some cases, the second therapeutic agent is a cytokine, TNFa (Tumor Necrosis Factor alpha), a PAP (phosphatidic acid phosphatase) inhibitor, an oncolytic virus, a kinase inhibitor, an IDO (Indoleamine-pyrrole 2,3-dioxygenase) inhibitor, a glutaminase GLS 1 inhibitor, a CAR (Chimeric Antigen Receptor)-T cell or T cell therapy, a TLR (Toll-Like Receptor) Agonist (*e*.*g*., TLR3, TLR4, TLR5, TLR7, TLR9), or a tumor vaccine.

In certain cases, the EpCAM binding proteins of the disclosure reduce the growth of tumor cells *in vivo* when administered to a subject who has tumor cells that express EpCAM. Measurement of the reduction of the growth of tumor cells can be determined by multiple different methodologies well known in the art. Nonlimiting examples include direct measurement of tumor dimension, measurement of excised tumor mass and comparison to control subjects, measurement via imaging techniques (*e*.*g*., CT or MRI) that may or may not use isotopes or luminescent molecules (*e*.*g*., luciferase) for enhanced analysis, and the like. In specific cases, administration of the EpCAM binding proteins of the disclosure results in a reduction of *in vivo* growth of tumor cells as compared to a control antigen binding agent by at least about 10%, 20%, 30%, 40%, 50%, 60%, 70%, 80%, 90% or 100%, with an about 100% reduction in tumor growth indicating a complete response and disappearance of the tumor. In further cases, administration of the EpCAM binding proteins of the disclosure results in a reduction of *in vivo* growth of tumor cells as compared to a control antigen binding agent by about 50-100%, about 75-100% or about 90-100%. In further cases, administration of the EpCAM binding proteins of the disclosure results in a reduction of *in vivo* growth of tumor cells as compared to a control antigen binding agent by about 50-60%, about 60-70%, about 70-80%, about 80-90%, or about 90-100%.

In some cases, the EpCAM binding proteins of the present disclosure are administered to treat a neoplastic condition. Neoplastic conditions, in some cases, are benign or malignant; solid tumors or other blood neoplasia; and, in some cases, are selected from the group including, but not limited to: adrenal gland tumors, AIDS-associated cancers, alveolar soft part sarcoma, astrocytic tumors, autonomic ganglia tumors, bladder cancer (squamous cell carcinoma and transitional cell carcinoma), blastocoelic disorders, bone cancer (adamantinoma, aneurismal bone cysts, osteochondroma, osteosarcoma), brain and spinal cord cancers, metastatic brain tumors, breast cancer including triple negative breast cancer, carotid body tumors, cervical cancer, chondrosarcoma, chordoma, chromophobe renal cell carcinoma, clear cell carcinoma, colon cancer, colorectal cancer, cutaneous benign fibrous histiocytomas, desmoplastic small round cell tumors, ependymomas, epithelial disorders, Ewing's tumors, extraskeletal myxoid chondrosarcoma, fibrogenesis imperfecta ossium, fibrous dysplasia of the bone, gallbladder and bile duct cancers, gastric cancer, gastrointestinal, gestational trophoblastic disease, germ cell tumors, glandular disorders, head and neck cancers, hypothalamic, intestinal cancer, islet cell tumors, Kaposi's Sarcoma, kidney cancer (nephroblastoma, papillary renal cell carcinoma), leukemias, lipoma/benign lipomatous tumors, liposarcoma/malignant lipomatous tumors, liver cancer (hepatoblastoma, hepatocellular carcinoma), lymphomas, lung cancers (small cell carcinoma, adenocarcinoma, squamous cell carcinoma, large cell carcinoma etc.), macrophagal disorders, medulloblastoma, melanoma, meningiomas, multiple endocrine neoplasia, multiple myeloma, myelodysplastic syndrome, neuroblastoma, neuroendocrine tumors, ovarian cancer, pancreatic cancers, papillary thyroid carcinomas, parathyroid tumors, pediatric cancers, peripheral nerve sheath tumors, phaeochromocytoma, pituitary tumors, prostate cancer, posterior unveal melanoma, rare hematologic disorders, renal metastatic cancer, rhabdoid tumor, rhabdomyosarcoma, sarcomas, skin cancer, soft-tissue sarcomas, squamous cell cancer, stomach cancer, stromal disorders, synovial sarcoma, testicular cancer, thymic carcinoma, thymoma, thyroid metastatic cancer, and uterine cancers (carcinoma of the cervix, endometrial carcinoma, and leiomyoma).

In certain cases the EpCAM binding proteins of the present disclosure are used as a front line therapy and administered to subjects who have not previously been treated for the cancerous condition. In other cases the EpCAM binding proteins of the present disclosure are used to treat subjects that have previously been treated (with an EpCAM binding protein of this disclosure or with other anti-cancer agent) and have relapsed or determined to be refractory to the previous treatment. In some cases the EpCAM binding proteins of the present disclosure are used to treat subjects that have recurrent tumors.

In some cases, an EpCAM binding protein as described herein, including a multispecific protein, a CAR or a ProCAR as described herein, is administered to treat a cancer with widespread EpCAM expression and prevalence, including, but not limited to, colorectal, prostate, neuroendocrine, thyroid, lung (both non-small cell lung and small cell lung cancers), gastric, ovarian, endometrial, pancreatic, biliary tract and gallbladder cancer, esophageal, breast, and all adenocarcinoma.

In some aspects, the EpCAM binding proteins of the present disclosure are administered to treat a proliferative disorder comprising a solid tumor including, but not limited to, adrenal, liver, kidney, bladder, breast, gastric, ovarian, cervical, uterine, esophageal, colorectal, prostate, pancreatic, lung (both small cell and non-small cell), thyroid, carcinomas, sarcomas, glioblastomas and various head and neck tumors.

In some cases, the EpCAM binding proteins of the present disclosure are administered to a subject suffering from melanoma. In some cases, the EpCAM binding proteins of the present disclosure are used to diagnose, monitor, treat or prevent melanoma. The term "melanoma," as used herein, includes all types of melanoma including, but not limited to, primary melanoma, malignant melanoma, cutaneous melanoma, extracutaneous melanoma, superficial spreading melanoma, polypoid melanoma, melanocarcinomas, melanoepitheliomas, melanosarcomas, melanoma in situ, nodular malignant melanoma, lentigo maligna melanoma, lentiginous melanoma, lentiginous malignant melanoma, mucosal lentiginous melanoma, mucosal melanoma, acral lentiginous melanoma, soft tissue melanoma, ocular melanoma, invasive melanoma, familial atypical mole and melanoma (FAM-M) syndrome, desmoplastic malignant melanoma or uveal melanoma.

In some cases, possible indications for administration of the EpCAM binding proteins of this disclosure or pharmaceutical compositions comprising the same are tumorous diseases especially epithelial cancers/carcinomas such as breast cancer, colon cancer, prostate cancer, head and neck cancer, skin cancer, cancers of the genito-urinary tract, *e*.*g*., ovarian cancer, endometrial cancer, cervix cancer and kidney cancer, lung cancer, gastric cancer, cancer of the small intestine, liver cancer, pancreas cancer, gall bladder cancer, cancers of the bile duct, esophagus cancer, cancer of the salivatory glands and cancer of the thyroid gland. In some cases, the administration of the EpCAM binding proteins of this disclosure or pharmaceutical compositions comprising the same is indicated for minimal residual disease, such as early solid tumor, advanced solid tumor or metastatic solid tumor, which is characterized by the local and non-local reoccurrence of the tumor caused by the survival of single cells.

In selected aspects an EpCAM binding proteins of the disclosure is incorporated into a chimeric antigen receptors (CAR) and the EpCAM CAR is administered in a CAR based therapy effective at treating a cancer, such as: epithelial cancers/carcinomas such as breast cancer, colon cancer, prostate cancer, head and neck cancer, skin cancer, cancers of the genito-urinary tract, *e*.*g*., ovarian cancer, endometrial cancer, cervix cancer and kidney cancer, lung cancer, gastric cancer, cancer of the small intestine, liver cancer, pancreas cancer, gall bladder cancer, cancers of the bile duct, esophagus cancer, cancer of the salivatory glands and cancer of the thyroid gland, small cell lung cancer, non-small cell lung cancer (*e*.*g*., squamous cell non-small cell lung cancer or squamous cell small cell lung cancer) and large cell neuroendocrine carcinoma (LCNEC).

A chimeric antigen receptor is generally an artificially constructed hybrid protein or polypeptide containing or comprising an antigen binding domain of an antibody linked to a signaling domain (*e*.*g*., T-cell signaling or T-cell activation domains). In some cases, CARs comprising the EpCAM binding protein of the present disclosure have the ability to redirect the specificity and reactivity of sensitized lymphocytes (*e*.*g*., T-cells) toward EpCAM positive target cells in a non-MHC-restricted manner by exploiting the antigen-binding properties of antibodies or antigen binding fragments thereof. The non-MHC-restricted antigen recognition gives T-cells expressing EpCAM CARs the ability to recognize tumorigenic EpCAM independent of antigen processing, thus bypassing a major mechanism of tumor escape. Moreover, when expressed in T-cells, CARs advantageously do not dimerize with endogenous T cell receptor (TCR) alpha and beta chains.

In some cases the disclosed EpCAM binding proteins are administered to refractory patients (*i.e.,* those whose disease recurs during or shortly after completing a course of initial therapy); sensitive patients (*i.e.,* those whose relapse is longer than 2-3 months after primary therapy); or patients exhibiting resistance to a platinum based agent (*e*.*g*., carboplatin, cisplatin, oxaliplatin) and/or a taxane (*e*.*g*., docetaxel, paclitaxel, larotaxel or cabazitaxel). In another case the disclosed EpCAM CAR treatments are effective at treating ovarian cancer, including ovarian-serous carcinoma and ovarian-papillary serous carcinoma.

In another case the EpCAM binding proteins of the disclosure, the EpCAM CAR, or the EpCAM sensitized lymphocytes, or any combination thereof are used in maintenance therapy to reduce or eliminate the chance of tumor recurrence following the initial presentation of the disease. In some cases, the disorder has been treated and the initial tumor mass eliminated, reduced or otherwise ameliorated so the patient is asymptomatic or in remission. At such time the subject is administered pharmaceutically effective amounts of the disclosed the EpCAM binding proteins of the disclosure, the EpCAM CAR, or the EpCAM sensitized lymphocytes, or any combination thereof one or more times regardless of if there is little or no indication of disease using standard diagnostic procedures. In some cases, the EpCAM binding proteins of the disclosure, the EpCAM CAR, or the EpCAM sensitized lymphocytes, or any combination thereof is administered on a regular schedule over a period of time, such as weekly, every two weeks, monthly, every six weeks, every two months, every three months every six months or annually, for example, to reduce the potential of disease recurrence. Moreover such treatments are in some cases continued for a period of weeks, months, years or even indefinitely depending on the patient response and clinical and diagnostic parameters.

In yet another case, the EpCAM binding proteins of the disclosure, the EpCAM CAR, or the EpCAM sensitized lymphocytes, or any combination thereof are used to prophylactically or as an adjuvant therapy to prevent or reduce the possibility of tumor metastasis following a debulking procedure. As used in the present disclosure a "debulking procedure," is means any procedure, technique or method that eliminates, reduces, treats or ameliorates a tumor or tumor proliferation. Exemplary debulking procedures include, but are not limited to, surgery, radiation treatments (*i.e.,* beam radiation), chemotherapy, immunotherapy or ablation. In some cases, at appropriate times, the EpCAM binding proteins of the disclosure, the EpCAM CAR, or the EpCAM sensitized lymphocytes, or any combination thereof are administered as suggested by clinical, diagnostic or theranostic procedures to reduce tumor metastasis. In some cases, the dosing regimen is accompanied by appropriate diagnostic or monitoring techniques that allow it to be modified.

Yet other cases of the disclosure comprise administering the EpCAM binding protein of the disclosure, the EpCAM CAR, or the EpCAM sensitized lymphocytes, or any combination thereof to subjects that are asymptomatic but at risk of developing a proliferative disorder. That is, in some cases, the EpCAM binding protein of the disclosure, the EpCAM CAR, or the EpCAM sensitized lymphocytes, or any combination thereof are used in preventative sense and given to patients that have been examined or tested and have one or more noted risk factors (*e*.*g*., genomic indications, family history, in vivo or in vitro test results, etc.) but have not developed neoplasia. In such cases those skilled in the art would be able to determine an effective dosing regimen through empirical observation or through accepted clinical practices.

In some cases of the methods described herein, the EpCAM binding proteins, or compositions as described herein are administered in combination with an agent for treatment of the particular disease, disorder or condition. Agents include but are not limited to, therapies involving antibodies, small molecules (*e*.*g*., chemotherapeutics), hormones (steroidal, peptide, and the like), radiotherapies (γ-rays, X-rays, and/or the directed delivery of radioisotopes, microwaves, UV radiation and the like), gene therapies (*e*.*g*., antisense, retroviral therapy and the like) and other immunotherapies. In some cases, an EpCAM binding protein as described herein is administered in combination with anti-diarrheal agents, anti-emetic agents, analgesics, opioids and/or non-steroidal anti-inflammatory agents. In some cases, an EpCAM binding protein as described herein is administered in combination with anti-cancer agents. Nonlimiting examples of anti-cancer agents that can be used in the various cases of the disclosure, including pharmaceutical compositions and dosage forms and kits of the disclosure, include: acivicin; aclarubicin; acodazole hydrochloride; acronine; adozelesin; aldesleukin; altretamine; ambomycin; ametantrone acetate; aminoglutethimide; amsacrine; anastrozole; anthramycin; asparaginase; asperlin; azacitidine; azetepa; azotomycin; batimastat; benzodepa; bicalutamide; bisantrene hydrochloride; bisnafide dimesylate; bizelesin; bleomycin sulfate; brequinar sodium; bropirimine; busulfan; cactinomycin; calusterone; caracemide; carbetimer; carboplatin; carmustine; carubicin hydrochloride; carzelesin; cedefingol; chlorambucil; cirolemycin; cisplatin; cladribine; crisnatol mesylate; cyclophosphamide; cytarabine; dacarbazine; dactinomycin; daunorubicin hydrochloride; decitabine; dexormaplatin; dezaguanine; dezaguanine mesylate; diaziquone; docetaxel; doxorubicin; doxorubicin hydrochloride; droloxifene; droloxifene citrate; dromostanolone propionate; duazomycin; edatrexate; eflornithine hydrochloride; elsamitrucin; enloplatin; enpromate; epipropidine; epirubicin hydrochloride; erbulozole; esorubicin hydrochloride; estramustine; estramustine phosphate sodium; etanidazole; etoposide; etoposide phosphate; etoprine; fadrozole hydrochloride; fazarabine; fenretinide; floxuridine; fludarabine phosphate; fluorouracil; flurocitabine; fosquidone; fostriecin sodium; gemcitabine; gemcitabine hydrochloride; hydroxyurea; idarubicin hydrochloride; ifosfamide; ilmofosine; interleukin II (including recombinant interleukin II, or rIL2), interferon alpha-2a; interferon alpha-2b; interferon alpha-n1 interferon alpha-n3; interferon beta-I a; interferon gamma-I b; iproplatin; irinotecan hydrochloride; lanreotide acetate; letrozole; leuprolide acetate; liarozole hydrochloride; lometrexol sodium; lomustine; losoxantrone hydrochloride; masoprocol; maytansine; mechlorethamine hydrochloride; megestrol acetate; melengestrol acetate; melphalan; menogaril; mercaptopurine; methotrexate; methotrexate sodium; metoprine; meturedepa; mitindomide; mitocarcin; mitocromin; mitogillin; mitomalcin; mitomycin; mitosper; mitotane; mitoxantrone hydrochloride; mycophenolic acid; nocodazole; nogalamycin; ormaplatin; oxisuran; paclitaxel; pegaspargase; peliomycin; pentamustine; peplomycin sulfate; perfosfamide; pipobroman; piposulfan; piroxantrone hydrochloride; plicamycin; plomestane; porfimer sodium; porfiromycin; prednimustine; procarbazine hydrochloride; puromycin; puromycin hydrochloride; pyrazofurin; riboprine; rogletimide; safingol; safingol hydrochloride; semustine; simtrazene; sparfosate sodium; sparsomycin; spirogermanium hydrochloride; spiromustine; spiroplatin; streptonigrin; streptozocin; sulofenur; talisomycin; tecogalan sodium; tegafur; teloxantrone hydrochloride; temoporfin; teniposide; teroxirone; testolactone; thiamiprine; thioguanine; thiotepa; tiazofurin; tirapazamine; toremifene citrate; trestolone acetate; triciribine phosphate; trimetrexate; trimetrexate glucuronate; triptorelin; tubulozole hydrochloride; uracil mustard; uredepa; vapreotide; verteporfin; vinblastine sulfate; vincristine sulfate; vindesine; vindesine sulfate; vinepidine sulfate; vinglycinate sulfate; vinleurosine sulfate; vinorelbine tartrate; vinzolidine sulfate; vinzolidine sulfate; vorozole; zeniplatin; zinostatin; zorubicin hydrochloride. Other examples of anti-cancer drugs include, but are not limited to: 20-epi-1,25 dihydroxyvitamin D3; 5-ethynyluracil; abiraterone; aclarubicin; acylfulvene; adecypenol; adozelesin; aldesleukin; ALL-TK antagonists; altretamine; ambamustine; amidox; amifostine; aminolevulinic acid; amrubicin; amsacrine; anagrelide; anastrozole; andrographolide; angiogenesis inhibitors; antagonist D; antagonist G; antarelix; anti-dorsalizing morphogenetic protein-1; antiandrogen, prostatic carcinoma; antiestrogen; antineoplaston; antisense oligonucleotides; aphidicolin glycinate; apoptosis gene modulators; apoptosis regulators; apurinic acid; ara-CDP-DL-PTBA; arginine deaminase; asulacrine; atamestane; atrimustine; axinastatin 1; axinastatin 2; axinastatin 3; azasetron; azatoxin; azatyrosine; baccatin III derivatives; balanol; batimastat; BCR/ABL antagonists; benzochlorins; benzoylstaurosporine; beta lactam derivatives; beta-alethine; betaclamycin B; betulinic acid; bFGF inhibitor; bicalutamide; bisantrene; bisaziridinylspermine; bisnafide; bistratene A; bizelesin; breflate; bropirimine; budotitane; buthionine sulfoximine; calcipotriol; calphostin C; camptothecin derivatives; canarypox IL-2; capecitabine; carboxamide-amino-triazole; carboxyamidotriazole; CaRest M3; CARN 700; cartilage derived inhibitor; carzelesin; casein kinase inhibitors (ICOS); castanospermine; cecropin B; cetrorelix; chlorins; chloroquinoxaline sulfonamide; cicaprost; cis-porphyrin; cladribine; clomifene analogues; clotrimazole; collismycin A; collismycin B; combretastatin A4; combretastatin analogue; conagenin; crambescidin 816; crisnatol; cryptophycin 8; cryptophycin A derivatives; curacin A; cyclopentanthraquinones; cycloplatam; cypemycin; cytarabine ocfosfate; cytolytic factor; cytostatin; dacliximab; decitabine; dehydrodidemnin B; deslorelin; dexamethasone; dexifosfamide; dexrazoxane; dexverapamil; diaziquone; didemnin B; didox; diethylnorspermine; dihydro-5-azacytidine; dihydrotaxol, 9-; dioxamycin; diphenyl spiromustine; docetaxel; docosanol; dolasetron; doxifluridine; droloxifene; dronabinol; duocarmycin SA; ebselen; ecomustine; edelfosine; edrecolomab; eflornithine; elemene; emitefur; epirubicin; epristeride; estramustine analogue; estrogen agonists; estrogen antagonists; etanidazole; etoposide phosphate; exemestane; fadrozole; fazarabine; fenretinide; filgrastim; finasteride; flavopiridol; flezelastine; fluasterone; fludarabine; fluorodaunorunicin hydrochloride; forfenimex; formestane; fostriecin; fotemustine; gadolinium texaphyrin; gallium nitrate; galocitabine; ganirelix; gelatinase inhibitors; gemcitabine; glutathione inhibitors; hepsulfam; heregulin; hexamethylene bisacetamide; hypericin; ibandronic acid; idarubicin; idoxifene; idramantone; ilmofosine; ilomastat; imidazoacridones; imiquimod; immunostimulant peptides; insulin-like growth factor-I receptor inhibitor; interferon agonists; interferons; interleukins; iobenguane; iododoxorubicin; ipomeanol, 4-; iroplact; irsogladine; isobengazole; isohomohalicondrin B; itasetron; jasplakinolide; kahalalide F; lamellarin-N triacetate; lanreotide; leinamycin; lenograstim; lentinan sulfate; leptolstatin; letrozole; leukemia inhibiting factor; leukocyte alpha interferon; leuprolide+estrogen+progesterone; leuprorelin; levamisole; liarozole; linear polyamine analogue; lipophilic disaccharide peptide; lipophilic platinum compounds; lissoclinamide 7; lobaplatin; lombricine; lometrexol; lonidamine; losoxantrone; HMG-CoA reductase inhibitor (such as but not limited to, Lovastatin, Pravastatin, Fluvastatin, Statin, Simvastatin, and Atorvastatin); loxoribine; lurtotecan; lutetium texaphyrin; lysofylline; lytic peptides; maitansine; mannostatin A; marimastat; masoprocol; maspin; matrilysin inhibitors; matrix metalloproteinase inhibitors; menogaril; merbarone; meterelin; methioninase; metoclopramide; MIF inhibitor; mifepristone; miltefosine; mirimostim; mismatched double stranded RNA; mitoguazone; mitolactol; mitomycin analogues; mitonafide; mitotoxin fibroblast growth factor-saporin; mitoxantrone; mofarotene; molgramostim; monoclonal antibody, human chorionic gonadotrophin; monophosphoryl lipid A+myobacterium cell wall sk; mopidamol; multiple drug resistance gene inhibitor; multiple tumor suppressor 1-based therapy; mustard anticancer agent; mycaperoxide B; mycobacterial cell wall extract; myriaporone; N-acetyldinaline; N-substituted benzamides; nafarelin; nagrestip; naloxone+pentazocine; napavin; naphterpin; nartograstim; nedaplatin; nemorubicin; neridronic acid; neutral endopeptidase; nilutamide; nisamycin; nitric oxide modulators; nitroxide antioxidant; nitrullyn; O6-benzylguanine; octreotide; okicenone; oligonucleotides; onapristone; ondansetron; ondansetron; oracin; oral cytokine inducer; ormaplatin; osaterone; oxaliplatin; oxaunomycin; paclitaxel; paclitaxel analogues; paclitaxel derivatives; palauamine; palmitoylrhizoxin; pamidronic acid; panaxytriol; panomifene; parabactin; pazelliptine; pegaspargase; peldesine; pentosan polysulfate sodium; pentostatin; pentrozole; perflubron; perfosfamide; perillyl alcohol; phenazinomycin; phenylacetate; phosphatase inhibitors; picibanil; pilocarpine hydrochloride; pirarubicin; piritrexim; placetin A; placetin B; plasminogen activator inhibitor; platinum complex; platinum compounds; platinum-triamine complex; porfimer sodium; porfiromycin; prednisone; propyl bis-acridone; prostaglandin J2; proteasome inhibitors; protein A-based immune modulator; protein kinase C inhibitor; protein kinase C inhibitors, microalgal; protein tyrosine phosphatase inhibitors; purine nucleoside phosphorylase inhibitors; purpurins; pyrazoloacridine; pyridoxylated hemoglobin polyoxyethylene conjugate; raf antagonists; raltitrexed; ramosetron; ras farnesyl protein transferase inhibitors; ras inhibitors; ras-GAP inhibitor; retelliptine demethylated; rhenium Re 186 etidronate; rhizoxin; ribozymes; RII retinamide; rogletimide; rohitukine; romurtide; roquinimex; rubiginone B1; ruboxyl; safingol; saintopin; SarCNU; sarcophytol A; sargramostim; Sdi 1 mimetics; semustine; senescence derived inhibitor 1; sense oligonucleotides; signal transduction inhibitors; signal transduction modulators; single chain antigen binding protein; sizofiran; sobuzoxane; sodium borocaptate; sodium phenylacetate; solverol; somatomedin binding protein; sonermin; sparfosic acid; spicamycin D; spiromustine; splenopentin; spongistatin 1; squalamine; stem cell inhibitor; stem-cell division inhibitors; stipiamide; stromelysin inhibitors; sulfinosine; superactive vasoactive intestinal peptide antagonist; suradista; suramin; swainsonine; synthetic glycosaminoglycans; tallimustine; tamoxifen methiodide; tauromustine; tazarotene; tecogalan sodium; tegafur; tellurapyrylium; telomerase inhibitors; temoporfin; temozolomide; teniposide; tetrachlorodecaoxide; tetrazomine; thaliblastine; thiocoraline; thrombopoietin; thrombopoietin mimetic; thymalfasin; thymopoietin receptor agonist; thymotrinan; thyroid stimulating hormone; tin ethyl etiopurpurin; tirapazamine; titanocene bichloride; topsentin; toremifene; totipotent stem cell factor; translation inhibitors; tretinoin; triacetyluridine; triciribine; trimetrexate; triptorelin; tropisetron; turosteride; tyrosine kinase inhibitors; tyrphostins; UBC inhibitors; ubenimex; urogenital sinus-derived growth inhibitory factor; urokinase receptor antagonists; vapreotide; variolin B; vector system, erythrocyte gene therapy; velaresol; veramine; verdins; verteporfin; vinorelbine; vinxaltine; Vitaxin^{®}; vorozole; zanoterone; zeniplatin; zilascorb; and zinostatin stimalamer. Additional anti-cancer drugs are 5-fluorouracil and leucovorin. These two agents are particularly useful when used in methods employing thalidomide and a topoisomerase inhibitor. In some cases, the EpCAM binding protein of the present disclosure is used in combination with gemcitabine. In some cases, the EpCAM binding protein as described herein is administered before, during, or after surgery.

### Methods of detection of EpCAM expression and diagnosis of EpCAM associated cancer

According to another case of the disclosure, kits for detecting expression of EpCAM *in vitro* or *in vivo* are provided. The kits include the foregoing EpCAM binding protein (*e.g.,* an EpCAM binding protein containing a labeled anti-EpCAM single domain antibody or antigen binding fragments thereof), and one or more compounds for detecting the label. In some cases, the label is selected from the group consisting of a fluorescent label, an enzyme label, a radioactive label, a nuclear magnetic resonance active label, a luminescent label, and a chromophore label.

In some cases, EpCAM expression is detected in a biological sample. The sample can be any sample, including, but not limited to, tissue from biopsies, autopsies and pathology specimens. Biological samples also include sections of tissues, for example, frozen sections taken for histological purposes. Biological samples further include body fluids, such as blood, serum, plasma, sputum, spinal fluid or urine. A biological sample is typically obtained from a mammal, such as a human or non-human primate.

In one case, provided is a method of determining if a subject has cancer by contacting a sample from the subject with an anti-EpCAM single domain antibody as disclosed herein; and detecting binding of the single domain antibody to the sample. An increase in binding of the antibody to the sample as compared to binding of the antibody to a control sample identifies the subject as having cancer.

In another case, provided is a method of confirming a diagnosis of cancer in a subject by contacting a sample from a subject diagnosed with cancer with an anti-EpCAM single domain antibody as disclosed herein; and detecting binding of the antibody to the sample. An increase in binding of the antibody to the sample as compared to binding of the antibody to a control sample confirms the diagnosis of cancer in the subject.

In some examples of the disclosed methods, the EpCAM single domain antibody is directly labeled. In some examples, the methods further include contacting a second antibody that specifically binds the anti-EpCAM single domain antibody with the sample; and detecting the binding of the second antibody. An increase in binding of the second antibody to the sample as compared to binding of the second antibody to a control sample detects cancer in the subject or confirms the diagnosis of cancer in the subject. In some cases, the cancer is a neuroendocrine cancer, prostate cancer, lung cancer, stomach cancer, squamous cell carcinoma, pancreatic cancer, cholangiocarcinoma, triple negative breast cancer or ovarian cancer (such as epithelial ovarian carcinoma), or any other type of cancer that expresses EpCAM. In some examples, the control sample is a sample from a subject without cancer. In particular examples, the sample is a blood or tissue sample.

In some cases, the antibody that binds (for example specifically binds) EpCAM is directly labeled with a detectable label. In another case, the antibody that binds (for example, specifically binds) EpCAM (the first antibody) is unlabeled and a second antibody or other molecule that can bind the antibody that specifically binds EpCAM is labeled. A second antibody is chosen such that it is able to specifically bind the specific species and class of the first antibody. For example, if the first antibody is a llama IgG, then the secondary antibody may be an anti-llama-IgG. Other molecules that can bind to antibodies include, without limitation, Protein A and Protein G, both of which are available commercially. Suitable labels for the antibody or secondary antibody are described above, and include various enzymes, prosthetic groups, fluorescent materials, luminescent materials, magnetic agents and radioactive materials. Non-limiting examples of suitable enzymes include horseradish peroxidase, alkaline phosphatase, beta-galactosidase, or acetylcholinesterase. Non-limiting examples of suitable prosthetic group complexes include streptavidin/biotin and avidin/biotin. Non-limiting examples of suitable fluorescent materials include umbelliferon, fluorescein, fluorescein isothiocyanate, rhodamine, dichlorotriazinylamine fluorescein, dansyl chloride or phycoerythrin. A non-limiting exemplary luminescent material is luminol; a non-limiting exemplary a magnetic agent is gadolinium, and non-limiting exemplary radioactive labels include 125I, 131I, 35S or 3H.

In an alternative case, EpCAM can be assayed in a biological sample by a competition immunoassay utilizing EpCAM standards labeled with a detectable substance and an unlabeled antibody that specifically binds EpCAM. In this assay, the biological sample, the labeled EpCAM standards and the antibody that specifically bind EpCAM are combined and the amount of labeled EpCAM standard bound to the unlabeled antibody is determined. The amount of EpCAM in the biological sample is inversely proportional to the amount of labeled EpCAM standard bound to the antibody that specifically binds EpCAM.

The immunoassays and method disclosed herein can be used for a number of purposes. In one case, the antibody that specifically binds EpCAM may be used to detect the production of EpCAM in cells in cell culture. In another case, the antibody can be used to detect the amount of EpCAM in a biological sample, such as a tissue sample, or a blood or serum sample. In some examples, the EpCAM is cell-surface EpCAM. In other examples, the EpCAM is soluble EpCAM (*e.g*., EpCAM in a cell culture supernatant or soluble EpCAM in a body fluid sample, such as a blood or serum sample).

In one case, a kit is provided for detecting EpCAM in a biological sample, such as a blood sample or tissue sample. For example, to confirm a cancer diagnosis in a subject, a biopsy can be performed to obtain a tissue sample for histological examination. Alternatively, a blood sample can be obtained to detect the presence of soluble EpCAM protein or fragment. Kits for detecting a polypeptide will typically comprise a single domain antibody, according to the present disclosure, that specifically binds EpCAM. In some cases, an antibody fragment, such as an scFv fragment, a VH domain, or a Fab is included in the kit. In a further case, the antibody is labeled (for example, with a fluorescent, radioactive, or an enzymatic label).

In one case, a kit includes instructional materials disclosing means of use of an antibody that binds EpCAM. The instructional materials may be written, in an electronic form (such as a computer diskette or compact disk), may be visual (such as video files), or provided through an electronic network, for example, over the internet, World Wide Web, an intranet, or other network. The kits may also include additional components to facilitate the particular application for which the kit is designed. Thus, for example, the kit may additionally contain means of detecting a label (such as enzyme substrates for enzymatic labels, filter sets to detect fluorescent labels, appropriate secondary labels such as a secondary antibody, or the like). The kits may additionally include buffers and other reagents routinely used for the practice of a particular method. Such kits and appropriate contents are well known to those of skill in the art.

In one case, the diagnostic kit comprises an immunoassay. Although the details of the immunoassays may vary with the particular format employed, the method of detecting EpCAM in a biological sample generally includes the steps of contacting the biological sample with an antibody which specifically reacts, under immunologically reactive conditions, to an EpCAM polypeptide. The antibody is allowed to specifically bind under immunologically reactive conditions to form an immune complex, and the presence of the immune complex (bound antibody) is detected directly or indirectly.

Methods of determining the presence or absence of a cell surface marker are well known in the art. For example, the antibodies can be conjugated to other compounds including, but not limited to, enzymes, magnetic beads, colloidal magnetic beads, haptens, fluorochromes, metal compounds, radioactive compounds or drugs. The antibodies can also be utilized in immunoassays such as but not limited to radioimmunoassays (RIAs), ELISA, or immunohistochemical assays. The antibodies can also be used for fluorescence activated cell sorting (FACS). FACS employs a plurality of color channels, low angle and obtuse light-scattering detection channels, and impedance channels, among other more sophisticated levels of detection, to separate or sort cells. *See* U.S. Patent No. 5, 061,620). Any of the single domain antibodies that bind EPCAM, as disclosed herein, can be used in these assays. Thus, the antibodies can be used in a conventional immunoassay, including, without limitation, an ELISA, an RIA, FACS, tissue immunohistochemistry, Western blot or immunoprecipitation.

### EXAMPLES

The invention provides multispecific proteins as defined by the claims. Other sequences disclosed in the Examples are provided for better understanding of the claimed invention.

### Example 1: Screening of Phage Display Library for Identification of EpCAM Binding Domains

Llamas were immunized with purified EpCAM protein expressed in Expi293 cells. A phage display library for expression of heavy variable antibody domains was constructed from circulating B cells. *See* van der Linden , de Geus , Stok, Bos ,van Wassenaar, Verrips, and Frenken. 2000. J Immunol Methods 240:185-195. Phage clones were screened for binding to EpCAM by expressing anti-EpCAM proteins in *E coli,* preparing periplasmic extracts, and proteins were screened for human and cynomolgus EpCAM binding activity using a colorimetric ELISA. Thirty-eight unique heavy chain only sequences were identified (SEQ ID Nos. 1 to 38) that produced a signal in the ELISA screening relative to the control with human and/or cynomolgus EpCAM proteins (as shown in **Table 2).** The CDR1, CDR2, and CDR3 sequences for these heavy variable domains are, respectively, SEQ ID Nos. 39 to 76, SEQ ID Nos. 77 to 114, and SEQ ID Nos. 115 to 152.

**Table 2: Binding of Llama anti-Human EpCAM heavy chain only single domain antibodies to Human and Cynomolgus EpCAM, as demonstrated by signal in an ELISA Assay (absorbance readings in a colorimetric ELISA assay), relative to control heavy chain only single domain antibodies**

| **Sequence name** | **ELISA Human EpCAM** | **ELISA Cynomolgus EpCAM** | **ELISA Control** | **Human EpCAM / Control** | **Cynomolgus EpCAM / Control** |
|---|---|---|---|---|---|
| EPL90 | 1.6 | 1.7 | 0.2 | 10 | 10 |
| EPL 118 | 3.4 | 2.9 | 0.7 | 5 | 4 |
| EPL138 | 3.1 | 2.7 | 0.9 | 3 | 3 |
| EPL145 | 0.6 | 0.4 | 0.2 | 3 | 2 |
| EPL164 | 0.6 | 2.8 | 0.1 | 7 | 34 |
| EPL31 | 0.6 | 0.5 | 0.1 | 7 | 6 |
| EPL55 | 0.4 | 0.6 | 0.1 | 5 | 7 |
| EPL57 | 1.7 | 3.4 | 0.1 | 14 | 27 |
| EPL136 | 0.9 | 1.3 | 0.1 | 9 | 13 |
| EPL15 | 0.7 | 3.2 | 0.1 | 8 | 35 |
| EPL34 | 0.9 | 3.1 | 0.1 | 10 | 36 |
| EPL86 | 0.8 | 3.0 | 0.1 | 8 | 31 |
| EPL153 | 3.1 | 2.2 | 0.1 | 31 | 21 |
| EPL20 | 2.9 | 2.2 | 0.5 | 6 | 4 |
| EPL70 | 3.2 | 2.2 | 0.1 | 24 | 16 |
| EPL125 | 3.5 | 3.5 | 0.5 | 8 | 8 |
| EPL13 | 2.9 | 4.0 | 0.2 | 17 | 23 |
| EPL129 | 2.0 | 3.1 | 0.1 | 20 | 29 |
| EPL159 | 0.4 | 0.2 | 0.1 | 3 | 2 |
| EPL120 | 3.2 | 2.3 | 0.6 | 5 | 4 |
| EPL126 | 3.4 | 2.8 | 0.7 | 5 | 4 |
| EPL60 | 1.1 | 3.5 | 0.1 | 10 | 33 |
| EPL156 | 3.6 | 4.0 | 0.2 | 16 | 17 |
| EPL2 | 2.2 | 3.6 | 0.1 | 15 | 24 |
| EPL43 | 1.8 | 3.8 | 0.1 | 13 | 27 |
| EPL10 | 2.7 | 2.0 | 0.2 | 12 | 9 |
| EPL49 | 1.1 | 0.6 | 0.1 | 8 | 4 |
| EPL58 | 1.1 | 0.5 | 0.1 | 8 | 4 |
| EPL74 | 1.4 | 0.7 | 0.1 | 10 | 5 |
| EPL78 | 3.1 | 2.0 | 0.2 | 17 | 11 |
| EPL82 | 0.9 | 1.1 | 0.2 | 4 | 5 |
| EPL83 | 2.0 | 1.1 | 0.6 | 3 | 2 |
| EPL97 | 2.5 | 1.7 | 0.3 | 8 | 5 |
| EPL109 | 0.4 | 0.1 | 0.1 | 5 | 2 |
| EPL117 | 0.9 | 0.6 | 0.2 | 4 | 3 |
| EPL127 | 0.4 | 0.9 | 0.1 | 5 | 11 |
| EPL152 | 3.4 | 2.8 | 0.8 | 4 | 4 |
| EPL189 | 1.3 | 0.9 | 0.1 | 12 | 8 |

### Example 2: Incorporation of EpCAM Binding Heavy Chain Only Single Domain Antibodies Into Fusion Proteins and T Cell Dependent Cellular Cytotoxicity Assays

Selected anti-EpCAM heavy chain only single domain antibodies from Example 1 were cloned into DNA constructs for expression of recombinant proteins. These expression constructs all encoded a signal peptide. One set of anti-EpCAM constructs (SEQ ID Nos. 153 to 179) was designed to express a fusion protein with a humanized anti-CD3 scFv domain on the N-terminus of the mature secreted fusion protein followed by a llama anti-EpCAM domain, with the two domains linked by the sequence GGGGSGGGS, and with a HHHHHH on the C-terminus. One second of anti-EpCAM constructs (SEQ ID Nos. 180 to 206) was designed to express a fusion protein with a llama anti-EpCAM domain on the N-terminus of the mature secreted fusion protein followed a humanized anti-CD3-scFv domain, with the two domains linked by the sequence GGGGSGGGS, and with a HHHHHH on the C-terminus.

These anti-EpCAM/anti-CD3 (from N-terminus to C-terminus) or anti-CD3/anti-EpCAM (from N terminus to C terminus) fusion protein constructs were transfected into Expi293 cells. The amount of anti-EpCAM/anti-CD3 fusion protein in the conditioned media from the transfected Expi293 cells was quantitated using by using an Octet instrument with streptavidin and loaded with biotinylated CD3-Fc fusion protein using an anti-CD3 fusion protein of similar molecular weight to the anti-EPCAM/ant-CD3 proteins as a standard.

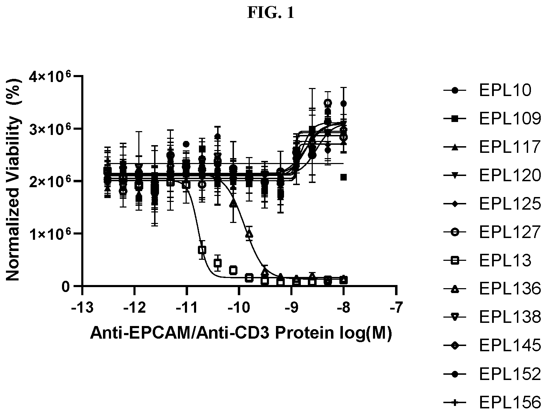

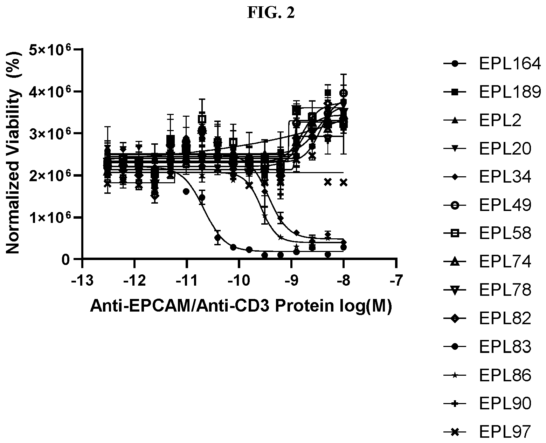

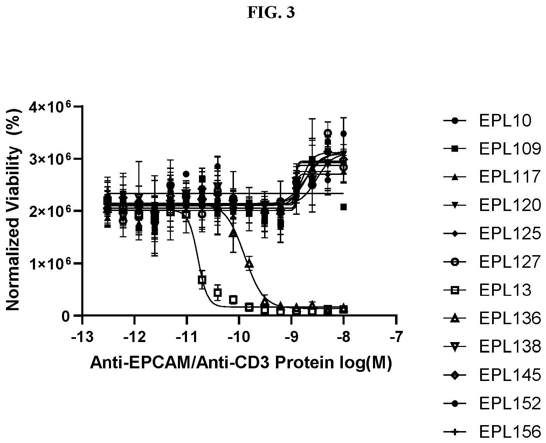

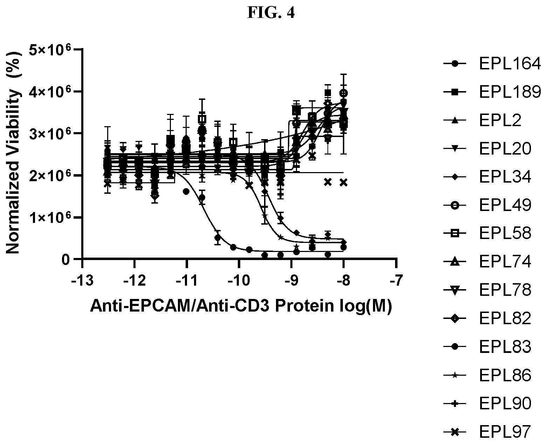

The conditioned media were tested in a T-cell dependent cellular cytotoxicity assay. *See* Nazarian AA, Archibeque IL, Nguyen YH, Wang P, Sinclair AM, Powers DA. 2015. J Biomol Screen. 20:519-27. In this assay, luciferase labelled NCI-H508 cells, which express EpCAM, were combined with purified human T cells and a titration of the anti-EpCAM/anti-CD3 fusion protein or the anti-CD3/anti-EpCAM. It was hypothesized that if the fusion protein directs T cells to kill the NCI-H508 cells, the signal in a luciferase assay performed at 48 hours after starting the experiment should decrease. **FIGS. 1-4** provide the TDCC data in graphical format .

EC₅₀ values from the TDCC assays are listed in **Table 3** (lists EC₅₀ data for SEQ ID Nos. 153-179) and **Table 4** (lists EC₅₀ data for SEQ ID Nos. 180-206). The most potent molecule (EPL13) had an EC₅₀ value of about 1.6 pM. Some of the anti-EpCAM binding proteins were only active when present in an anti-CD3/anti-EpCAM configuration. One anti-EPCAM sequence, EPL34, was only active in the anti-EpCAM/anti-CD3 configuration. A negative control for the TDCC assays was anti-GFP / anti-CD3 protein, and this protein did not direct the T cells to kill the NCI-H508 cells (data not shown).

**Table 3: EC₅₀ Values for Redirected T Cell Killing of NCI-H508 Cells by Anti-CD3/Anti-EPCAM Proteins Containing Llama Anti-EpCAM Sequences (n/a= insufficient activity to calculate an EC₅₀ using the protein concentrations tested)**

| **Anti-EpCAM Sequence** | **NCI-H508 Cell Killing EC₅₀ (M)** |
|---|---|
| EPL10 | n/a |
| EPL109 | 2.2E-09 |
| EPL117 | n/a |
| EPL120 | 5.8e-010 |
| EPL125 | 1.3E-09 |
| EPL127 | n/a |
| EPL13 | 1.6E-12 |
| EPL136 | 6.1E-12 |
| EPL138 | 7.9E-12 |
| EPL145 | n/a |
| EPL152 | n/a |
| EPL153 | 1.4E-10 |
| EPL156 | n/a |
| EPL164 | 3.6E-10 |
| EPL189 | n/a |
| EPL2 | n/a |
| EPL20 | n/a |
| EPL34 | n/a |
| EPL49 | n/a |
| EPL58 | n/a |
| EPL74 | 2.6E-09 |
| EPL78 | n/a |
| EPL82 | n/a |
| EPL83 | 3.1E-10 |
| EPL86 | 4.7E-10 |
| EPL90 | 9.2E-12 |
| EPL97 | n/a |

**Table 4: EC₅₀ Values for Redirected T Cell Killing of NCI-H508 Cells by Anti-EpCAM/Anti-CD3 Proteins Containing Llama Anti-EpCAM Sequences(n/a= insufficient activity to calculate an EC₅₀ using the protein concentrations tested)**

| **Anti-EpCAM Sequence** | **NCI-H508 Cell Killing EC50 (M)** |
|---|---|
| EPL10 | n/a |
| EPL109 | n/a |
| EPL117 | n/a |
| EPL120 | n/a |
| EPL125 | n/a |
| EPL127 | n/a |
| EPL13 | 1.6E-11 |
| EPL136 | 1.3E-10 |
| EPL138 | n/a |
| EPL145 | n/a |
| EPL152 | n/a |
| EPL153 | not expressed |
| EPL156 | n/a |
| EPL164 | n/a |
| EPL189 | n/a |
| EPL2 | n/a |
| EPL20 | n/a |
| EPL34 | 3.7E-10 |
| EPL49 | n/a |
| EPL58 | n/a |
| EPL74 | n/a |
| EPL78 | n/a |
| EPL82 | n/a |
| EPL83 | 2.1E-11 |
| EPL86 | 2.7E-10 |
| EPL90 | n/a |
| EPL97 | n/a |

Using conditioned media with known concentrations of anti-EpCAM/anti-CD3 or anti-CD3/anti-EpCAM fusion proteins, the binding affinities of the fusion proteins for human and cynomolgus monkey EpCAM proteins were measured. An Octet instrument with streptavidin tips were loaded with biotinylated human or cynomolgus EpCAM protein, and K_{D} values were calculated by measuring the on rate and off rate of binding of the anti-EPCAM/anti-CD3 fusion or anti-CD3/anti-EpCAM fusion proteins to the biotinylated EpCAM proteins. The K_{D} measurements were made using a single 50 nM concentration of the anti-EPCAM/anti-CD3 or anti-CD3/anti-EpCAM fusion proteins, which allowed for rank ordering potency. The measured relative affinities are listed in **Table 5** All of the fusion proteins bound to cynomolgus EpCAM, with K_{D} values ranging from 1.6 to 56 nM. Most, but not all of the fusion proteins were measured binding to human EpCAM with K_{D} values ranging from 0.8 to 74 nM.

**Table 5: Binding Affinities to Human and Cyno EpCAM of Anti-EPCAM/Anti-CD3 or Anti-CD3/Anti-EpCAM Fusion Proteins Containing Llama Anti-EpCAM Sequences**

| | **Anti-CD3 / Anti-EpCAM** | | **Anti-EpCAM / Anti-CD3** | |
|---|---|---|---|---|
| **Humanized anti-EpCAM Binder** | **hu KD (nM)** | **cy KD (nM)** | **hu KD (nM)** | **cy KD (nM)** |
| EPL13 | 29 | 13 | 14 | 7.6 |
| EPL136 | 74 | 56 | 32 | 31 |
| EPL138 | 2.2 | 2.2 | 0.8 | 1.3 |
| EPL153 | n/q | 3.5 | No expression | |
| EPL164 | 3.1 | 3.6 | 2.1 | 2.9 |
| EPL34 | n/q | 14 | n/q | 6.4 |
| EPL83 | 1.1 | 3 | 1.6 | 3 |
| EPL86 | n/q | 8.3 | n/q | 7 |
| EPL90 | 1.9 | 1.6 | 0.8 | 1.2 |

### Example 3: Humanization of EpCAM Binding Heavy Chain Only Single Domain Antibodies and T Cell Dependent Cellular Cytotoxicity Assays

Three of the llama anti-EpCAM antibodies sequences identified in Example 1 were humanized by grafting their CDR sequences onto human germline sequences, while retaining some llama framework sequences to ensure the antibodies did not lose activity (SEQ ID Nos. 207 to 209).

These sequences were cloned into expression constructs for expression of anti-EpCAM/anti-CD3 fusion proteins (SEQ ID Nos. 210 to 212) in Expi293 cells, as described in **Example 2.**

The amount of anti-EpCAM/anti-CD3 fusion proteins present in the conditioned medium was quantitated as described in **Example 2.** The affinities of these humanized proteins for human, cynomolgus, and mouse EpCAM were measured as described in **Example 2.** The relative K_{D} values calculated from these measurements are listed in Table 6. All three sequences bound to human and cynomolgus EpCAM, with relative K_{D} values ranging from about 0.3 to about 18 nM. Two of the sequences also bound to mouse EpCAM, with K_{D} values ranging from about 1.4 to about 1.8 nM.

**Table 6: Binding Affinities to Human, Cynomolgus, and Mouse EpCAM of Anti-EpCAM/Anti-CD3 Fusion Proteins Containing Llama Anti-EpCAM Sequences (n/q= not quantifiable under the experimental conditions used)**

| **Humanized Anti-EpCAM Sequence** | **Human EpCAM (nM)** | **Cynomolgus EpCAM (nM)** | **Mouse EpCAM (nM)** |
|---|---|---|---|
| H13 | 17 | 18 | n/q |
| H90 | 0.3 | 1.3 | 1.4 |
| H138 | 0.3 | 1.8 | 1.8 |

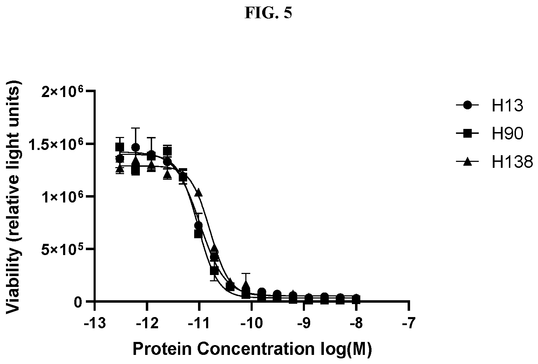

T cell killing potential of the anti-EpCAM/anti-CD3 fusion proteins present in the conditioned medium was assessed as described in Example 2. Results are provided in Table 7 and in **FIG. 5****.**

**Table 7: EC₅₀ Values for Redirected T Cell Killing of NCI-H508 Cells by Purified Anti-CD3/Anti-EpCAM Proteins Containing Humanized Anti-EpCAM Sequences**

| **Humanized anti-EpCAM Binder Sequence** | **EC₅₀ (pM)** |
|---|---|
| H13 | 10 |
| H90 | 10 |
| H138 | 16 |

### Example 4: Xenograft tumor model

An EpCAM targeting fusion protein of this disclosure (*e*.*g*., a fusion protein which is a trispecific protein comprising an anti-EpCAM heavy chain only single domain antibody, an anti-CD3 scFv, and an anti-Albumin domain) is evaluated in a xenograft model. In order to determine efficacy of the exemplary EpCAM targeting fusion protein *in vivo,* multiple xenograft tumor models are used. Examples of common tumor cell lines for use in xenograft tumor studies include A549 (non-small cell lung carcinoma) cells, DU-145 (prostate) cells, MCF-7 (breast) cells, Colo 205 (colon) cells, 3T3/]GF-IR (mouse fibroblast) cells, NCI H441 cells, HEP G2 (hepatoma) cells, MDA MB 231 (breast) cells, HT-29 (colon) cells, MDA-MB-435s (breast) cells, U266 cells, SH-SYSY cells, Sk-Mel-2 cells, NCI-H929, RPM18226, and A431 cells. Immune-deficient NOD/SCID mice are sub-lethally irradiated (2 Gy) and subcutaneously inoculated with 1X 10⁶ tumor cells (*e.g*., NCI H441 cells) into their right dorsal flank. When tumors reach 100 to 200 mm³, animals are allocated into 3 treatment groups. Groups 2 and 3 are intraperitoneally injected with 1.5x10⁷ activated human T-cells. Three days later, animals from Group 3 are subsequently treated with the exemplary EpCAM targeting trispecific antigen-binding protein of. Groups 1 and 2 are only treated with vehicle. Body weight and tumor volume are determined for 30 days, beginning at least 5 days post treatment with the exemplary EpCAM targeting trispecific protein.

It is expected that animals treated with the exemplary EpCAM targeting trispecific protein have a statistically significant delay in tumor growth in comparison to the respective vehicle-treated control group.

### Example 5: Proof-of-Concept clinical trial protocol for administration of the EpCAM targeting trispecific antigen-binding protein of Example 4 to ovarian cancer patients

This is a Phase I/II clinical trial for studying an exemplary EpCAM targeting trispecific antigen-binding protein of this disclosure as a treatment for an epithelial ovarian cancer.

### Study Outcomes:

*Primary:* Maximum tolerated dose of the exemplary EpCAM targeting trispecific protein.

*Secondary:* To determine whether *in vitro* response of the exemplary EpCAM targeting trispecific protein is associated with clinical response

### Phase I

The maximum tolerated dose (MTD) will be determined in the phase I section of the trial.
1.1 The maximum tolerated dose (MTD) will be determined in the phase I section of the trial.
1.2 Patients who fulfill eligibility criteria will be entered into the trial to EpCAM targeting trispecific proteins of the previous examples.
1.3 The goal is to identify the highest dose of EpCAM targeting trispecific proteins of the previous examples that can be administered safely without severe or unmanageable side effects in participants. The dose given will depend on the number of participants who have been enrolled in the study prior and how well the dose was tolerated. Not all participants will receive the same dose.

### Phase II

2.1 A subsequent phase **II** section will be treated at the MTD with a goal of determining if therapy with therapy of the exemplary EpCAM targeting trispecific protein results in at least a 20% response rate.
Primary Outcome for the Phase II ---To determine if therapy of EpCAM targeting trispecific protein results in at least 20% of patients achieving a clinical response (blast response, minor response, partial response, or complete response)

### Eligibility:

- Histologically or cytologically confirmed epithelial ovarian cancer. May have Recurrent epithelial ovarian carcinoma or disease progression following failure of first-line, platinum-based chemotherapy with no more than one prior platinum based regimen therapy
- Adequate laboratory values of bone marrow function, renal function, liver function, and echocardiogram tests

### Phase III

3.1A subsequent phase III section will carried out with the exemplary EpCAM targeting trispecific protein, wherein secondary endpoints such as response rate (RR), patient recorded outcomes (PRO), progression-free survival (PFS), duration of progression free survival, time to progression (TIP), overall survival, health-related quality of life assessment, number of participants with overall survival, duration of response, time to response, number of participants with response, and time to tumor growth etc. will be assessed.

### Example 6: Construction and testing of exemplary multivalent target binding proteins

### Constructs

FIG. 6: The following ProCAR constructs were made, using EpCAM binder sequences provided herein. An exemplary construct including an anti-human EpCAM sdAb, a FLAG epitope, a CD8 hinge/transmembrane domain, a 4-1BB intracellular domain, and a CD3 zeta intracellular domain (SEQ ID NO: 485). An exemplary construct including an anti-human serum albumin sdAb, an anti-human EpCAM sdAb, a FLAG epitope, a CD8 hinge/transmembrane domain, a 4-1BB intracellular domain, and a CD3 zeta intracellular domain (SEQ ID NO: 486). An exemplary construct including an anti-human serum albumin sdAb, an anti-human EpCAM sdAb, a FLAG epitope, a CD8 hinge/transmembrane domain, a 4-1BB intracellular domain, and a CD3 zeta intracellular domain (SEQ ID NO: 487). An exemplary construct including an anti-human serum albumin sdAb, an anti-human EpCAM sdAb, a FLAG epitope, a CD8 hinge/transmembrane domain, a 4-1BB intracellular domain, and a CD3 zeta intracellular domain (SEQ ID NO: 488). An exemplary construct including an anti-human serum albumin sdAb, a protease cleavage site 3, an anti-human EpCAM sdAb, a FLAG epitope, a CD8 hinge/transmembrane domain, a 4-1BB intracellular domain, and a CD3 zeta intracellular domain (SEQ ID NO: 489). An exemplary construct including an anti-human serum albumin sdAb, a protease cleavage site 3, an anti-human EpCAM sdAb, a FLAG epitope, a CD8 hinge/transmembrane domain, a 4-1BB intracellular domain, and a CD3 zeta intracellular domain (SEQ ID NO: 490). An exemplary construct including an anti-GFP sdAb, a FLAG epitope, a CD8 hinge/transmembrane domain, a 4-1BB intracellular domain, and a CD3 zeta intracellular domain (SEQ ID NO: 491).

### EpCAM Mask 1 Blocks ProCAR EpCAM-Binding Activity

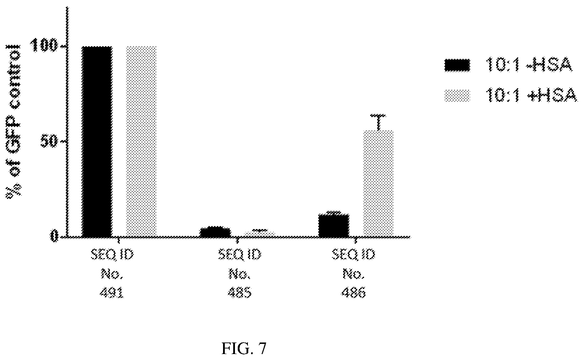

300,000 primary human T cells isolated from healthy donors were infected with 1 mL lentiviral supernatant made from the indicated constructs from **FIG. 6** to generate anti-EpCAM CAR-T cells, which were subsequently stained with anti-FLAG antibodies and EpCAM-Fc along with the indicated secondary antibodies. The data was analyzed by flow cytometry. **FIG. 7** provides histograms of EpCAM-Fc/Alexa Fluor 647 staining of CAR-T cells that have been grouped into low **(FIG. 7A),** medium **(FIG. 7B),** or high **(FIG. 7C)** CAR expression based on anti-FLAG staining.

This data demonstrates the efficacy of EpCAM mask 1 blocking ProCAR EpCAM-binding activity.

### EpCAM Mask 2 Blocks ProCAR EpCAM-Binding Activity

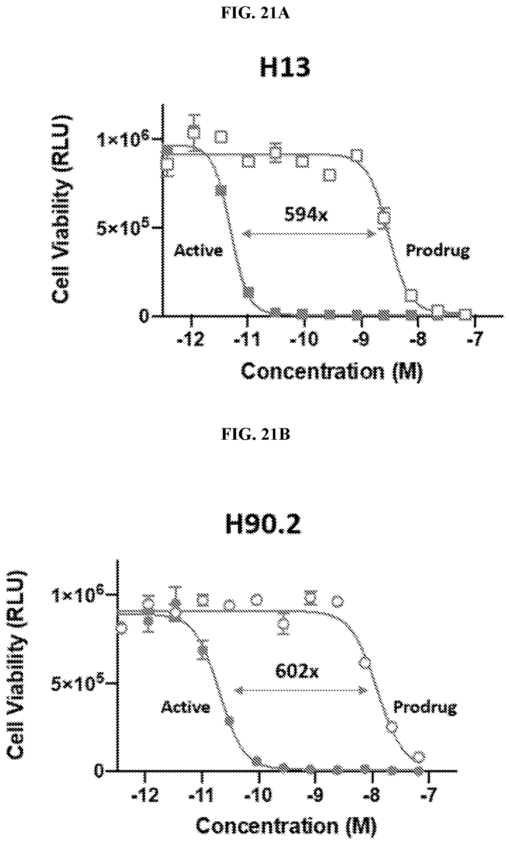

300,000 primary human T cells isolated from healthy donors were infected with 1 mL lentiviral supernatant made from the indicated constructs from **FIG. 21** to generate anti-EpCAM CAR-T cells, which were subsequently stained with anti-FLAG antibodies and EpCAM-Fc along with the indicated secondary antibodies. The data was analyzed by flow cytometry.

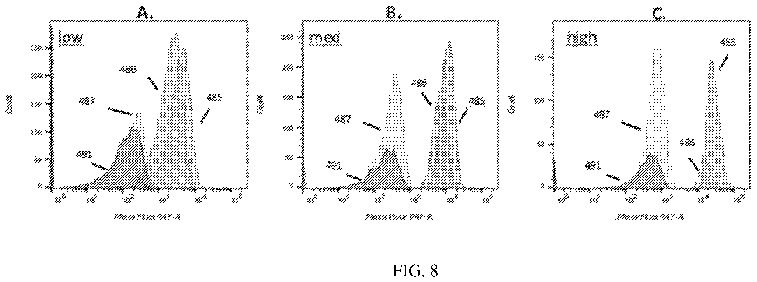

**FIG. 8** provides histograms of EpCAM-Fc/Alexa Fluor 647 staining of CAR-T cells from **FIG. 6** that have been grouped into low **(****FIG. 8A****),** medium **(****FIG. 8B****),** or high **(****FIG. 8C****)** CAR expression based on anti-FLAG staining that demonstrate the efficacy of EpCAM mask 2 in blocking ProCAR EpCAM-binding activity.

### Masking of Anti-EpCAM sdAb H90

300,000 primary human T cells isolated from healthy donors were infected with 1 mL lentiviral supernatant made from the indicated constructs from **FIG. 6** to generate anti-EpCAM CAR-T cells, which were subsequently co-cultured with various ratios (CAR-T:Target cells) with EpCAM-expressing cancer cells that stably express luciferase. Luciferase activity was read 72 hours later as a proxy for cancer cell viability and normalized to the anti-GFP control CAR-T cells, C1081.

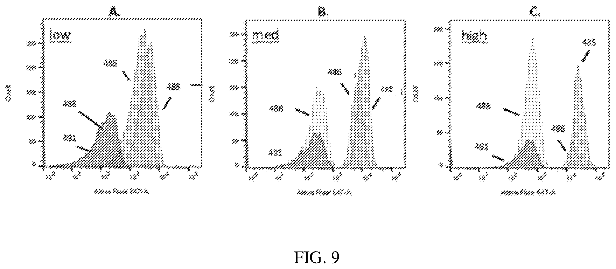

The data provided in **FIG. 9** demonstrates masking of the anti-EpCAM sdAb H90. SEQ ID No. 485 is the "naked" CAR, *i.e.,* no anti-ALB domain. Addition of the anti-ALB domain (SEQ ID No. 486) has a small impact on cell killing activity. Addition of a mask to the CC' loop of the anti-ALB domain has a large impact on cell killing activity due to specific blocking of EpCAM binding.

### Steric blocking by HSA of Anti-EpCAM sdAb H90

300,000 primary human T cells isolated from healthy donors were infected with 1 mL lentiviral supernatant made from the indicated constructs from **FIG. 6** to generate anti-EpCAM CAR-T cells, which were subsequently co-cultured with various ratios (CAR-T:Target cells) with EpCAM-expressing cancer cells that stably express luciferase in the presence or absence of human serum albumin (HSA). Luciferase activity was read 72 hours later as a proxy for cancer cell viability and normalized to the anti-GFP control CAR-T cells, C1081.

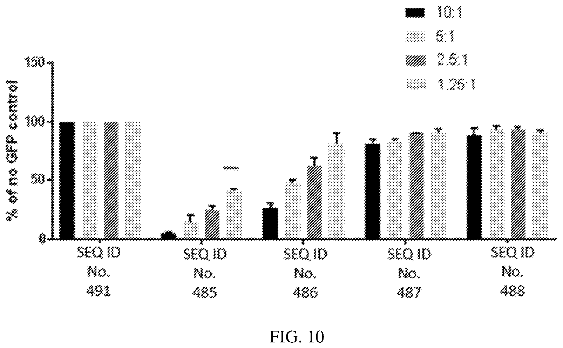

The data provided in **FIG. 10** demonstrate steric blocking by HSA of the anti-EpCAM sdAb H90.

### Example 7: EpCAM ProCAR Protease Site-Dependent Cell Killing

Chimeric antigen receptor-expressing T-cells (CAR T-cells) were generated by infecting isolated CD4/CD8 positive T cells from a healthy donor with lentivirus expressing the indicated constructs. **FIG. 6** shows a diagram with the constructs used. Cells were incubated with Fc-tagged EpCAM extracellular domain (ECD). Cells were washed to remove unbound Fc-tagged EpCAM ECD. Cells were incubated with a secondary antibody conjugated to DyLight 650 that recognizes human Fc. Binding of the secondary antibody to cells was measured by flow cytometry.

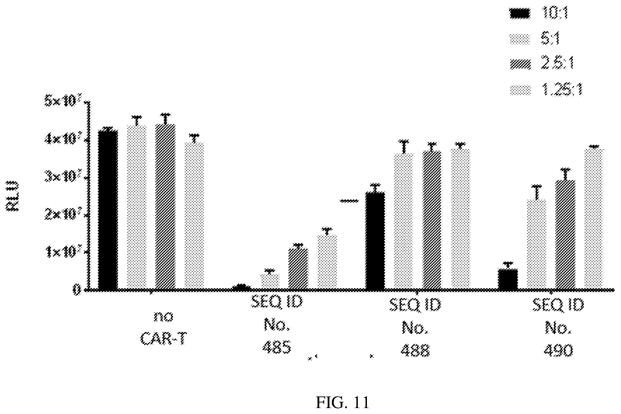

FIG. 11 demonstrates protease site-dependent activation of EpCAM ProCAR mask 2 cell killing activity.

### Example 8: Protease Activation of EpCAM Mask 1 ProCAR Antigen Binding Activity

300,000 primary human T cells isolated from healthy donors were infected with 1 mL lentiviral supernatant made from the indicated constructs from **FIG. 6** to generate anti-EpCAM CAR-T cells, which were subsequently washed in PBS, and then treated for 1 hr. at room temp with either PBS or PBS containing 400 nM recombinant uPA protease and then stained with anti-FLAG antibodies and EpCAM-Fc along with the anti-mouse BV421 and anti-human Fc Alexa Fluor 647 secondary antibodies and analyzed by flow cytometry.

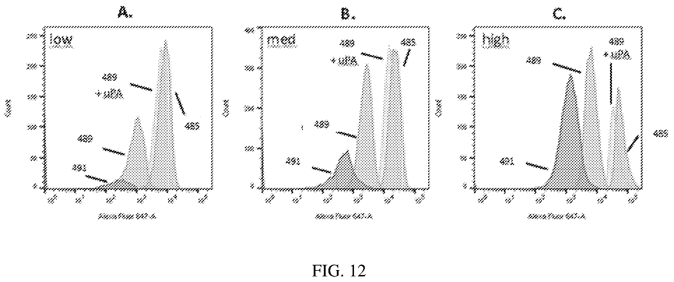

Histograms of EpCAM-Fc staining of CAR-T cells that have been grouped into low **(****FIG. 12A****),** medium **(****FIG. 12B****),** or high **(****FIG. 12C****)** CAR expression based on anti-FLAG staining. These results demonstrate protease activation of EpCAM Mask 1 ProCAR antigen binding activity.

### Example 9: Protease Activation of EpCAM Mask 2 ProCAR Antigen Binding Activity

300,000 primary human T cells isolated from healthy donors were infected with 1 mL lentiviral supernatant made from the indicated constructs from **FIG. 6** to generate anti-EpCAM CAR-T cells, which were subsequently washed in PBS, and then treated for 1 hr. at room temp with either PBS or PBS containing 400 nM recombinant uPA protease and then stained with anti-FLAG antibodies and EpCAM-Fc along with the anti-mouse BV421 and anti-human Fc Alexa Fluor 647 secondary antibodies and analyzed by flow cytometry.

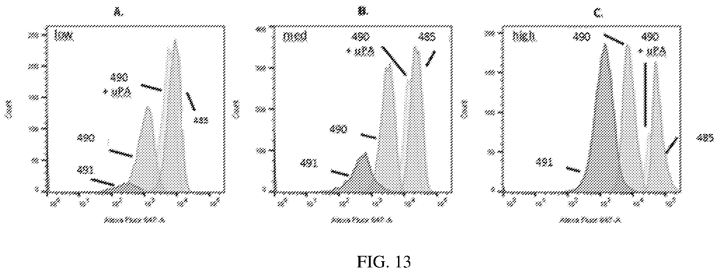

Histograms of EpCAM-Fc staining of CAR-T cells that have been grouped into low **(****FIG. 13A****),** medium **(****FIG. 13B****),** or high **(****FIG. 13C****)** CAR expression based on anti-FLAG staining. These results demonstrate protease activation of EpCAM Mask 2 ProCAR antigen binding activity.

### Example 10: Demonstration of improved tolerability in mouse, conferred by an exemplary EpCAM targeting ProTriTAC molecule

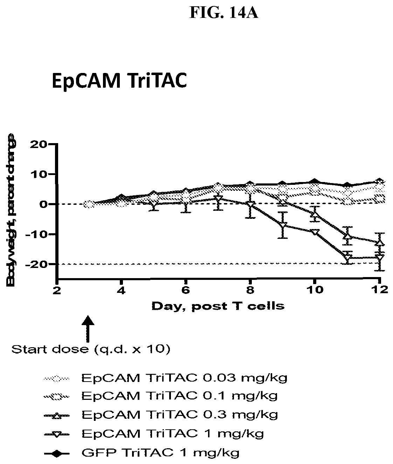

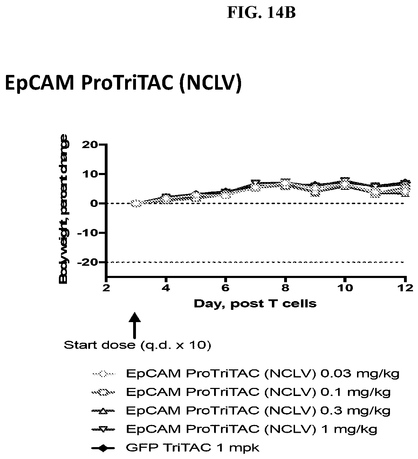

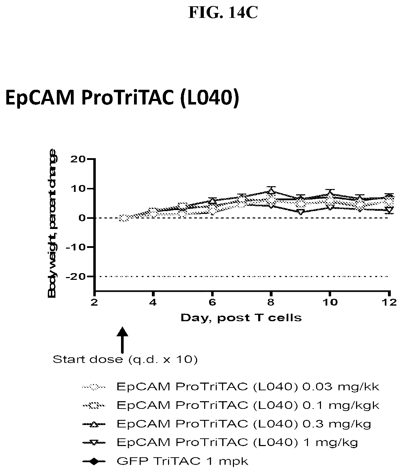

In this study, the tolerability of an exemplary EpCAM targeting ProTriTAC molecule was assessed. Seven weeks old NSG female tumor free mice were intraperitoneally injected with 2 x 10⁷ expanded human T cells at the commencement of the study, *i.e.,* at day 0. On day 2, treatment was started by dividing the mice into various groups and administering to them varying concentrations of the exemplary EpCAM targeting ProTriTAC molecule containing the linker sequence L040 (SEQ ID NO: 494), an EpCAM targeting TriTAC molecule (SEQ ID NO: 492), an EpCAM targeting ProTriTAC molecule containing a non-cleavable linker (EpCAM ProTriTAC (NCLV) (SEQ ID NO: 495), and a GFP TriTAC molecule as a control (SEQ ID NO: 493). The molecules were administered once daily for 10 days, at the following dosages: 0.03 mg/kg, 0.1 mg/kg, 0.3 mg/kg, and 1 mg/kg. Starting from day 2, body weight of the animals were recorded daily. As shown in **FIGS. 14A-14C****,** the EpCAM targeting ProTriTAC molecule containing a non-cleavable linker (ProTriTAC (NCLV)) and a GFP TriTAC (used as a negative control) were very well tolerated in mice even at the highest dose of 1 mg/kg. The EpCAM targeted ProTriTAC molecule containing the linker sequence of L040 was well tolerated at the dosage of 1 mg/kg, whereas the EpCAM targeted TriTAC was well tolerated 0.1 mg/kg. It was thus observed that the EpCAM targeting ProTriTAC containing the L040 linker sequence conferred at least about 10 times improved tolerability, in mouse, compared to the EpCAM targeting TriTAC.

### Example 11: Affinity of EpCAM targeting ProTriTAC proteins toward human, cynomolgus, or mouse EpCAM

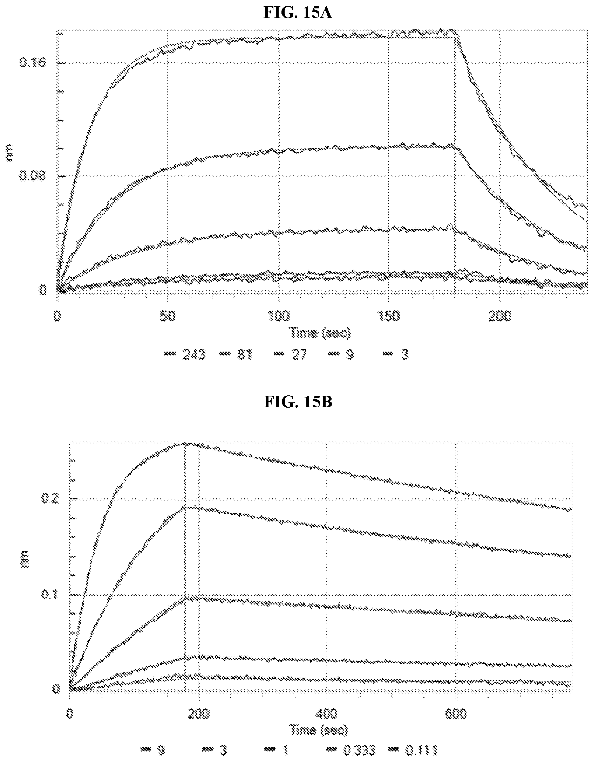

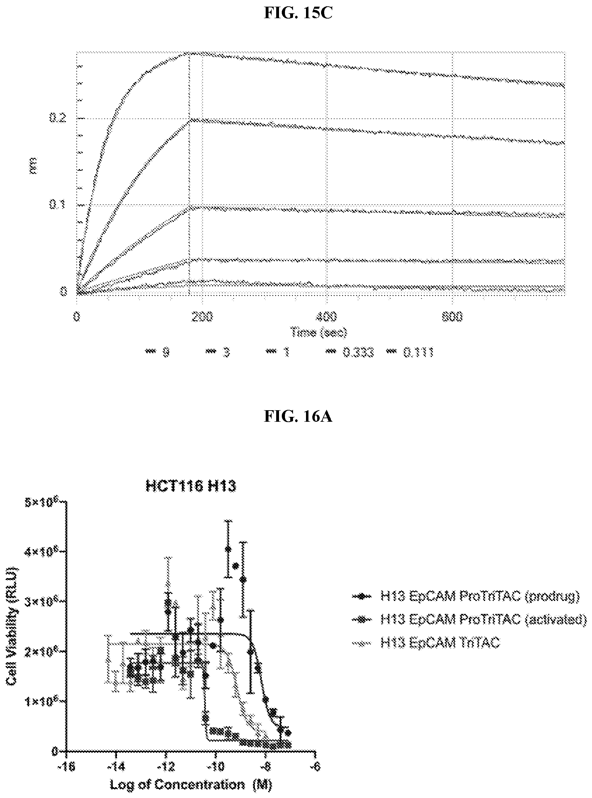

The affinities of various exemplary EpCAM targeting ProTriTAC proteins, toward human, cynomolgus, or mouse EpCAM were measured in a binding assay, which also looked at the binding kinetics for the interaction between the tested ProTriTAC proteins and human EpCAM. The binding kinetics are shown in **FIGS. 15A, 15B****,** and **15C****.**

The affinity summary for the EpCAM targeting ProTriTAC proteins, containing exemplary EpCAM binding domains H13 (SEQ ID No. 207), H90 (SEQ ID No. 209), or H90.2 (SEQ ID No. 497), is provided in **Table 8.**

**Table 8: Affinity summary for EpCAM targeting ProTriTAC proteins**

| | **H13 (SEQ ID No. 576) (nM)** | **(H90) (SEQ ID No. 577) (nM)** | **H90.2 (SEQ ID No. 578) (nM)** |
|---|---|---|---|
| huEpCAM:Fc (SEQ ID No. 579) | 115 | 0.23 | 0.10 |
| cyEpCAM:Fc (SEQ ID No. 580) | 137 | 0.42 | 0.21 |
| muEpCAM (biotinylated version of mouse EpCAM antigen) | No binding | 0.26 | 0.15 |

### Example 12: T cell engaging capability and specificity of exemplary EpCAM targeting proteins in TriTAC format, a non-cleavable prodrug format, or active drug format

Three exemplary humanized EpCAM targeting ProTriTAC proteins containing an exemplary EpCAM binding domain as described herein, H13 (SEQ ID NO. 207), H138.2 (SEQ ID No. 496), or H90.2 (SEQ ID No. 497), were tested in a T cell dependent cellular cytotoxicity (TDCC) assay (*see* Nazarian AA, Archibeque IL, Nguyen YH, Wang P, Sinclair AM, Powers DA. 2015. J Biomol Screen. 20:519-27), using EpCAM expressing colon cancer cells HCT116. The H90.2 and H138.2 EpCAM binding domain sequences were largely similar to the EpCAM binding domain sequences H90 and H138, respectively, however, the first amino acid of framework 4 was modified (*see* SEQ ID No. 582). In particular, the EpCAM binding domain sequences H90 and H138 contained an Asn deamidation (NG) sites, which were altered to was altered to WG to remove the Asn deamidation, and an improvement in terms of protein stability (which in turn is likely to be advantageous for improving the manufacturability of the drug) was contemplated.

The results are shown in **FIGS. 16A** (H13), **16B** (H90.2), **and 16C** (H138.2). The tested EpCAM binding proteins contained an EpCAM binding domain (H13, H90.2, or H138.2), an albumin binding domain (anti-ALB), and a CD3 binding domain (anti-CD3), wherein the anti-ALB domain further included a non-cleavable linker (NCLV) (the non-cleavable prodrug format), or a cleavable linker plus a masking domain (ACT). The activated EpCAM binding proteins (CT) (the active drug format) contained an EpCAM binding domain (H13, H90.2, or H138.2) and a CD3 binding domain (anti-CD3).

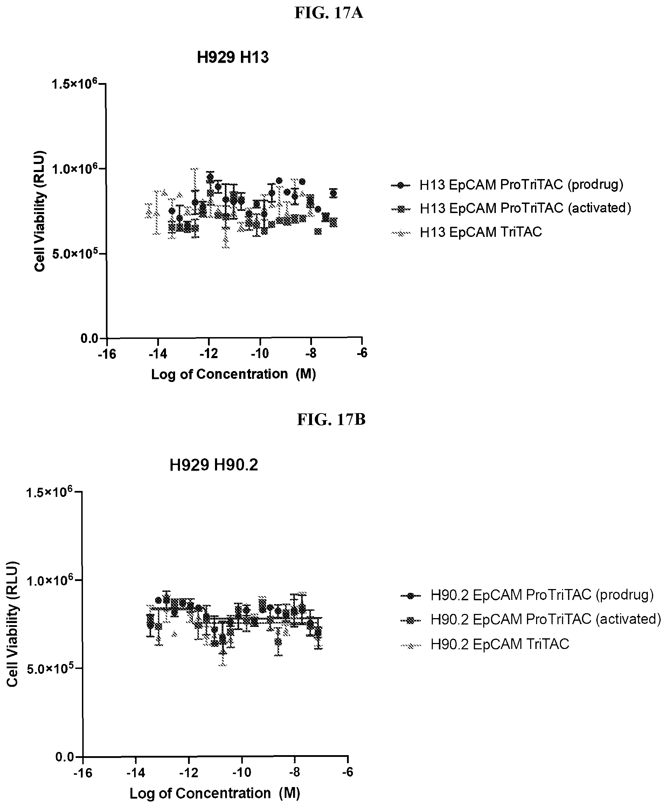

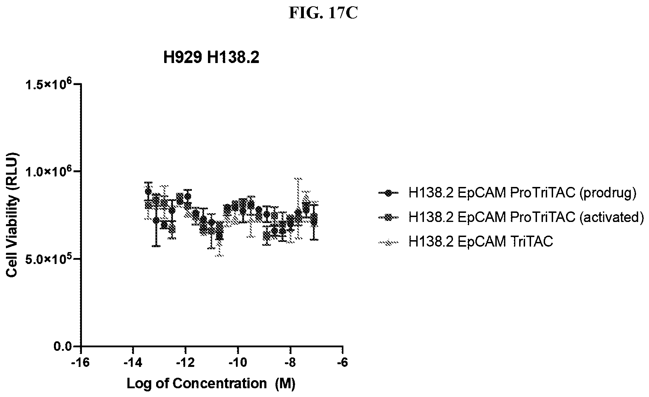

In this assay, luciferase labelled HCT116 cells were combined with purified human T cells and exposed to a titration of the exemplary EpCAM binding proteins as described above (NCLV, CT, ACT). It was hypothesized that if an EpCAM binding protein directed T cells to kill the EpCAM expressing HCT116 cells, then the viability of those cells, as determined by running a luciferase assay at 48 hours after starting the experiment, should decrease. A similar assay was also carried out using EpCAM-negative NCI-H929 myeloma cells, and, as shown in **FIGS. 17A, 17B****, and** **17C****,** none of the proteins tested were able to engage T cells in killing the NCI-H929 cells.

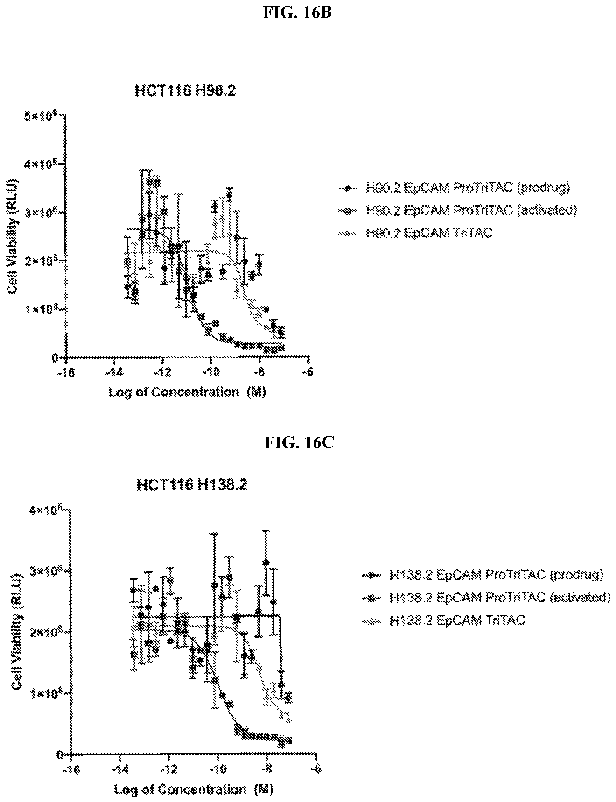

**FIGS. 16A****,** **16B,** **and 16C** illustrate representative TDCC data from the assay using HCT116 cells. EC₅₀ values from the TDCC assay are listed in **Table 9.** As seen in the plots and indicated by the EC₅₀ values, binding proteins containing any of the three EpCAM binding domains (H13, H90.2, or H138.2) functioned as T cell engagers in EpCAM expressing HCT116 colon cancer cells. Further, for the binding proteins with cleavable linker and masking domain (ACT), the EC₅₀ values were about 7 to 10 fold (H13 and H138.2) lower compared to that of the binding proteins with non-cleavable linker (NCLV) (the non-cleavable prodrug format); whereas for the active drug (CT), the EC₅₀ values were up to about 350 fold (H138.2) lower than that of the NCLV. The EC₅₀ of the active drug containing the H13 EpCAM binding domain was about 37 pM; H90.2 binding domain was about 11 pM; and H138.2 binding domain was about 107 pM.

**Table 9: ECso values for TDCC assays using EpCAM ProTriTAC proteins, on HCT116 cells**

| | **EC₅₀ (M)** | **Fold Shift to NCLV** |
|---|---|---|
| EpCAM H13 ProTriTAC (NCLV) (SEQ ID No. 569) | 6.87E-09 | |
| EpCAM H13 ProTriTAC (CT) (SEQ ID No. 210) | 3.67E-11 | 187 |
| EpCAM H13 ProTriTAC (ACT) (SEQ ID No. 570) | 7.07E-10 | 10 |
| EpCAM H90.2 ProTriTAC (NCLV) (SEQ ID No. 502) | | |
| EpCAM H90.2 ProTriTAC (CT) (SEQ ID No. 571) | 1.07E-11 | |
| EpCAM H90.2 ProTriTAC (ACT) (SEQ ID No. 572) | 2.67E-09 | |
| EpCAM H138.2 ProTriTAC (NCLV) (SEQ ID No. 573) | 3.73E-08 | |
| EpCAM H138.2 ProTriTAC (CT) (SEQ ID No. 574) | 1.07E-10 | 349 |
| EpCAM H138.2 ProTriTAC (ACT) (SEQ ID No. 575) | 5.01E-09 | 7 |

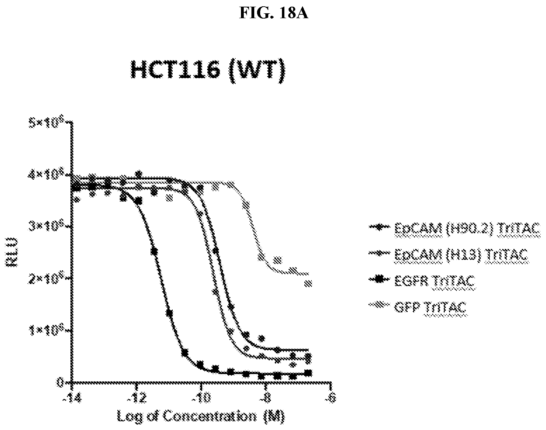

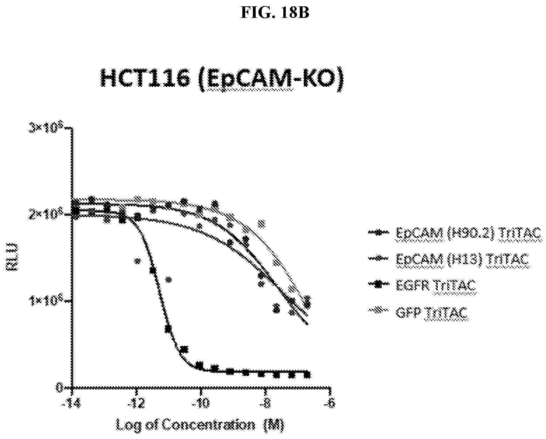

To further assess the EpCAM specific cell killing potential of the test EpCAM binding proteins , a TDCC assay as describe above, was carried out with HCT116 colon cancer cells that express EpCAM (wild-type) or HCT116 (EpCAM-KO) cells that do not express EpCAM. The EpCAM TriTAC proteins containing EpCAM binding domains H13 or H90.2 were tested in this assay, and controls proteins were a GFP TriTAC protein and an EGFR TriTAC protein. Results for this assay is shown in **FIGS. 18A** and **18B****,** and the EC₅₀ values are listed in **Table 10.**

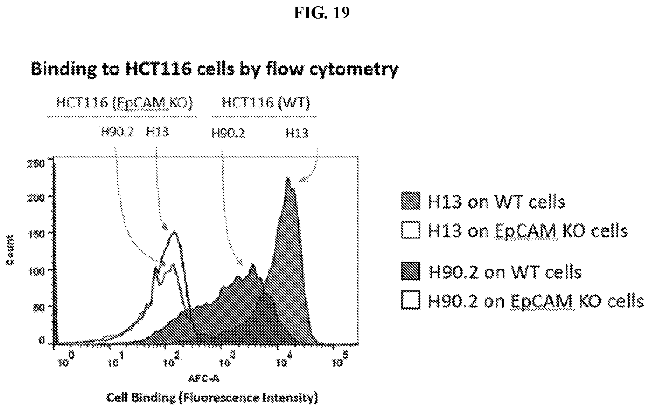

The EpCAM binding proteins exhibited differential killing of cells, based on expression of EpCAM. Specificity of binding was also confirmed by flow cytometry, as shown in **FIG. 19****.** For the flow cytometry assay the cells were stained with 300 nM H13 or H90.2 in the CT format (active drug) and detected with 11D3-AF650 antibody.

**Table 10: ECso values for TDCC assays using EpCAM TriTAC proteins, on HCT116 cells and HCT116 (EpCAM-KO) cells**

| | **HCT116 EC₅₀ (M)** | **HCT116(EpCAM-KO) EC₅₀ (M)** |
|---|---|---|
| EpCAM (H90.2) TriTAC | 3.8E-10 | 3.3E-09 |
| EpCAM (H13) TriTAC | 2.3E-10 | 3.5E-06 |
| EGFR TriTAC | 5.6E-12 | 5.3E-12 |
| GFP TriTAC | 4.0E-09 | 5.8E-07 |

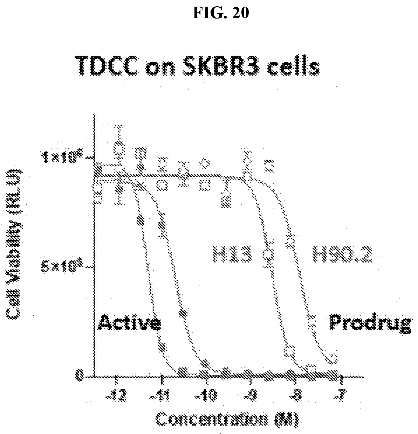

In further TDCC assays, using various other cell lines, the cell killing potential of two EpCAM binding proteins in CT format (the active drug, containing either the H13 or H90.2 EpCAM binding domain and an anti-CD3 domain) or the non-cleavable prodrug format (containing either H13 or H90.2 and anti-CD3 and anti-HSA with non-cleavable linker, NCLV), were compared. Results are provided in **Table 11** and the plot of **FIG. 20** is for the TDCC assay using SKBR3 (human breast cancer cells).

It was observed that the active drug containing the EpCAM binding domain H13 was about 3x more potent than the active drug containing the EpCAM binding domain H90.2 in TDCC assays *in vitro.* The proteins, when compared between their active drug and non-cleavable prodrug formats (CT vs. NCLV), exhibited similar 400-600x masking, in TDCC assays with multiple EpCAM-expressing cells. Representative plots demonstrating the masking effect achieved by the non-cleavable prodrug format, is shown in **FIGS. 21A and 21B****.**

**Table 11: ECso values for TDCC assays using active EpCAM targeting active drugs (CT format), tested in various cell lines**

| **TDCC EC₅₀ (pM)** | | **Active Drug Potency (pM)** | | **H90.2/H13 Ratio** |
|---|---|---|---|---|
| | | **H13** | **H90.2** | |
| CAPAN2 | Pancreas | 4 | 11 | 2.6 |
| SKBR3 | Breast | 5 | 20 | 4.0 |
| TT | Esophagus | 8 | 13 | 1.7 |
| HEPG2 | Liver | 13 | 44 | 3.3 |
| HCT116 | Colon | 14 | 19 | 1.4 |
| SW780 | Bladder | 16 | 56 | 3.6 |
| DMS53 | Lung | 18 | 60 | 3.4 |
| OVCAR8 | Ovary | 57 | 160 | 2.8 |
| HELA | Cervix | 62 | 280 | 4.4 |
| MDAPCA2b | Prostate | 98 | 380 | 3.8 |
| PECAPJ41 | Head & Neck | 120 | 100 | 0.8 |
| **Average** | | **34** | **90** | **2.9x** |

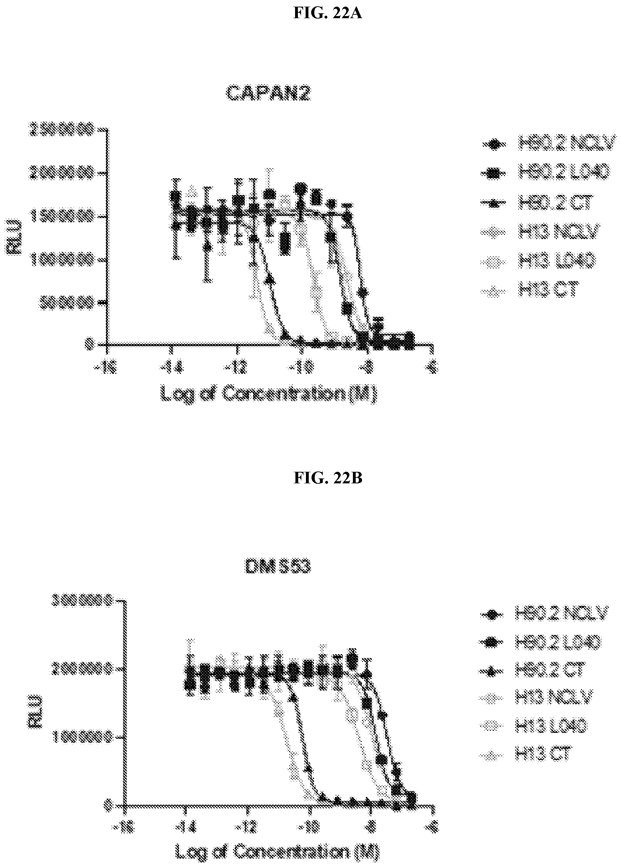

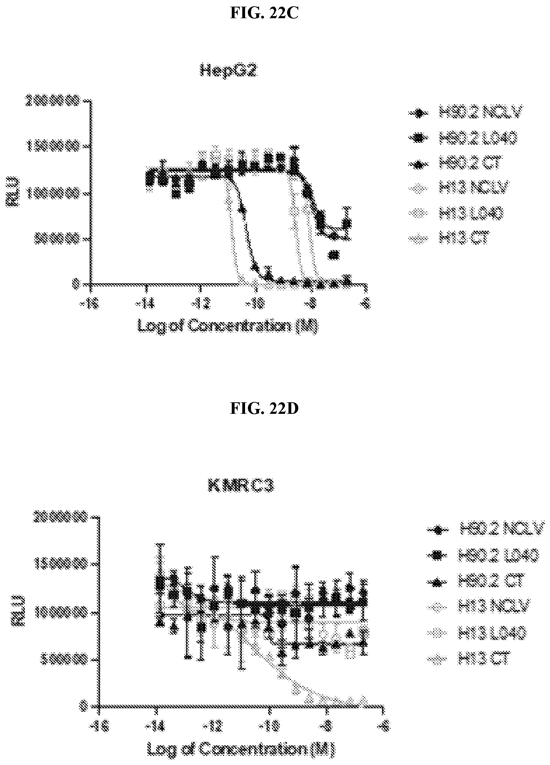

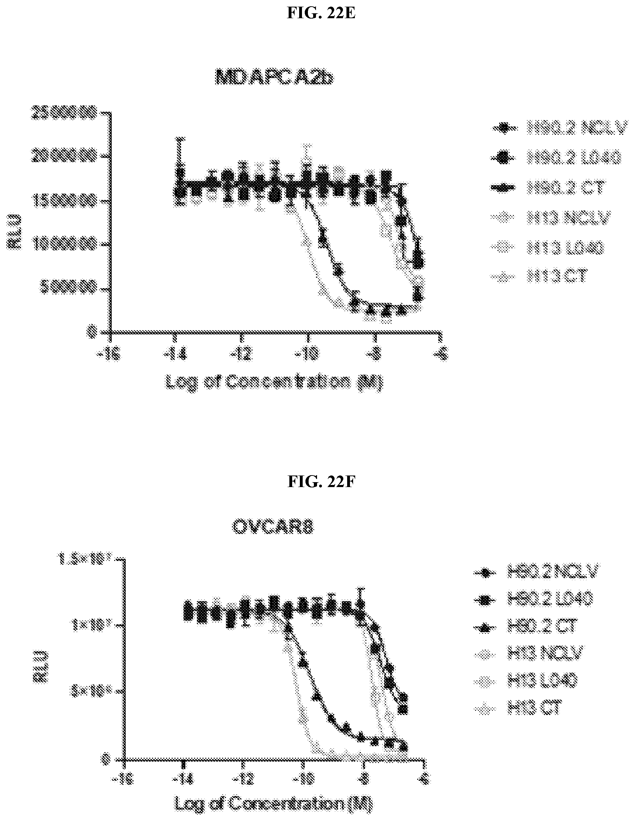

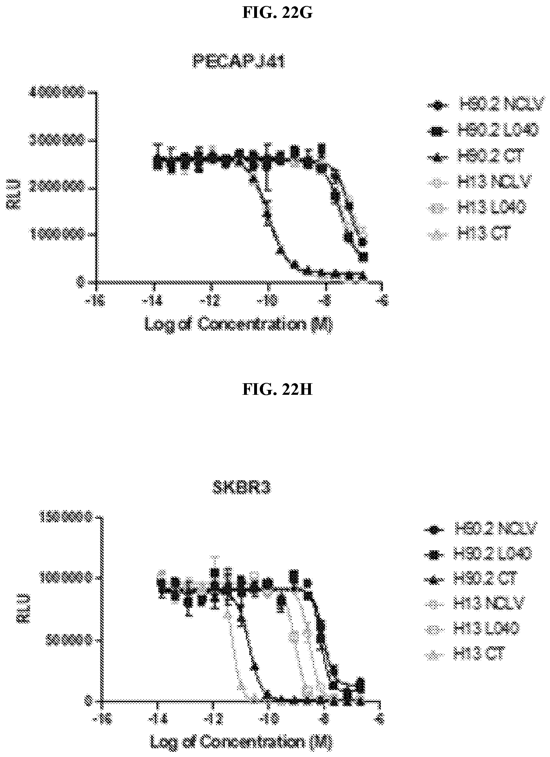

Additional TDCC assays were carried out with cell lines (including CAPAN2, DMS53, HepG2, KMRC3, MDAPCA2b, and SKBR3) for a comparison of H13 containing EpCAM binding protein NCLV (non-cleavable prodrug); ProTriTAC (with cleavable linker L040), and active drug CT. The EC₅₀ values are provided in **Table 12** and representative plots are shown in **FIGS. 22A, 22B****,** **22C, 22D****,** **22E, 22F****,** **22G,** and **22H****.**

**Table 12: ECso values for TDCC assays using active EpCAM targeting active drugs (CT format), tested in various cell lines**

| **Cell Line** | **EpCAM binding domain** | **Format** | **EC₅₀ (M)** |
|---|---|---|---|
| CAPAN2 (Pancreas) | H90.2 | NCLV | 6.31E-09 |
| | | L040 | 1.53E-09 |
| | | CT | 1.06E-11 |
| | H13 | NCLV | 2.49E-09 |
| | | L040 | 2.23E-10 |
| | | CT | 4.06E-12 |
| DMS53 (Lung) | H90.2 | NCLV | 3.26E-08 |
| | | L040 | 1.42E-08 |
| | | CT | 6.04E-11 |
| | H13 | NCLV | 1.01E-08 |
| | | L040 | 4.11E-09 |
| | | CT | 1.76E-11 |
| HepG2 (Liver) | H90.2 | NCLV | 1.18E-08 |
| | | L040 | 1.08E-08 |
| | | CT | 4.37E-11 |
| | H13 | NCLV | 8.63E-09 |
| | | L040 | 2.79E-09 |
| | | CT | 1.32E-11 |
| OVCAR8 (Ovary) | H90.2 | NCLV | 5.14E-08 |
| | | L040 | 3.53E-08 |
| | | CT | 1.61E-10 |
| | H13 | NCLV | 3.49E-08 |
| | | L040 | 1.83E-08 |
| | | CT | 5.66E-11 |
| PECAPJ41 (Head &Neck) | H90.2 | NCLV | 6.48E-08 |
| | | L040 | 3.17E-08 |
| | | CT | 1.01E-10 |
| | H13 | NCLV | 7.57E-08 |
| | | L040 | 4.53E-08 |
| | | CT | 1.24E-10 |
| SKBR3 (Breast) | H90.2 | NCLV | 9.53E-09 |
| | | L040 | 7.52E-09 |
| | | CT | 2.02E-11 |
| | H13 | NCLV | 2.95E-09 |
| | | L040 | 8.76E-10 |
| | | CT | 4.98E-12 |
| KMRC3 | H90.2 | NCLV | |
| | | L040 | |
| | | CT | |
| | H13 | NCLV | |
| | | L040 | |
| | | CT | 1.60E-11 |
| MDAPCA2b | H90.2 | NCLV | |
| | | L040 | 6.74E-08 |
| | | CT | 3.77E-10 |
| | H13 | NCLV | 5.34E-08 |
| | | L040 | 3.07E-08 |
| | | CT | 9.81E-11 |

### Example 13: Anti-tumor efficacy and tolerability of EpCAM targeting ProTriTAC compared to EpCAM targeting TriTAC

The *in vivo* efficacy of EpCAM ProTriTAC containing EpCAM binding domain H13 was assessed in an established HT29 colorectal tumor model and compared to that of an EpCAM TriTAC containing the EpCAM binding domain H13.

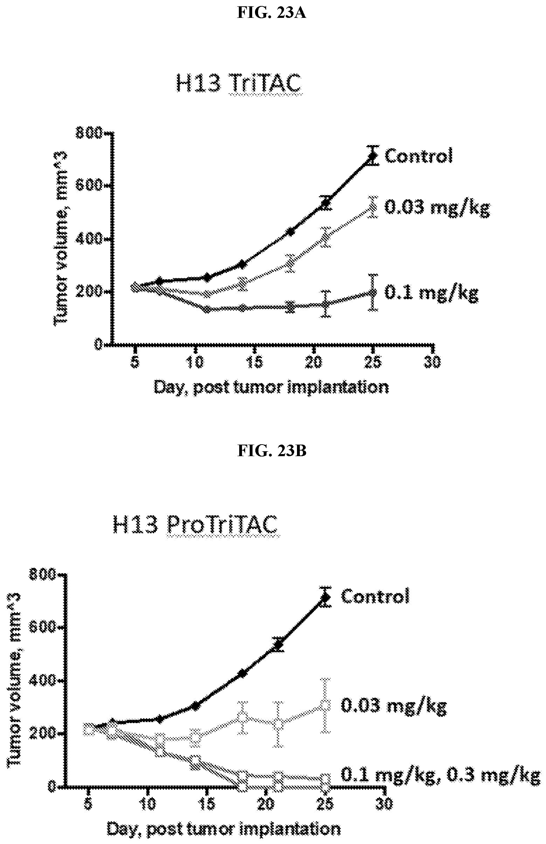

On day 5, following tumor implantation, mice were injected with either the test EpCAM H13 ProTriTAC at 0.3 mg/kg or 0.1 mg/kg; the test EpCAM H13 TriTAC at 0.3 mg/kg; or a control TriTAC at comparable doses, and tumor volumes were measured for a period of about 20 days. As shown in **FIGS. 23A** and **23B****,** The EpCAM H13 ProTriTAC was more efficacious than the EpCAM H13 TriTAC in the mouse tumor model.

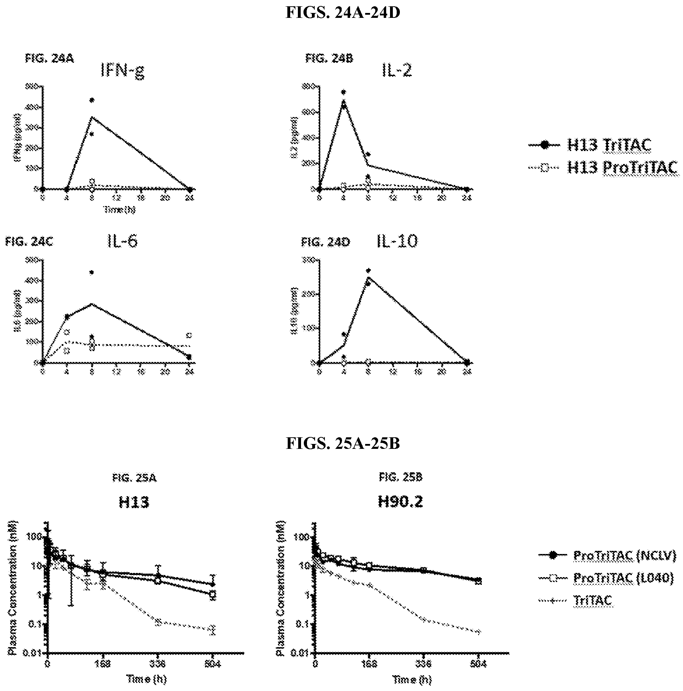

In another study, cynomolgus monkeys were injected with comparable doses (30 µg/kg) of the EpCAM H13 ProTriTAC or the EpCAM H13 TriTAC and the cytokine levels (IFN-gamma; IL-2; IL-6; and IL-10). Results are shown in **FIGS. 24A, 24B, 24C,** and **24D****,** which indicates that the EpCAM H13 ProTriTAC exhibited markedly reduced cytokine production compared to the EpCAM H13 TriTAC, in a single-dose cyno study at 30 µg/kg.

### Example 14: Pharmacokinetic properties of exemplary EpCAM binding proteins, in Cynomolgus monkeys

Binding proteins containing the EpCAM binding domain H13 or H90.2 (in TriTAC format; ProTriTAC NCLV format; and ProTriTAC with cleavable linker L040 format) were administered to cynomolgus monkeys and the pharmacokinetic properties of the same were assessed. **FIGS. 25A** and **25B** show the plasma concentration of the tested proteins, over time, in the cynomolgus monkeys. In addition, **Table 13** summarizes the pharmacokinetic parameters.

**Table 13: PKparameters for EpCAM binding trispecific proteins, in cynomolgus monkeys**

| | | **Cmax (nM)** | **AUC_{0-inf} (hour* nM)** | **Half-Life (day)** |
|---|---|---|---|---|
| H13 | ProTriTAC (NCLV) | 77 | 4586 | 9.7 |
| | ProTriTAC (L040) | 47 | 3643 | 5.8 |
| | TriTAC | 38 | 1588 | 2.5 |
| H90.2 | ProTriTAC (NCLV) | 44 | 5603 | 10.5 |
| | ProTriTAC (L040) | 47 | 6387 | 7.7 |
| | TriTAC | 25 | 1126 | 2.6 |

### Example 15: Assessing therapeutic index (efficacy and toxicity) of EpCAM targeting ProTriTAC/TriTAC proteins

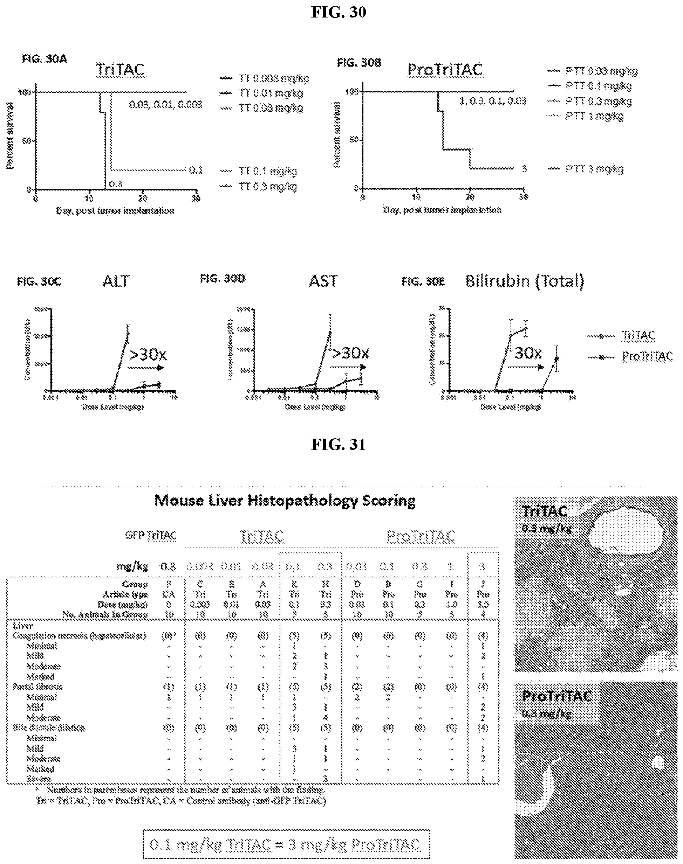

In this study, various doses of an EpCAM targeting TriTAC protein or an EpCAM targeting ProTriTAC protein, containing EpCAM binding domain H90, or a control TriTAC protein, were injected to mice bearing LoVo tumor model (colon cancer model). Results are shown in **FIGS. 29A-29K****.** The safety and tolerability of the ProTriTAC and TriTAC versions were also tested and the results are shown in **FIGS. 30A-30E.** The percent survival is shown in **FIGS. 30A-30B** and the clinical chemistry parameters (ALT, AST, and total bilirubin) are shown in **FIGS. 30C, 30D,** and **30E.** The overall results demonstrate that the ProTriTAC format is about 30x better tolerated than the TriTAC format in the same LoVo tumor-bearing mice. This observation was further corroborated through histopathological studies, and the summary is provided in **FIG. 31** and **Table 14.**

Thus, this study demonstrated a therapeutic index expansion of about 10X, for the ProTriTAC format, based on the efficacy and safety of the drug in the same animal.

**Table 14: Comparative therapeutic index of TriTAC and ProTriTAC formats**

| | **Minimum Efficacious Dose** | **Maximum Tolerated Dose** | **Therapeutic Index (TI)** |
|---|---|---|---|
| **EpCAM TriTAC** | 0.03 mg/kg | 0.03 mg/kg | 1 |
| **EpCAM ProTriTAC** | 0.1 mg/kg | 1 mg/kg | 10 |
| **ProTriTAC Advantage** | 0.3x | 30x | 10x |

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

### SEQUENCES

| SEQ ID NO: | Anti-EpCAM sequence name | Amino Acid Sequence |
|---|---|---|
| 1 | EPL90 | QVQLQESGGGLVQAGGSLRLSCAASGFIFRAASMAWYRQSPGNERELVASISSGAFTNY ADSVKARFTISRDNAKNTVYLQMNSLKPEDTAVYFCGATFLRSDGHHTINGQGTQVTVS S |
| 2 | EPL118 | QVQLQESGGGLVQAGGSLRLSCAASGFIFRAASMGWFRQSPGNERELVATVSSGDFTNY ADSVKGRFTISRDNAKNTVYLQMNSLKPEDTAVYFCGATFVRSDGHHTIYGQGTQVTVS S |
| 3 | EPL138 | QVQLQESGGGLVQAGGSLRLSCAASGFIFRAASMDWYRQFPGNERESIATISSGGFTNY ADSVKGRFTISRDNAKNTVYLQMNSLKPEDTAVYFCGATFLRSDGHHTINGQGTQVTVS S |
| 4 | EPL145 | QVQLQESGGGLVQAGGSLRLSCAASGFIFRAASMGWFRQSPGNERELVATVSSGGFTNY ADSVKGRFTISRDNAKNTVYLQMNSLKPEDTAVYFCGATFVRSDGHHTIYGQGTQVTVS S |
| 5 | EPL164 | QVQLQESGGGLVQAGGSLRLSCAASGFIFRAASMDWYRQSPGTQPELVATISSTGFTNY ANSVKGRFTISRDNAKNTVYLQMNSLKPEDTAVYFCGATFLRSDGQHSIYGQGTQVTVS S |
| 6 | EPL31 | QVQLQESGGGLVHTGGSLRLSCAASGDTFLRYAMGWFRQAPGKEREFVAAITWNGGNTD YAGSLKGRFTISRDNTKNTVYLQMNSLKPEDTAVYYCAADLTFGLASSHYQYDYWGQGT QVTVSS |
| 7 | EPL55 | QVQLQESGGGLVQAGGSLRLSCAASGDTFLRYAMGWFRQAPGKEREFVAAITWNGGNTD YAGSLKGRFTISRDNTKNTVYLQMNSLKPEDTAVYYCAADLTFGLASSHYQYDYWGQGT QVTVSS |
| 8 | EPL57 | QVQLQESGGGLVHTGGSLRLSCAFSGDTFLRYAMGWFRQAPGKEREFVAAITWNGGNTD YADSLKGRFTISRDNTKNTVYLQMNSLRPEDTAVYYCAADLTFGLASSHYQYDYWGQGT QVTVSS |
| 9 | EPL136 | QVQLQESGGGLVQPGGSLRLSCAASGDTFLRYAMGWFRQAPGKEREFVAAITWNGGNTD YAGSLKGRFTISRDNTKNTVYLQMNSLKPEDTAVYYCAADLTFGLASSHYQYDYWGQGT QVTVSS |
| 10 | EPL15 | QVQLQESGGGSVLAGGSLRLSCAASGFTFSSYYMSWVRQAPGKGLEWVSGIHYTGDWTN YADSVKGRFTISRDNAKNELYLEMNNLKPEDTAVYYCARGSDKGQGTQVTVSS |
| 11 | EPL34 | QVQLQESGGGSVQAGGSLRLSCAASGFTFSSYYMSWVRQAPGKGLEWVSGIHYTGDWTN YADSVKGRFTISRDNAKNELYLEMNNLKPEDTAVYYCARGSDKGQGTQVTVSS |
| 12 | EPL86 | QVQLQESGGGLVQPGGSLRLSCAASGFTFSDWAMSWVRQAPGKGLEWVSGIHYGDHTTH YADFVKGRFTISRDDAKNTLYLQMNSLKPEDTAVYYCARGSTKGQGTQVTVSS |
| 13 | EPL153 | QVQLQESGGGLVQPGGSLRLSCAASGFTFSDWAMSWVRQAPGKGLEWVSSIHYGDHTTH YADFVKGRFTISRDDAKNTLYLQMNSLKPEDTAVYYCEKGTTRGQGTQVTVSS |
| 14 | EPL20 | QVQLQESGGGLVQAGGSLKLSCAASGNVFRAATMAWYRQAPEKQREMVATIASGGTTNY ADFVKGRFTISRDNAKNTVYLQMNTLKPEDTAVYYCNAGYLTSLGPKNYWGQGTQVTVS S |
| 15 | EPL70 | QVQLQESGGGLVQPGGSLRLSCAASGNVFRAATMAWYRQAPEKXREMVATIASGGTTNY ADFVKGRFTISRDNAKNTVYLQMNTLKPEDTAVYYCNAGYLTSLGPKNYWGQGTQVTVS S |
| 16 | EPL125 | QVQLQESGGGLVQAGGSLRLSCAASGNVFRAATMAWYRQVPEKQREMVATIASGGTTNY ADFVKGRFTISRDNAKNTVYLQMNTLKPEDTAVYYCNALYLTSLGPKSYWGQGTQVTVS S |
| 17 | EPL13 | QVQLQESGGGLVQPGGSLRLSCAASGFAFGNHWMYWYRQAPGRGRELVASISSGGSTNY VDSVKGRFTISRDNARNTVYLQMYSLKPEDTAVYYCGTSDNWGQGTQVTVSS |
| 18 | EPL129 | QVQLQESGGGLVQAGGSLRLSCAASGFAFGNHWMYWYRQAPGRGRELVASISSGGSTNY VDSVKGRFTISRDNARNTVYLQMYSLRPEDTAVYYCGTSDNWGQGTQVTVSS |
| 19 | EPL159 | QVQLQESGGGLVQAGGSLRLSCAASGFAFGNHWMYWYRQAPGRGRELVASISSGGSTNY VDSVKGRFTISRDNARNTVYLQMYSLKPEDTAVYYCGTSDNWGQGTQVTVSS |
| 20 | EPL120 | QVQLQESGGGLVQAGGSLRLSCAASGFIFRAASISWYRQSPGNERELVATINSGGFTNY ADSVLGRFTISRDNAKNTGYLQMNSLKPEDTAVYFCAATFLRSDGQPPIWGQGTQVTVS S |
| 21 | EPL126 | QVQLQESGGGLVQAGGSLRLSCAASGFIFRAASMGWYRQSPGNERELVATINSGGFTNY ADSVKGRFTISRDNAKNTGYLQMNSLKPEDTAVYFCAATFLRSDGQPPIWGQGTQVTVS S |
| 22 | EPL60 | QVQLQESGGGLVQAGGSLRLSCAASEYILSMYRMAWYRQAPGKVRELVADMSSGGTTNY ADFVKGRFTISRDNDRNTVYLQMNRLQPEDTAAYYCNVAGRTGPPSYDAFNNWGQGTQV TVSS |
| 23 | EPL156 | QVQLQESGGGLVQPGASLRVSCAASEYILSMYRMAWYRQAPGKVRELVADMSSGGTTNY ADFVKGRFTISRDNDRNTVYLQMNRLQPEDTAAYYCNVAGRTGPPSYDAFNNWGQGTQV TVSS |
| 24 | EPL2 | QVQLQESGGGLVQPGGSLRLSCAASESISSFIAVGWYRQAPGKERELVAGINRSGFTYY TDSVKGRFSISRDNAKNTVLLQMTSLKPEDTAVYYCNAGGLYFSNAYTQGDYWGQGTQV TVSS |
| 25 | EPL43 | QVQLQESGGGLVQTGGSLRLSCAASESISSFIAVGWYRQAPGKERELVAGINRSGFTYY TDSVKGRFSISRDNAKNTVLLQMTSLKPEDTAVYYCNAGGLYFSNAYTQGDYWGQGTQV TVSS |
| 26 | EPL10 | QVQLQESGGGLVQAGGSLRLSCAASGSVFRANVMGWYRQAPGKQHELVARIDPGGTTTY ADPVKGRFTISRDNAKKTVYLQMNSLKPDDTAVYYCNAIILLSGGPKDYWGQGTQVTVS S |
| 27 | EPL49 | QVQLQESGGGLVQAGGSLRLSCAPSGRTSSIFGMGWFRQAPGKEREFVASINWSGGSTS YADSVKGRFTISRDNAKNEMYLQMNSLKFEDTAVYVCAAAVLTNKPSWNFWGQGTQVTV SS |
| 28 | EPL58 | QVQLQESGGGLVQAGGSLRLSCAASGPIFSDTIRTMGWYRQAAGKQRELVATIASFPSR TNYVDSVKGRFTISRDIAKNTVYLQMDSLKPEDTAVYYCNVDLASIPTKTYWGQGTQVT VSS |
| 29 | EPL74 | QVQLQESGGGLVQAGGSLRLSCAASGSIFGINAMGWYRQAPGKQRESVAFITIGGNTNY LDSVKGRFTISRDNAKNTVYLQMNGLKPEDTAVYYCNTNPPLILTAGGLYWGQGTQVTV SS |
| 30 | EPL78 | QVQLQESGGGLVQPGGSLRLSCATSANRFNINVMGWYRQAPGQQRELVATINIGGSTDY ADSVKGRFTISRDNAKNTVYLQLSDLKPEDTAVYYCNVKLRVSGPTGPNVYWGQGTQVT VSS |
| 31 | EPL82 | QVQLQESGGGLVQAGGSLKLSCTASGTILSTMAWYRQAPGKQRELVATISRGGTTNYSD SVKGRFAISRDSTKNTVYLQMNSLKPEDTAVYYCNTPLTDYGMGYNWGQGTQVTVSS |
| 32 | EPL83 | QVQLQESGGGLVQAGGSLRLSCAVSGSIFSLNTLAWYRQAPGRQRDLIARITGGGTTVY ADSVKGRFTISRDNAKNTVYLQMNSLKPEDTAVYYCNLMVRHPSGSTYEYWGQGTQVTV SS |
| 33 | EPL97 | QVQLQESGGGLVQAGGSLRLSCAASGIIFRGTTMGWFRQAPGKQRESVASISPLGTTSY SGSVEGRFTVSRDNAKNTLFLQMNSLKSEDTAVYYCNAIQVTNVGPRVYWGQGTQVTVS S |
| 34 | EPL109 | QVQLQESGGGLVQPGGSLRLSCASSGFTLDDYTIGWFRQAPGKEREGVSCISRRDDSTY YADSVKGRFTISRDNAKNTVDLQMISLRPEDTAVYYCAATPRSYTLRCLGKFDFQGQGT QVTVSS |
| 35 | EPL117 | QVQLQESGGGLVQAGGSLRLSCAASGNIVRMTNMAWYRQAPGKQREFVATISAGGSTTY VDSVKDRFTISRDNTKNTVYLQMNYLKPEDTAVYYCATGSILTNRGAIPGSWGHGTQVT VSS |
| 36 | EPL127 | QVQLQESGGGLVQAGGSLRLSCAAPGFAFNDHAILWFRQAPGKEREGVSEICRDGTTYY TDSVKGRFTISSDNAKNTVYLQMNSVKTDDTAVYYCAVDRRRYYCSGNRAFSSDYYYWG QGTQVTVSS |
| 37 | EPL152 | QVQLQESGGGLVQAGGSLRLSCVHSGSIFRASTMAWYRQAPGKQRELVAQIMSGGGTNY AGSVKGRFTISRDNANNTVYLQMNSLKPEDTAVYYCNAAQITSWGPKVYWGQGTQVTVS S |
| 38 | EPL189 | QVQLQESGGGLVQPGGSLRLSCAASGRINSINTMGWYRQAPGNQRELVAEITRGGTTNY ADSVQGRYAISRDNAKNLVYLQMNSLKPEDTDVYYCNAQTFPTFSRPTGLDYWGQGTQV TVSS |

| SEQ ID NO: | Anti-EpCAM sequence CDR1 | Amino Acid Sequence |
|---|---|---|
| 39 | EPL90 | GFIFRAASMA |
| 40 | EPL118 | GFIFRAASMG |
| 41 | EPL138 | GFIFRAASMD |
| 42 | EPL145 | GFIFRAASMG |
| 43 | EPL164 | GFIFRAASMD |
| 44 | EPL31 | GDTFLRYAMG |
| 45 | EPL55 | GDTFLRYAMG |
| 46 | EPL57 | GDTFLRYAMG |
| 47 | EPL136 | GDTFLRYAMG |
| 48 | EPL15 | GFTFSSYYMS |
| 49 | EPL34 | GFTFSSYYMS |
| 50 | EPL86 | GFTFSDWAMS |
| 51 | EPL153 | GFTFSDWAMS |
| 52 | EPL20 | GNVFRAATMA |
| 53 | EPL70 | GNVFRAATMA |
| 54 | EPL125 | GNVFRAATMA |
| 55 | EPL13 | GFAFGNHWMY |
| 56 | EPL129 | GFAFGNHWMY |
| 57 | EPL159 | GFAFGNHWMY |
| 58 | EPL120 | GFIFRAASIS |
| 59 | EPL126 | GFIFRAASMG |
| 60 | EPL60 | EYILSMYRMA |
| 61 | EPL156 | EYILSMYRMA |
| 62 | EPL2 | ESISSFIAVG |
| 63 | EPL43 | ESISSFIAVG |
| 64 | EPL10 | GSVFRANVMG |
| 65 | EPL49 | GRTSSIFGMG |
| 66 | EPL58 | GPIFSDTIRTMG |
| 67 | EPL74 | GSIFGINAMG |
| 68 | EPL78 | ANRFNINVMG |
| 69 | EPL82 | GTILSTMA |
| 70 | EPL83 | GSIFSLNTLA |
| 71 | EPL97 | GIIFRGTTMG |
| 72 | EPL109 | GFTLDDYTIG |
| 73 | EPL117 | GNIVRMTNMA |
| 74 | EPL127 | GFAFNDHAIL |
| 75 | EPL152 | GSIFRASTMA |
| 76 | EPL189 | GRINSINTMG |

| SEQ ID NO: | Anti-EpCAM sequence CDR2 | Amino Acid Sequence |
|---|---|---|
| 77 | EPL90 | SISSGAFTNYADSVKA |
| 78 | EPL118 | TVSSGDFTNYADSVKG |
| 79 | EPL138 | TISSGGFTNYADSVKG |
| 80 | EPL145 | TVSSGGFTNYADSVKG |
| 81 | EPL164 | TISSTGFTNYANSVKG |
| 82 | EPL31 | AITWNGGNTDYAGSLKG |
| 83 | EPL55 | AITWNGGNTDYAGSLKG |
| 84 | EPL57 | AITWNGGNTDYADSLKG |
| 85 | EPL136 | AITWNGGNTDYAGSLKG |
| 86 | EPL15 | GIHYTGDWTNYADSVKG |
| 87 | EPL34 | GIHYTGDWTNYADSVKG |
| 88 | EPL86 | GIHYGDHTTHYADFVKG |
| 89 | EPL153 | SIHYGDHTTHYADFVKG |
| 90 | EPL20 | TIASGGTTNYADFVKG |
| 91 | EPL70 | TIASGGTTNYADFVKG |
| 92 | EPL125 | TIASGGTTNYADFVKG |
| 93 | EPL13 | SISSGGSTNYVDSVKG |
| 94 | EPL129 | SISSGGSTNYVDSVKG |
| 95 | EPL159 | SISSGGSTNYVDSVKG |
| 96 | EPL120 | TINSGGFTNYADSVLG |
| 97 | EPL126 | TINSGGFTNYADSVKG |
| 98 | EPL60 | DMSSGGTTNYADFVKG |
| 99 | EPL156 | DMSSGGTTNYADFVKG |
| 100 | EPL2 | GINRSGFTYYTDSVKG |
| 101 | EPL43 | GINRSGFTYYTDSVKG |
| 102 | EPL10 | RIDPGGTTTYADPVKG |
| 103 | EPL49 | SINWSGGSTSYADSVKG |
| 104 | EPL58 | TIASFPSRTNYVDSVKG |
| 105 | EPL74 | FITIGGNTNYLDSVKG |
| 106 | EPL78 | TINIGGSTDYADSVKG |
| 107 | EPL82 | TISRGGTTNYSDSVKG |
| 108 | EPL83 | RITGGGTTVYADSVKG |
| 109 | EPL97 | SISPLGTTSYSGSVEG |
| 110 | EPL109 | CISRRDDSTYYADSVKG |
| 111 | EPL117 | TISAGGSTTYVDSVKD |
| 112 | EPL127 | EICRDGTTYYTDSVKG |
| 113 | EPL152 | QIMSGGGTNYAGSVKG |
| 114 | EPL189 | EITRGGTTNYADSVQG |

| **SEQ ID NO:** | **Anti-EpCAM sequence CDR3** | **Amino Acid Sequence** |
|---|---|---|
| 115 | EPL90 | TFLRSDGHHTI |
| 116 | EPL118 | TFVRSDGHHTI |
| 117 | EPL138 | TFLRSDGHHTI |
| 118 | EPL145 | TFVRSDGHHTI |
| 119 | EPL164 | TFLRSDGQHSI |
| 120 | EPL31 | DLTFGLASSHYQYDY |
| 121 | EPL55 | DLTFGLASSHYQYDY |
| 122 | EPL57 | DLTFGLASSHYQYDY |
| 123 | EPL136 | DLTFGLASSHYQYDY |
| 124 | EPL15 | GSD |
| 125 | EPL34 | GSD |
| 126 | EPL86 | GST |
| 127 | EPL153 | GTT |
| 128 | EPL20 | GYLTSLGPKNY |
| 129 | EPL70 | GYLTSLGPKNY |
| 130 | EPL125 | LYLTSLGPKSY |
| 131 | EPL13 | SDN |
| 132 | EPL129 | SDN |
| 133 | EPL159 | SDN |
| 134 | EPL120 | TFLRSDGQPPI |
| 135 | EPL126 | TFLRSDGQPPI |
| 136 | EPL60 | AGRTGPPSYDAFNN |
| 137 | EPL156 | AGRTGPPSYDAFNN |
| 138 | EPL2 | GGLYFSNAYTQGDY |
| 139 | EPL43 | GGLYFSNAYTQGDY |
| 140 | EPL10 | IILLSGGPKDY |
| 141 | EPL49 | AVLTNKPSWNF |
| 142 | EPL58 | DLASIPTKTY |
| 143 | EPL74 | NPPLILTAGGLY |
| 144 | EPL78 | KLRVSGPTGPNVY |
| 145 | EPL82 | PLTDYGMGYN |
| 146 | EPL83 | MVRHPSGSTYEY |
| 147 | EPL97 | IQVTNVGPRVY |
| 148 | EPL109 | TPRSYTLRCLGKFDF |
| 149 | EPL117 | GSILTNRGAIPGS |
| 150 | EPL127 | DRRRYYCSGNRAFSSDYYY |
| 151 | EPL152 | AQITSWGPKVY |
| 152 | EPL189 | QTFPTFSRPTGLDY |

| **SEQ ID NO:** | **Anti-CD3/Anti-EpCAM sequence name** | **Amino Acid Sequence** |
|---|---|---|
| 153 | EPL10 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAASGSVFR ANVMGWYRQAPGKQHELVARIDPGGTTTYADPVKGRFTISRDNAKKTVYLQMNSLKPDD TAVYYCNAIILLSGGPKDYWGQGTQVTVSSHHHHHH* |
| 154 | EPL109 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQPGGSLRLSCASSGFTLD DYTIGWFRQAPGKEREGVSCISRRDDSTYYADSVKGRFTISRDNAKNTVDLQMISLRPE DTAVYYCAATPRSYTLRCLGKFDFQGQGTQVTVSSHHHHHH* |
| 155 | EPL117 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAASGNIVR MTNMAWYRQAPGKQREFVATISAGGSTTYVDSVKDRFTISRDNTKNTVYLQMNYLKPED TAVYYCATGSILTNRGAIPGSWGHGTQVTVSSHHHHHH* |
| 156 | EPL120 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAASGFIFR AASISWYRQSPGNERELVATINSGGFTNYADSVLGRFTISRDNAKNTGYLQMNSLKPED TAVYFCAATFLRSDGQPPIWGQGTQVTVSSHHHHHH* |
| 157 | EPL125 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAASGNVFR |
| | | AATMAWYRQVPEKQREMVATIASGGTTNYADFVKGRFTISRDNAKNTVYLQMNTLKPED TAVYYCNALYLTSLGPKSYWGQGTQVTVSSHHHHHH* |
| 158 | EPL127 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAAPGFAFN DHAILWFRQAPGKEREGVSEICRDGTTYYTDSVKGRFTISSDNAKNTVYLQMNSVKTDD TAVYYCAVDRRRYYCSGNRAFSSDYYYWGQGTQVTVSSHHHHHH* |
| 159 | EPL13 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQPGGSLRLSCAASGFAFG NHWMYWYRQAPGRGRELVASISSGGSTNYVDSVKGRFTISRDNARNTVYLQMYSLKPED TAVYYCGTSDNWGQGTQVTVSSHHHHHH* |
| 160 | EPL136 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQPGGSLRLSCAASGDTFL RYAMGWFRQAPGKEREFVAAITWNGGNTDYAGSLKGRFTISRDNTKNTVYLQMNSLKPE DTAVYYCAADLTFGLASSHYQYDYWGQGTQVTVSSHHHHHH* |
| 161 | EPL138 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAASGFIFR AASMDWYRQFPGNERESIATISSGGFTNYADSVKGRFTISRDNAKNTVYLQMNSLKPED TAVYFCGATFLRSDGHHTINGQGTQVTVSSHHHHHH* |
| 162 | EPL145 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAASGFIFR AASMGWFRQSPGNERELVATVSSGGFTNYADSVKGRFTISRDNAKNTVYLQMNSLKPED TAVYFCGATFVRSDGHHTIYGQGTQVTVSSHHHHHH* |
| 163 | EPL152 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE |
| | | WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCVHSGSIFR ASTMAWYRQAPGKQRELVAQIMSGGGTNYAGSVKGRFTISRDNANNTVYLQMNSLKPED TAVYYCNAAQITSWGPKVYWGQGTQVTVSSHHHHHH* |
| 164 | EPL153 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQPGGSLRLSCAASGFTFS DWAMSWVRQAPGKGLEWVSSIHYGDHTTHYADFVKGRFTISRDDAKNTLYLQMNSLKPE DTAVYYCEKGTTRGQGTQVTVSSHHHHHH* |
| 165 | EPL156 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQPGASLRVSCAASEYILS MYRMAWYRQAPGKVRELVADMSSGGTTNYADFVKGRFTISRDNDRNTVYLQMNRLQPED TAAYYCNVAGRTGPPSYDAFNNWGQGTQVTVSSHHHHHH* |
| 166 | EPL164 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAASGFIFR AASMDWYRQSPGTQPELVATISSTGFTNYANSVKGRFTISRDNAKNTVYLQMNSLKPED TAVYFCGATFLRSDGQHSIYGQGTQVTVSSHHHHHH* |
| 167 | EPL189 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQPGGSLRLSCAASGRINS INTMGWYRQAPGNQRELVAEITRGGTTNYADSVQGRYAISRDNAKNLVYLQMNSLKPED TDVYYCNAQTFPTFSRPTGLDYWGQGTQVTVSSHHHHHH* |
| 168 | EPL2 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQPGGSLRLSCAASESISS FIAVGWYRQAPGKERELVAGINRSGFTYYTDSVKGRFSISRDNAKNTVLLQMTSLKPED TAVYYCNAGGLYFSNAYTQGDYWGQGTQVTVSSHHHHHH* |
| 169 | EPL20 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLKLSCAASGNVFR AATMAWYRQAPEKQREMVATIASGGTTNYADFVKGRFTISRDNAKNTVYLQMNTLKPED TAVYYCNAGYLTSLGPKNYWGQGTQVTVSSHHHHHH* |
| 170 | EPL34 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGSVQAGGSLRLSCAASGFTFS SYYMSWVRQAPGKGLEWVSGIHYTGDWTNYADSVKGRFTISRDNAKNELYLEMNNLKPE DTAVYYCARGSDKGQGTQVTVSSHHHHHH* |
| 171 | EPL49 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAPSGRTSS IFGMGWFRQAPGKEREFVASINWSGGSTSYADSVKGRFTISRDNAKNEMYLQMNSLKFE DTAVYVCAAAVLTNKPSWNFWGQGTQVTVSSHHHHHH* |
| 172 | EPL58 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAASGPIFS DTIRTMGWYRQAAGKQRELVATIASFPSRTNYVDSVKGRFTISRDIAKNTVYLQMDSLK PEDTAVYYCNVDLASIPTKTYWGQGTQVTVSSHHHHHH* |
| 173 | EPL74 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAASGSIFG INAMGWYRQAPGKQRESVAFITIGGNTNYLDSVKGRFTISRDNAKNTVYLQMNGLKPED TAVYYCNTNPPLILTAGGLYWGQGTQVTVSSHHHHHH* |
| 174 | EPL78 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQPGGSLRLSCATSANRFN |
| | | INVMGWYRQAPGQQRELVATINIGGSTDYADSVKGRFTISRDNAKNTVYLQLSDLKPED TAVYYCNVKLRVSGPTGPNVYWGQGTQVTVSSHHHHHH* |
| 175 | EPL82 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLKLSCTASGTILS TMAWYRQAPGKQRELVATISRGGTTNYSDSVKGRFAISRDSTKNTVYLQMNSLKPEDTA VYYCNTPLTDYGMGYNWGQGTQVTVSSHHHHHH* |
| 176 | EPL83 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAVSGSIFS LNTLAWYRQAPGRQRDLIARITGGGTTVYADSVKGRFTISRDNAKNTVYLQMNSLKPED TAVYYCNLMVRHPSGSTYEYWGQGTQVTVSSHHHHHH* |
| 177 | EPL86 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQPGGSLRLSCAASGFTFS DWAMSWVRQAPGKGLEWVSGIHYGDHTTHYADFVKGRFTISRDDAKNTLYLQMNSLKPE DTAVYYCARGSTKGQGTQVTVSSHHHHHH* |
| 178 | EPL90 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAASGFIFR AASMAWYRQSPGNERELVASISSGAFTNYADSVKARFTISRDNAKNTVYLQMNSLKPED TAVYFCGATFLRSDGHHTINGQGTQVTVSSHHHHHH* |
| 179 | EPL97 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSQVQLQESGGGLVQAGGSLRLSCAASGIIFR GTTMGWFRQAPGKQRESVASISPLGTTSYSGSVEGRFTVSRDNAKNTLFLQMNSLKSED TAVYYCNAIQVTNVGPRVYWGQGTQVTVSSHHHHHH* |

| **SEQ ID NO:** | **Anti- EpCAM /Anti-CD3 sequence name** | **Amino Acid Sequence** |
|---|---|---|
| 180 | EPL10 | GGGSQVQLQESGGGLVQAGGSLRLSCAASGSVFRANVMGWYRQAPGKQHELVARIDPGG TTTYADPVKGRFTISRDNAKKTVYLQMNSLKPDDTAVYYCNAIILLSGGPKDYWGQGTQ VTVSSGGGGSGGGSEVQLVESGGGLVQPGGSLKLSCAASGFTFNKYAINWVRQAPGKGL EWVARIRSKYNNYATYYADQVKDRFTISRDDSKNTAYLQMNNLKTEDTAVYYCVRHANF GNSYISYWAYWGQGTLVTVSSGGGGSGGGGSGGGGSQTVVTQEPSLTVSPGGTVTLTCA SSTGAVTSGNYPNWVQQKPGQAPRGLIGGTKFLVPGTPARFSGSLLGGKAALTLSGVQP EDEAEYYCTLWYSNRWVFGGGTKLTVLHHHHHH* |
| 181 | EPL109 | GGGSQVQLQESGGGLVQPGGSLRLSCASSGFTLDDYTIGWFRQAPGKEREGVSCISRRD DSTYYADSVKGRFTISRDNAKNTVDLQMISLRPEDTAVYYCAATPRSYTLRCLGKFDFQ GQGTQVTVSSGGGGSGGGSEVQLVESGGGLVQPGGSLKLSCAASGFTFNKYAINWVRQA PGKGLEWVARIRSKYNNYATYYADQVKDRFTISRDDSKNTAYLQMNNLKTEDTAVYYCV RHANFGNSYISYWAYWGQGTLVTVSSGGGGSGGGGSGGGGSQTVVTQEPSLTVSPGGTV TLTCASSTGAVTSGNYPNWVQQKPGQAPRGLIGGTKFLVPGTPARFSGSLLGGKAALTL SGVQPEDEAEYYCTLWYSNRWVFGGGTKLTVLHHHHHH* |
| 182 | EPL117 | GGGSQVQLQESGGGLVQAGGSLRLSCAASGNIVRMTNMAWYRQAPGKQREFVATISAGG STTYVDSVKDRFTISRDNTKNTVYLQMNYLKPEDTAVYYCATGSILTNRGAIPGSWGHG TQVTVSSGGGGSGGGSEVQLVESGGGLVQPGGSLKLSCAASGFTFNKYAINWVRQAPGK GLEWVARIRSKYNNYATYYADQVKDRFTISRDDSKNTAYLQMNNLKTEDTAVYYCVRHA NFGNSYISYWAYWGQGTLVTVSSGGGGSGGGGSGGGGSQTVVTQEPSLTVSPGGTVTLT CASSTGAVTSGNYPNWVQQKPGQAPRGLIGGTKFLVPGTPARFSGSLLGGKAALTLSGV QPEDEAEYYCTLWYSNRWVFGGGTKLTVLHHHHHH* |
| 183 | EPL120 | GGGSQVQLQESGGGLVQAGGSLRLSCAASGFIFRAASISWYRQSPGNERELVATINSGG FTNYADSVLGRFTISRDNAKNTGYLQMNSLKPEDTAVYFCAATFLRSDGQPPIWGQGTQ VTVSSGGGGSGGGSEVQLVESGGGLVQPGGSLKLSCAASGFTFNKYAINWVRQAPGKGL EWVARIRSKYNNYATYYADQVKDRFTISRDDSKNTAYLQMNNLKTEDTAVYYCVRHANF GNSYISYWAYWGQGTLVTVSSGGGGSGGGGSGGGGSQTVVTQEPSLTVSPGGTVTLTCA SSTGAVTSGNYPNWVQQKPGQAPRGLIGGTKFLVPGTPARFSGSLLGGKAALTLSGVQP EDEAEYYCTLWYSNRWVFGGGTKLTVLHHHHHH* |
| 184 | EPL125 | GGGSQVQLQESGGGLVQAGGSLRLSCAASGNVFRAATMAWYRQVPEKQREMVATIASGG TTNYADFVKGRFTISRDNAKNTVYLQMNTLKPEDTAVYYCNALYLTSLGPKSYWGQGTQ VTVSSGGGGSGGGSEVQLVESGGGLVQPGGSLKLSCAASGFTFNKYAINWVRQAPGKGL EWVARIRSKYNNYATYYADQVKDRFTISRDDSKNTAYLQMNNLKTEDTAVYYCVRHANF GNSYISYWAYWGQGTLVTVSSGGGGSGGGGSGGGGSQTVVTQEPSLTVSPGGTVTLTCA SSTGAVTSGNYPNWVQQKPGQAPRGLIGGTKFLVPGTPARFSGSLLGGKAALTLSGVQP EDEAEYYCTLWYSNRWVFGGGTKLTVLHHHHHH* |
| 185 | EPL127 | GGGSQVQLQESGGGLVQAGGSLRLSCAAPGFAFNDHAILWFRQAPGKEREGVSEICRDG TTYYTDSVKGRFTISSDNAKNTVYLQMNSVKTDDTAVYYCAVDRRRYYCSGNRAFSSDY YYWGQGTQVTVSSGGGGSGGGSEVQLVESGGGLVQPGGSLKLSCAASGFTFNKYAINWV RQAPGKGLEWVARIRSKYNNYATYYADQVKDRFTISRDDSKNTAYLQMNNLKTEDTAVY YCVRHANFGNSYISYWAYWGQGTLVTVSSGGGGSGGGGSGGGGSQTVVTQEPSLTVSPG |
| | | GTVTLTCASSTGAVTSGNYPNWVQQKPGQAPRGLIGGTKFLVPGTPARFSGSLLGGKAA LTLSGVQPEDEAEYYCTLWYSNRWVFGGGTKLTVLHHHHHH* |
| 186 | EPL13 | GGGSQVQLQESGGGLVQPGGSLRLSCAASGFAFGNHWMYWYRQAPGRGRELVASISSGG STNYVDSVKGRFTISRDNARNTVYLQMYSLKPEDTAVYYCGTSDNWGQGTQVTVSSGGG GSGGGSEVQLVESGGGLVQPGGSLKLSCAASGFTFNKYAINWVRQAPGKGLEWVARIRS KYNNYATYYADQVKDRFTISRDDSKNTAYLQMNNLKTEDTAVYYCVRHANFGNSYISYW AYWGQGTLVTVSSGGGGSGGGGSGGGGSQTVVTQEPSLTVSPGGTVTLTCASSTGAVTS GNYPNWVQQKPGQAPRGLIGGTKFLVPGTPARFSGSLLGGKAALTLSGVQPEDEAEYYC TLWYSNRWVFGGGTKLTVLHHHHHH* |
| 187 | EPL136 | GGGSQVQLQESGGGLVQPGGSLRLSCAASGDTFLRYAMGWFRQAPGKEREFVAAITWNG GNTDYAGSLKGRFTISRDNTKNTVYLQMNSLKPEDTAVYYCAADLTFGLASSHYQYDYW GQGTQVTVSSGGGGSGGGSEVQLVESGGGLVQPGGSLKLSCAASGFTFNKYAINWVRQA PGKGLEWVARIRSKYNNYATYYADQVKDRFTISRDDSKNTAYLQMNNLKTEDTAVYYCV RHANFGNSYISYWAYWGQGTLVTVSSGGGGSGGGGSGGGGSQTVVTQEPSLTVSPGGTV TLTCASSTGAVTSGNYPNWVQQKPGQAPRGLIGGTKFLVPGTPARFSGSLLGGKAALTL SGVQPEDEAEYYCTLWYSNRWVFGGGTKLTVLHHHHHH* |
| 188 | EPL138 | GGGSQVQLQESGGGLVQAGGSLRLSCAASGFIFRAASMDWYRQFPGNERESIATISSGG FTNYADSVKGRFTISRDNAKNTVYLQMNSLKPEDTAVYFCGATFLRSDGHHTINGQGTQ VTVSSGGGGSGGGSEVQLVESGGGLVQPGGSLKLSCAASGFTFNKYAINWVRQAPGKGL EWVARIRSKYNNYATYYADQVKDRFTISRDDSKNTAYLQMNNLKTEDTAVYYCVRHANF GNSYISYWAYWGQGTLVTVSSGGGGSGGGGSGGGGSQTVVTQEPSLTVSPGGTVTLTCA SSTGAVTSGNYPNWVQQKPGQAPRGLIGGTKFLVPGTPARFSGSLLGGKAALTLSGVQP EDEAEYYCTLWYSNRWVFGGGTKLTVLHHHHHH* |
| 189 | EPL145 | GGGSQVQLQESGGGLVQAGGSLRLSCAASGFIFRAASMGWFRQSPGNERELVATVSSGG FTNYADSVKGRFTISRDNAKNTVYLQMNSLKPEDTAVYFCGATFVRSDGHHTIYGQGTQ VTVSSGGGGSGGGSEVQLVESGGGLVQPGGSLKLSCAASGFTFNKYAINWVRQAPGKGL EWVARIRSKYNNYATYYADQVKDRFTISRDDSKNTAYLQMNNLKTEDTAVYYCVRHANF GNSYISYWAYWGQGTLVTVSSGGGGSGGGGSGGGGSQTVVTQEPSLTVSPGGTVTLTCA SSTGAVTSGNYPNWVQQKPGQAPRGLIGGTKFLVPGTPARFSGSLLGGKAALTLSGVQP EDEAEYYCTLWYSNRWVFGGGTKLTVLHHHHHH* |
| 190 | EPL152 | GGGSQVQLQESGGGLVQAGGSLRLSCVHSGSIFRASTMAWYRQAPGKQRELVAQIMSGG GTNYAGSVKGRFTISRDNANNTVYLQMNSLKPEDTAVYYCNAAQITSWGPKVYWGQGTQ VTVSSGGGGSGGGSEVQLVESGGGLVQPGGSLKLSCAASGFTFNKYAINWVRQAPGKGL EWVARIRSKYNNYATYYADQVKDRFTISRDDSKNTAYLQMNNLKTEDTAVYYCVRHANF GNSYISYWAYWGQGTLVTVSSGGGGSGGGGSGGGGSQTVVTQEPSLTVSPGGTVTLTCA SSTGAVTSGNYPNWVQQKPGQAPRGLIGGTKFLVPGTPARFSGSLLGGKAALTLSGVQP EDEAEYYCTLWYSNRWVFGGGTKLTVLHHHHHH* |
| 191 | EPL153 | GGGSQVQLQESGGGLVQPGGSLRLSCAASGFTFSDWAMSWVRQAPGKGLEWVSSIHYGD HTTHYADFVKGRFTISRDDAKNTLYLQMNSLKPEDTAVYYCEKGTTRGQGTQVTVSSGG GGSGGGSEVQLVESGGGLVQPGGSLKLSCAASGFTFNKYAINWVRQAPGKGLEWVARIR |
| | | SKYNNYATYYADQVKDRFTISRDDSKNTAYLQMNNLKTEDTAVYYCVRHANFGNSYISY WAYWGQGTLVTVSSGGGGSGGGGSGGGGSQTVVTQEPSLTVSPGGTVTLTCASSTGAVT SGNYPNWVQQKPGQAPRGLIGGTKFLVPGTPARFSGSLLGGKAALTLSGVQPEDEAEYY CTLWYSNRWVFGGGTKLTVLHHHHHH* |
| 192 | EPL156 | GGGSQVQLQESGGGLVQPGASLRVSCAASEYILSMYRMAWYRQAPGKVRELVADMSSGG TTNYADFVKGRFTISRDNDRNTVYLQMNRLQPEDTAAYYCNVAGRTGPPSYDAFNNWGQ GTQVTVSSGGGGSGGGSEVQLVESGGGLVQPGGSLKLSCAASGFTFNKYAINWVRQAPG KGLEWVARIRSKYNNYATYYADQVKDRFTISRDDSKNTAYLQMNNLKTEDTAVYYCVRH ANFGNSYISYWAYWGQGTLVTVSSGGGGSGGGGSGGGGSQTVVTQEPSLTVSPGGTVTL TCASSTGAVTSGNYPNWVQQKPGQAPRGLIGGTKFLVPGTPARFSGSLLGGKAALTLSG VQPEDEAEYYCTLWYSNRWVFGGGTKLTVLHHHHHH* |
| 193 | EPL164 | GGGSQVQLQESGGGLVQAGGSLRLSCAASGFIFRAASMDWYRQSPGTQPELVATISSTG FTNYANSVKGRFTISRDNAKNTVYLQMNSLKPEDTAVYFCGATFLRSDGQHSIYGQGTQ VTVSSGGGGSGGGSEVQLVESGGGLVQPGGSLKLSCAASGFTFNKYAINWVRQAPGKGL EWVARIRSKYNNYATYYADQVKDRFTISRDDSKNTAYLQMNNLKTEDTAVYYCVRHANF GNSYISYWAYWGQGTLVTVSSGGGGSGGGGSGGGGSQTVVTQEPSLTVSPGGTVTLTCA SSTGAVTSGNYPNWVQQKPGQAPRGLIGGTKFLVPGTPARFSGSLLGGKAALTLSGVQP EDEAEYYCTLWYSNRWVFGGGTKLTVLHHHHHH* |
| 194 | EPL189 | GGGSQVQLQESGGGLVQPGGSLRLSCAASGRINSINTMGWYRQAPGNQRELVAEITRGG TTNYADSVQGRYAISRDNAKNLVYLQMNSLKPEDTDVYYCNAQTFPTFSRPTGLDYWGQ GTQVTVSSGGGGSGGGSEVQLVESGGGLVQPGGSLKLSCAASGFTFNKYAINWVRQAPG KGLEWVARIRSKYNNYATYYADQVKDRFTISRDDSKNTAYLQMNNLKTEDTAVYYCVRH ANFGNSYISYWAYWGQGTLVTVSSGGGGSGGGGSGGGGSQTVVTQEPSLTVSPGGTVTL TCASSTGAVTSGNYPNWVQQKPGQAPRGLIGGTKFLVPGTPARFSGSLLGGKAALTLSG VQPEDEAEYYCTLWYSNRWVFGGGTKLTVLHHHHHH* |
| 195 | EPL2 | GGGSQVQLQESGGGLVQPGGSLRLSCAASESISSFIAVGWYRQAPGKERELVAGINRSG FTYYTDSVKGRFSISRDNAKNTVLLQMTSLKPEDTAVYYCNAGGLYFSNAYTQGDYWGQ GTQVTVSSGGGGSGGGSEVQLVESGGGLVQPGGSLKLSCAASGFTFNKYAINWVRQAPG KGLEWVARIRSKYNNYATYYADQVKDRFTISRDDSKNTAYLQMNNLKTEDTAVYYCVRH ANFGNSYISYWAYWGQGTLVTVSSGGGGSGGGGSGGGGSQTVVTQEPSLTVSPGGTVTL TCASSTGAVTSGNYPNWVQQKPGQAPRGLIGGTKFLVPGTPARFSGSLLGGKAALTLSG VQPEDEAEYYCTLWYSNRWVFGGGTKLTVLHHHHHH* |
| 196 | EPL20 | GGGSQVQLQESGGGLVQAGGSLKLSCAASGNVFRAATMAWYRQAPEKQREMVATIASGG TTNYADFVKGRFTISRDNAKNTVYLQMNTLKPEDTAVYYCNAGYLTSLGPKNYWGQGTQ VTVSSGGGGSGGGSEVQLVESGGGLVQPGGSLKLSCAASGFTFNKYAINWVRQAPGKGL EWVARIRSKYNNYATYYADQVKDRFTISRDDSKNTAYLQMNNLKTEDTAVYYCVRHANF GNSYISYWAYWGQGTLVTVSSGGGGSGGGGSGGGGSQTVVTQEPSLTVSPGGTVTLTCA SSTGAVTSGNYPNWVQQKPGQAPRGLIGGTKFLVPGTPARFSGSLLGGKAALTLSGVQP EDEAEYYCTLWYSNRWVFGGGTKLTVLHHHHHH* |
| 197 | EPL34 | GGGSQVQLQESGGGSVQAGGSLRLSCAASGFTFSSYYMSWVRQAPGKGLEWVSGIHYTG DWTNYADSVKGRFTISRDNAKNELYLEMNNLKPEDTAVYYCARGSDKGQGTQVTVSSGG GGSGGGSEVQLVESGGGLVQPGGSLKLSCAASGFTFNKYAINWVRQAPGKGLEWVARIR SKYNNYATYYADQVKDRFTISRDDSKNTAYLQMNNLKTEDTAVYYCVRHANFGNSYISY WAYWGQGTLVTVSSGGGGSGGGGSGGGGSQTVVTQEPSLTVSPGGTVTLTCASSTGAVT SGNYPNWVQQKPGQAPRGLIGGTKFLVPGTPARFSGSLLGGKAALTLSGVQPEDEAEYY CTLWYSNRWVFGGGTKLTVLHHHHHH* |
| 198 | EPL49 | GGGSQVQLQESGGGLVQAGGSLRLSCAPSGRTSSIFGMGWFRQAPGKEREFVASINWSG GSTSYADSVKGRFTISRDNAKNEMYLQMNSLKFEDTAVYVCAAAVLTNKPSWNFWGQGT QVTVSSGGGGSGGGSEVQLVESGGGLVQPGGSLKLSCAASGFTFNKYAINWVRQAPGKG LEWVARIRSKYNNYATYYADQVKDRFTISRDDSKNTAYLQMNNLKTEDTAVYYCVRHAN FGNSYISYWAYWGQGTLVTVSSGGGGSGGGGSGGGGSQTVVTQEPSLTVSPGGTVTLTC ASSTGAVTSGNYPNWVQQKPGQAPRGLIGGTKFLVPGTPARFSGSLLGGKAALTLSGVQ PEDEAEYYCTLWYSNRWVFGGGTKLTVLHHHHHH* |
| 199 | EPL58 | GGGSQVQLQESGGGLVQAGGSLRLSCAASGPIFSDTIRTMGWYRQAAGKQRELVATIAS FPSRTNYVDSVKGRFTISRDIAKNTVYLQMDSLKPEDTAVYYCNVDLASIPTKTYWGQG TQVTVSSGGGGSGGGSEVQLVESGGGLVQPGGSLKLSCAASGFTFNKYAINWVRQAPGK GLEWVARIRSKYNNYATYYADQVKDRFTISRDDSKNTAYLQMNNLKTEDTAVYYCVRHA NFGNSYISYWAYWGQGTLVTVSSGGGGSGGGGSGGGGSQTVVTQEPSLTVSPGGTVTLT CASSTGAVTSGNYPNWVQQKPGQAPRGLIGGTKFLVPGTPARFSGSLLGGKAALTLSGV QPEDEAEYYCTLWYSNRWVFGGGTKLTVLHHHHHH* |
| 200 | EPL74 | GGGSQVQLQESGGGLVQAGGSLRLSCAASGSIFGINAMGWYRQAPGKQRESVAFITIGG NTNYLDSVKGRFTISRDNAKNTVYLQMNGLKPEDTAVYYCNTNPPLILTAGGLYWGQGT QVTVSSGGGGSGGGSEVQLVESGGGLVQPGGSLKLSCAASGFTFNKYAINWVRQAPGKG LEWVARIRSKYNNYATYYADQVKDRFTISRDDSKNTAYLQMNNLKTEDTAVYYCVRHAN FGNSYISYWAYWGQGTLVTVSSGGGGSGGGGSGGGGSQTVVTQEPSLTVSPGGTVTLTC ASSTGAVTSGNYPNWVQQKPGQAPRGLIGGTKFLVPGTPARFSGSLLGGKAALTLSGVQ PEDEAEYYCTLWYSNRWVFGGGTKLTVLHHHHHH* |
| 201 | EPL78 | GGGSQVQLQESGGGLVQPGGSLRLSCATSANRFNINVMGWYRQAPGQQRELVATINIGG STDYADSVKGRFTISRDNAKNTVYLQLSDLKPEDTAVYYCNVKLRVSGPTGPNVYWGQG TQVTVSSGGGGSGGGSEVQLVESGGGLVQPGGSLKLSCAASGFTFNKYAINWVRQAPGK GLEWVARIRSKYNNYATYYADQVKDRFTISRDDSKNTAYLQMNNLKTEDTAVYYCVRHA NFGNSYISYWAYWGQGTLVTVSSGGGGSGGGGSGGGGSQTVVTQEPSLTVSPGGTVTLT CASSTGAVTSGNYPNWVQQKPGQAPRGLIGGTKFLVPGTPARFSGSLLGGKAALTLSGV QPEDEAEYYCTLWYSNRWVFGGGTKLTVLHHHHHH* |
| 202 | EPL82 | GGGSQVQLQESGGGLVQAGGSLKLSCTASGTILSTMAWYRQAPGKQRELVATISRGGTT NYSDSVKGRFAISRDSTKNTVYLQMNSLKPEDTAVYYCNTPLTDYGMGYNWGQGTQVTV SSGGGGSGGGSEVQLVESGGGLVQPGGSLKLSCAASGFTFNKYAINWVRQAPGKGLEWV ARIRSKYNNYATYYADQVKDRFTISRDDSKNTAYLQMNNLKTEDTAVYYCVRHANFGNS YISYWAYWGQGTLVTVSSGGGGSGGGGSGGGGSQTVVTQEPSLTVSPGGTVTLTCASST |
| | | GAVTSGNYPNWVQQKPGQAPRGLIGGTKFLVPGTPARFSGSLLGGKAALTLSGVQPEDE AEYYCTLWYSNRWVFGGGTKLTVLHHHHHH* |
| 203 | EPL83 | GGGSQVQLQESGGGLVQAGGSLRLSCAVSGSIFSLNTLAWYRQAPGRQRDLIARITGGG TTVYADSVKGRFTISRDNAKNTVYLQMNSLKPEDTAVYYCNLMVRHPSGSTYEYWGQGT QVTVSSGGGGSGGGSEVQLVESGGGLVQPGGSLKLSCAASGFTFNKYAINWVRQAPGKG LEWVARIRSKYNNYATYYADQVKDRFTISRDDSKNTAYLQMNNLKTEDTAVYYCVRHAN FGNSYISYWAYWGQGTLVTVSSGGGGSGGGGSGGGGSQTVVTQEPSLTVSPGGTVTLTC ASSTGAVTSGNYPNWVQQKPGQAPRGLIGGTKFLVPGTPARFSGSLLGGKAALTLSGVQ PEDEAEYYCTLWYSNRWVFGGGTKLTVLHHHHHH* |
| 204 | EPL86 | GGGSQVQLQESGGGLVQPGGSLRLSCAASGFTFSDWAMSWVRQAPGKGLEWVSGIHYGD HTTHYADFVKGRFTISRDDAKNTLYLQMNSLKPEDTAVYYCARGSTKGQGTQVTVSSGG GGSGGGSEVQLVESGGGLVQPGGSLKLSCAASGFTFNKYAINWVRQAPGKGLEWVARIR SKYNNYATYYADQVKDRFTISRDDSKNTAYLQMNNLKTEDTAVYYCVRHANFGNSYISY WAYWGQGTLVTVSSGGGGSGGGGSGGGGSQTVVTQEPSLTVSPGGTVTLTCASSTGAVT SGNYPNWVQQKPGQAPRGLIGGTKFLVPGTPARFSGSLLGGKAALTLSGVQPEDEAEYY CTLWYSNRWVFGGGTKLTVLHHHHHH* |
| 205 | EPL90 | GGGSQVQLQESGGGLVQAGGSLRLSCAASGFIFRAASMAWYRQSPGNERELVASISSGA FTNYADSVKARFTISRDNAKNTVYLQMNSLKPEDTAVYFCGATFLRSDGHHTINGQGTQ VTVSSGGGGSGGGSEVQLVESGGGLVQPGGSLKLSCAASGFTFNKYAINWVRQAPGKGL EWVARIRSKYNNYATYYADQVKDRFTISRDDSKNTAYLQMNNLKTEDTAVYYCVRHANF GNSYISYWAYWGQGTLVTVSSGGGGSGGGGSGGGGSQTVVTQEPSLTVSPGGTVTLTCA SSTGAVTSGNYPNWVQQKPGQAPRGLIGGTKFLVPGTPARFSGSLLGGKAALTLSGVQP EDEAEYYCTLWYSNRWVFGGGTKLTVLHHHHHH* |
| 206 | EPL97 | GGGSQVQLQESGGGLVQAGGSLRLSCAASGIIFRGTTMGWFRQAPGKQRESVASISPLG TTSYSGSVEGRFTVSRDNAKNTLFLQMNSLKSEDTAVYYCNAIQVTNVGPRVYWGQGTQ VTVSSGGGGSGGGSEVQLVESGGGLVQPGGSLKLSCAASGFTFNKYAINWVRQAPGKGL EWVARIRSKYNNYATYYADQVKDRFTISRDDSKNTAYLQMNNLKTEDTAVYYCVRHANF GNSYISYWAYWGQGTLVTVSSGGGGSGGGGSGGGGSQTVVTQEPSLTVSPGGTVTLTCA SSTGAVTSGNYPNWVQQKPGQAPRGLIGGTKFLVPGTPARFSGSLLGGKAALTLSGVQP EDEAEYYCTLWYSNRWVFGGGTKLTVLHHHHHH* |

| **SEQ ID NO:** | **Humanized Anti-EpCAM sequence name** | **Amino Acid Sequence** |
|---|---|---|
| 207 | H13 | EVQLVESGGGLVQPGGSLTLSCAASGFAFGNHWMYWYRQAPGRGRELVASISSGGSTNY VDSVKGRFTISRDNAKNTLYLQMNSLRAEDTAVYYCGTSDNWGQGTLVTVSS |
| 208 | H138 | EVQLLESGGGLVQPGGSLTLSCAASGFIFRAASMDWYRQFPGNERESIATISSGGFTNY ADSVKGRFTISRDNSKNTLYLQMNSLRAEDTAVYYCGATFLRSDGHHTINGQGTLVTVS S |
| 209 | H90 | EVQLLESGGGLVQPGGSLTLSCAASGFIFRAASMAWYRQSPGNERELVASISSGAFTNY ADSVKARFTISRDNSKNTLYLQMNSLRAEDTAVYYCGATFLRSDGHHTINGQGTLVTVS S |

| **SEQ ID NO:** | **Humanized Anti-CD3/Anti-EpCAM sequence name** | **Amino Acid Sequence** |
|---|---|---|
| 210 | H13 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSEVQLVESGGGLVQPGGSLTLSCAASGFAFG NHWMYWYRQAPGRGRELVASISSGGSTNYVDSVKGRFTISRDNAKNTLYLQMNSLRAED TAVYYCGTSDNWGQGTLVTVSSHHHHHH* |
| 211 | H138 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSEVQLLESGGGLVQPGGSLTLSCAASGFIFR AASMDWYRQFPGNERESIATISSGGFTNYADSVKGRFTISRDNSKNTLYLQMNSLRAED TAVYYCGATFLRSDGHHTINGQGTLVTVSSHHHHHH* |
| 212 | H90 | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSEVQLLESGGGLVQPGGSLTLSCAASGFIFR AASMAWYRQSPGNERELVASISSGAFTNYADSVKARFTISRDNSKNTLYLQMNSLRAED TAVYYCGATFLRSDGHHTINGQGTLVTVSSHHHHHH* |

| **Anti-EpCAM sequen ce name** | **SEQ ID No.** | **Framework 1 sequence** |
|---|---|---|
| 213 | EPL90 | QVQLQESGGGLVQAGGSLRLSCAAS |
| 214 | EPL118 | QVQLQESGGGLVQAGGSLRLSCAAS |
| 215 | EPL138 | QVQLQESGGGLVQAGGSLRLSCAAS |
| 216 | EPL145 | QVQLQESGGGLVQAGGSLRLSCAAS |
| 217 | EPL164 | QVQLQESGGGLVQAGGSLRLSCAAS |
| 218 | EPL31 | QVQLQESGGGLVHTGGSLRLSCAAS |
| 219 | EPL55 | QVQLQESGGGLVQAGGSLRLSCAAS |
| 220 | EPL57 | QVQLQESGGGLVHTGGSLRLSCAFS |
| 221 | EPL136 | QVQLQESGGGLVQPGGSLRLSCAAS |
| 222 | EPL15 | QVQLQESGGGSVLAGGSLRLSCAAS |
| 223 | EPL34 | QVQLQESGGGSVQAGGSLRLSCAAS |
| 224 | EPL86 | QVQLQESGGGLVQPGGSLRLSCAAS |
| 225 | EPL153 | QVQLQESGGGLVQPGGSLRLSCAAS |
| 226 | EPL20 | QVQLQESGGGLVQAGGSLKLSCAAS |
| 227 | EPL70 | QVQLQESGGGLVQPGGSLRLSCAAS |
| 228 | EPL125 | QVQLQESGGGLVQAGGSLRLSCAAS |
| 229 | EPL13 | QVQLQESGGGLVQPGGSLRLSCAAS |
| 230 | EPL129 | QVQLQESGGGLVQAGGSLRLSCAAS |
| 231 | EPL159 | QVQLQESGGGLVQAGGSLRLSCAAS |
| 232 | EPL120 | QVQLQESGGGLVQAGGSLRLSCAAS |
| 233 | EPL126 | QVQLQESGGGLVQAGGSLRLSCAAS |
| 234 | EPL60 | QVQLQESGGGLVQAGGSLRLSCAAS |
| 235 | EPL156 | QVQLQESGGGLVQPGASLRVSCAAS |
| 236 | EPL2 | QVQLQESGGGLVQPGGSLRLSCAAS |
| 237 | EPL43 | QVQLQESGGGLVQTGGSLRLSCAAS |
| 238 | EPL10 | QVQLQESGGGLVQAGGSLRLSCAAS |
| 239 | EPL49 | QVQLQESGGGLVQAGGSLRLSCAPS |
| 240 | EPL58 | QVQLQESGGGLVQAGGSLRLSCAAS |
| 241 | EPL74 | QVQLQESGGGLVQAGGSLRLSCAAS |
| 242 | EPL78 | QVQLQESGGGLVQPGGSLRLSCATS |
| 243 | EPL82 | QVQLQESGGGLVQAGGSLKLSCTAS |
| 244 | EPL83 | QVQLQESGGGLVQAGGSLRLSCAVS |
| 245 | EPL97 | QVQLQESGGGLVQAGGSLRLSCAAS |
| 246 | EPL109 | QVQLQESGGGLVQPGGSLRLSCASS |
| 247 | EPL117 | QVQLQESGGGLVQAGGSLRLSCAAS |
| 248 | EPL127 | QVQLQESGGGLVQAGGSLRLSCAAP |
| 249 | EPL152 | QVQLQESGGGLVQAGGSLRLSCVHS |
| 250 | EPL189 | QVQLQESGGGLVQPGGSLRLSCAAS |

| **Anti-EpCAM sequen ce name** | **SEQ ID No.** | **Framework 2 sequence** |
|---|---|---|
| EPL90 | 251 | WYRQSPGNERELVA |
| EPL118 | 252 | WFRQSPGNERELVA |
| EPL138 | 253 | WYRQFPGNERESIA |
| EPL145 | 254 | WFRQSPGNERELVA |
| EPL164 | 255 | WYRQSPGTQPELVA |
| EPL31 | 256 | WFRQAPGKEREFVA |
| EPL55 | 257 | WFRQAPGKEREFVA |
| EPL57 | 258 | WFRQAPGKEREFVA |
| EPL136 | 259 | WFRQAPGKEREFVA |
| EPL15 | 260 | WVRQAPGKGLEWVS |
| EPL34 | 261 | WVRQAPGKGLEWVS |
| EPL86 | 262 | WVRQAPGKGLEWVS |
| EPL153 | 263 | WVRQAPGKGLEWVS |
| EPL20 | 264 | WYRQAPEKQREMVA |
| EPL70 | 265 | WYRQAPEKXREMVA |
| EPL125 | 266 | WYRQVPEKQREMVA |
| EPL13 | 267 | WYRQAPGRGRELVA |
| EPL129 | 268 | WYRQAPGRGRELVA |
| EPL159 | 269 | WYRQAPGRGRELVA |
| EPL120 | 270 | WYRQSPGNERELVA |
| EPL126 | 271 | WYRQSPGNERELVA |
| EPL60 | 272 | WYRQAPGKVRELVA |
| EPL156 | 273 | WYRQAPGKVRELVA |
| EPL2 | 274 | WYRQAPGKERELVA |
| EPL43 | 275 | WYRQAPGKERELVA |
| EPL10 | 276 | WYRQAPGKQHELVA |
| EPL49 | 277 | WFRQAPGKEREFVA |
| EPL58 | 278 | WYRQAAGKQRELVA |
| EPL74 | 279 | WYRQAPGKQRESVA |
| EPL78 | 280 | WYRQAPGQQRELVA |
| EPL82 | 281 | WYRQAPGKQRELVA |
| EPL83 | 282 | WYRQAPGRQRDLIA |
| EPL97 | 283 | WFRQAPGKQRESVA |
| EPL109 | 284 | WFRQAPGKEREGVS |
| EPL117 | 285 | WYRQAPGKQREFVA |
| EPL127 | 286 | WFRQAPGKEREGVS |
| EPL152 | 287 | WYRQAPGKQRELVA |
| EPL189 | 288 | WYRQAPGNQRELVA |

| **Anti-EpCAM** | **SEQ ID No.** | **Framework 3 sequence** |
|---|---|---|
| **sequen ce name** | | |
| EPL90 | 289 | RFTISRDNAKNTVYLQMNSLKPEDTAVYFCGA |
| EPL118 | 290 | RFTISRDNAKNTVYLQMNSLKPEDTAVYFCGA |
| EPL138 | 291 | RFTISRDNAKNTVYLQMNSLKPEDTAVYFCGA |
| EPL145 | 292 | RFTISRDNAKNTVYLQMNSLKPEDTAVYFCGA |
| EPL164 | 293 | RFTISRDNAKNTVYLQMNSLKPEDTAVYFCGA |
| EPL31 | 294 | RFTISRDNTKNTVYLQMNSLKPEDTAVYYCAA |
| EPL55 | 295 | RFTISRDNTKNTVYLQMNSLKPEDTAVYYCAA |
| EPL57 | 296 | RFTISRDNTKNTVYLQMNSLRPEDTAVYYCAA |
| EPL136 | 297 | RFTISRDNTKNTVYLQMNSLKPEDTAVYYCAA |
| EPL15 | 298 | RFTISRDNAKNELYLEMNNLKPEDTAVYYCAR |
| EPL34 | 299 | RFTISRDNAKNELYLEMNNLKPEDTAVYYCAR |
| EPL86 | 300 | RFTISRDDAKNTLYLQMNSLKPEDTAVYYCAR |
| EPL153 | 301 | RFTISRDDAKNTLYLQMNSLKPEDTAVYYCEK |
| EPL20 | 302 | RFTISRDNAKNTVYLQMNTLKPEDTAVYYCNA |
| EPL70 | 303 | RFTISRDNAKNTVYLQMNTLKPEDTAVYYCNA |
| EPL125 | 304 | RFTISRDNAKNTVYLQMNTLKPEDTAVYYCNA |
| EPL13 | 305 | RFTISRDNARNTVYLQMYSLKPEDTAVYYCGT |
| EPL129 | 306 | RFTISRDNARNTVYLQMYSLRPEDTAVYYCGT |
| EPL159 | 307 | RFTISRDNARNTVYLQMYSLKPEDTAVYYCGT |
| EPL120 | 308 | RFTISRDNAKNTGYLQMNSLKPEDTAVYFCAA |
| EPL126 | 309 | RFTISRDNAKNTGYLQMNSLKPEDTAVYFCAA |
| EPL60 | 310 | RFTISRDNDRNTVYLQMNRLQPEDTAAYYCNV |
| EPL156 | 311 | RFTISRDNDRNTVYLQMNRLQPEDTAAYYCNV |
| EPL2 | 312 | RFSISRDNAKNTVLLQMTSLKPEDTAVYYCNA |
| EPL43 | 313 | RFSISRDNAKNTVLLQMTSLKPEDTAVYYCNA |
| EPL10 | 314 | RFTISRDNAKKTVYLQMNSLKPDDTAVYYCNA |
| EPL49 | 315 | RFTISRDNAKNEMYLQMNSLKFEDTAVYVCAA |
| EPL58 | 316 | RFTISRDIAKNTVYLQMDSLKPEDTAVYYCNV |
| EPL74 | 317 | RFTISRDNAKNTVYLQMNGLKPEDTAVYYCNT |
| EPL78 | 318 | RFTISRDNAKNTVYLQLSDLKPEDTAVYYCNV |
| EPL82 | 319 | RFAISRDSTKNTVYLQMNSLKPEDTAVYYCNT |
| EPL83 | 320 | RFTISRDNAKNTVYLQMNSLKPEDTAVYYCNL |
| EPL97 | 321 | RFTVSRDNAKNTLFLQMNSLKSEDTAVYYCNA |
| EPL109 | 322 | RFTISRDNAKNTVDLQMISLRPEDTAVYYCAA |
| EPL117 | 323 | RFTISRDNTKNTVYLQMNYLKPEDTAVYYCAT |
| EPL127 | 324 | RFTISSDNAKNTVYLQMNSVKTDDTAVYYCAV |
| EPL152 | 325 | RFTISRDNANNTVYLQMNSLKPEDTAVYYCNA |
| EPL189 | 326 | RYAISRDNAKNLVYLQMNSLKPEDTDVYYCNA |

| **Anti-EpCAM sequen ce name** | **SEQ ID No.** | **Framework 4 sequence** |
|---|---|---|
| EPL90 | 327 | NGQGTQVTVSS |
| EPL118 | 328 | YGQGTQVTVSS |
| EPL138 | 329 | NGQGTQVTVSS |
| EPL145 | 330 | YGQGTQVTVSS |
| EPL164 | 331 | YGQGTQVTVSS |
| EPL31 | 332 | WGQGTQVTVSS |
| EPL55 | 333 | WGQGTQVTVSS |
| EPL57 | 334 | WGQGTQVTVSS |
| EPL136 | 335 | WGQGTQVTVSS |
| EPL15 | 336 | KGQGTQVTVSS |
| EPL34 | 337 | KGQGTQVTVSS |
| EPL86 | 338 | KGQGTQVTVSS |
| EPL153 | 339 | RGQGTQVTVSS |
| EPL20 | 340 | WGQGTQVTVSS |
| EPL70 | 341 | WGQGTQVTVSS |
| EPL125 | 342 | WGQGTQVTVSS |
| EPL13 | 343 | WGQGTQVTVSS |
| EPL129 | 344 | WGQGTQVTVSS |
| EPL159 | 345 | WGQGTQVTVSS |
| EPL120 | 346 | WGQGTQVTVSS |
| EPL126 | 347 | WGQGTQVTVSS |
| EPL60 | 348 | WGQGTQVTVSS |
| EPL156 | 349 | WGQGTQVTVSS |
| EPL2 | 350 | WGQGTQVTVSS |
| EPL43 | 351 | WGQGTQVTVSS |
| EPL10 | 352 | WGQGTQVTVSS |
| EPL49 | 353 | WGQGTQVTVSS |
| EPL58 | 354 | WGQGTQVTVSS |
| EPL74 | 355 | WGQGTQVTVSS |
| EPL78 | 356 | WGQGTQVTVSS |
| EPL82 | 357 | WGQGTQVTVSS |
| EPL83 | 358 | WGQGTQVTVSS |
| EPL97 | 359 | WGQGTQVTVSS |
| EPL109 | 360 | QGQGTQVTVSS |
| EPL117 | 361 | WGHGTQVTVSS |
| EPL127 | 362 | WGQGTQVTVSS |
| EPL152 | 363 | WGQGTQVTVSS |
| EPL189 | 364 | WGQGTQVTVSS |

| **SEQ ID NO:** | **Description** | **Amino Acid Sequence** |
|---|---|---|
| 365 | Linker | (GS) n |
| 366 | Linker | (GGS) n |
| 367 | Linker | (GGGS) n |
| 368 | Linker | (GGSG) n |
| 369 | Linker | (GGSGG) n |
| 370 | Linker | (GGGGS) n |
| 371 | Linker | (GGGGG) n |
| 372 | Linker | (GGG) n |
| 373 | Linker | GGGGSGGGGSGGGGSGGGGS |
| 374 | Linker | GGGGSGGGGSGGGGS |
| 375 | Linker | GGGGSGGGS |
| 376 | Sortase | LPETG |
| 377 | Histidine tag | HHHHHH |
| 378 | Anti-HSA sdAb clone 10G | EVQLVESGGGLVQPGNSLRLSCAASGFTFSKFGMSWVRQAPGKGLEWVSSISGSGRDTL YADSVKGRFTISRDNAKTTLYLQMNSLRPEDTAVYYCTIGGSLSVSSQGTLVTVSS |
| 379 | Anti-CD3, clone 2B2 | EVQLVESGGGLVQPGGSLKLSCAASGFTFNKYAINWVRQAPGKGLEWVARIRSKYNNYA TYYADQVKDRFTISRDDSKNTAYLQMNNLKTEDTAVYYCVRHANFGNSYISYWAYWGQG TLVTVSSGGGGSGGGGSGGGGSQTVVTQEPSLTVSPGGTVTLTCASSTGAVTSGNYPNW VQQKPGQAPRGLIGGTKFLVPGTPARFSGSLLGGKAALTLSGVQPEDEAEYYCTLWYSN RWVFGGGTKLTVL |
| 380 | Exemplary masking sequence | GGGGGLDGNEEPGG |
| 381 | Exemplary masking sequence | APGKG |
| 382 | Exemplary masking sequence | GGQDGNEEMGGG |
| 383 | Exemplary masking sequence | GGQDGNEEGG |
| 384 | Exemplary masking sequence | GGGGQDGNEEMGGGGG |
| 385 | Exemplary masking sequence | APFGSEM |
| 386 | Exemplary masking sequence | AWNGPYE |
| 387 | Exemplary masking sequence | AQDNGDTKTG |
| 388 | Exemplary masking sequence | AEAKETQG |
| 389 | Exemplary masking sequence | ATRREQVEG |
| 390 | Exemplary masking sequence | AQAPSQP |
| 391 | Exemplary masking sequence | ATRSRTRNDG |
| 392 | Exemplary masking sequence | ADVDAPDGLG |
| 393 | Exemplary masking sequence | AADISDPGG |
| 394 | Exemplary masking sequence | ALSVDPSG |
| 395 | Exemplary masking sequence | ARLSVDPG |
| 396 | Exemplary masking sequence | AVEAADRG |
| 397 | Exemplary masking sequence | GGPDGNEEMGGG |
| 398 | Exemplary masking sequence | GGFDGNEEMGGG |
| 399 | Exemplary masking sequence | GGGDGNEEMGGG |
| 400 | Exemplary masking sequence | GGEMDGEGQNGG |
| 401 | Exemplary masking sequence | GGGGGPDGNEEPGG |
| 402 | Exemplary masking sequence | GGGGSLDGNEEPGG |
| 403 | Exemplary masking sequence | GGGGALDGNEEPGG |
| 404 | Exemplary masking sequence | GGGGGLDGNEEPGG |
| 405 | Exemplary masking sequence | GGGALDGNEEPGG |
| 406 | Exemplary masking sequence | GGGGGPDGNEEPGGG |
| 407 | Exemplary masking sequence | GGSGALDGNEEPGG |
| 408 | Exemplary masking sequence | GGSGSLDGNEEPGG |
| 409 | Exemplary masking sequence | GGSGGPDGNEEPGG |
| 410 | Exemplary masking sequence | GGVRDGPDGNEEPGG |
| 411 | Exemplary masking sequence | GGSGGPDGNEEPGGGG |
| 412 | Exemplary masking sequence | GGGRGPDGNEEPGG |
| 413 | Exemplary masking sequence | GGSGGLDGNEEPGG |
| 414 | Exemplary masking sequence | GGGVGPDGNEEPGG |
| 415 | Exemplary masking sequence | GGGEGPDGNEEPGG |
| 416 | Exemplary masking sequence | GGGVALDGNEEPGG |
| 417 | Exemplary masking sequence | GGGRALDGNEEPGG |
| 418 | Exemplary masking sequence | GGYAGLDGNEEPGG |
| 419 | Exemplary masking sequence | GGAGGPDGNEEPGG |
| 420 | Exemplary masking sequence | GGRGGPDGNEEPGG |
| 421 | Exemplary masking sequence | GGGGPDGNEEPGGGG |
| 422 | Exemplary masking sequence | GGGEALDGNEEPGG |
| 423 | Exemplary masking sequence | GGDASLDGNEEPGG |
| 424 | Exemplary masking sequence | GGRDAPDGNEEGG |
| 425 | Exemplary cleavable linker sequence | KRALGLPG |
| 426 | Exemplary cleavable linker sequence | (DE)₈RPLALWRS(DR)₈ |
| 427 | Exemplary cleavable linker sequence | PR(S/T) (L/I) (S/T) |
| 428 | Exemplary cleavable linker sequence | LEATA |
| 429 | Exemplary cleavable linker sequence | GGAANLVRGG |
| 430 | Exemplary cleavable linker sequence | SGRIGFLRTA |
| 431 | Exemplary cleavable linker sequence | PLGLAG |
| 432 | Exemplary cleavable linker sequence | PLGLAX |
| 433 | Exemplary cleavable linker sequence | PLGC(me)AG |
| 434 | Exemplary cleavable linker sequence | ESPAYYTA |
| 435 | Exemplary cleavable linker sequence | RLQLKL |
| 436 | Exemplary cleavable linker sequence | RLQLKAC |
| 437 | Exemplary cleavable linker sequence | EP(Cit)G(Hof)YL |
| 438 | Exemplary cleavable linker sequence | SGRSA |
| 439 | Exemplary cleavable linker sequence | DAFK |
| 440 | Exemplary cleavable linker sequence | GGGRR |
| 441 | Exemplary cleavable linker sequence | GFLG |
| 442 | Exemplary cleavable linker sequence | ALAL |
| 443 | Exemplary cleavable linker sequence | FK |
| 444 | Exemplary cleavable linker sequence | NLL |
| 445 | Exemplary cleavable linker sequence | PIC (Et) FF |
| 446 | Exemplary cleavable linker sequence | GGPRGLPG |
| 447 | Exemplary cleavable linker sequence | HSSKLQ |
| 448 | Exemplary cleavable linker sequence | HSSKLQL |
| 449 | Exemplary cleavable linker sequence | HSSKLQEDA |
| 450 | Exemplary cleavable linker sequence | LVLASSSFGY |
| 451 | Exemplary cleavable linker sequence | GVSQNYPIVG |
| 452 | Exemplary cleavable linker sequence | GVVQASCRLA |
| 453 | Exemplary cleavable linker sequence | F(Pip)RS |
| 454 | Exemplary cleavable linker sequence | DPRSFL |
| 455 | Exemplary cleavable linker sequence | PPRSFL |
| 456 | Exemplary cleavable linker sequence | DEVD |
| 457 | Exemplary cleavable linker sequence | DEVDP |
| 458 | Exemplary cleavable linker sequence | KGSGDVEG |
| 459 | Exemplary cleavable linker sequence | GWEHDG |
| 460 | Exemplary cleavable linker sequence | EDDDDKA |
| 461 | Exemplary cleavable linker sequence | KQEQNPGST |
| 462 | Exemplary cleavable linker sequence | GKAFRR |
| 463 | Exemplary cleavable linker sequence | DAFK |
| 464 | Exemplary cleavable linker sequence | DVLK |
| 465 | Exemplary cleavable linker sequence | DAFK |
| 466 | Exemplary cleavable linker sequence | ALLLALL |
| 467 | Exemplary cleavable linker sequence | KPLGLQARVV |
| 468 | Exemplary cleavable linker sequence | PQASTGRSGG |
| 469 | Exemplary cleavable linker sequence | PQGSTGRAAG |
| 470 | Exemplary cleavable linker sequence | PPASSGRAGG |
| 471 | Exemplary cleavable linker sequence | PIPVQGRAH |
| 472 | Exemplary anti-albumin (HSA) sequence with masking moiety (identified in bold and italics) | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSS |
| 473 | Exemplary anti-albumin (HSA) sequence with masking moiety (identified in bold and italics) and cleavable linker (identified in bold and underline) | EVQLVESGGGLVQPGNSLRLSCAASGFTFSKFGMSWVRQ***GGGGGLDGNEEPGG***LEWVSS ISGSGRDTLYADSVKGRFTISRDNAKTTLYLQMNSLRPEDTAVYYCTIGGSLSVSSQGT LVTVSSGGGG**KPLGLQARVV**GGGGT |
| 474 | Exemplary anti-CD3 scFv of a proTriTAC molecule containing an anti-EpCAM binding domain of this disclosure | QTVVTQEPSLTVSPGGTVTLTCASSTGAVTSGNYPNWVQQKPGQAPRGLIGGTKFLVPG TPARFSGSLLGGKAALTLSGVQPEDEAEYYCTLWYSNRWVFGGGTKLTVLGGGGSGGGG SGGGGSEVQLVESGGGLVQPGGSLKLSCAASGFTFNKYAINWVRQAPGKGLEWVARIRS KYNNYATYYADQVKDRFTISRDDSKNTAYLQMNNLKTEDTAVYYCVRHANFGNSYISYW AYWGQGTLVTVSS |
| 475 | Human EpCAM | >tr;B5MCA4;B5MCA4_HUMAN Epithelial cell adhesion molecule OS=Homo sapiens OX=9606 GN=EPCAM PE=1 SV=1 METKHLGRGGAGRAGPHLWRGPRPNCSAGAGGGEPTHSPNSRAVTHQRAPAARECVCEN YKLAVNCFVNNNRQCQCTSVGAQNTVICSKLAAKCLVMKAEMNGSKLGRRAKPEGALQN NDGLYDPDCDESGLFKAKQCNGTSMCWCVNTAGVRRTDKDTEITCSERVRTYWIIIELK HKAREKPYDSKSLRTALQKEITTRYQLDPKFITSILYENNVITIDLVQNSSQKTQNDVD IADVAYYFEKDVKGESLFHSKKMDLTVNGEQLDLDPGQTLIYYVDEKAPEFSMQGLKAG VIAVIVVVVIAVVAGIVVLVISRKKRMAKYEKAEIKEMGEMHRELNA |
| 476 | Cynomolgus EpCAM | OS=Macaca fascicularis OX=9541 GN=EPCAM PE=4 SV=1 CVCENYKLAVNCFLNDNGQCQCTSIGAQNTVLCSKLAAKCLVMKAEMNGSKLGRRAKPE GALQNNDGLYDPDCDESGLFKAKQCNGTSTCWCVNTAGVRRTDKDTEITCSERVRTYWI IIELKHKAREKPYDVQSLRTALEEAIKTRYQLDPKFITNILYEDNVITIDLVQNSSQKT QNDVDIADVAYYFEKDVKGESLFHSKKMDLRVNGEQLDLDPGQTLIYYVDEKAPEFSMQ GLKAGVIAVIVVVVIAIVAGIVVLVISRKKRMAKYEKAEIKEMGEIHRELNA |
| 477 | Mouse EpCAM | >sp;Q99JW5;EPCAM_MOUSE Epithelial cell adhesion molecule OS=Mus musculus OX=10090 GN=Epcam PE=1 SV=1 MAGPQALAFGLLLAVVTATLAAAQRDCVCDNYKLATSCSLNEYGECQCTSYGTQNTVIC SKLASKCLAMKAEMTHSKSGRRIKPEGAIQNNDGLYDPDCDEQGLFKAKQCNGTATCWC VNTAGVRRTDKDTEITCSERVRTYWIIIELKHKERESPYDHQSLQTALQEAFTSRYKLN QKFIKNIMYENNVITIDLMQNSSQKTQDDVDIADVAYYFEKDVKGESLFHSSKSMDLRV NGEPLDLDPGQTLIYYVDEKAPEFSMQGLTAGIIAVIVVVSLAVIAGIVVLVISTRKKS AKYEKAEIKEMGEIHRELNA |
| 478 | Human EpCAM | >sp;P16422;EPCAM_HUMAN Epithelial cell adhesion molecule OS=Homo sapiens OX=9606 GN=EPCAM PE=1 SV=2 MAPPQVLAFGLLLAAATATFAAAQEECVCENYKLAVNCFVNNNRQCQCTSVGAQNTVIC SKLAAKCLVMKAEMNGSKLGRRAKPEGALQNNDGLYDPDCDESGLFKAKQCNGTSMCWC VNTAGVRRTDKDTEITCSERVRTYWIIIELKHKAREKPYDSKSLRTALQKEITTRYQLD PKFITSILYENNVITIDLVQNSSQKTQNDVDIADVAYYFEKDVKGESLFHSKKMDLTVN GEQLDLDPGQTLIYYVDEKAPEFSMQGLKAGVIAVIVVVVIAVVAGIVVLVISRKKRMA KYEKAEIKEMGEMHRELNA |
| 479 | Human EpCAM extracellul ar domain sequence | QEECVCENYKLAVNCFVNNNRQCQCTSVGAQNTVICSKLAAKCLVMKAEMNGSKLGRRA KPEGALQNNDGLYDPDCDESGLFKAKQCNGTSMCWCVNTAGVRRTDKDTEITCSERVRT YWIIIELKHKAREKPYDSKSLRTALQKEITTRYQLDPKFITSILYENNVITIDLVQNSS QKTQNDVDIADVAYYFEKDVKGESLFHSKKMDLTVNGEQLDLDPGQTLIYYVDEKAPEF SMQGLK |
| 480 | Cynomolgus EpCAM extracellul ar domain sequence | QKECVCENYKLAVNCFLNDNGQCQCTSIGAQNTVLCSKLAAKCLVMKAEMNGSKLGRRA KPEGALQNNDGLYDPDCDESGLFKAKQCNGTSTCWCVNTAGVRRTDKDTEITCSERVRT YWIIIELKHKAREKPYDVQSLRTALEEAIKTRYQLDPKFITNILYEDNVITIDLVQNSS QKTQNDVDIADVAYYFEKDVKGESLFHSKKMDLRVNGEQLDLDPGQTLIYYVDEKAPEF SMQGLK |
| 481 | Mouse EpCAM extracellular domain sequence | QRDCVCDNYKLATSCSLNEYGECQCTSYGTQNTVICSKLASKCLAMKAEMTHSKSGRRI KPEGAIQNNDGLYDPDCDEQGLFKAKQCNGTATCWCVNTAGVRRTDKDTEITCSERVRT YWIIIELKHKERESPYDHQSLQTALQEAFTSRYKLNQKFIKNIMYENNVITIDLMQNSS |
| | | QKTQDDVDIADVAYYFEKDVKGESLFHSSKSMDLRVNGEPLDLDPGQTLIYYVDEKAPE FSMQGLT |
| 482 | 10G sdAb H90 Mask 1 | EVQLVESGGGLVQPGNSLRLSCAASGFTFSKFGMSWVRQRQSTFMAMDMPILEWVSSIS GSGRDTLYADSVKGRFTISRDNAKTTLYLQMNSLRPEDTAVYYCTIGGSLSVSSQGTLV TVSS |
| 483 | 10G sdAb H90 Mask 2 | EVQLVESGGGLVQPGNSLRLSCAASGFTFSKFGMSWVRQQAYWWGNSRDLEWVSSISGS GRDTLYADSVKGRFTISRDNAKTTLYLQMNSLRPEDTAVYYCTIGGSLSVSSQGTLVTV SS |
| 484 | H90 sdAb | EVQLLESGGGLVQPGGSLTLSCAASGFIFRAASMAWYRQSPGNERELVASISSGAFTNY ADSVKARFTISRDNSKNTLYLQMNSLRAEDTAVYYCGATFLRSDGHHTINGQGTLVTVS S |
| 485 | Exemplary construct | MALPVTALLLPLALLLHAARPEVQLLESGGGLVQPGGSLTLSCAASGFIFRAASMAWYR QSPGNERELVASISSGAFTNYADSVKARFTISRDNSKNTLYLQMNSLRAEDTAVYYCGA TFLRSDGHHTINGQGTLVTVSSTSDYKDDDDKTTTPAPRPPTPAPTIASQPLSLRPEAC RPAAGGAVHTRGLDFACDIYIWAPLAGTCGVLLLSLVITLYKRGRKKLLYIFKQPFMRP VQTTQEEDGCSCRFPEEEEGGCELRVKFSRSADAPAYQQGQNQLYNELNLGRREEYDVL DKRRGRDPEMGGKPRRKNPQEGLYNELQKDKMAEAYSEIGMKGERRRGKGHDGLYQGLS TATKDTYDALHMQALPPR |
| 486 | Exemplary construct | MALPVTALLLPLALLLHAARPEVQLVESGGGLVQPGNSLRLSCAASGFTFSKFGMSWVR QAPGKGLEWVSSISGSGRDTLYADSVKGRFTISRDNAKTTLYLQMNSLRPEDTAVYYCT IGGSLSVSSQGTLVTVSSGSGGSGGGGSGGGGGSGEVQLLESGGGLVQPGGSLTLSCAA SGFIFRAASMAWYRQSPGNERELVASISSGAFTNYADSVKARFTISRDNSKNTLYLQMN SLRAEDTAVYYCGATFLRSDGHHTINGQGTLVTVSSTSDYKDDDDKTTTPAPRPPTPAP TIASQPLSLRPEACRPAAGGAVHTRGLDFACDIYIWAPLAGTCGVLLLSLVITLYKRGR KKLLYIFKQPFMRPVQTTQEEDGCSCRFPEEEEGGCELRVKFSRSADAPAYQQGQNQLY NELNLGRREEYDVLDKRRGRDPEMGGKPRRKNPQEGLYNELQKDKMAEAYSEIGMKGER RRGKGHDGLYQGLSTATKDTYDALHMQALPPR |
| 487 | Exemplary construct | MALPVTALLLPLALLLHAARPEVQLVESGGGLVQPGNSLRLSCAASGFTFSKFGMSWVR QRQSTFMAMDMPILEWVSSISGSGRDTLYADSVKGRFTISRDNAKTTLYLQMNSLRPED TAVYYCTIGGSLSVSSQGTLVTVSSGSGGSGGGGSGGGGGSGEVQLLESGGGLVQPGGS LTLSCAASGFIFRAASMAWYRQSPGNERELVASISSGAFTNYADSVKARFTISRDNSKN TLYLQMNSLRAEDTAVYYCGATFLRSDGHHTINGQGTLVTVSSTSDYKDDDDKTTTPAP RPPTPAPTIASQPLSLRPEACRPAAGGAVHTRGLDFACDIYIWAPLAGTCGVLLLSLVI TLYKRGRKKLLYIFKQPFMRPVQTTQEEDGCSCRFPEEEEGGCELRVKFSRSADAPAYQ QGQNQLYNELNLGRREEYDVLDKRRGRDPEMGGKPRRKNPQEGLYNELQKDKMAEAYSE IGMKGERRRGKGHDGLYQGLSTATKDTYDALHMQALPPR |
| 488 | Exemplary construct | MALPVTALLLPLALLLHAARPEVQLVESGGGLVQPGNSLRLSCAASGFTFSKFGMSWVR QQAYWWGNSRDLEWVSSISGSGRDTLYADSVKGRFTISRDNAKTTLYLQMNSLRPEDTA VYYCTIGGSLSVSSQGTLVTVSSGSGGSGGGGSGGGGGSGEVQLLESGGGLVQPGGSLT LSCAASGFIFRAASMAWYRQSPGNERELVASISSGAFTNYADSVKARFTISRDNSKNTL YLQMNSLRAEDTAVYYCGATFLRSDGHHTINGQGTLVTVSSTSDYKDDDDKTTTPAPRP PTPAPTIASQPLSLRPEACRPAAGGAVHTRGLDFACDIYIWAPLAGTCGVLLLSLVITL YKRGRKKLLYIFKQPFMRPVQTTQEEDGCSCRFPEEEEGGCELRVKFSRSADAPAYQQG QNQLYNELNLGRREEYDVLDKRRGRDPEMGGKPRRKNPQEGLYNELQKDKMAEAYSEIG MKGERRRGKGHDGLYQGLSTATKDTYDALHMQALPPR |
| 489 | Exemplary construct | MALPVTALLLPLALLLHAARPEVQLVESGGGLVQPGNSLRLSCAASGFTFSKFGMSWVR QRQSTFMAMDMPILEWVSSISGSGRDTLYADSVKGRFTISRDNAKTTLYLQMNSLRPED TAVYYCTIGGSLSVSSQGTLVTVSSGGGGPQGSTGRAAGGGGGTEVQLLESGGGLVQPG GSLTLSCAASGFIFRAASMAWYRQSPGNERELVASISSGAFTNYADSVKARFTISRDNS KNTLYLQMNSLRAEDTAVYYCGATFLRSDGHHTINGQGTLVTVSSTSDYKDDDDKTTTP APRPPTPAPTIASQPLSLRPEACRPAAGGAVHTRGLDFACDIYIWAPLAGTCGVLLLSL VITLYKRGRKKLLYIFKQPFMRPVQTTQEEDGCSCRFPEEEEGGCELRVKFSRSADAPA YQQGQNQLYNELNLGRREEYDVLDKRRGRDPEMGGKPRRKNPQEGLYNELQKDKMAEAY SEIGMKGERRRGKGHDGLYQGLSTATKDTYDALHMQALPPR |
| 490 | Exemplary construct | MALPVTALLLPLALLLHAARPEVQLVESGGGLVQPGNSLRLSCAASGFTFSKFGMSWVR QQAYWWGNSRDLEWVSSISGSGRDTLYADSVKGRFTISRDNAKTTLYLQMNSLRPEDTA VYYCTIGGSLSVSSQGTLVTVSSGGGGPQGSTGRAAGGGGGTEVQLLESGGGLVQPGGS LTLSCAASGFIFRAASMAWYRQSPGNERELVASISSGAFTNYADSVKARFTISRDNSKN TLYLQMNSLRAEDTAVYYCGATFLRSDGHHTINGQGTLVTVSSTSDYKDDDDKTTTPAP RPPTPAPTIASQPLSLRPEACRPAAGGAVHTRGLDFACDIYIWAPLAGTCGVLLLSLVI |
| | | TLYKRGRKKLLYIFKQPFMRPVQTTQEEDGCSCRFPEEEEGGCELRVKFSRSADAPAYQ QGQNQLYNELNLGRREEYDVLDKRRGRDPEMGGKPRRKNPQEGLYNELQKDKMAEAYSE IGMKGERRRGKGHDGLYQGLSTATKDTYDALHMQALPPR |
| 491 | Exemplary construct | MALPVTALLLPLALLLHAARPQVQLVESGGALVQPGGSLRLSCAASGFPVNRYSMRWYR QAPGKEREWVAGMSSAGDRSSYEDSVKGRFTISRDDARNTVYLQMNSLKPEDTAVYYCN VNVGFEYWGQGTQVTVSSTSDYKDDDDKTTTPAPRPPTPAPTIASQPLSLRPEACRPAA GGAVHTRGLDFACDIYIWAPLAGTCGVLLLSLVITLYKRGRKKLLYIFKQPFMRPVQTT QEEDGCSCRFPEEEEGGCELRVKFSRSADAPAYQQGQNQLYNELNLGRREEYDVLDKRR GRDPEMGGKPRRKNPQEGLYNELQKDKMAEAYSEIGMKGERRRGKGHDGLYQGLSTATK DTYDALHMQALPPR |
| 492 | EpCAM TriTAC | EVQLVESGGGLVQPGNSLRLSCAASGFTFSKFGMSWVRQAPGKGLEWVSSISGSGRDTL YADSVKGRFTISRDNAKTTLYLQMNSLRPEDTAVYYCTIGGSLSVSSQGTLVTVSSGGG GSGGGSQTVVTQEPSLTVSPGGTVTLTCASSTGAVTSGNYPNWVQQKPGQAPRGLIGGT KFLVPGTPARFSGSLLGGKAALTLSGVQPEDEAEYYCTLWYSNRWVFGGGTKLTVLGGG GSGGGGSGGGGSEVQLVESGGGLVQPGGSLKLSCAASGFTFNKYAINWVRQAPGKGLEW VARIRSKYNNYATYYADQVKDRFTISRDDSKNTAYLQMNNLKTEDTAVYYCVRHANFGN SYISYWAYWGQGTLVTVSSGGGGSGGGSEVQLLESGGGLVQPGGSLTLSCAASGFIFRA ASMAWYRQSPGNERELVASISSGAFTNYADSVKARFTISRDNSKNTLYLQMNSLRAEDT AVYYCGATFLRSDGHHTINGQGTLVTVSSHHHHHH |
| 493 | GFP TriTAC | QVQLVESGGALVQPGGSLRLSCAASGFPVNRYSMRWYRQAPGKEREWVAGMSSAGDRSS YEDSVKGRFTISRDDARNTVYLQMNSLKPEDTAVYYCNVNVGFEYWGQGTQVTVSSGGG GSGGGSEVQLVESGGGLVQPGNSLRLSCAASGFTFSKFGMSWVRQAPGKGLEWVSSISG SGRDTLYADSVKGRFTISRDNAKTTLYLQMNSLRPEDTAVYYCTIGGSLSVSSQGTLVT VSSGGGGSGGGSEVQLVESGGGLVQPGGSLKLSCAASGFTFNKYAINWVRQAPGKGLEW VARIRSKYNNYATYYADQVKDRFTISRDDSKNTAYLQMNNLKTEDTAVYYCVRHANFGN SYISYWAYWGQGTLVTVSSGGGGSGGGGSGGGGSQTVVTQEPSLTVSPGGTVTLTCASS TGAVTSGNYPNWVQQKPGQAPRGLIGGTKFLVPGTPARFSGSLLGGKAALTLSGVQPED EAEYYCTLWYSNRWVFGGGTKLTVLHHHHHH |
| 494 | EpCAM ProTriTAC L040 (CT) | QTVVTQEPSLTVSPGGTVTLTCASSTGAVTSGNYPNWVQQKPGQAPRGLIGGTKFLVPG TPARFSGSLLGGKAALTLSGVQPEDEAEYYCTLWYSNRWVFGGGTKLTVLGGGGSGGGG SGGGGSEVQLVESGGGLVQPGGSLKLSCAASGFTFNKYAINWVRQAPGKGLEWVARIRS KYNNYATYYADQVKDRFTISRDDSKNTAYLQMNNLKTEDTAVYYCVRHANFGNSYISYW AYWGQGTLVTVSSGGGGSGGGSEVQLLESGGGLVQPGGSLTLSCAASGFIFRAASMAWY RQSPGNERELVASISSGAFTNYADSVKARFTISRDNSKNTLYLQMNSLRAEDTAVYYCG ATFLRSDGHHTINGQGTLVTVSSHHHHHH |
| 495 | EpCAM ProTriTAC non-cleavable | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSSGGGGSGGGGSGGVVGGGGTQTVVTQEPSLTVSPGGTVTLTCASSTGAVTSGNY PNWVQQKPGQAPRGLIGGTKFLVPGTPARFSGSLLGGKAALTLSGVQPEDEAEYYCTLW YSNRWVFGGGTKLTVLGGGGSGGGGSGGGGSEVQLVESGGGLVQPGGSLKLSCAASGFT FNKYAINWVRQAPGKGLEWVARIRSKYNNYATYYADQVKDRFTISRDDSKNTAYLQMNN LKTEDTAVYYCVRHANFGNSYISYWAYWGQGTLVTVSSGGGGSGGGSEVQLLESGGGLV QPGGSLTLSCAASGFIFRAASMAWYRQSPGNERELVASISSGAFTNYADSVKARFTISR DNSKNTLYLQMNSLRAEDTAVYYCGATFLRSDGHHTINGQGTLVTVSSHHHHHH |

| **SEQ ID NO:** | **Anti-EpCAM sequence name** | **Amino Acid Sequence** |
|---|---|---|
| 496 | H138.2 | EVQLLESGGGLVQPGGSLTLSCAASGFIFRAASMDWYRQFPGNERESIATISSGGFTNY ADSVKGRFTISRDNSKNTLYLQMNSLRAEDTAVYYCGATFLRSDGHHTIWGQGTLVTVS S |
| 497 | H90.2 | EVQLLESGGGLVQPGGSLTLSCAASGFIFRAASMAWYRQSPGNERELVASISSGAFTNY ADSVKARFTISRDNSKNTLYLQMNSLRAEDTAVYYCGATFLRSDGHHTIWGQGTLVTVS S |

| **SEQ ID NO:** | **EpCAM ProTriTAC sequence (Anti-Alb: Anti-CD3:Anti-EpCAM)** | **Amino Acid Sequence** |
|---|---|---|
| 498 | Exemplary EpCAM ProTriTAC (L040) (H90. 2) | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSSGGGGPQASTGRSGGGGGGTQTVVTQEPSLTVSPGGTVTLTCASSTGAVTSGNY PNWVQQKPGQAPRGLIGGTKFLVPGTPARFSGSLLGGKAALTLSGVQPEDEAEYYCTLW YSNRWVFGGGTKLTVLGGGGSGGGGSGGGGSEVQLVESGGGLVQPGGSLKLSCAASGFT FNKYAINWVRQAPGKGLEWVARIRSKYNNYATYYADQVKDRFTISRDDSKNTAYLQMNN LKTEDTAVYYCVRHANFGNSYISYWAYWGQGTLVTVSSGGGGSGGGSEVQLLESGGGLV QPGGSLTLSCAASGFIFRAASMAWYRQSPGNERELVASISSGAFTNYADSVKARFTISR DNSKNTLYLQMNSLRAEDTAVYYCGATFLRSDGHHTIWGQGTLVTVSSHHHHHH |
| 499 | Exemplary EpCAM ProTriTAC (L041) (H90. 2) | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSSGGGGPQGSTGRAAGGGGGTQTVVTQEPSLTVSPGGTVTLTCASSTGAVTSGNY PNWVQQKPGQAPRGLIGGTKFLVPGTPARFSGSLLGGKAALTLSGVQPEDEAEYYCTLW YSNRWVFGGGTKLTVLGGGGSGGGGSGGGGSEVQLVESGGGLVQPGGSLKLSCAASGFT FNKYAINWVRQAPGKGLEWVARIRSKYNNYATYYADQVKDRFTISRDDSKNTAYLQMNN LKTEDTAVYYCVRHANFGNSYISYWAYWGQGTLVTVSSGGGGSGGGSEVQLLESGGGLV QPGGSLTLSCAASGFIFRAASMAWYRQSPGNERELVASISSGAFTNYADSVKARFTISR DNSKNTLYLQMNSLRAEDTAVYYCGATFLRSDGHHTIWGQGTLVTVSSHHHHHH |
| 500 | Exemplary EpCAM ProTriTAC (L043) (H90. 2) | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSSGGGGPQGSTARSAGGGGGTQTVVTQEPSLTVSPGGTVTLTCASSTGAVTSGNY PNWVQQKPGQAPRGLIGGTKFLVPGTPARFSGSLLGGKAALTLSGVQPEDEAEYYCTLW YSNRWVFGGGTKLTVLGGGGSGGGGSGGGGSEVQLVESGGGLVQPGGSLKLSCAASGFT FNKYAINWVRQAPGKGLEWVARIRSKYNNYATYYADQVKDRFTISRDDSKNTAYLQMNN LKTEDTAVYYCVRHANFGNSYISYWAYWGQGTLVTVSSGGGGSGGGSEVQLLESGGGLV QPGGSLTLSCAASGFIFRAASMAWYRQSPGNERELVASISSGAFTNYADSVKARFTISR DNSKNTLYLQMNSLRAEDTAVYYCGATFLRSDGHHTIWGQGTLVTVSSHHHHHH* |
| 501 | Exemplary EpCAM ProTriTAC (L001) (H90. 2) | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSSGGGGKPLGLQARVVGGGGTQTVVTQEPSLTVSPGGTVTLTCASSTGAVTSGNY PNWVQQKPGQAPRGLIGGTKFLVPGTPARFSGSLLGGKAALTLSGVQPEDEAEYYCTLW YSNRWVFGGGTKLTVLGGGGSGGGGSGGGGSEVQLVESGGGLVQPGGSLKLSCAASGFT FNKYAINWVRQAPGKGLEWVARIRSKYNNYATYYADQVKDRFTISRDDSKNTAYLQMNN LKTEDTAVYYCVRHANFGNSYISYWAYWGQGTLVTVSSGGGGSGGGSEVQLLESGGGLV QPGGSLTLSCAASGFIFRAASMAWYRQSPGNERELVASISSGAFTNYADSVKARFTISR DNSKNTLYLQMNSLRAEDTAVYYCGATFLRSDGHHTINGQGTLVTVSSHHHHHH |
| 502 | Exemplary EpCAM non-cleavable prodrug (NCLV) (H90.2) | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSSGGGGSGGGGSGGVVGGGGTQTVVTQEPSLTVSPGGTVTLTCASSTGAVTSGNY PNWVQQKPGQAPRGLIGGTKFLVPGTPARFSGSLLGGKAALTLSGVQPEDEAEYYCTLW YSNRWVFGGGTKLTVLGGGGSGGGGSGGGGSEVQLVESGGGLVQPGGSLKLSCAASGFT FNKYAINWVRQAPGKGLEWVARIRSKYNNYATYYADQVKDRFTISRDDSKNTAYLQMNN LKTEDTAVYYCVRHANFGNSYISYWAYWGQGTLVTVSSGGGGSGGGSEVQLLESGGGLV QPGGSLTLSCAASGFIFRAASMAWYRQSPGNERELVASISSGAFTNYADSVKARFTISR DNSKNTLYLQMNSLRAEDTAVYYCGATFLRSDGHHTIWGQGTLVTVSSHHHHHH |

| **SEQ ID NO:** | **Linker** | **Amino Acid Sequence** |
|---|---|---|
| 503 | Exemplary linker sequence (L040) | GGGGPQASTGRSGGGGGG |
| 504 | Exemplary linker sequence (L041) | GGGGPQGSTGRAAGGGGG |
| 505 | Exemplary linker sequence (L043) | GGGGPQGSTARSAGGGGG |
| 506 | Exemplary linker sequence (L001) | GGGGKPLGLQARVVGGGG |
| 507 | Exemplary linker sequence (NCLV) | GGGGSGGGGSGGVVGGGG |

| **SEQ ID NO:** | **EpCAM Binding Protein sequence** | **Amino Acid Sequence** |
|---|---|---|
| 569 | H13 non-cleavable prodrug (NCLV) | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSSGGGGSGGGGSGGVVGGGGTQTVVTQEPSLTVSPGGTVTLTCASSTGAVTSGNY PNWVQQKPGQAPRGLIGGTKFLVPGTPARFSGSLLGGKAALTLSGVQPEDEAEYYCTLW YSNRWVFGGGTKLTVLGGGGSGGGGSGGGGSEVQLVESGGGLVQPGGSLKLSCAASGFT FNKYAINWVRQAPGKGLEWVARIRSKYNNYATYYADQVKDRFTISRDDSKNTAYLQMNN LKTEDTAVYYCVRHANFGNSYISYWAYWGQGTLVTVSSGGGGSGGGSEVQLVESGGGLV QPGGSLTLSCAASGFAFGNHWMYWYRQAPGRGRELVASISSGGSTNYVDSVKGRFTISR DNAKNTLYLQMNSLRAEDTAVYYCGTSDNWGQGTLVTVSSHHHHHH* |
| 570 | EpCAM-H13 TriTAC (ACT) | EVQLVESGGGLVQPGNSLRLSCAASGFTFSKFGMSWVRQAPGKGLEWVSSISGSGRDTL YADSVKGRFTISRDNAKTTLYLQMNSLRPEDTAVYYCTIGGSLSVSSQGTLVTVSSGGG GSGGGSQTVVTQEPSLTVSPGGTVTLTCASSTGAVTSGNYPNWVQQKPGQAPRGLIGGT KFLVPGTPARFSGSLLGGKAALTLSGVQPEDEAEYYCTLWYSNRWVFGGGTKLTVLGGG GSGGGGSGGGGSEVQLVESGGGLVQPGGSLKLSCAASGFTFNKYAINWVRQAPGKGLEW VARIRSKYNNYATYYADQVKDRFTISRDDSKNTAYLQMNNLKTEDTAVYYCVRHANFGN SYISYWAYWGQGTLVTVSSGGGGSGGGSEVQLVESGGGLVQPGGSLTLSCAASGFAFGN HWMYWYRQAPGRGRELVASISSGGSTNYVDSVKGRFTISRDNAKNTLYLQMNSLRAEDT AVYYCGTSDNWGQGTLVTVSSHHHHHH* |
| 571 | EpCAM-H90.2 (CT) | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSEVQLLESGGGLVQPGGSLTLSCAASGFIFR AASMAWYRQSPGNERELVASISSGAFTNYADSVKARFTISRDNSKNTLYLQMNSLRAED TAVYYCGATFLRSDGHHTIWGQGTLVTVSSHHHHHH* |
| 572 | EpCAM TriTAC-H90.2 (ACT) | EVQLVESGGGLVQPGNSLRLSCAASGFTFSKFGMSWVRQAPGKGLEWVSSISGSGRDTL YADSVKGRFTISRDNAKTTLYLQMNSLRPEDTAVYYCTIGGSLSVSSQGTLVTVSSGGG GSGGGSQTVVTQEPSLTVSPGGTVTLTCASSTGAVTSGNYPNWVQQKPGQAPRGLIGGT KFLVPGTPARFSGSLLGGKAALTLSGVQPEDEAEYYCTLWYSNRWVFGGGTKLTVLGGG GSGGGGSGGGGSEVQLVESGGGLVQPGGSLKLSCAASGFTFNKYAINWVRQAPGKGLEW VARIRSKYNNYATYYADQVKDRFTISRDDSKNTAYLQMNNLKTEDTAVYYCVRHANFGN SYISYWAYWGQGTLVTVSSGGGGSGGGSEVQLLESGGGLVQPGGSLTLSCAASGFIFRA ASMAWYRQSPGNERELVASISSGAFTNYADSVKARFTISRDNSKNTLYLQMNSLRAEDT AVYYCGATFLRSDGHHTIWGQGTLVTVSSHHHHHH* |
| 573 | EpCAM-H138.2 ProTriTAC (NCLV) | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSSGGGGSGGGGSGGVVGGGGTQTVVTQEPSLTVSPGGTVTLTCASSTGAVTSGNY PNWVQQKPGQAPRGLIGGTKFLVPGTPARFSGSLLGGKAALTLSGVQPEDEAEYYCTLW YSNRWVFGGGTKLTVLGGGGSGGGGSGGGGSEVQLVESGGGLVQPGGSLKLSCAASGFT FNKYAINWVRQAPGKGLEWVARIRSKYNNYATYYADQVKDRFTISRDDSKNTAYLQMNN LKTEDTAVYYCVRHANFGNSYISYWAYWGQGTLVTVSSGGGGSGGGSEVQLLESGGGLV QPGGSLTLSCAASGFIFRAASMDWYRQFPGNERESIATISSGGFTNYADSVKGRFTISR DNSKNTLYLQMNSLRAEDTAVYYCGATFLRSDGHHTIWGQGTLVTVSSHHHHHH* |
| 574 | EpCAM ProTriTAC-H138.2 (CT) | VVGGGGTQTVVTQEPSLTVSPGGTVTLTCASSTGAVTSGNYPNWVQQKPGQAPRGLIGG TKFLVPGTPARFSGSLLGGKAALTLSGVQPEDEAEYYCTLWYSNRWVFGGGTKLTVLGG GGSGGGGSGGGGSEVQLVESGGGLVQPGGSLKLSCAASGFTFNKYAINWVRQAPGKGLE WVARIRSKYNNYATYYADQVKDRFTISRDDSKNTAYLQMNNLKTEDTAVYYCVRHANFG NSYISYWAYWGQGTLVTVSSGGGGSGGGSEVQLLESGGGLVQPGGSLTLSCAASGFIFR |
| | | AASMDWYRQFPGNERESIATISSGGFTNYADSVKGRFTISRDNSKNTLYLQMNSLRAED TAVYYCGATFLRSDGHHTIWGQGTLVTVSSHHHHHH* |
| 575 | EpCAM H138.2 TriTAC DG/WG | EVQLVESGGGLVQPGNSLRLSCAASGFTFSKFGMSWVRQAPGKGLEWVSSISGSGRDTL YADSVKGRFTISRDNAKTTLYLQMNSLRPEDTAVYYCTIGGSLSVSSQGTLVTVSSGGG GSGGGSQTVVTQEPSLTVSPGGTVTLTCASSTGAVTSGNYPNWVQQKPGQAPRGLIGGT KFLVPGTPARFSGSLLGGKAALTLSGVQPEDEAEYYCTLWYSNRWVFGGGTKLTVLGGG GSGGGGSGGGGSEVQLVESGGGLVQPGGSLKLSCAASGFTFNKYAINWVRQAPGKGLEW VARIRSKYNNYATYYADQVKDRFTISRDDSKNTAYLQMNNLKTEDTAVYYCVRHANFGN SYISYWAYWGQGTLVTVSSGGGGSGGGSEVQLLESGGGLVQPGGSLTLSCAASGFIFRA ASMDWYRQFPGNERESIATISSGGFTNYADSVKGRFTISRDNSKNTLYLQMNSLRAEDT AVYYCGATFLRSDGHHTIWGQGTLVTVSSHHHHHH* |
| 576 | EpCAM H13 Mask27 L040 | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSSGGGGPQASTGRSGGGGGGSQTVVTQEPSLTVSPGGTVTLTCASSTGAVTSGNY PNWVQQKPGQAPRGLIGGTKFLVPGTPARFSGSLLGGKAALTLSGVQPEDEAEYYCTLW YSNRWVFGGGTKLTVLGGGGSGGGGSGGGGSEVQLVESGGGLVQPGGSLKLSCAASGFT FNKYAINWVRQAPGKGLEWVARIRSKYNNYATYYADQVKDRFTISRDDSKNTAYLQMNN LKTEDTAVYYCVRHANFGNSYISYWAYWGQGTLVTVSSGGGGSGGGSEVQLVESGGGLV QPGGSLTLSCAASGFAFGNHWMYWYRQAPGRGRELVASISSGGSTNYVDSVKGRFTISR DNAKNTLYLQMNSLRAEDTAVYYCGTSDNWGQGTLVTVSSHHHHHH* |
| 577 | EpCAM H90 Mask27 L040 | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSSGGGGPQASTGRSGGGGGGTQTVVTQEPSLTVSPGGTVTLTCASSTGAVTSGNY PNWVQQKPGQAPRGLIGGTKFLVPGTPARFSGSLLGGKAALTLSGVQPEDEAEYYCTLW YSNRWVFGGGTKLTVLGGGGSGGGGSGGGGSEVQLVESGGGLVQPGGSLKLSCAASGFT FNKYAINWVRQAPGKGLEWVARIRSKYNNYATYYADQVKDRFTISRDDSKNTAYLQMNN LKTEDTAVYYCVRHANFGNSYISYWAYWGQGTLVTVSSGGGGSGGGSEVQLLESGGGLV QPGGSLTLSCAASGFIFRAASMAWYRQSPGNERELVASISSGAFTNYADSVKARFTISR DNSKNTLYLQMNSLRAEDTAVYYCGATFLRSDGHHTINGQGTLVTVSSHHHHHH* |
| 578 | EpCAM H90.2 Mask27 L040 | EVQLVESGGGLVQPGNSLRLSCAASGFTFSKFGMSWVRQGGGGGLDGNEEPGGLEWVSS ISGSGRDTLYADSVKGRFTISRDNAKTTLYLQMNSLRPEDTAVYYCTIGGSLSVSSQGT LVTVSSGGGGPQASTGRSGGGGGGSQTVVTQEPSLTVSPGGTVTLTCASSTGAVTSGNY PNWVQQKPGQAPRGLIGGTKFLVPGTPARFSGSLLGGKAALTLSGVQPEDEAEYYCTLW YSNRWVFGGGTKLTVLGGGGSGGGGSGGGGSEVQLVESGGGLVQPGGSLKLSCAASGFT FNKYAINWVRQAPGKGLEWVARIRSKYNNYATYYADQVKDRFTISRDDSKNTAYLQMNN LKTEDTAVYYCVRHANFGNSYISYWAYWGQGTLVTVSSGGGGSGGGSEVQLLESGGGLV QPGGSLTLSCAASGFIFRAASMAWYRQSPGNERELVASISSGAFTNYADSVKARFTISR DNSKNTLYLQMNSLRAEDTAVYYCGATFLRSDGHHTIWGQGTLVTVSSHHHHHH* |

| **SEQ ID NO:** | | **Amino Acid Sequence** |
|---|---|---|
| 579 | 1.7 Bn huEpCAM aa22-265 FLAG huIgG1 Fc (biotin-huEpCAM-Fc) | QEECVCENYKLAVNCFVNNNRQCQCTSVGAQNTVICSKLAAKCLVMKAEMNGSKLGRRA KPEGALQNNDGLYDPDCDESGLFKAKQCNGTSMCWCVNTAGVRRTDKDTEITCSERVRT YWIIIELKHKAREKPYDSKSLRTALQKEITTRYQLDPKFITSILYENNVITIDLVQNSS QKTQNDVDIADVAYYFEKDVKGESLFHSKKMDLTVNGEQLDLDPGQTLIYYVDEKAPEF SMQGLKDYKDDDDKGGGGSDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNGKEY KCKVSNKALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHEALHNHYT QKSLSLSPGK* |
| 580 | 1.9-Bn cyEpCAM aa24-265 FLAG huIgG1 Fc (biotin-cyEpCAM-Fc) | QKECVCENYKLAVNCFLNDNGQCQCTSIGAQNTVLCSKLAAKCLVMKAEMNGSKLGRRA KPEGALQNNDGLYDPDCDESGLFKAKQCNGTSTCWCVNTAGVRRTDKDTEITCSERVRT YWIIIELKHKAREKPYDVQSLRTALEEAIKTRYQLDPKFITNILYEDNVITIDLVQNSS QKTQNDVDIADVAYYFEKDVKGESLFHSKKMDLRVNGEQLDLDPGQTLIYYVDEKAPEF SMQGLKDYKDDDDKGGGGSDKTHTCPPCPAPELLGGPSVFLFPPKPKDTLMISRTPEVT CVVVDVSHEDPEVKFNWYVDGVEVHNAKTKPREEQYNSTYRVVSVLTVLHQDWLNGKEY KCKVSNKALPAPIEKTISKAKGQPREPQVYTLPPSREEMTKNQVSLTCLVKGFYPSDIA VEWESNGQPENNYKTTPPVLDSDGSFFLYSKLTVDKSRWQQGNVFSCSVMHEALHNHYT QKSLSLSPGK* |
| 581 | Linker | GGGGT |
| 582 | Framework 4 for H90.2 and H138.2 | WGQGTLVTVSS |

## Claims

1. A multispecific protein comprising (a) an EpCAM binding domain, (b) a CD3ε binding domain, and (c) a bulk serum protein binding domain that binds to a human serum albumin protein, wherein the multispecific protein comprises an amino acid sequence of any one of SEQ ID NOS: 576, 577, and 578.

2. The multispecific protein according to claim 1, wherein the multispecific protein comprises the amino acid sequence of SEQ ID NO: 576.

3. The multispecific protein according to claim 1, wherein the multispecific protein comprises the amino acid sequence of SEQ ID NO: 577.

4. The multispecific protein according to claim 1, wherein the multispecific protein comprises the amino acid sequence of SEQ ID NO: 578.

5. A pharmaceutical composition comprising (i) a multispecific protein according to any one of claims 1-4, and (ii) a pharmaceutically acceptable carrier.

6. A polynucleotide encoding the multispecific protein according to any one of claims 1-4.

7. The multispecific protein according to any one of claims 1-4, the pharmaceutical composition of claim 5, or a vector comprising the polynucleotide of claim 6 for use in the treatment of a cancer in a subject in need thereof.

8. The multispecific protein, the pharmaceutical composition, or a vector comprising the polynucleotide for use according to claim 7, wherein the subject is human.

9. The multispecific protein, the pharmaceutical composition, or a vector comprising the polynucleotide for use according to claim 7 or 8, wherein the cancer comprises a solid tumor.

10. The multispecific protein, the pharmaceutical composition, or a vector comprising the polynucleotide for use according to claim 9, wherein the solid tumor is metastatic.

11. The multispecific protein, the pharmaceutical composition, or a vector comprising the polynucleotide for use according to any one of claims 7-10, wherein the cancer comprises a colorectal cancer, a prostate cancer, a neuroendocrine cancer, a thyroid cancer, a non-small cell lung cancer, a small cell lung cancer, a gastric cancer, an ovarian cancer, an endometrial cancer, a pancreatic cancer, a biliary track cancer, a gall bladder cancer, an esophageal cancer, a breast cancer, or an adenocarcinoma.

## Patentansprüche

1. Ein multispezifisches Protein, umfassend (a) eine EpCAM-Bindungsdomäne, (b) eine CD3ε-Bindungsdomäne und (c) eine Bulk-Serumprotein-Bindungsdomäne, die an ein humanes Serumalbumin-Protein bindet, wobei das multispezifische Protein eine Aminosäuresequenz von einer der Sequenzen SEQ ID NOS: 576, 577 und 578 umfasst.

2. Das multispezifische Protein gemäß Anspruch 1, wobei das multispezifische Protein die Aminosäuresequenz SEQ ID NO: 576 umfasst.

3. Das multispezifische Protein gemäß Anspruch 1, wobei das multispezifische Protein die Aminosäuresequenz SEQ ID NO: 577 umfasst.

4. Das multispezifische Protein gemäß Anspruch 1, wobei das multispezifische Protein die Aminosäuresequenz SEQ ID NO: 578 umfasst.

5. Eine pharmazeutische Zusammensetzung, umfassend (i) ein multispezifisches Protein gemäß einem der Ansprüche 1-4 und (ii) einen pharmazeutisch annehmbaren Träger.

6. Ein Polynukleotid, das für das multispezifische Protein gemäß einem der Ansprüche 1-4 kodiert.

7. Das multispezifische Protein gemäß einem der Ansprüche 1-4, die pharmazeutische Zusammensetzung nach Anspruch 5 oder ein Vektor, der das Polynukleotid nach Anspruch 6 umfasst, zur Verwendung bei der Behandlung eines Karzinoms bei einem Subjekt, das diese benötigt.

8. Das multispezifische Protein, die pharmazeutische Zusammensetzung oder ein Vektor, der das Polynukleotid umfasst, zur Verwendung gemäß Anspruch 7, wobei das Subjekt ein Mensch ist.

9. Das multispezifische Protein, die pharmazeutische Zusammensetzung oder ein Vektor, der das Polynukleotid umfasst, zur Verwendung gemäß Anspruch 7 oder 8, wobei das Karzinom einen soliden Tumor umfasst.

10. Das multispezifische Protein, die pharmazeutische Zusammensetzung oder ein Vektor, der das Polynukleotid umfasst, zur Verwendung gemäß Anspruch 9, wobei der solide Tumor metastatisch ist.

11. Das multispezifische Protein, die pharmazeutische Zusammensetzung oder ein Vektor, der das Polynukleotid umfasst, zur Verwendung gemäß einem der Ansprüche 7-10, wobei das Karzinom ein kolorektales Karzinom, ein Prostatakarzinom, ein neuroendokrines Karzinom, ein Schilddrüsenkarzinom, ein nicht-kleinzelliges Lungenkarzinom, ein kleinzelliges Lungenkarzinom, ein Magenkarzinom, ein Ovarialkarzinom, ein Endometriumkarzinom, ein Bauchspeicheldrüsenkarzinom, ein Gallengangkarzinom, ein Gallenblasenkarzinom, ein Ösophaguskarzinom, Brustkrebs oder ein Adenokarzinom umfasst.

## Revendications

1. Protéine multi-spécifique comprenant (a) un domaine de liaison à EpCAM, (b) un domaine de liaison à CD3ε, et (c) un domaine de liaison aux protéines sériques totales qui se lie à une protéine d'albumine sérique humaine, où la protéine multi-spécifique comprend une séquence d'acides aminés parmi l'une quelconque des SEQ ID n° 576, 577 et 578.

2. Protéine multi-spécifique selon la revendication 1, où la protéine multi-spécifique comprend la séquence d'acides aminés de la SEQ ID n° 576.

3. Protéine multi-spécifique selon la revendication 1, où la protéine multi-spécifique comprend la séquence d'acides aminés de la SEQ ID n° 577.

4. Protéine multi-spécifique selon la revendication 1, où la protéine multi-spécifique comprend la séquence d'acides aminés de la SEQ ID n° 578.

5. Composition pharmaceutique comprenant (i) une protéine multi-spécifique selon l'une quelconque des revendications 1 à 4 et (ii) un support pharmaceutiquement acceptable.

6. Polynucléotide codant pour la protéine multi-spécifique selon l'une quelconque des revendications 1 à 4.

7. Protéine multi-spécifique selon l'une quelconque des revendications 1 à 4, composition pharmaceutique selon la revendication 5, ou vecteur comprenant le polynucléotide selon la revendication 6 pour utilisation dans le traitement d'un cancer chez un sujet en ayant besoin.

8. Protéine multi-spécifique, composition pharmaceutique, ou vecteur comprenant le polynucléotide pour utilisation selon la revendication 7, où le sujet est un être humain.

9. Protéine multi-spécifique, composition pharmaceutique, ou vecteur comprenant le polynucléotide pour utilisation selon la revendication 7 ou la revendication 8, où le cancer comprend une tumeur solide.

10. Protéine multi-spécifique, composition pharmaceutique, ou vecteur comprenant le polynucléotide pour utilisation selon la revendication 9, où la tumeur solide est métastatique.

11. Protéine multi-spécifique, composition pharmaceutique, ou vecteur comprenant le polynucléotide pour utilisation selon l'une quelconque des revendications 7 à 10, où le cancer comprend un cancer colorectal, un cancer de la prostate, un cancer neuroendocrinien, un cancer de la thyroïde, un cancer du poumon non à petites cellules, un cancer du poumon à petites cellules, un cancer gastrique, un cancer de l'ovaire, un cancer de l'endomètre, un cancer du pancréas, un cancer du tractus biliaire, un cancer de la vésicule biliaire, un cancer de l'œsophage, un cancer du sein ou un adénocarcinome.
